(19) Europäisches Patentamt
European Patent Office
Office européen des brevets

(11) EP 4 428 138 A1

(12) # EUROPEAN PATENT APPLICATION
published in accordance with Art. 153(4) EPC

(43) Date of publication:
**11.09.2024 Bulletin 2024/37**

(21) Application number: **21962965.6**

(22) Date of filing: **05.11.2021**

(51) International Patent Classification (IPC):
***C07F 9/24*** (2006.01) ***C07F 9/564*** (2006.01)
***A61K 31/664*** (2006.01) ***A61K 31/396*** (2006.01)
***A61K 31/04*** (2006.01) ***A61K 31/06*** (2006.01)
***A61P 35/00*** (2006.01)

(52) Cooperative Patent Classification (CPC):
**A61K 31/04; A61K 31/06; A61K 31/396; A61K 31/664; A61P 35/00; C07F 9/24; C07F 9/564**

(86) International application number:
**PCT/CN2021/129077**

(87) International publication number:
**WO 2023/077452 (11.05.2023 Gazette 2023/19)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Ascentawits Pharmaceuticals, Ltd.**
**Shenzhen, Guangdong 518122 (CN)**

(72) Inventors:
- **LI, Anrong**
  **Shenzhen, Guangdong 518122 (CN)**
- **DUAN, Jianxin**
  **Shenzhen, Guangdong 518122 (CN)**
- **MENG, Fanying**
  **San Francisco, California 94121 (US)**
- **QI, Tianyang**
  **Shenzhen, Guangdong 518122 (CN)**
- **MENG, Teng**
  **Shenzhen, Guangdong 518122 (CN)**
- **ZHANG, Mengyun**
  **Shenzhen, Guangdong 518122 (CN)**

(74) Representative: **Studio Torta S.p.A.**
**Via Viotti, 9**
**10121 Torino (IT)**

(54) **AKR1C3-ACTIVATED DNA ALKYLATING AGENT, AND MEDICAL APPLICATION THEREOF**

(57) An AKR1C3-activated DNA alkylating agent having any one of the following structural formulae (A, B, C), or a pharmaceutically acceptable salt thereof, or a prodrug thereof, or a solvate thereof, or an isotopic variant thereof, and an anticancer medical application thereof.

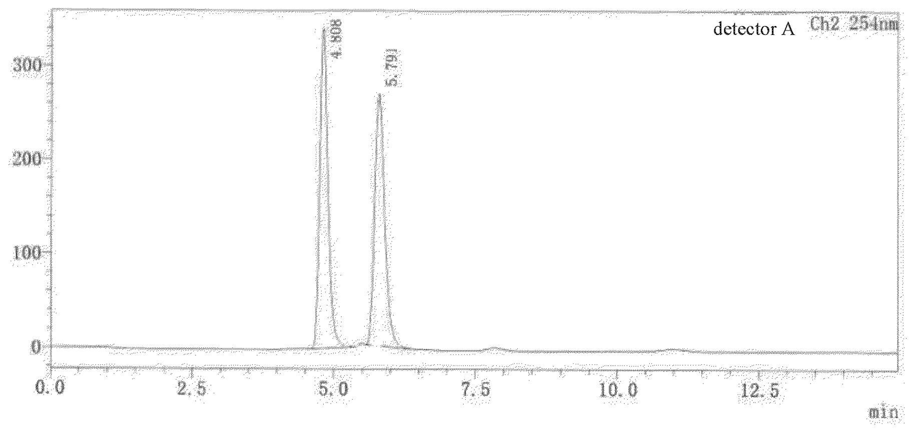


Fig. 1



Processed by Luminess, 75001 PARIS (FR)

## Description

### Technical Field

The present invention relates to the field of compounds for cancer therapy, belonging to the field of small molecule drug research.

### Background Art

The patent PCT/CN2020/089692 (filed on May 12, 2020) by inventors DUAN Jianxin, LI Anrong, et al. discloses a series of compounds with the following general formulae and the anticancer use thereof:

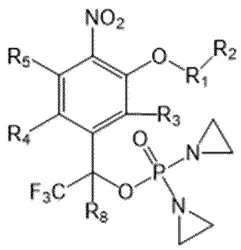

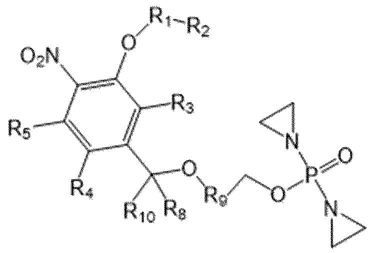

In this invention, in order to overcome the shortcomings of AST-3424 being an oil that cannot be conveniently formulated, stored and transported, solid (partially waxy) compounds are obtained by introducing trifluoromethyl groups at specific positions in the molecular structure of AST-3424 and compounds with similar structures. The above compounds with the introduction of trifluoromethyl groups have been confirmed to be still AKR1C3-activated DNA alkylating agents through preliminary cell tests.

AST-3424 was found in further research to be a substrate of P-gp, unable to pass through the blood-brain barrier and thus ineffective against tumors in the brain.

Therefore, further research and development should be carried out on AKR1C3-activated small molecule compounds that can enter the brain or other parts of the central nervous system.

### Summary of the Invention

In subsequent research and screening, the inventors found that Compound No. 16 disclosed in PCT/CN2020/089692 had the ability to penetrate the brain or other parts of the central nervous system, and was expected to be further developed into a drug for the treatment of tumors of the brain or other parts of the central nervous system.

The present invention provides an AKR1C3-activated small molecule compound that can enter the brain or other parts of the central nervous system, the mechanism of action of which is to inhibit and kill tumor cells by releasing DNA alkylating agents through the action of AKR1C3 enzyme.

In particular, the above Compound No. 16 has the following structural formula:

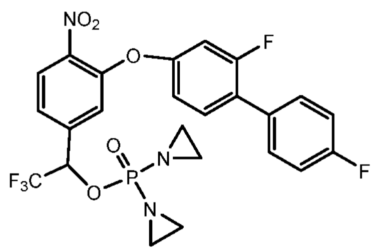
.

Experiments showed that this compound had high cancer cell proliferation inhibition activity, especially under activation of AKR1C3 enzyme, this compound had higher cancer cell inhibition activity; and these compounds have been shown not to be substrates of Pgp (P-Glycoprotein), being able to pass through the blood-brain barrier and enter the brain or other parts of the central nervous system for action to exert their effects; the experiments in serum and liver microsomes and animal models further showed that the compound had high stability, and good clearance and inhibition effect on tumors.

Based on the above findings, the present invention provides the following AKR1C3-activated DNA alkylating agents and makes clear that they have related anticancer and antitumor medical uses.

The present invention provides a compound with any one of the following structural formulae A, B, and C, or a pharmaceutically acceptable salt, a prodrug, a solvate, or an isotopic variant thereof:

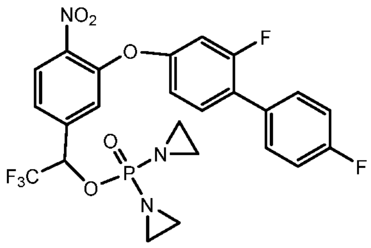
,
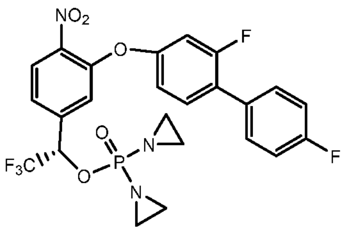
,
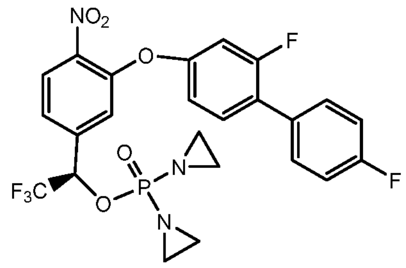

A B C .

The salt is a basic salt or an acid salt, and the solvate is a hydrate or alcoholate.

Preferably, the alcoholate is an ethanolate.

The present invention provides pharmaceutically acceptable salts of the above three compounds, wherein the salts may be basic salts, including salts of the compounds formed with inorganic bases (such as alkali metal hydroxides, alkaline earth metal hydroxides, or the like) or with organic bases (such as mono-, di-, or triethanolamine or the like). Alternatively, the salts may be acid salts including salts of the compound formed with inorganic acids (such as hydrochloric acid, hydrobromic acid, hydroiodic acid, nitric acid, perchloric acid, sulfuric acid, phosphoric acid, or the like) or with organic acids (such as methanesulfonic acid, trifluoromethanesulfonic acid, ethanesulfonic acid, benzenesulfonic acid, p-toluenesulfonic acid, fumaric acid, oxalic acid, maleic acid, citric acid, or the like). The selection and preparation of acceptable salts, solvates, and the like of a compound are well known in the art.

The compounds herein may further be used in the form of solvates; that is, the present invention provides pharmaceutically acceptable solvates of the above three compounds, wherein the solvates are hydrates, alcoholates, and the like, and the alcoholates include ethanolates.

The term "isotopic variant" refers to a compound containing non-natural proportions of isotopes in one or more of the atoms that make up such a compound. In some embodiments, the "isotopic variants" of the compounds contain one or more isotopes in non-natural proportions, including (but not limited to) hydrogen ($^{1}$H), deuterium ($^{2}$H), tritium ($^{3}$H), carbon-11 ($^{11}$C), carbon-12 ($^{12}$C), carbon-13 ($^{13}$C), carbon-14 ($^{14}$C), nitrogen-13 ($^{13}$N), nitrogen-14 ($^{14}$N), nitrogen-15 ($^{15}$N), oxygen-14 ($^{14}$O), oxygen-15 ($^{15}$O), oxygen-16 ($^{16}$O), oxygen-17 ($^{17}$O), oxygen-18 ($^{18}$O), fluorine-17 ($^{17}$F), fluorine-18 ($^{18}$F), phosphorus-31 ($^{31}$P), phosphorus-32 ($^{32}$P), phosphorus-33 ($^{33}$P), sulfur-32 ($^{32}$S), sulfur-33 ($^{33}$S), sulfur-34 ($^{34}$S), sulfur-35 ($^{35}$S), sulfur-36 ($^{36}$S), chlorine-35 ($^{35}$Cl), chlorine-36 ($^{36}$Cl), chlorine-37 ($^{37}$Cl), bromine-79 ($^{79}$Br), bromine-81 ($^{81}$Br), iodine-123 ($^{123}$I), iodine-125 ($^{125}$I), iodine-127 ($^{127}$I), iodine-129 ($^{129}$I), and iodine-131 ($^{131}$I), In some embodiments, the "isotopic variants" of the compounds are in stable (i.e. nonradioactive) form. In some embodiments, the "isotopic variants" of the compounds contain one or more isotopes in non-natural proportions, including (but not limited to) hydrogen ($^{1}$H), deuterium ($^{2}$H), carbon-12 ($^{12}$C), carbon-13 ($^{13}$C), nitrogen-14 ($^{14}$N), nitrogen-15 ($^{15}$N), oxygen-16 ($^{16}$O), oxygen-17 ($^{17}$O), oxygen-18 ($^{18}$O), fluorine-17 ($^{17}$F), phosphorus-31 ($^{31}$P), sulfur-32 ($^{32}$S), sulfur-33 ($^{33}$S), sulfur-34 ($^{34}$S), sulfur-36 ($^{36}$S), chlorine-35 ($^{35}$Cl), chlorine-37 ($^{37}$Cl), bromine-79 ($^{79}$Br), bromine-81 ($^{81}$Br), and iodine-127 ($^{127}$I). In some embodiments, the "isotopic variants" of the compounds are in unstable (i.e. radioactive) form. In some embodiments, the "isotopic variants" of the compounds contain one or more isotopes in non-natural proportions, including (but not limited to) tritium ($^{3}$H), carbon-11 ($^{11}$C), carbon-14 ($^{14}$C), nitrogen-13 ($^{13}$N), oxygen-14 ($^{14}$O), oxygen-15 ($^{15}$O), fluorine-18 ($^{18}$F), phosphorus-32 ($^{32}$P), phosphorus-33 ($^{33}$P), sulfur-35 ($^{35}$S), chlorine-36 ($^{36}$Cl), iodine-123 ($^{123}$I), iodine-125 ($^{125}$I), iodine-129 ($^{129}$I), and iodine-131 ($^{131}$I), It should be understood that in the compounds as provided herein, when deemed feasible by those skilled in the art, any hydrogen may be for example $^{2}$H (i.e., D), or any carbon may be for example $^{13}$C, or any nitrogen may be for example $^{15}$N, and any oxygen may be $^{18}$O. In some embodiments, the "isotopic variants" of the compounds contain non-natural proportions of deuterium (D).

The isotopic variants provided by the present invention correspond to deuterated compounds selected from the following structures:

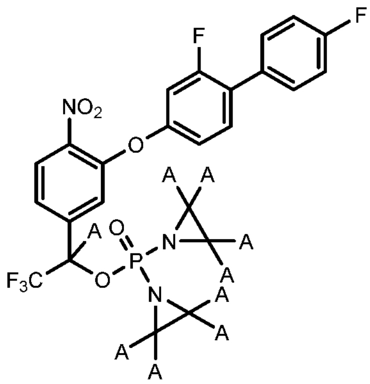
,
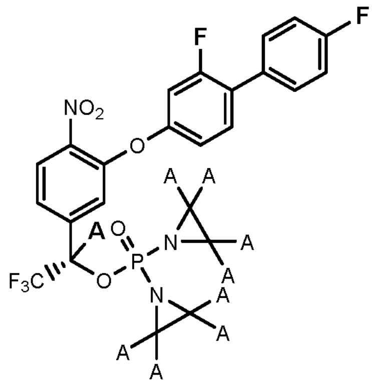
,
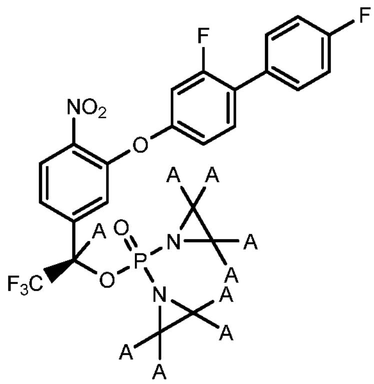
,

wherein As are each independently H or D, and at least one of the nine As is D.

Preferably, the isotopic variants correspond to the deuterated compounds with the following structures:

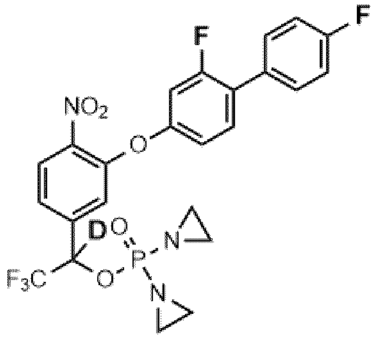
,
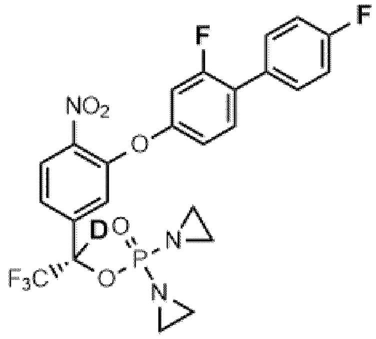
,
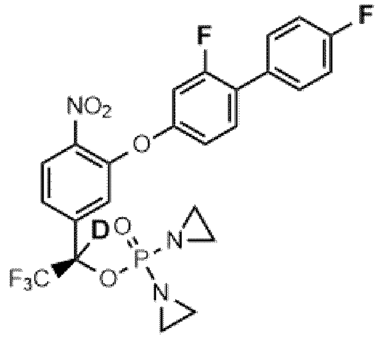
,

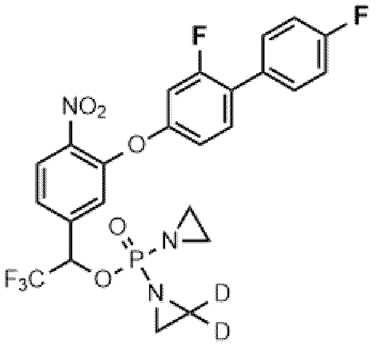
,
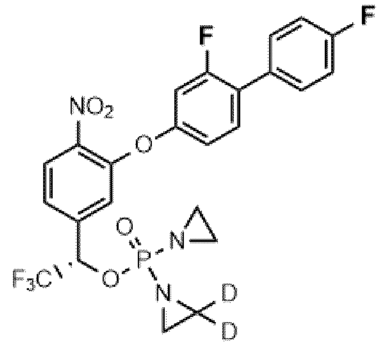
,
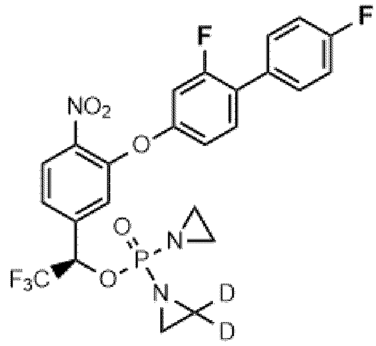
,

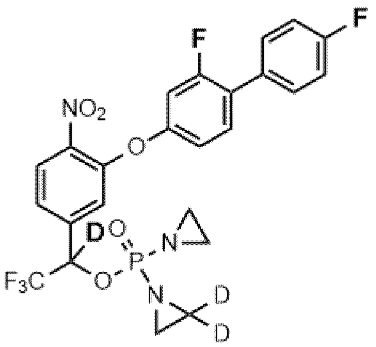
,
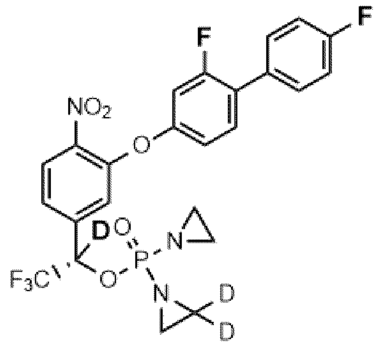
,
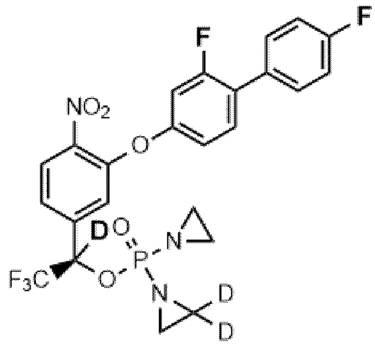
,

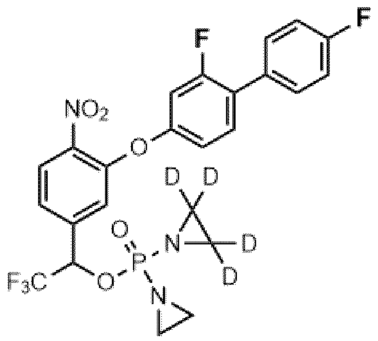,
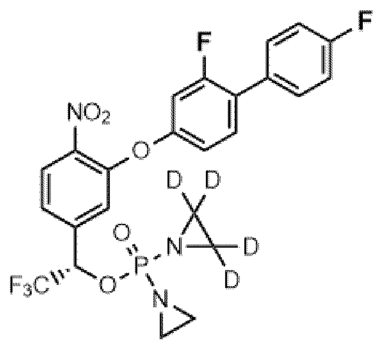,
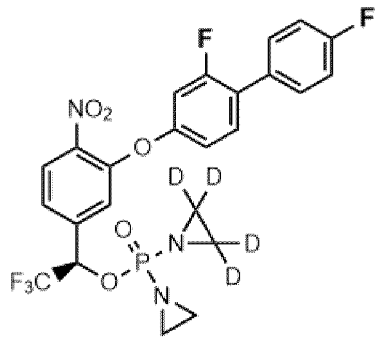,

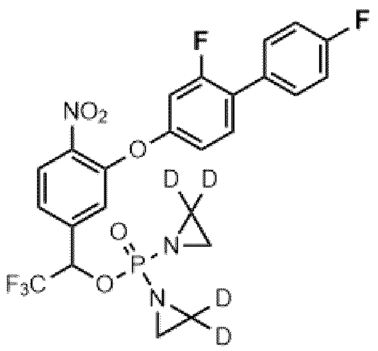,
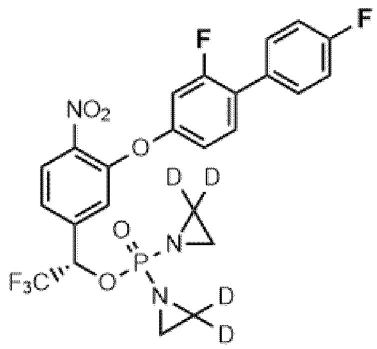,
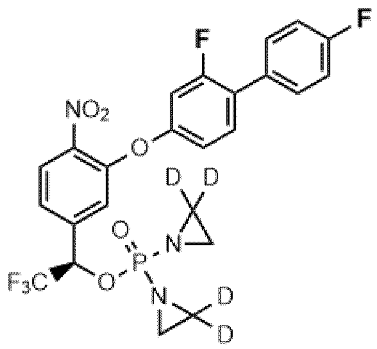,

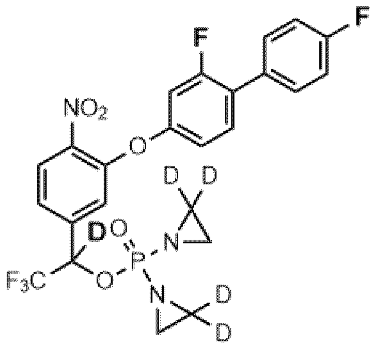,
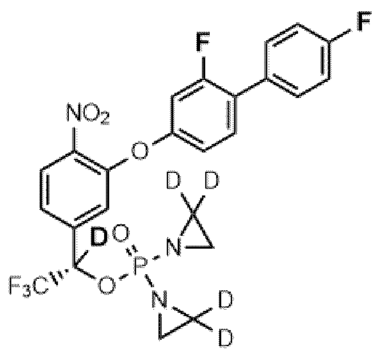,
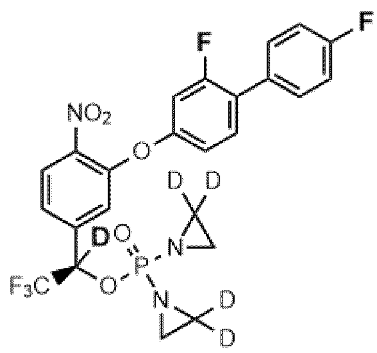,

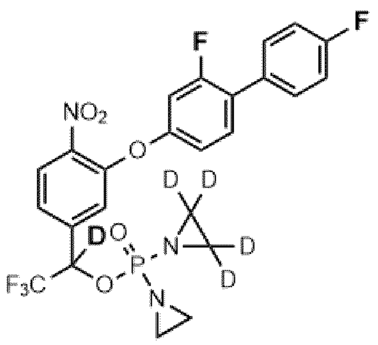,
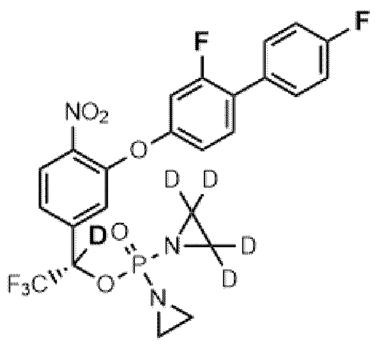,
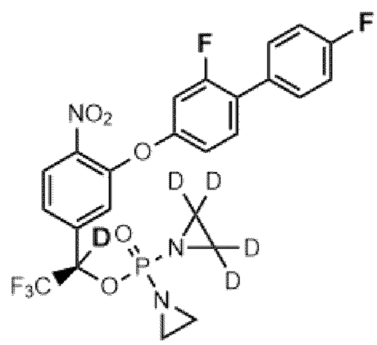,

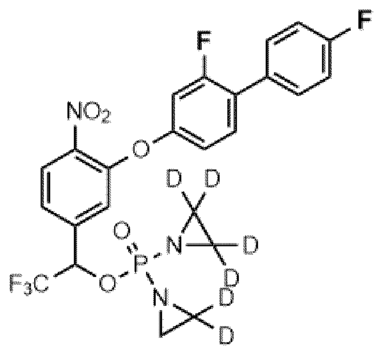 , 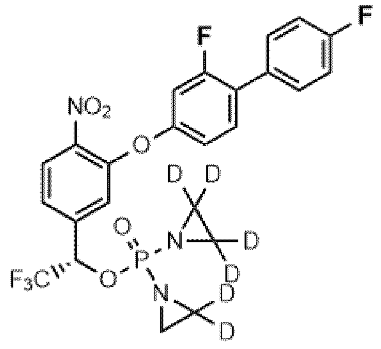 , 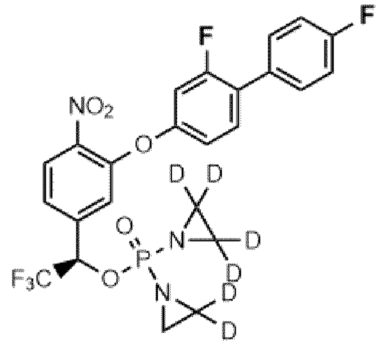 ,

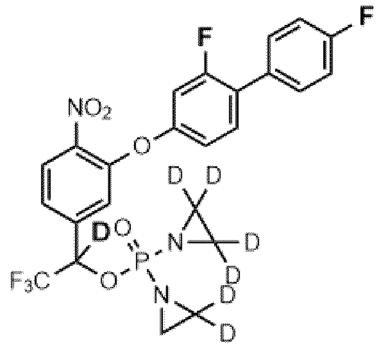 , 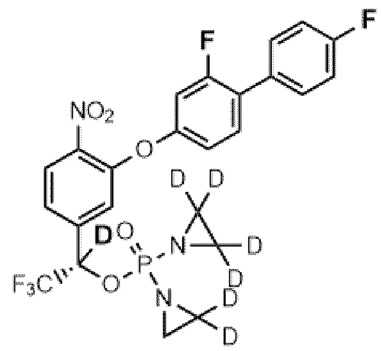 , 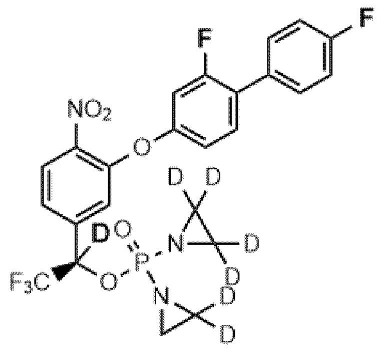 ,

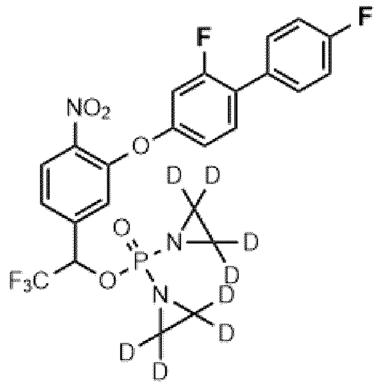 , 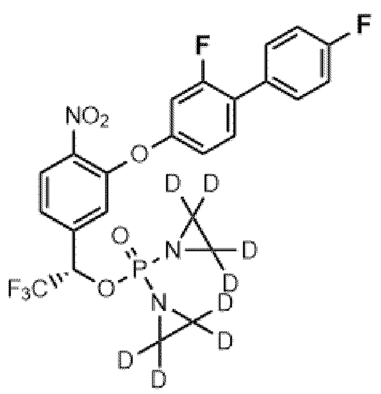 , 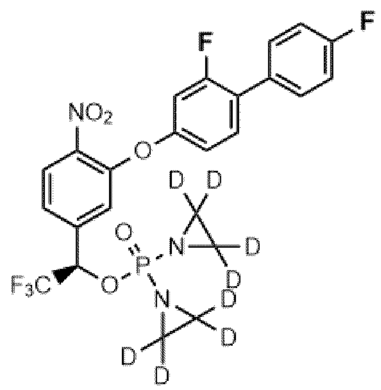 ,

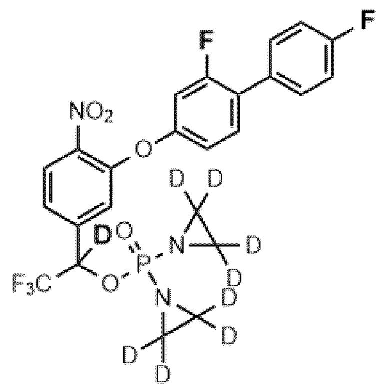 , 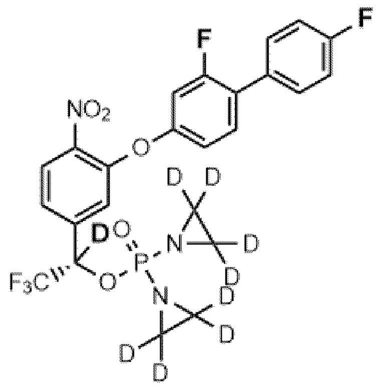 , 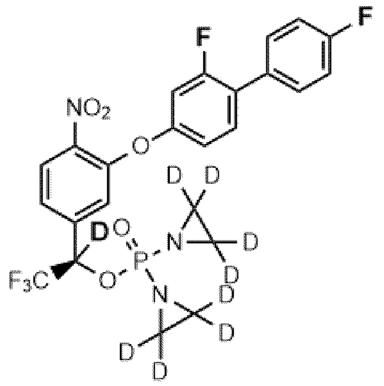 .

The present invention provides a drug containing the compound of any one of the above structures A, B, and C, or a pharmaceutically acceptable salt, a prodrug, a solvate, or an isotopic variant thereof.

The present invention also provides use of the above drug for the treatment of cancer or tumor patients, conditions caused by cancer or tumor, or cell proliferative diseases.

Treatment includes monotherapy and combination therapy.

Monotherapy refers to a single drug therapy. Combination refers to a combined drug therapy. Single drug therapy refers to the use of only one anticancer drug in a course of treatment. Combination therapy refers to the simultaneous or sequential use of two or more anticancer drugs in a course of treatment.

Generally speaking, for combination therapy, different administration dosages and administration cycles need to be explored according to the characteristics of the disease and the types of drugs to be used in combination; and only according to the above situation, the combination therapy plan obtained from the exploration may achieve better therapeutic effect as compared with single drug therapy.

The administration dosages and administration cycles of drugs in both monotherapy and combination therapy regimens need to be explored through clinical trials with the reference to the dosages and administration regimens of the above compounds A, B, and C and analogues thereof and other drugs.

Furthermore, the present invention provides use of the compound of any one of the above structures A, B, and C, or a pharmaceutically acceptable salt, a prodrug, a solvate, or an isotopic variant thereof in the treatment of cancer or tumor patients, conditions caused by cancer or tumor, or cell proliferative diseases.

Specifically, the above cancer or tumor is lung cancer, pancreatic cancer, liver cancer, or gastric cancer.

Specifically, the above cancer or tumor is primary brain cancer, brain tumor or metastatic cancer or tumor that metastasizes to the brain.

The present invention provides use of the compound of any one of the above structures A, B, and C, or a pharmaceutically acceptable salt, a prodrug, a solvate, or an isotopic variant thereof in the preparation of a drug for the treatment of cancer or tumor patients, conditions caused by cancer or tumor, or cell proliferative diseases.

The above cancer or tumor includes: lung cancer, non-small cell lung cancer, liver cancer, pancreatic cancer, stomach cancer, bone cancer, esophagus cancer, breast cancer, prostate cancer, testicular cancer, colon cancer, ovarian cancer, bladder cancer, cervical cancer, melanoma, squamous cell carcinoma, basal cell carcinoma, adenocarcinoma, sweat gland carcinoma, sebaceous gland carcinoma, papillary carcinoma, papillary adenocarcinoma, cystic adenocarcinoma, cystic carcinoma, medullary carcinoma, bronchial carcinoma, osteocyte carcinoma, epithelial carcinoma, cholangiocarcinoma, choriocarcinoma, embryonal carcinoma, seminoma, Wilm's tumor, glioblastoma, astrocytoma, medulloblastoma, craniopharyngioma, ependymoma, pineal tumor, hemocytoblastoma, vocal cord neuroma, meningioma, neuroblastoma, optic neuroblastoma, retinoblastoma, neurofibroma, fibrosarcoma, fibroblastoma, fibroma, fibroadenoma, fibrochondroma, fibrocystoma, fibromyxoma, fibroosteoma, fibromyxosarcoma, fibropapilloma, myxosarcoma, myxocystoma, myxochondroma, myxochondrosarcoma, myxochondrofibrosarcoma, myxadenoma, myxoblastoma, liposarcoma, lipoma, lipoadenoma, lipoblastoma, lipochondroma, lipofibroma, lipoangioma, myxolipoma, chondrosarcoma, chondroma, chondromyoma, chordoma, chorioadenoma, chorioepithelioma, chorioblastoma, osteosarcoma, osteoblastoma, osteochondrofibroma, osteochondrosarcoma, osteochondroma, osteocystoma, osteodentinoma, osteofibroma, fibrosarcoma of bone, angiosarcoma, hemangioma, angiolipoma, angiochondroma, hemangioblastoma, angiokeratoma, angioglioma, angioendothelioma, angiofibroma, angiomyoma, angiolipoma, angiolymphangioma, angiolipoleiomyoma, angiomyolipoma, angiomyoneuroma, angiomyxoma, angioreticuloma, lymphangiosarcoma, lymphogranuloma, lymphangioma, lymphoma, lymphomyxoma, lymphosarcoma, lymphangiofibroma, lymphocytoma, lymphoepithelioma, lymphoblastoma, endothelioma, endothelioblastoma, synovioma, synovial sarcoma, mesothelioma, connective tissue tumor, Ewing's tumor, leiomyoma, leiomyosarcoma, leiomyoblastoma, leiomyofibroma, rhabdomyoma, rhabdomyosarcoma, rhabdomyomyxoma, acute lymphatic leukemia, acute myelogenous leukemia, anemia of chronic disease, polycythemia, lymphoma, endometrial cancer, glioma, colorectal cancer, thyroid cancer, urothelial cancer or multiple myeloma.

Preferably, the cancer or tumor is primary brain cancer, brain tumor or metastatic cancer or tumor that metastasizes to the brain.

Preferably, the cancer or tumor is lung cancer, pancreatic cancer, liver cancer, or gastric cancer; more preferably, the lung cancer is non-small cell lung cancer.

The present invention provides a method for treating cancer or tumor, comprising step a or step b:

a. determining the AKR1C3 reductase content of cancer cells or tissues in a patient, and administering the above drug or the compound with any one of the above structures A, B, and C, or a pharmaceutically acceptable salt, a prodrug, a solvate, or an isotopic variant thereof to the patient if the measured AKR1C3 reductase content is equal to or greater than a predetermined value;

b. determining the corresponding RNA expression level of AKR1C3 reductase of cancer cells or tissues in a patient, and administering the above drug or the compound with any one of the above structures A, B, and C, or a pharmaceutically acceptable salt, a prodrug, a solvate, or an isotopic variant thereof to the patient if the measured RNA expression level is in a predetermined range.

The AKR1C3 reductase content can be determined using methods including but not limited to ELISA assay

and IHC assay.

Liquid samples such as plasma and blood sample can be directly detected using a commercially available human aldo-keto reductase 1C3 (AKR1C3) ELISA assay kit. Other samples are detected after being treated.

The immunohistochemical (IHC) method is suitable for detecting solid tumor samples.

Studies show that after radiotherapy, the content of AKR1C3 enzyme in the tumor tissue of patients with head and neck cancer is increased. Therefore, it is possible to increase the expression level of AKR1C3 enzyme by irradiating the tumor tissue in a patient with radioactive rays used in radiotherapy. The radioactive rays include α-, β-, and γ-rays produced by radioisotopes, and X-rays, electron rays, proton beams, and other particle beams produced by various X-ray therapeutic machines or accelerators.

The present invention provides another method for treating cancer or tumor, comprising a step of adjusting the content of AKR1C3 reductaset, wherein the above drug or the compound with any one of the above structures A, B, and C, or a pharmaceutically acceptable salt, a prodrug, a solvate, or an isotopic variant thereof is administered to the patient when the content of AKR1C3 reductase is adjusted to be equal to or greater than a predetermined value.

This method is mainly for the situation where the content of AKR1C3 reductase in a patient is relatively low, and is performed by adjusting the content of AKR1C3 reductase in the patient to an appropriate level through a certain adjustment treatment/administration process.

The present invention provides a method for preparing an AKR1C3-activated compound of the following structure I (compound A, B, or C), comprising subjecting compound I-1, I-2, or I-3 to a ring-closure reaction to obtain the corresponding compound A, B, or C:

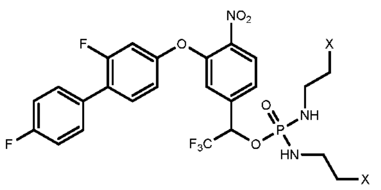
I-1

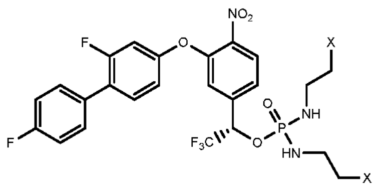
I-2

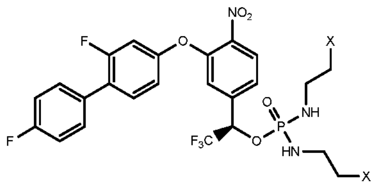
I-3

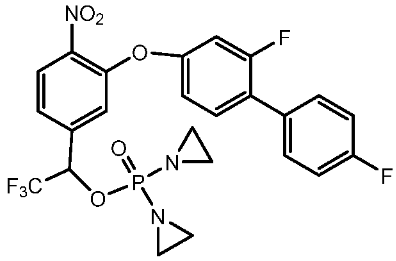
A

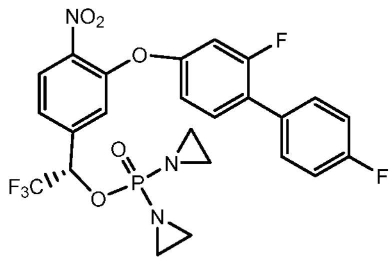
B

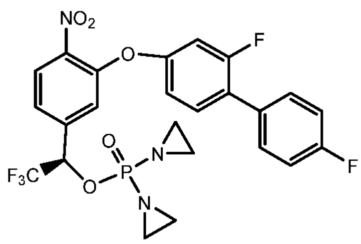
C

wherein Xs are each independently fluorine, chlorine, bromine, or iodine, preferably bromine.

In the above preparation method, silver oxide or silver nitrate is used as a catalyst in the ring-closure reaction, with or without the addition of an organic base. The organic base is preferably N,N-diisopropylethylamine or triethylamine.

The present invention provides a compound with any one of the following structural formulae:

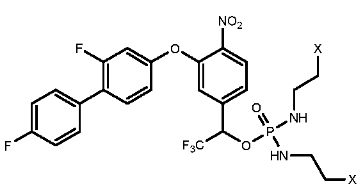
I-1

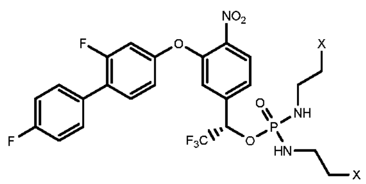
I-2

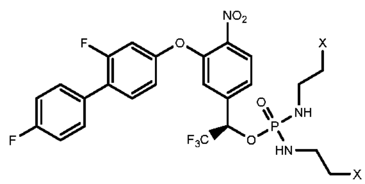
I-3

wherein Xs are each independently fluorine, chlorine, bromine, or iodine, preferably bromine.

The present invention provides use of the compound of the above structure I-1, I-2, or I-3, as an intermediate for the synthesis of the above AKR1C3-activated DNA alkylating agent (compound A, B, or C, or a pharmaceutically

acceptable salt, a prodrug, a solvate, or an isotopic variant thereof), or in the preparation of a drug for the treatment of cancer or tumor patients, conditions caused by cancer or tumor, or cell proliferative diseases.

The present invention provides a method for synthesizing the compound with the following structure I-1, I-2, or I-3, comprising performing the first reaction of compound II-1, II-2, or II-3 as the starting reactant with phosphorus oxyhalide to obtain an intermediate, respectively, then performing the second reaction of the intermediate with haloethylamine or haloethylamine hydrohalide, and finally obtaining the corresponding compound I-1, I-2, or I-3, respectively:

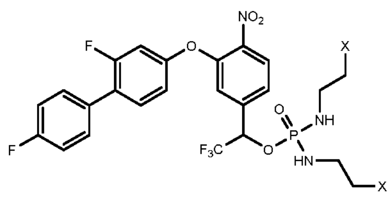

I-1

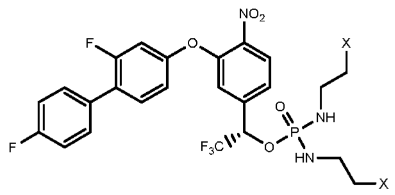

I-2

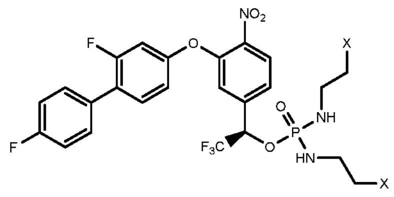

I-3

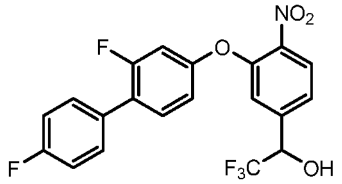

II-1

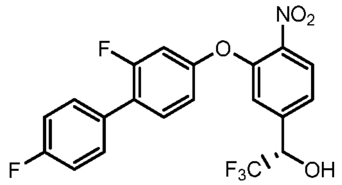

II-2

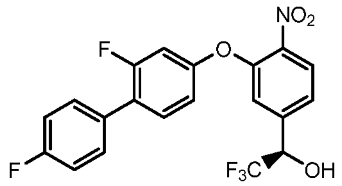

II-3

wherein the two Xs in compound I-1, I-2, or I-3 are each independently F, Cl, Br, or I, preferably Br;

wherein the phosphorus oxyhalide is selected from phosphorus oxyfluoride, phosphorus oxychloride, phosphorus oxybromide, and phosphorus oxyiodide, and is preferably phosphorus oxychloride;

wherein the halogen atoms in the phosphorus oxyhalide and the two Xs in the compound I-1, I-2, or I-3 may be the same or different;

preferably, the haloethylamine is bromoethylamine, and the haloethylamine hydrohalide is bromoethylamine hydrobromide.

The present invention provides a compound with any one of the following structures:

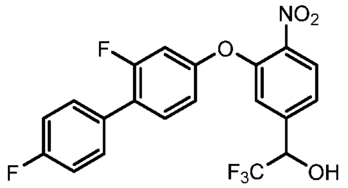

II-1

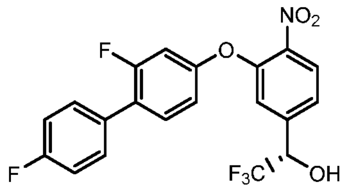

II-2

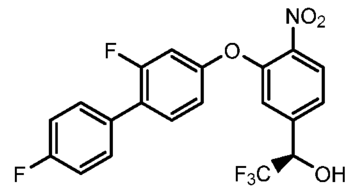

II-3

The present invention provides a method for synthesizing any one of the compounds with the following structures:

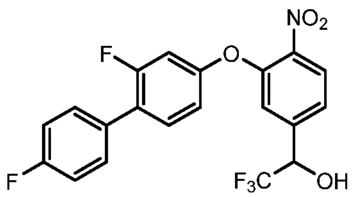

II-1

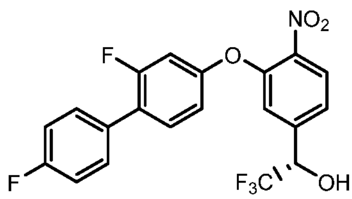

II-2

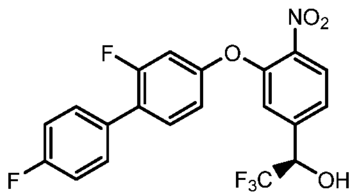

II-3

Compound III-3 is reacted with $TMSCF_3$ in contact and hydrolyzed after deprotection to obtain racemic alcohol II-1, and chiral resolution operation is performed on racemic alcohol II-1 to obtain chiral alcohol II-2 and chiral alcohol II-3, respectively.

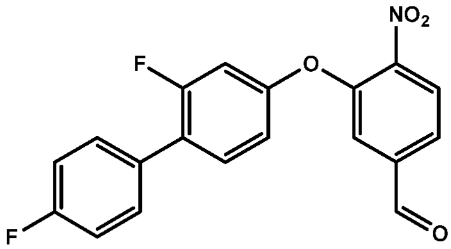

III-3

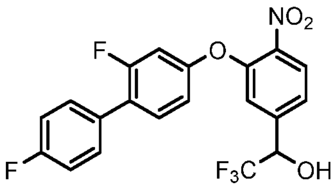

II-1

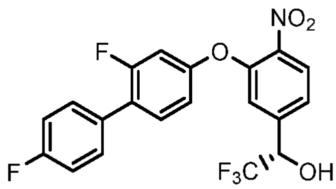

II-2

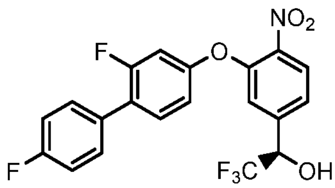

II-3 .

The $TMSCF_3$ is trimethyl(trifluoromethyl)silane, and the deprotection reagent used for the deprotection operation is tetrabutylammonium fluoride (TBAF), and ammonium fluoride, aqueous ammonium chloride or hydrochloric acid is used for hydrolysis operation.

Methods of the chiral resolution include crystallization method and chemical resolution method.

The present invention provides a method for separating the racemate of compound A, to obtain an enantiomer or to increase the concentration of either of the enantiomers of the compound in the compound to be excessive, comprising the following steps:

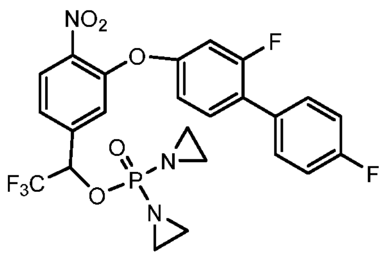

A

subjecting the racemic compound to optical resolution operation, wherein the operation is performed by chiral chromatography comprising stationary phase and mobile phase, wherein the stationary phase comprises silica gel impregnated with a functionalized polysaccharide, and wherein the mobile phase comprises an alcohol and a further solvent.

Preferably, the alcohol is ethanol and the further solvent is n-hexane; the temperature of the chromatography

column during the operation of the chiral chromatography is 38°C; and the functionalized polysaccharide is amylose tris(3,5-dimethylphenylcarbamate).

The present invention provides a further HPLC method for separating the racemate of compound A, to obtain an enantiomer or to increase the concentration of either of the enantiomers of the racemate in the compound to be excessive, wherein the chromatography column used in the HPLC method is CHIRALPAK® AD, and the mobile phase is ethanol, and the column temperature is 35°C.

The present invention provides a method for preparing any one of the deuterated compounds with the following structures, comprising subjecting compounds IV-1 to IV-21 to a ring-closure reaction to obtain the corresponding deuterated compounds V-1 to V-15, respectively:

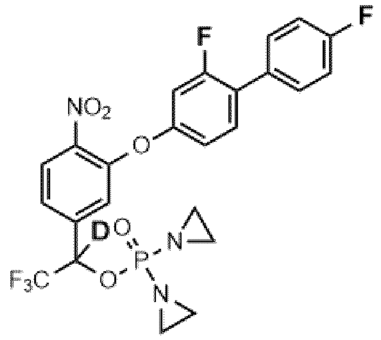,

V-1

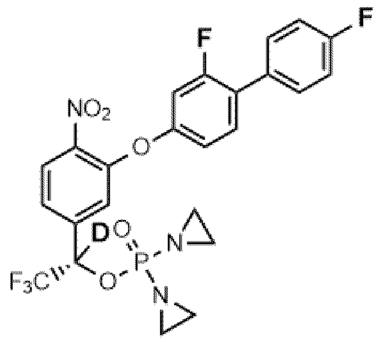,

V-2

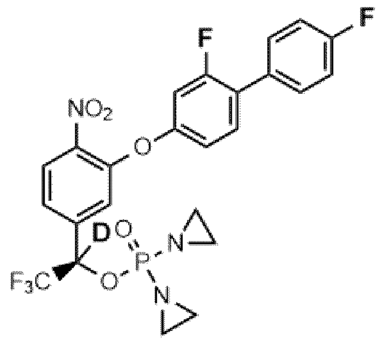,

V-3

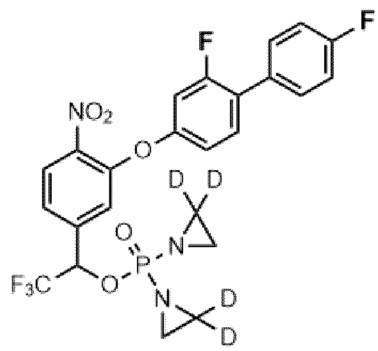,

V-4

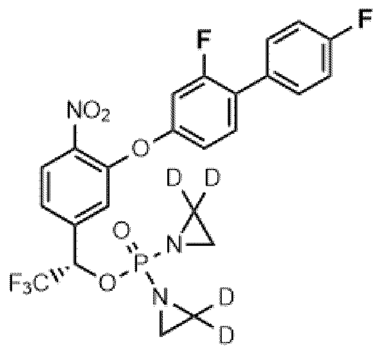,

V-5

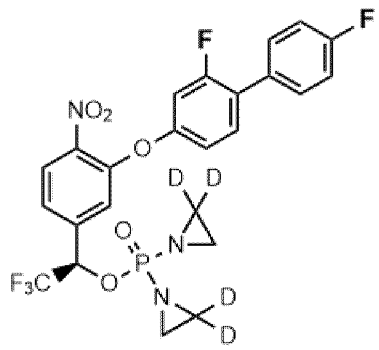,

V-6

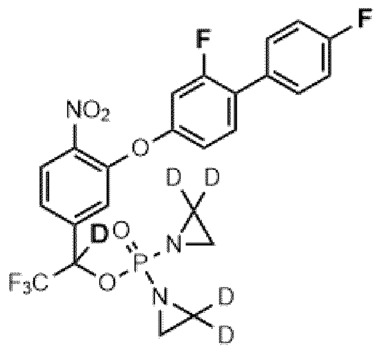,

V-7

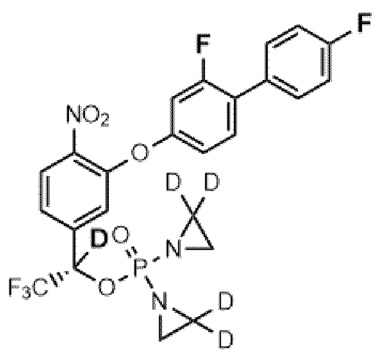,

V-8

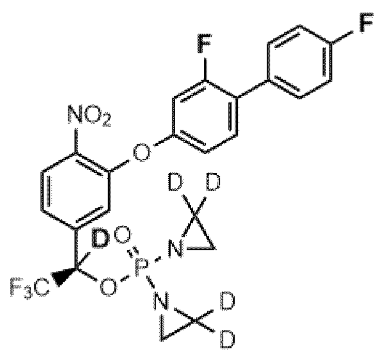,

V-9

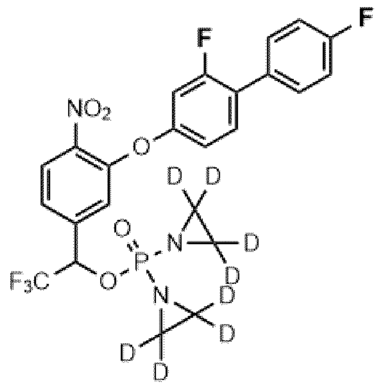

,

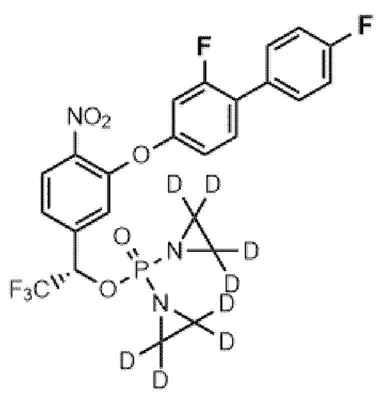

,

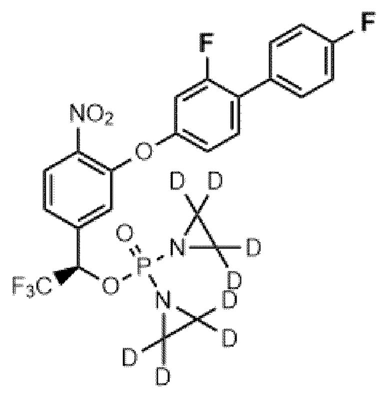

,

V-10

V-11

V-12

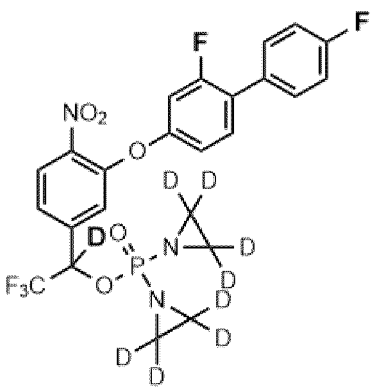

,

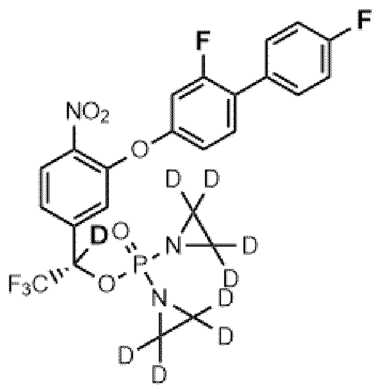

,

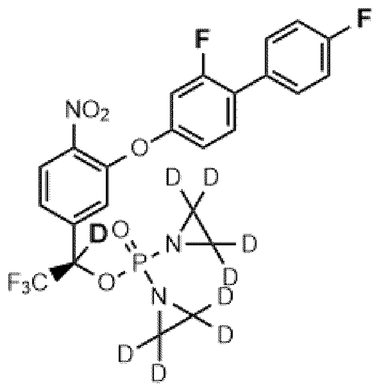

,

V-13

V-14

V-15

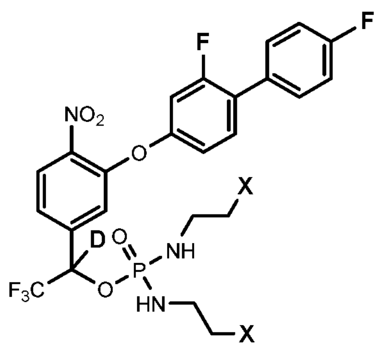

,

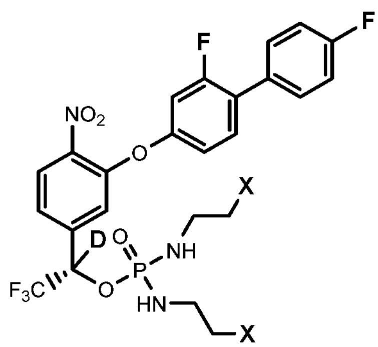

,

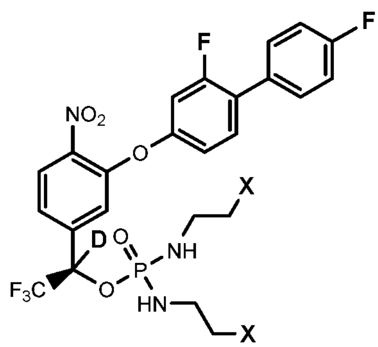

,

IV-1

IV-2

IV-3

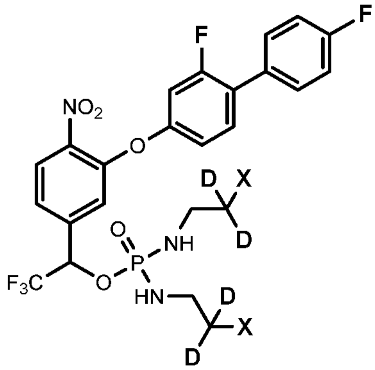

,

IV-4

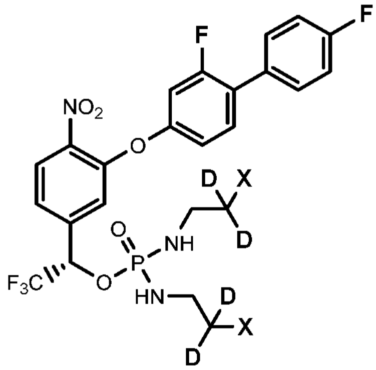

,

IV-5

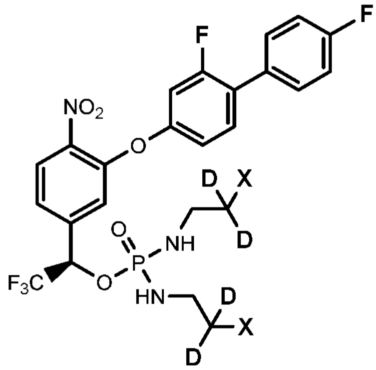

,

IV-6

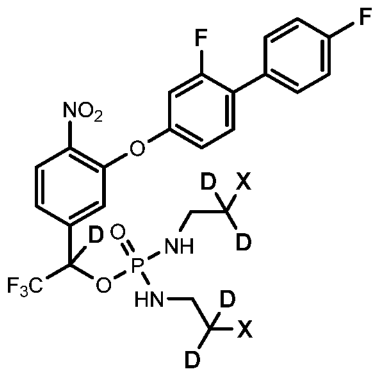

,

IV-7

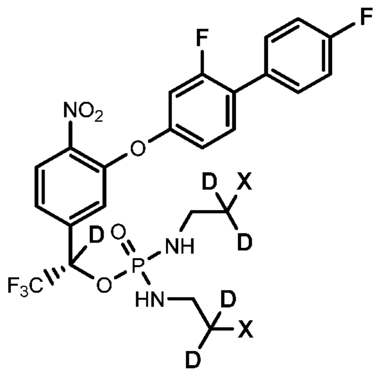

,

IV-8

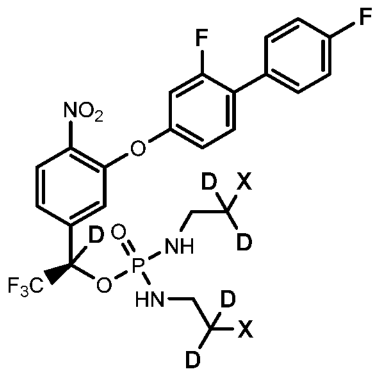

,

IV-9

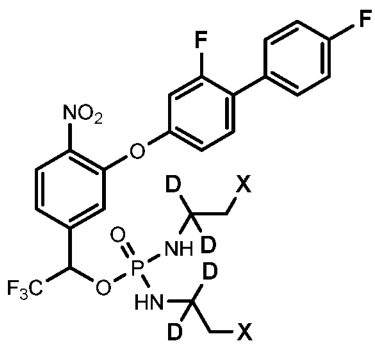

,

IV-10

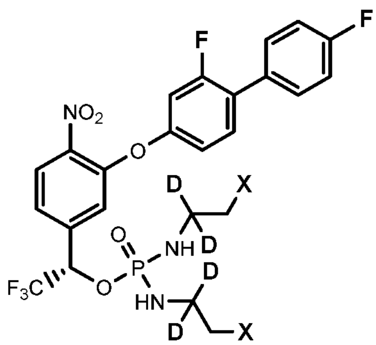

,

IV-11

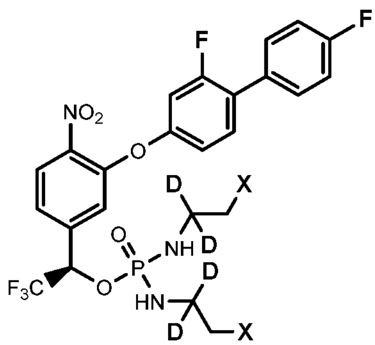

,

IV-12

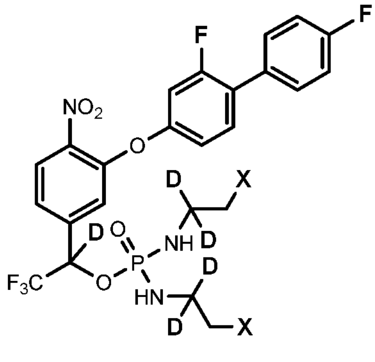

,

IV-13

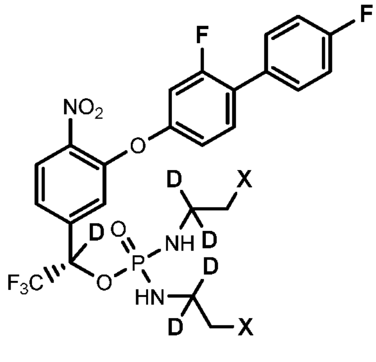

,

IV-14

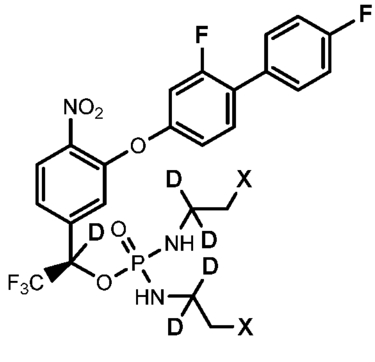

,

IV-15

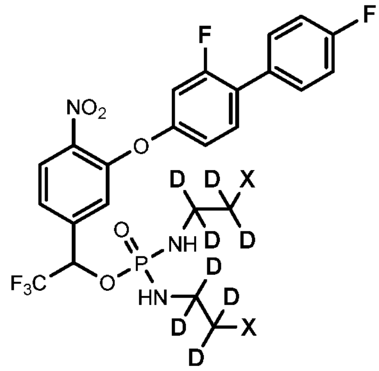,

IV-16

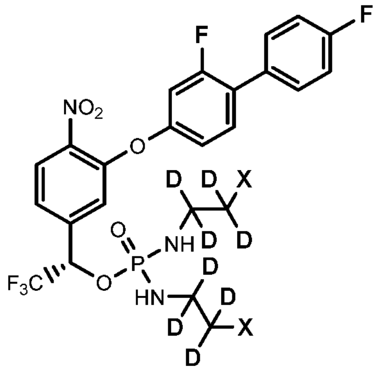,

IV-17

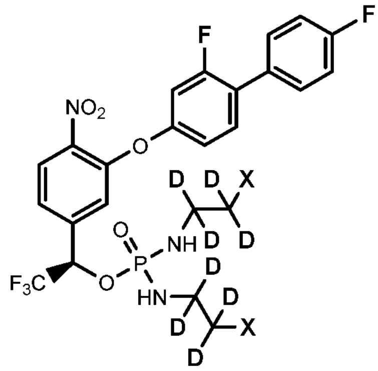,

IV-18

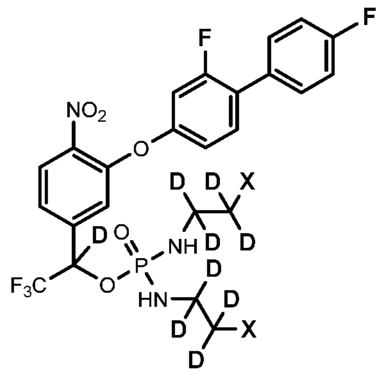,

IV-19

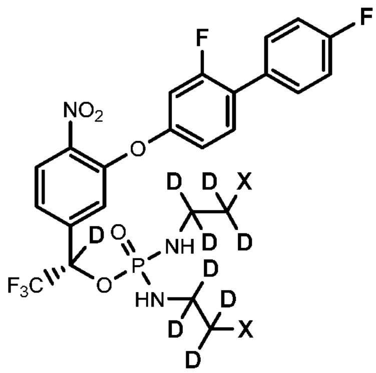,

IV-20

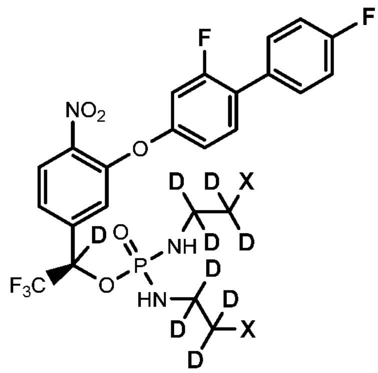,

IV-21

wherein the two X atoms in any one of compounds IV-1 to IV-21 are each independently fluorine, chlorine, bromine, or iodine, preferably bromine;

wherein silver oxide or silver nitrate is used as a catalyst in the ring-closure reaction, with or without the addition of a base.

The present invention provides a method for preparing any one of the above compounds IV-1 to IV-21, comprising performing the first reaction of compound II-1, II-2, or II-3 or the deuterated compound thereof II-1a, II-2a, or II-3a as the starting reactant with phosphorus oxyhalide to obtain an intermediate, respectively, then performing the second reaction of the intermediate with the corresponding deuterated haloethylamine VI-1 or VI-2 or hydrohalide of the deuterated haloethylamine VI-1 or VI-2, and finally obtaining the corresponding compounds IV-1 to IV-21:

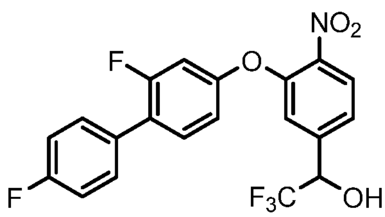,

II-1

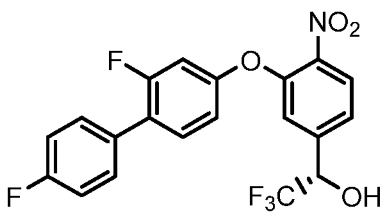,

II-2

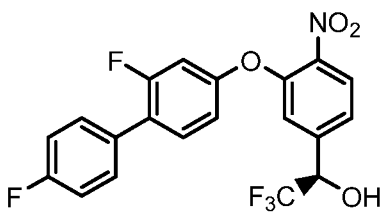,

II-3

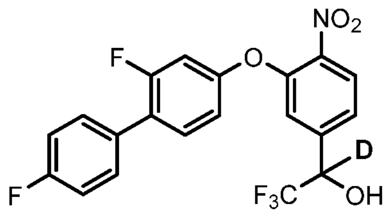, 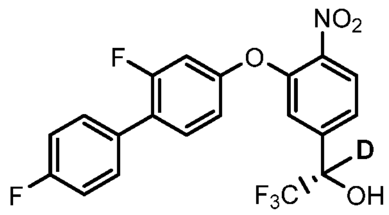, 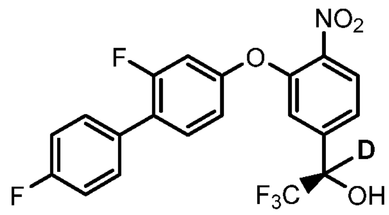

II-1a II-2a II-3a

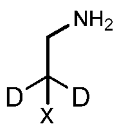 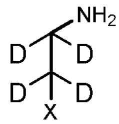

VI-1 VI-2

wherein the X atom in compound VI-1 or VI-2 is independently fluorine, chlorine, bromine, or iodine, and corresponds to the X atom in compounds IV-1 to IV-21;

wherein the phosphorus oxyhalide is selected from phosphorus oxyfluoride, phosphorus oxychloride, phosphorus oxybromide, and phosphorus oxyiodide, and is preferably phosphorus oxychloride;

wherein the halogen in the phosphorus oxyhalide and the two X atoms in any one of compounds IV-1 to IV-21 or the X atom in compound VI-1 or VI-2 may be the same or different;

wherein in the hydrohalide of the deuterated haloethylamine, the halogen in hydrohalide and the halogen in deuterated haloethylamine are the same;

preferably, the haloethylamine is bromoethylamine, and the haloethylamine hydrohalide is bromoethylamine hydrobromide.

The present invention provides a method for preparing any one of the compounds with the following structures, comprising the following steps: reacting compound

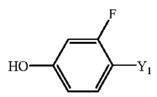

as the starting material to obtain compound

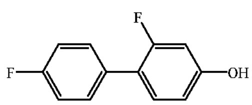,

then reacting the compound

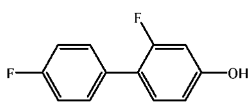

with formula I-7-1, I-7-2, or I-7-3 to obtain the corresponding compound of formula A, B, or C, respectively.

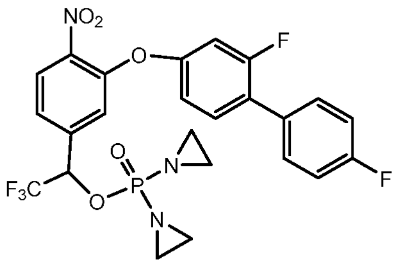,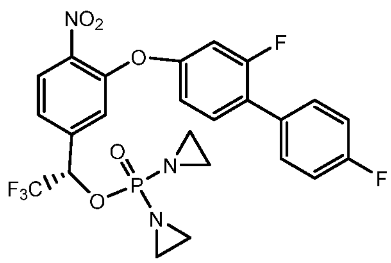,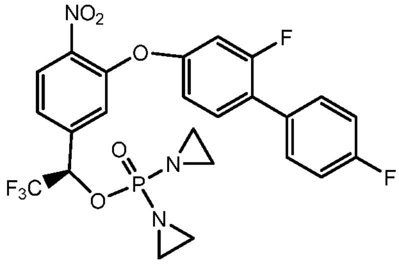

A B C

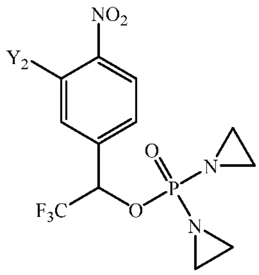,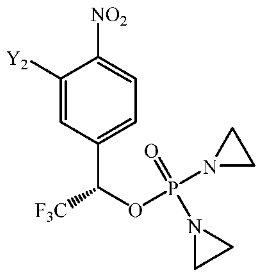,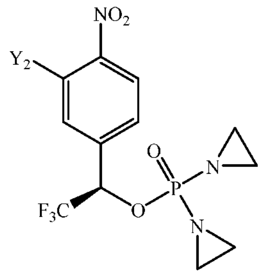

I-7-1 I-7-2 I-7-3

Specifically, $Y_1$ and $Y_2$ in the structures of the compounds are halogen or OM, and M is hydrogen, sodium, potassium, magnesium, or calcium, and halogen is preferably bromine.

Specifically, when M is magnesium or calcium, the amount of other groups in the structures of the compounds is twice as much as when M is hydrogen, sodium, or potassium.

Regarding the drug described herein, the prepared drug contains a specific dosage range of the shown compounds or salts or solvates or isotopic variants thereof, and/or the prepared drug is in a specific dosage form and is administered using a specific administration method.

Regarding the use described herein, the prepared drug may also contain pharmaceutically acceptable auxiliaries or excipients. The medicament can be any dosage form for clinical administration, such as tablets, suppositories, dispersible tablets, enteric-coated tablets, chewable tablets, orally disintegrating tablets, capsules, sugar coated agents, granules, dry powders, oral solutions, a small needle for injection, lyophilized powder for injection, or infusion solutions. According to the specific dosage form and the mode of administration, the pharmaceutically acceptable auxiliaries or excipients in the medicament may include one or more of the following: diluent, solubilizer, disintegrant, suspension, lubricant, adhesive, filler, flavoring agent, sweetener, antioxidant, surfactant, preservative, wrapping agent and pigment, etc.

Preferably, the patient is a mammal, more preferably a human.

## Brief Description of the Drawings

Fig. 1 shows the high performance liquid chromatography analysis spectrum of compound A.

Fig. 2 shows the high performance liquid chromatography analysis spectrum of compound A-P1 obtained after separation of compound A.

Fig. 3 shows the high performance liquid chromatography analysis spectrum of compound A-P2 obtained after separation of compound A.

Fig. 4 shows the $^1$HNMR spectrum of a specific compound V-10.

Fig. 5 shows the curves of inhibition rates of compounds A, B, C, and AST-3424 at different concentrations on H460 cells under normoxic conditions, wherein Log.conc (nM) represents the logarithm value of the concentration value in nmol/L with base 10.

Fig. 6 shows the curves of inhibition rates of compounds A, B, and C at different concentrations on different liver cancer cell lines, wherein Log.conc (nM) represents the logarithm value of the concentration value in nmol/L with base 10.

Fig. 7 shows the curves of inhibition rates of compounds A, B, and C at different concentrations on H460 cancer cells in the presence or absence of AKR1C3 inhibitor AST3021, wherein Log.conc (nM) represents the logarithm value of the concentration value in nmol/L with base 10.

Fig. 8 shows the curve of AKR1C3 dependence of compound A on 10 strains of non-small cell lung cancer, wherein FPKM is Fragments Per Kilobase Million in RNA-seq, then logarithm with base 2 is taken to obtain $Log_2FPKM$, and $IC_{50}$ is 50% inhibition concentration, i.e., the corresponding concentration when cell survival is half of that of the control sample.

Fig. 9 shows the curve of AKR1C3 dependence of compound AST-3424 on 10 strains of non-small cell lung cancer, wherein FPKM is Fragments Per Kilobase Million in RNA-seq, then logarithm with base 2 is taken to obtain $Log_2FPKM$, and $IC_{50}$ is 50% inhibition concentration, i.e., the corresponding concentration when cell survival is half of that of the control sample.

Fig. 10 shows the curves of tumor volume change on different days in the *in vivo* pharmacodynamic experiment of AKR1C3 dependence of compound A and Sorafenib in the liver cancer LI6280 PDX model.

Fig. 11 shows the curves of tumor volume change on different days in the *in vivo* pharmacodynamic experiment of compound A, AST-3424, and Ifosfamide in the gastric cancer GA6201 PDX model.

Fig. 12 shows the curves of tumor volume change on different days after drug withdrawal on Day 14 in the *in vivo* pharmacodynamic experiment of compound A and AST-3424 in the gastric cancer GA6201 PDX model.

Fig. 13 shows the curves of body weight change rates of mice on different days in the *in vivo* pharmacodynamic experiment of compound A, AST-3424, and Ifosfamide in the gastric cancer GA6201 PDX model.

Fig. 14 shows the curves of tumor volume change on different days in the *in vivo* pharmacodynamic experiment of compound A and Carboplatin in the human-derived non-small cell lung cancer NCI-H460 subcutaneous xenograft model.

Fig. 15 shows the curves of body weight change rates of mice on different days in the *in vivo* pharmacodynamic experiment of compound A and Carboplatin in the human-derived non-small cell lung cancer NCI-H460 subcutaneous xenograft model.

Fig. 16 shows the curves of tumor volume change on different days in the *in vivo* pharmacodynamic experiment of compound A and Sorafenib in the human liver cancer LI6652 subcutaneous xenograft PDX model.

Fig. 17 shows the curves of body weight change rates of mice on different days in the *in vivo* pharmacodynamic experiment of compound A and Sorafenib in the human liver cancer LI6652 subcutaneous xenograft PDX model.

Fig. 18 shows the curves of biofluorescence value change on different days in the *in vivo* pharmacodynamic experiment of compound A and Paclitaxel in the human-derived non-small cell lung cancer NCI-H460-Luc2 intracranial inoculation CDX model, wherein a high biofluorescence value indicates a large tumor volume.

Fig. 19 shows the curves of body weight change rates of mice on different days in the *in vivo* pharmacodynamic experiment of compound A and Paclitaxel in the human-derived non-small cell lung cancer NCI-H460-Luc2 intracranial inoculation CDX model.

Fig. 20 shows the curves of tumor volume change on different days in the *in vivo* pharmacodynamic experiment of compound A and Gemcitabine in the pancreatic cancer PA1222 PDX model.

Fig. 21 shows the curves of body weight change rates of mice for different days in the *in vivo* pharmacodynamic experiment of compound A and Gemcitabine in the pancreatic cancer PA1222 PDX model.

Fig. 22 shows the curves of tumor volume change on different days in the *in vivo* pharmacodynamic experiment of compound A and Paclitaxel in the lung cancer LU2505 PDX model.

Fig. 23 shows the curves of body weight change rates of mice on different days in the *in vivo* pharmacodynamic experiment of compound A and Paclitaxel in the lung cancer LU2505 PDX model.

Fig. 24 shows the curves of tumor volume change on different days in the *in vivo* pharmacodynamic experiment of Sorafenib, compound A 0.5 mg/kg, 1 mg/kg, and 2 mg/kg, and AST-3424 at different concentrations in the liver cancer LI6643 PDX model.

Fig. 25 shows the curves of tumor volume change on different days in the *in vivo* pharmacodynamic experiment of Sorafenib and compound A 1.25 mg/kg, 2.5 mg/kg, 5 mg/kg, and 10 mg/kg in the liver cancer LI6643 PDX model.

Fig. 26 shows the curves of body weight change rates of mice on different days in the *in vivo* pharmacodynamic experiment of Sorafenib and compound A 1.25 mg/kg, 2.5 mg/kg, 5 mg/kg, and 10 mg/kg in the liver cancer LI6643 PDX model.

Fig. 27 shows the curves of tumor volume change on different days in the *in vivo* pharmacodynamic experiment of compound A and Sorafenib in the liver cancer LI1005 PDX model.

Fig. 28 shows the curves of body weight change rates of mice on different days in the *in vivo* pharmacodynamic experiment of compound A and Sorafenib in the liver cancer LI1005 PDX model.

Fig. 29 shows the curves of tumor volume change on different days in the *in vivo* pharmacodynamic experiment of compound A and Sorafenib in the liver cancer HepG2 CDX model.

Fig. 30 shows the curves of body weight change rates of mice on different days in the *in vivo* pharmacodynamic experiment of compound A and Sorafenib in the liver cancer HepG2 CDX model.

Fig. 31 shows the curves of tumor volume change on different days in the *in vivo* pharmacodynamic experiment of compound A and Paclitaxel in the lung cancer LU0884 PDX model.

Fig. 32 shows the curves of body weight change rates of mice on different days in the *in vivo* pharmacodynamic experiment of compound A and Paclitaxel in the lung cancer LU0884 PDX model.

Fig. 33 shows the curves of tumor volume change on different days in the *in vivo* pharmacodynamic experiment of compound A, AST-3424, and Sorafenib in the liver cancer LI6664 PDX model.

Fig. 34 shows the curves of body weight change rates of mice on different days in the *in vivo* pharmacodynamic experiment of compound A, AST-3424, and Sorafenib in the liver cancer LI6664 PDX model.

Fig. 35 shows the curves of concentrations of compound A versus time in plasma and in brain tissues.

Fig. 36 shows the curve of the ratio between concentrations of compound A in plasma and in brain tissues versus time.

Fig. 37 shows the curves of concentrations of compound AST-2870 versus time in plasma and in brain tissues.

Fig. 38 shows the curve of the ratio between concentrations of compound AST-2870 in plasma and in brain tissues versus time.

Fig. 39 shows the curves of the remaining amount of compound A with the addition of NADPH, without the addition of NADPH, and with the addition of AKR1C3 inhibitor and of control drug progesterone after reaction in mouse, monkey, rat, and human hepatocyte cytosol solution over time, wherein Remaining represents metabolic remaining rate, and Time represents time, and AST represents compound A, and Progesterone represents progesterone.

Fig. 40 shows the curves of the remaining amount of compound AST-3424 with the addition of NADPH, without the addition of NADPH, and with the addition of AKR1C3 inhibitor and of control drug progesterone after reaction in mouse, monkey, rat, and human hepatocyte cytosol solution over time, wherein Remaining represents metabolic

remaining rate, and Time represents time, and Progesterone represents progesterone.

## Detailed Description of the Invention

The present invention will be described below with the reference to specific examples. Those skilled in the art will understand that these examples are only used to illustrate the present invention and do not limit the scope of the present invention in any way.

The experimental methods in the following examples are conventional methods unless specified otherwise. The medicinal raw materials, reagent materials, etc., used in the following examples are all commercially available products unless specified otherwise.

"Prodrug" refers to a compound that, after administration, is metabolized or otherwise converted to a biologically active or more active compound (or drug) with respect to at least one property. A prodrug, relative to the drug, is modified chemically in a manner that renders it, relative to the drug, less active or inactive, but the chemical modification is such that the corresponding drug is generated by metabolic or other biological processes after the prodrug is administered. A prodrug may have, relative to the active drug, altered metabolic stability or transport characteristics, fewer side effects or lower toxicity, or improved flavor. A prodrug may be synthesized using reactants other than the corresponding drug.

"Administering" or "administration of" a drug to a patient (and the grammatical equivalents of this phrase) refers to direct administration, which may be administration to a patient by a medical professional or may be self-administration, and/or indirect administration, which may be the act of prescribing a drug. For example, a physician who instructs a patient to self-administer a drug and/or provides a patient with a prescription for a drug is administering the drug to the patient.

"Cancer" refers to leukemia, lymphoma, carcinoma, and other malignant tumors (including solid tumors) with potentially unrestrained growth that can expand locally by invasion and systemically by metastasis. Examples of cancer include (but are not limited to) cancer of the adrenal gland, bone, brain, breast, bronchus, colon and/or rectum, gallbladder, head and neck, kidney, larynx, liver, lung, nervous tissue, pancreas, prostate, accessory thyroid gland, skin, stomach, and thyroid gland. Certain other examples of cancer include acute and chronic lymphocytic and granulocytic neoplasms, adenocarcinoma, adenoma, basal cell carcinoma, cervical epithelial dysplasia and carcinoma *in situ,* Ewing's sarcoma, epidermoid carcinoma, giant cell tumor, glioblastoma multiforme, pilocytic tumor, intestinal ganglioneuroma, hyperplastic corneal nerve tumor, islet cell carcinoma, Kaposi's sarcoma, leiomyoma, leukemia, lymphoma, malignant carcinoid, malignant melanoma, malignant hypercalcemia, marfanoid habitus tumor, medullary epithelial carcinoma, metastatic skin cancer, mucosal neuroma, myeloma, mycosis fungoides, neuroblastoma, osteosarcoma, osteogenic and other sarcomas, ovarian tumor, pheochromocytoma, polycythemia vera, primary brain tumor, small cell lung cancer, ulcerative and papillary squamous cell carcinoma, hyperplasia, seminoma, soft tissue sarcoma, retinoblastoma, rhabdomyosarcoma, renal cell tumor, local skin lesion, reticulum cell sarcoma, and Wilm's tumor.

"Patient" and "individual" are used interchangeably and refer to a mammal in need of treatment for cancer. Typically, the patient is a human. Typically, the patient is a human diagnosed with cancer. In certain embodiments, a "patient" or "individual" may refer to a non-human mammal, such as a non-human primate, a dog, a cat, a rabbit, a pig, a mouse, or a rat used for screening, characterizing, and evaluating drugs and therapies.

"Solid tumors" refer to solid tumors including (but not limited to) metastatic tumors in the bone, brain, liver, lung, lymph node, pancreas, prostate, skin, and soft tissue (sarcoma).

"Therapeutically effective amount" of a drug refers to an amount of a drug that, when administered to a patient with cancer, has the intended therapeutic effect (e.g., alleviation, amelioration, palliation, or elimination of one or more clinical manifestations of cancer in the patient). A therapeutic effect does not necessarily occur by administration of one dose, and may occur only after administration of a series of doses. Thus, a therapeutically effective amount may be administered in one or more administrations.

"Treating", "treatment of" or "therapy of" a condition or a patient refers to taking steps to obtain beneficial or desired results (including clinical results). For purposes of the present invention, beneficial or desired clinical results include (but are not limited to) alleviation or amelioration of one or more symptoms of cancer; diminishment of extent of disease; delay or slowing of disease progression; amelioration, remission, or stabilization of disease status; or other beneficial results.

"Tumor cells" refer to tumor cells of any appropriate species (e.g., a mammal, such as a murine, a canine, a feline, an equine, or a human).

"Treatment" or "treatment of a patient" is to administer or use a therapeutically effective amount of a drug in relation to the present invention to a patient.

"Administering" or "administration of" or "use of" a drug to a patient refers to direct administration, which may be administration to a patient by a medical professional or may be self-administration, and/or indirect administration, which may be the act of prescribing a drug. For example, a physician who instructs a patient to self-administer a drug and/or provides a patient with a prescription for a drug is administering the drug to the patient.

The above description of specific embodiments of the present invention does not limit the present invention. Those skilled in the art can make various modifications and changes according to the present invention, and any modification and change within the spirit of the present invention shall be covered in the scope of the claims appended to the present invention.

## I. Synthesis, preparation and structure determination of compounds

Abbreviations note:

| Abbreviation | Full Name |
|---|---|
| THF | tetrahydrofuran |
| DCM | dichloromethane |
| EA | ethyl acetate |
| TEA | triethylamine |
| MTBE | methyl tert-butyl ether |
| DMAP | 4-dimethylaminopyridine |
| DBAD | di-tert-butyl azodicarboxylate |
| TFA | trifluoroacetic acid |
| EtOH | ethanol |
| t-BuOH | tert-butanol |
| DMF | N,N-dimethylformamide |
| PE | petroleum ether |
| DMF-DMA | N,N-dimethylformamide dimethyl acetal |
| TBAF | tetrabutylammonium fluoride |
| DIPEA or DIEA | N,N-diisopropylethylamine |
| DIAD | diisopropyl azodiformate |
| t-BuOK | potassium tert-butoxide |
| MS | mass spectrometry |
| HPLC | high performance liquid chromatograph |
| Calculated | theoretical calculating value of mass spectrometry |
| found | measured value of mass spectrometry |
| eq | equivalent |

Other abbreviations or terms that are not specified can be interpreted or operated according to the definitions or instructions in the Handbook for Organic Chemistry or Organic Synthesis.
All reagents or drugs that are not specified are purchased commercially.
For $^1$H-NMR NMR test, if not specified, 400MHz instrument is used for determination; for MS mass spectrometry test, LC-MS liquid chromatography-mass spectrometry instrument is used for determination.

1.1 Synthesis of Compound A (i.e., Compound 16)

Method 1:

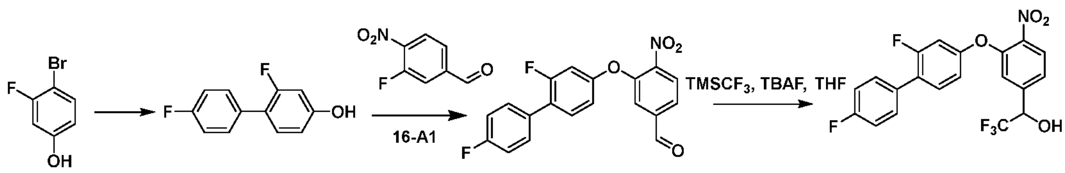


16-A0 16-A2 16-A3

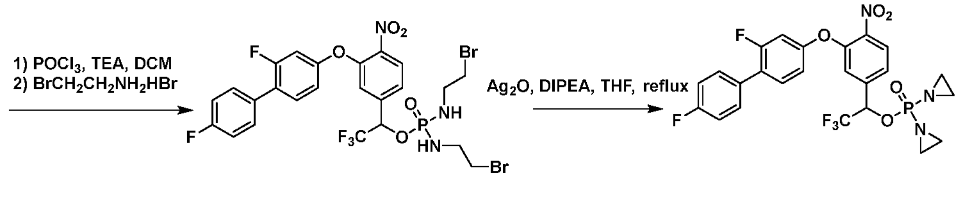


16-A4 16

Synthesis route of Compound A

The synthetic method of Compound A is the same as the synthetic method of Compound No. 16 in Patent PCT/CN2020/089692, and the synthetic method of Compound No. 16 in that patent is hereby incorporated herein by reference.

Method 2:

The target Compound A was obtained by a two-step reaction using 3-fluoro-4-bromo-phenol as starting material.

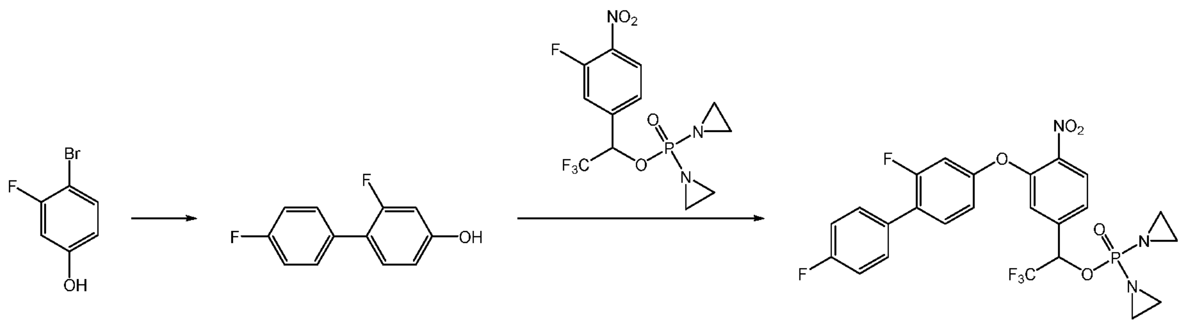

Method 3:

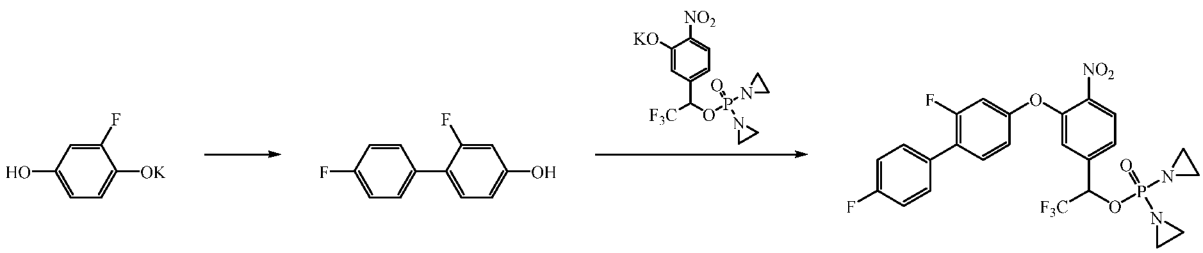

1.2 Chiral resolution of Compound A

The Compound A in pure form was subjected to chiral analysis

HPLC chiral analysis conditions were:

| | |
|---|---|
| Chromatographic column | CHIRALPAK AD-H(AD00CD-UE022) |
| Column size | internal diameter 0.46cm×length 15cm |
| Injection volumn | 0.2ul |
| Mobile phase | ethanol = 100% |
| Flow rate | 0.5 ml/min |
| Detection Wave length | UV 254nm |
| Column temperature | 35 °C |

The HPLC spectrum obtained upon test was shown in Fig. 1, and the specific results were shown in the following table.

| Compound | Separation Peak | Ret. Time (Retention time) | Area | Area% (Area ratio) |
|---|---|---|---|---|
| A-P1 | peak 1 | 4.808 | 3436401 | 51.036% |
| A-P2 | peak 2 | 5.791 | 3296947 | 48.964% |

4.4814g of Compound A was taken and dissolved in chromatographic grade ethanol. Chiral separation preparation was carried out using the following chiral separation preparation method and chiral separation preparation conditions.

HPLC chiral separation preparation method: the above chiral racemate (i.e., Compound A) were separated by HPLC method using HPLC preparation equipment and a chiral column, and the corresponding fractions were collected. The solvent was removed by rotary evaporation to obtain the pure product of the optical isomer.

The HPLC chiral separation preparation conditions were:

| | |
|---|---|
| Resolution chromatographic column | CHIRALPAK AD |
| Column size | internal diameter 5.0 cm, length 25 cm, filler particle size 10μm |
| Mobile phase | ethanol = 100% |
| Flow rate | 40 ml/min |
| Detection Wave length | UV 254 nm |
| Column temperature | 35°C |

After the separation preparation, 2.1432 g of pure product of the optical isomer corresponding to peak 1 was obtained, and the measured ee value was 99.2%; 2.0840 g of pure product of the optical isomer corresponding to peak 2 was obtained, and the measured ee value was 99.5%.

The optical isomer corresponding to peak 1 and the optical isomer corresponding to peak 2 from the chiral separation preparation were further analyzed using HPLC chiral analysis conditions, and the results were obtained as shown in Fig. 2 and Fig. 3.

The results of the HPLC analysis of the optical isomer corresponding to peak 1 were shown in the following table:

| Compound | Separatio n Peak | Ret. Time (Retention time) | Area | Area% (Area ratio) |
|---|---|---|---|---|
| A-P1 | peak 1 | 4.807 | 5645223 | 99.639% |
| A-P2 | peak 2 | 5.777 | 20453 | 0.361% |

The results of the HPLC analysis of the optical isomer corresponding to peak 2 were shown in the following table:

| Compound | Separation Peak | Ret. Time (Retention time) | Area | Area% (Area ratio) |
|---|---|---|---|---|
| A-P1 | peak 1 | 4.835 | 12783 | 0.216% |

(continued)

| Compound | Separation Peak | Ret. Time (Retention time) | Area | Area% (Area ratio) |
|---|---|---|---|---|
| A-P2 | peak 2 | 5.801 | 5918040 | 99.784% |

1.3 Study of chiral stability of Compound A and isomers A-P1 and A-P2 thereof

Isomer stability under long-term and accelerated conditions was studied.

Compound A and isomers A-P1 and A-P2 were investigated for traits and isomeric stability at - 20±5°C, 5°C±3°C, 25°C±2°C (60%±5% RH) and 40°C±2°C (75%±5% RH), respectively, and the results were shown in the following table:

| Compound | Isomer peak | Results of isomers (%) | | | | | | | | | | |
|---|---|---|---|---|---|---|---|---|---|---|---|---|
| | | 0 month | -20±5°C | 5°C±3°C | | | 25°C±2°C/60%±5%RH | | | 40°C±2°C/75%±5%RH | | |
| | | | 3 months | 1 month | 3 months | 6 months | 1 month | 3 months | 6 months | 1 month | 3 months | 6 months |
| A | Properties | Light yellow solid | NA | NA | NA | Light yellow solid | NA | NA | Light yellow solid | NA | NA | Light yellow solid |
| | A-P1 | 49.8 | NA | NA | NA | 49.9 | NA | NA | 49.9 | NA | NA | 49.9 |
| | A-P2 | 50.2 | NA | NA | NA | 50.1 | NA | NA | 50.1 | NA | NA | 50.1 |
| | Isomer transformation | **NA** | **NA** | **NA** | **NA** | **0.1** | **NA** | **NA** | **0.1** | **NA** | **NA** | **0.1** |
| A-P1 | Properties | Light yellow solid | Light yellow solid | Light yellow solid | Light yellow solid | / | Light yellow thick liquid | Light yellow thick liquid | / | Light yellow thick liquid | NA | NA |
| | A-P1 | 99.92 | 99.92 | 99.92 | 99.92 | / | 99.92 | 99.92 | / | 99.91 | NA | NA |
| | A-P2 | 0.08 | 0.08 | 0.08 | 0.08 | / | 0.08 | 0.08 | / | 0.09 | NA | NA |
| | Isomer transformation | **NA** | **0.00** | **0.00** | **0.00** | **/** | **0.00** | **0.00** | **/** | **0.01** | **NA** | **NA** |
| A-P2 | Properties | Light yellow solid | Light yellow solid | Light yellow solid | Light yellow solid | / | Light yellow thick liquid | Light yellow thick liquid | / | Light yellow thick liquid | NA | NA |
| | A-P1 | 0.25 | 0.26 | 0.25 | 0.25 | / | 0.25 | 0.25 | / | 0.26 | NA | NA |
| | A-P2 | 99.75 | 99.74 | 99.75 | 99.75 | / | 99.75 | 99.75 | / | 99.74 | NA | NA |
| | Isomer transformation | **NA** | **-0.01** | **0.00** | **0.00** | **/** | **0.00** | **0.00** | **/** | **0.01** | **NA** | **NA** |

Note: 1. The data in the table were calculated based on the peak areas in the original spectrum according to the formula "% content = individual isomer peak area ÷ (P1 peak area + P2 peak area) × 100%". 2. NA means not applicable, / means that the test was not performed.

The data in the above table demonstrated that after being placed at 5°C±3°C, 25°C±2°C (60%±5%RH) and 40°C±2°C (75%±5%RH) for 6 months, the properties and chirality of Compound A were essentially unchanged, indicating that the ratio of the two single isomers in Compound A was stable and the chirality of Compound A was stable.

Compounds A-P1 and A-P2 were placed at -20°C±5°C, 5°C±3°C, 25°C±2°C (60%±5%RH) for 3 months, and at 40°C±2°C (75%±5%RH) for 1 month, respectively, and there was essentially no change in the colour and chirality of the samples, indicating that under the above conditions the single isomers A-P1 and A-P would not be converted into each other and the chirality was stable.

1.4 Preparation of deuterated compounds

Deuterated compounds of formulas IV-1 to IV-21 were prepared using the following synthetic routes or steps:

Step 1, preparation of compounds IV-1 to IV-21, which comprises performing the first reaction of compound II-1, II-2, or II-3 or the deuterated compound thereof II-1a, II-2a, or II-3a as the starting reactant with phosphorus oxyhalide to obtain an intermediate, respectively, then performing the second reaction of the intermediate with the corresponding deuterated haloethylamine VI-1 or VI-2 or hydrohalide of the deuterated haloethylamine VI-1 or VI-2, and finally obtaining the corresponding compounds IV-1 to IV-21;

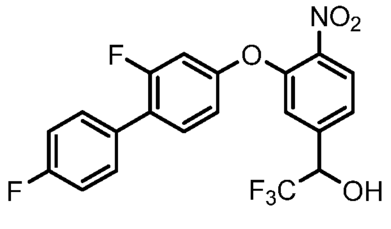
,
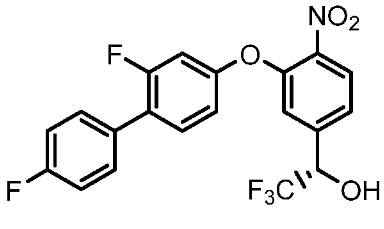
,
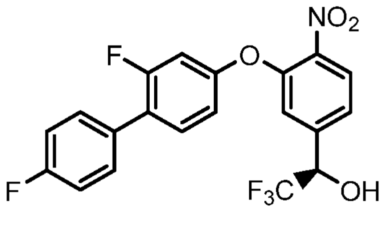
,

II-1 II-2 II-3

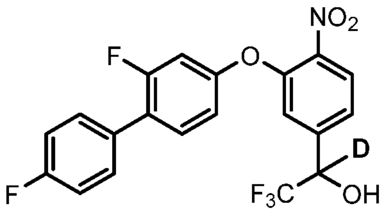
,
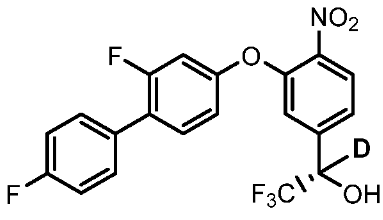
,
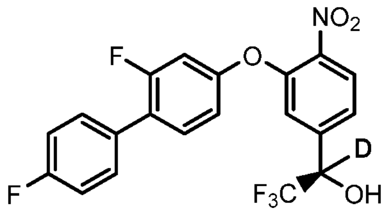

II-1a II-2a II-3a

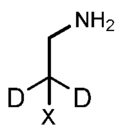
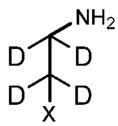

VI-1 VI-2

wherein X is each independently fluorine, chlorine, bromine, or iodine;

wherein in the hydrohalide of the deuterated haloethylamine, the halogen in hydrohalide and the halogen in deuterated haloethylamine are the same;

preferably, the haloethylamine is bromoethylamine and the haloethylamine hydrohalide is bromoethylamine hydrobromide.

Step 2, a method for preparation of deuterated compounds V-1 to V-15 with any one of the following structures, which comprises subjecting compounds IV-1 to IV-3 to a ring-closure reaction to obtain the corresponding the corresponding deuterated compounds V-1 to V-15, respectively;

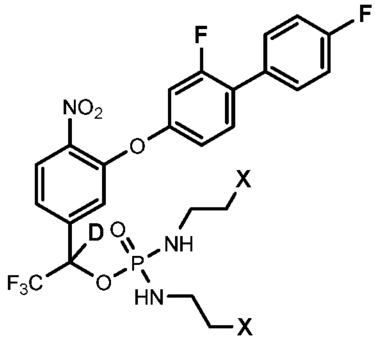
,

IV-1

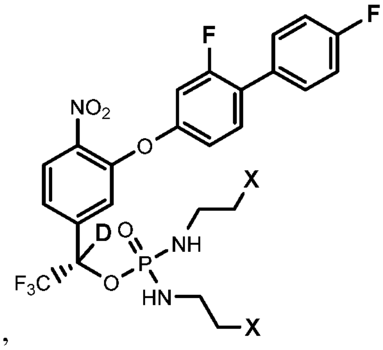
,

IV-2

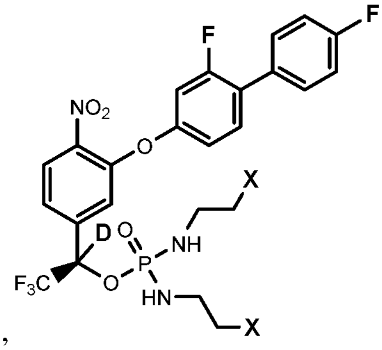
,

IV-3

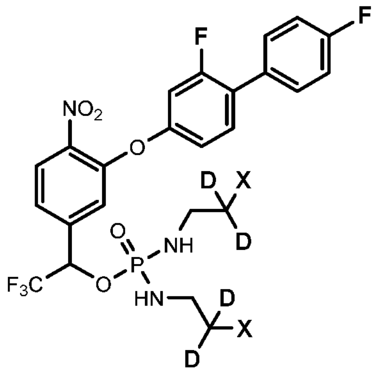
,

IV-4

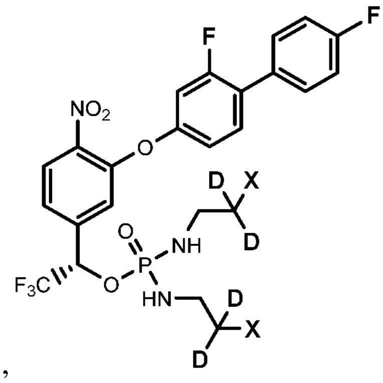
,

IV-5

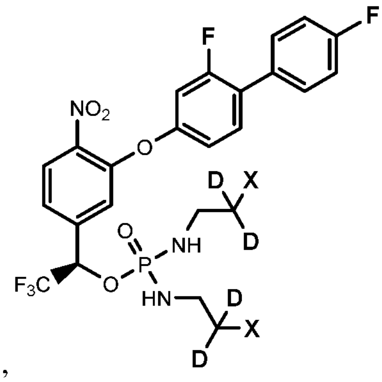
,

IV-6

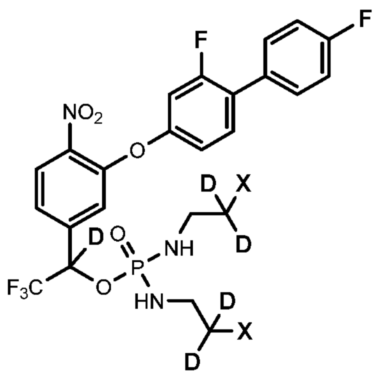
,

IV-7

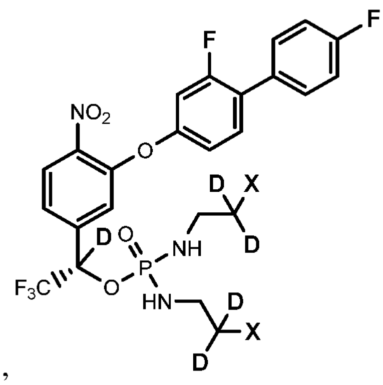
,

IV-8

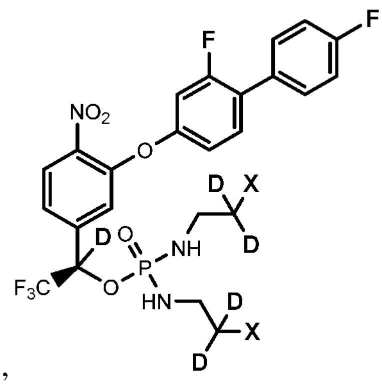
,

IV-9

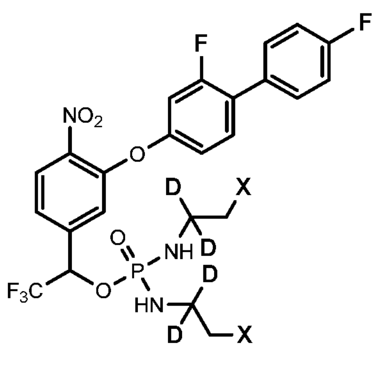
,

IV-10

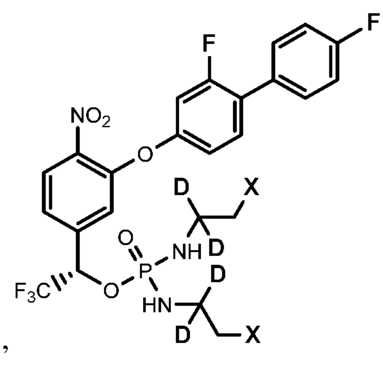
,

IV-11

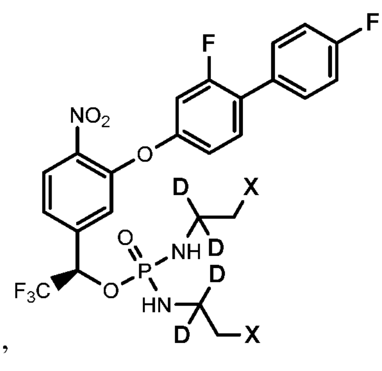
,

IV-12

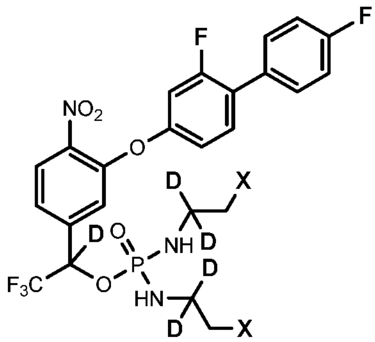
IV-13,

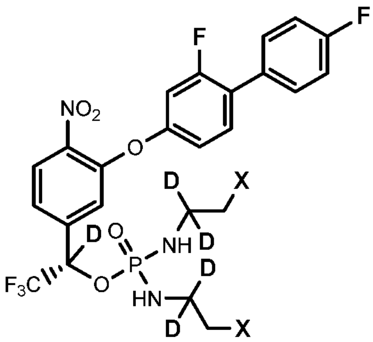
IV-14,

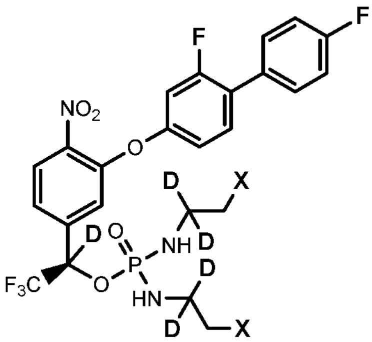
IV-15,

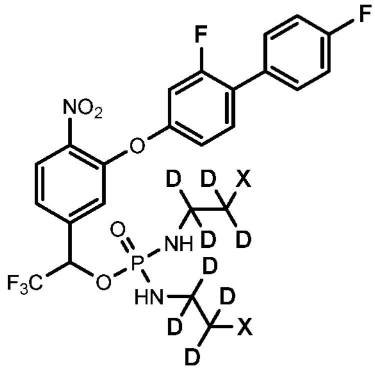
IV-16,

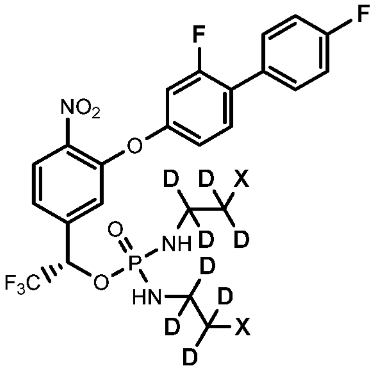
IV-17,

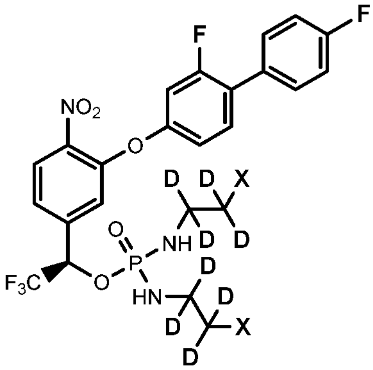
IV-18,

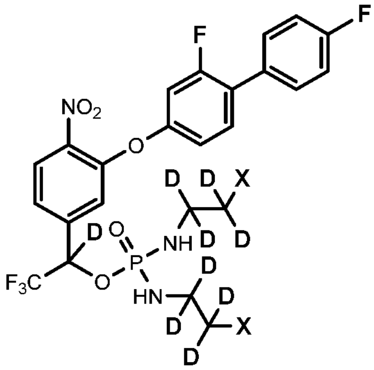
IV-19,

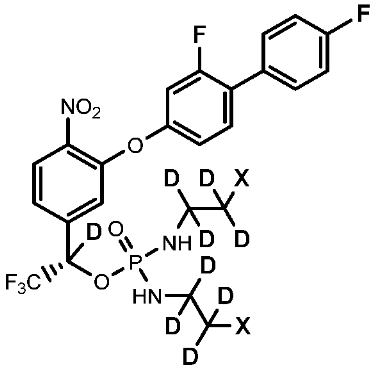
IV-20,

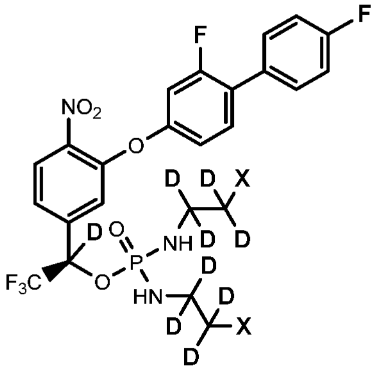
IV-21

wherein X is each independently fluorine, chlorine, bromine, or iodine, preferably bromine; wherein silver oxide or silver nitrate is used as a catalyst in the ring-closure reaction, with or without the addition of a base.

Specifically, with reference to the reaction conditions and material ratios of the synthesis method of Compound A in section 1.1 above, the synthesis preparation of the above 15 deuterated compounds (V-1 to V-15) may be achieved by choosing appropriate deuterated intermediates or starting materials.

The following deuterated compound of formula V-10 obtained was a light yellow solid, the $^{1}$HNMR spectrum of which was shown in Fig. 4, and the analytical data were as follows:

**1H NMR (400 MHz, CD3OD)** δ ppm 8.13 (d, J = 8.7 Hz, 1H), 7.60-7.51 (m, 4H), 7.45 (s, 1H), 7.19 (t, J = 8.7 Hz, 2H), 6.99-6.96 (m, 2H), 6.10-6.05 (m, 1H). HPLC (retention time: 9.817 min). MS: Calculated MS, 563.2, found, 564.2 ([M+H] +).

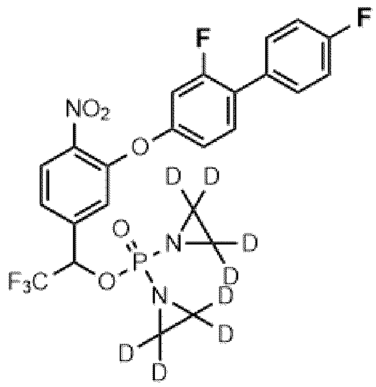

I-4-1

The meanings of abbreviations or vocabularies involved in the *in vivo* and *in vitro* experiments of sections 2 to 7 below can be interpreted according to the meanings of the vocabularies in textbooks of medicinal chemistry, biochemistry, physiology, pharmacology, or medicine.

## II. Inhibition Assays of Compounds on Cancer Cell

2.1 Inhibition Assays of Compounds A, B, C and AST-3424 on H460 cancer cells

Compound AST-3424 is a DNA alkylating medicament developed by the applicant targeting overexpressed aldo-keto reductase 1C3 (AKR1C3) for cancer therapy (DNA Alkylating Agent, PCT Application No. PCT/US2016/021581, Publication No. WO2016145092A1, Corresponding to the Chinese Application No. CN201680015078.8, the Publication No. CN107530556B), which is now in Phase II clinical stage, and has the following structural formula.

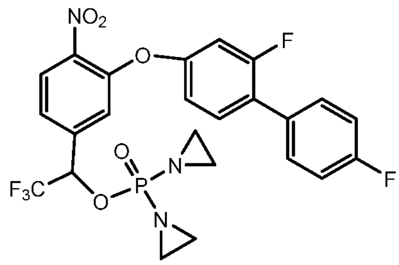

,

A

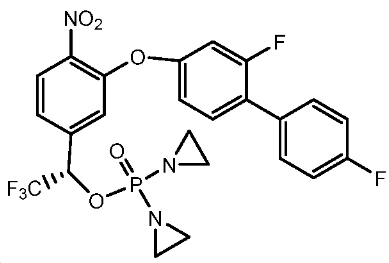

,

B

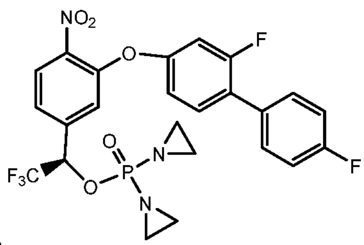

,

C

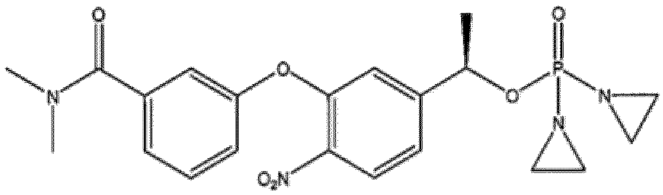

AST-3424

The quantification value $IC_{50}$ for cytotoxicity of a human tumor cell line *in vitro* was used to compare the cancer cell proliferation inhibition of compounds A, B, C and AST-3424.

If not otherwise specified, $IC_{50}$ values were measured by the following experimental methods:

(1) Cell culture

a) H460 cells were cultured in RPMI-1640 medium with 10% FBS and 1% double antibody and placed at 37°C and 5% $CO_2$.

(2) Cell plating

a) Cells were conventionally cultured until the cell saturation was 80%-90% and the count reached the requirement, then the cells were collected.

b) The cells were resuspended with the corresponding medium, counted, and formulated into the appropriate density of cell suspension.

c) The cell suspension was added to 96-well plate with 100μL per well and 2000 cells/well of cell density.

d) Cells were cultured overnight in a 37°C, 5% $CO_2$ incubator.

(3) Preparation of compounds

a) Compounds were prepared according to experimental requirements.

b) Cells with 0.5% DMSO were added into blank control wells as control wells for high readings.

c) The wells with no cells but only medium were used as control wells for low readings.

(4) Treating cells with compounds

a) Cells were plated for 24 hours, and then the compounds acted singly. 99μL of growth medium was supplemented per well, and then 1μL of 200X compound was added, gently shaken to ensure uniform mixing, and then placed in a 37°C, 5% $CO_2$ incubator.

b) The cell plate was placed in the incubator for 72 hours.

(5) CTG method detection

a) The cell plate to be tested was placed in equilibrium at room temperature for 30 minutes, and 100μL of medium was discarded from each well.

b) 100μL of CTG reagent (CelltiterGlo kit) was added to each well, the plate was placed in a rapid oscillator and shaken for 2 minutes, and then placed at room temperature for 30 minutes away from light.

c) The chemiluminescence signal value was read with Envision instrument.

(6) Data analysis

The $IC_{50}$ was calculated using GraphPad Prism 8 software and the $IC_{50}$ (half inhibitory concentration) of the compounds was obtained using the following non-linear fitting formula:

$$Y=Bottom+(Top-Bottom)/(1+10^\wedge((LogIC_{50}-X)*HillSlope))$$

X: log value of compound concentration, Y: inhibition rate (%inhibition)

Inhibition rate (%inhibition) = (control reading for high reading-reading for compound well)/ (control reading for high reading-control reading foe low reading)×100

The $IC_{50}$ values of compounds A, B, C and AST-3424 measured by the above experimental method were shown in Table 1 below.

Table 1: $IC_{50}$ data for the inhibitory effects of Compounds A, B, C and AST-3424 against H460 cancer cells

| Compound | $IC_{50}$ (nmol/L) |
|---|---|
| A | 6.87 |
| B | 8.77 |
| C | 23.37 |
| AST-3424 | 0.11 |

The $IC_{50}$ curves obtained from the test of compounds A, B, C and AST-3424 were shown in Fig. 5.

The test results showed that the two optical isomers (compounds B and C) had similar inhibitory toxicity against H460to that of the racemate (Compound A). The $IC_{50}$ of all three compounds A, B and C, was higher than that of AST-3424.

2.2 Cell inhibition experiments of Compounds A, B and C on different hepatocellular carcinoma (HCC) cell lines SNU354, SNU886, MHCC97H, huH-1

Using the same experimental methods as described above, Compounds A, B and C were further tested for cytotoxicity against different hepatocellular carcinoma (HCC) cell lines SNU354, SNU886, MHCC97H and huH-1, and the results were shown in Table 2 below:

Table 2: $IC_{50}$ data for the inhibitory effects of Compounds A, B and C on different hepatocellular carcinoma cells HCC

| Cell Line | $IC_{50}$ (nM) | | | ratio | | |
|---|---|---|---|---|---|---|
| | A | B | C | A/B | A/C | C/B |
| SNU354 | 962.3 | 754 | 1519 | 1.28 | 0.63 | 2.01 |
| SNU886 | 628.5 | 377.8 | 843.4 | 1.66 | 0.75 | 2.23 |
| MHCC97H | 395.8 | 242.4 | 563.1 | 1.63 | 0.70 | 2.32 |
| huH-1 | 305.7 | 257.8 | 269.3 | 1.19 | 1.14 | 1.49 |

The $IC_{50}$ curves corresponding to the tests for Compounds A, B, and C were shown in Fig. 6, and the test results indicated that the two optical isomers (Compounds B and C) have similar proliferation inhibitory activity for hepatocellular carcinoma (HCC) cell lines to that of the racemate (Compound A).

**III. AKR1C3 activation-dependent cell proliferation inhibition activity assay**

3.1 Inhibition assays of compounds A, B, C and AST-3424 on H460 cancer cells in the presence or absence of AKR1C3 inhibitors

Particularly, exponentially growing cells were inoculated in a 96-well plate at a density of $4\times10^3$ cells/well and incubated for 24 hours at 37°C and under the conditions of 5% $CO_2$, 95% air and 100% relative humidity. The tested compound was then added. The compound was dissolved in 100% DMSO at 200 times of the desired final test concentration. When the medicament was added, the compound was further diluted to 4 times of the desired final test concentration by using the complete medium. 50 $\mu$L of aliquots of the compound with specific concentration were added to the microwells containing 150 $\mu$L of medium, and then the final medicament concentration was obtained. After the medicament was added, the plate was incubated for another 2 hours at 37°C and under the conditions of 5% $CO_2$, 95% air and 100% relative humidity. The medicament was then washed. The fresh medium was added, and the plate was incubated for another 70 hours at 37°C and under the conditions of 5% $CO_2$, 95% air and 100% relative humidity. After the incubation was finished, the Alamar Blue was used to analyze and quantify the living cells. The concentration of medicament resulting in 50% growth inhibition ($IC_{50}$) was calculated by the computer software.

To further validate that the compounds are specifically activated by human AKR1C3 (Aldo-keto reductase family

1 member C3), assays of compound on H460 cancer cell proliferation were carried out in the presence or absence (at a concentration of 3 micromolar) of the specific AKR1C3 enzyme inhibitor AST-3021. Two hours prior to compound treatment, a solution of compound with the addition of the AKR1C3 enzyme inhibitor AST-3021 was added to cell cultures. The inhibitor used was compound 36, i.e.,

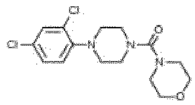,

described in Flanagan et al, Bioorganic and Medicinal Chemistry (2014), pages 962-977, abbreviated as AST-3021 or TH-3021 herein.

The $IC_{50}$ data for the inhibitory effects of compounds A, B, C and AST-3424 on H460 cancer cells in the presence or absence of AKR1C3 inhibitor AST-3021 were shown in Table 3, and the corresponding $IC_{50}$ curves were shown in Fig. 7.

Table 3: $IC_{50}$ data for the inhibitory effects of compounds A, B, C and AST-3424 on H460 cancer cells in the presence or absence of AKR1C3 inhibitor

| Compound | $IC_{50}$ (nmol/L) | | Fold |
|---|---|---|---|
| | Without the addition of AST-3021 | With the addition of AST-3021 | |
| A | 5.818 | 2136 | 367.13 |
| B | 3.727 | 1913 | 513.28 |
| C | 7.657 | 3329 | 434.76 |
| AST-3424 | 0.3486 | 44.87 | 128.71 |

The test results showed that although the $IC_{50}$ of all three Compounds A, B and C with or without the addition of AST-3021 was higher than that of Compound AST-3424, the folds of the $IC_{50}$ of the three Compounds A, B and C inhibited by AST-3021 was much higher than that of AST-3424, indicating that the *in vitro* activities of the Compounds A, B and C designed newly were much more closely related to the AKR1C3 enzyme.

3.2 AKR1C3-dependent assays of Compound A and AST-3424 on 10 cell lines of non-small cell lung cancer

The experimental method for determining $IC_{50}$ of 10 strains of non-small cell lung cancer was the same as that used in section 3.1 for H460 cancer cells. In addition, meanwhile, the expression amount of RNA corresponding to AKR1C3 enzyme was determined by the following method, based on $Log_2$ FPKM, and reference can be made to patent PCT/CN2021/079299. $Log_2$ FPKM and $IC_{50}$ of Compound A and AST-3424 were recited in the tables, and the experimental results were shown in tables 4 and 5.

Table 4: $Log_2$FPKM and $IC_{50}$ data of Compound A

| Non-small cell lung cancer cell lines | AKR1C3 RNA expression level ($Log_2$ FPKM) | $IC_{50}$ of Compound A (nM) |
|---|---|---|
| NCI-H 1944 | 11.06 | 71.27 |
| NCI-H2228 | 9.25 | 12.76 |
| NCIH1755 | 9.00 | 570 |
| NCI-H1563 | 8.61 | 454.4 |
| NCI-H2110 | 8.2334 | 67.77 |
| NCI-H1792 | 8.07 | 375.2 |
| CAL12T | 3.86 | 8076 |
| NCIH2106 | 2.68 | 6858 |
| NCI-H522 | -1.88 | 14147 |
| NCI-H23 | -1.98 | 11941 |

Table 5: $Log_2$FPKM and $IC_{50}$ data of Compound AST-3424

| NSCLC | AKR1C3 RNA expression level ($Log_2$ FPKM) | $IC_{50}$ of Compound AST-3424 (nM) |
|---|---|---|
| NCI-H1944 | 11.06 | 2.3 |
| NCI-H2228 | 9.25 | 0.21 |
| NCIH1755 | 9 | 8.2 |
| NCI-H1563 | 8.61 | 2.5 |
| NCI-H2110 | 8.23 | 1.1 |
| NCI-H1792 | 8.07 | 4.5 |
| CAL12T | 3.86 | 29.1 |
| NCIH2106 | 2.68 | >1000 |
| NCI-H23 | -1.98 | >1000 |
| NCI-H522 | -1.88 | >1000 |

Correlation analysis on RNA expression amount corresponding to AKR1C3 enzyme and $IC_{50}$ was carried out and the relationship plots between Compound A and AST-3424 were obtained, respectively, as shown in Fig. 8 and Fig. 9.

The experimental results showed that the in *vitro* cytotoxicity of Compound A and AST-3424 was positively correlated with AKR1C3 expression. For cell lines with high AKR1C3 expression, both Compound A and AST-3424 showed strong *in vitro* cytotoxicity; for cell lines with low AKR1C3 expression, very low Compound A and AST-3424-mediated cytotoxicity was shown. Compared with AST-3424 ($R^2$=0.796), Compound A correlated more closely with AKR1C3 expression levels ($R^2$=0.952), indicating that the activation of Compound A was more specific for AKR1C3.

3.3 AKR1C3-dependent *in vivo* pharmacodynamic experiment of Compound A and Sorafenib in liver cancer LI6280 PDX model

Experiment Instruction: BALB/c nude mice were subcutaneously inoculated with HuPrime® model LI6280 tumour blocks to establish a subcutaneous transplantation tumour model of human liver cancer. The test was divided into groups of test drug Sorafenib 30 mg/kg group, test drug Compound A 4.5 mg/kg group, and 7.5% anhydrous ethanol + 7.5% polyoxyethylene (35) castor oil + 85% glucose injection D5W (pH 7.4) vehicle control group, a total of 3 groups, with 6 mice per group. Among them, 7.5% anhydrous ethanol + 7.5% polyoxyethylene (35) castor oil + 85% glucose injection D5W (pH 7.4) vehicle control group was administered by tail vein,once a week for consecutive 3 weeks; the test drug Compound A 4.5 mg/kg group was administered by tail vein once a week for consecutive 3 weeks; and the test drug Sorafenib 30 mg/kg group was administered by gavage once a day for consecutive 21 days. The efficacy was evaluated according to the relative tumor inhibition rate TGI (%), and the safety was evaluated according to the change in body weight and the death of the animals. The specific dosing regimens and TGI (%) for each group were shown in Table 6.

Table 6: Table of pharmacodynamic analysis of each group in the HuPrime® liver cancer LI6280 model

| **Experimental group** | **Day 17 after the first administration (i.e., Day 17)** | | | | |
|---|---|---|---|---|---|
| | **Tumor volume** ($\bar{x}\pm$S) | **Relative tumor volume** ($\bar{x}\pm$S) | **TGI (%)** | **T/C (%)** | **P-value (compared with control group)** |
| Group 1<br>7.5% anhydrous ethanol + 7.5% polyoxyethylene (35) castor oil + 85% glucose injection DSW (pH7.4) | 2113.82±170.66 | 20.5±0.73 | - | - | - |
| Group 2<br>Sorafenib<br>(30 mg/kg) | 1076.45±230.21 | 10.18±1.86 | 50.35 | 49.65 | p< 0.001 |

(continued)

| Experimental group | Day 17 after the first administration (i.e., Day 17) | | | | |
|---|---|---|---|---|---|
| | **Tumor volume** ($\bar{x}\pm S$) | **Relative tumor volume** ($\bar{x}\pm S$) | **TGI (%)** | **T/C (%)** | **P-value (compared with control group)** |
| Group 3 Compound A (4.5 mg/kg) | 2201.20±287.44 | 20.79±1.15 | -1.41% | 101.41 | 0.987 |

Note: 1. Data were expressed as "mean ± standard error", i.e., $\bar{x}\pm S$;
2. T/C % = $T_{RTV}$ / $C_{RTV}$ × 100% or T/C % = $T_{TV}$ / $C_{TV}$ × 100% ($T_{RTV}$ : mean RTV of the treatment group; $C_{RTV}$ : mean RTV of the vehicle control group; RTV=$V_t/V_0$, $V_0$ was the tumour volume of the animal when grouped, Vε was the tumour volume of the animal after treatment, the same below); TGI% =(1-T/C)× 100% (T and C were the mean relative tumour volume (RTV) of the treatment group and the control group at a certain time point, respectively, the same below).

The size of tumour volume on different days was recorded and the results were shown in Table 7.

Table 7: Size of tumor volume in different experimental group on different days ($mm^3$)

| Experime ntal group | Day 0 | Day 3 | Day 7 | Day 10 | Day 14 | Day 17 | Day 21 | Day 24 | Day 28 | Day 31 |
|---|---|---|---|---|---|---|---|---|---|---|
| Group 1 | 103.74 | 169.79 | 401.62 | 775.57 | 1319.18 | 2113.82 | 2641.70 | 3149.10 | | |
| Group 2 | 103.93 | 168.82 | 269.83 | 439.29 | 731.99 | 1076.45 | 1241.19 | 1504.89 | 2218.52 | 2313.01 |
| Group 3 | 103.98 | 150.33 | 318.74 | 683.99 | 1404.42 | 2201.20 | 2739.12 | 3311.77 | 3362.07 | |

The curves of the corresponding tumour volume over time were shown in Fig. 10.

The test drug Sorafenib 30 mg/kg treatment group showed significant tumour inhibitory effect on day 17 after the first administration, with a statistically significant difference ($p<0.001$) compared with the control group and a relative tumor inhibition rate TGI (%) of 50.35%. The test drug Compound A 4.5 mg/kg treatment group had no tumour inhibitory effect on day 17 after the first administration, with no statistically significant difference compared with the control group ($p=0.987$) and a relative tumor inhibition rate TGI (%) of -1.41%.

The expression amount of RNA corresponding to AKR1C3 enzyme in this model was determined by the same method as described above, and was calculated as $Log_2FPKM$ = -0.943 based on $Log_2FPKM$.

It can be seen from the above experimental data that in the liver cancer LI6280 PDX model, the RNA expression level corresponding to AKR1C3 was relatively low ($Log_2$ FPKM=-0.943), and the efficacy of Compound A was almost indistinguishable from that of the vehicle control group, whereas there was a certain degree of efficacy for Sorafenib, which was not AKR1C3-dependent. Therefore, for Compound A to exert significant anticancer activity, specific activation of the AKR1C3 enzyme was required, in combination with the conclusions in Section 3.2, this indicated that Compound A was an anticancer prodrug activated by the AKR1C3 enzyme.

## IV. Experiments on animal models of PDX and CDX

4.1 Comparison of *in vivo* pharmacodynamic experiment of Compound A, AST-3424 and Isocyclophosphamide in gastric cancer GA6201 PDX model

Experiment Instruction: BALB/c nude mice were subcutaneously inoculated with HuPrime® model GA6201 tumour blocks to establish a subcutaneous transplantation tumour model of human gastric cancer. The test was divided into groups of the test drug Isocyclophosphamide 60 mg/kg group, the test drug AST-3424 5 mg/kg group, the test drug A compound 2.5 mg/kg and 5 mg/kg groups, and normal saline (pH 7.0-7.6) vehicle control group, a total of 5 groups, with 5 mice per group. Among them, normal saline (pH 7.0-7.6) vehicle control group was administered once a week for three weeks and observed for four weeks; the test drug AST-3424 5 mg/kg, test drug A compound 2.5 mg/kg and 5 mg/kg groups were administered by tail vein injection once a week for two weeks and observed for seven weeks; the test drug Isocyclophosphamide 60 mg/kg group was administered by intraperitoneal injection five times a week continuously and observed for seven weeks; and the test drug Isocyclophosphamide 60 mg/kg group was administered by intraperitoneal injection for consecutive 5 days followed by 2 days off every week for 2 weeks and observed for seven weeks. The efficacy was evaluated according to the relative tumor inhibition rate TGI (%), and the safety was evaluated according to the change in body weight and the death of the animals. The specific dosing regimens and TGI (%) for each group were shown in Table 8.

Table 8: Table of pharmacodynamic analysis of each group in gastric cancer GA6201 model

| Experimental group | Day 3 after the end of all administrations (i.e., Day38) | | | | |
|---|---|---|---|---|---|
| | **Tumor volume** ($\bar{x}\pm$S) | **Relative tumor volume** (x:!: S) | **TGI (%)** | **T/C (%)** | **P-value (compared with control group)** |
| Group 1<br>normal saline, pH 7.0-7.6 | 905.75±252.4 | 9.34±2.42 | - | - | - |
| Group 2<br>Isocyclophosphamide<br>60 mg/kg | 371.02±75.96 | 3.85±0.63 | 58.83 | 41.17 | 0.857 |
| Group 3<br>AST-3424<br>5 mg/kg | 12.65±7.91 | 0.11±0.07 | 98.79 | 1.21 | 0.00000265 |
| Group 4<br>Compound A<br>5 mg/kg | 19.97±10.18 | 0.19±0.08 | 97.96 | 2.04 | 0.0000182 |

(continued)

| Experimental group | Day 3 after the end of all administrations (i.e., Day38) | | | | |
|---|---|---|---|---|---|
| | **Tumor volume** ( $\bar{x}\pm$S) | **Relative tumor volume** (x:!: S) | **TGI (%)** | **T/C (%)** | **P-value (compared with control group)** |
| Group 5 Compound A 2.5 mg/kg | 28.69±8.2 | 0.29±0.08 | 96.85 | 3.15 | 0.000135 |

Note: 1. Data were expressed as "mean ± standard error", i.e. $\bar{x}\pm$S;
2. T/C % = $T_{RTV}$ / $C_{RTV}$ × 100% or T/C % = $T_{TV}$ / $C_{TV}$ × 100% ($T_{RTV}$ : mean RTV of the treatment group; $C_{RTV}$ : mean RTV of the vehicle control group; RTV=$V_t/V_0$, $V_0$ was the tumour volume of the animal when grouped, Vε was the tumour volume of the animal after treatment, the same below); TGI% =(1-T/C)× 100% (T and C were the mean relative tumour volume (RTV) of the treatment group and the control group at a certain time point, respectively).

In the above dosing regimen, Isocyclophosphamide was designed based on the best dosing regimen with maximum safe dosage as recited in the document (Jessica D. Sun, Qian Liu, Dharmendra Ahluwalia, Damien J. Ferraro, Yan Wang, Don Jung, Mark D. Matteucci, and Charles P. Hart. Comparison of hypoxia-activated prodrug evofosfamide (TH-302) and ifosfamide in preclinical non-small cell lung cancer models [J]. Cancer Biology & Therapy, 2016, 17(4): 371-380.).

The size of tumour volume on different days was recorded and the results were shown in Table 9.

Table 9: Size of tumor volume in different experimental group on different days ($mm^3$)

| Experiment group | 0 day | 3 days | 7 days | 10 days | 14 days | 17 days | 21 days | 24 days | 28 days | 31 days | 35 days | 38 days | 42 days | 45 days | 49 days |
|---|---|---|---|---|---|---|---|---|---|---|---|---|---|---|---|
| Group 1 | 94.22 | 123.26 | 161.08 | 182.31 | 242.26 | 297.28 | 398.37 | 479.52 | 579.90 | 648.12 | 831.15 | 905.75 | 1026.01 | 1130.43 | 1283.11 |
| Group 2 | 94.15 | 122.22 | 129.39 | 175.00 | 190.74 | 189.23 | 217.96 | 225.66 | 247.04 | 265.76 | 324.51 | 371.02 | 403.47 | 484.83 | 578.60 |
| Group 3 | 94.19 | 114.98 | 84.33 | 77.04 | 63.59 | 52.08 | 45.81 | 40.55 | 30.28 | 23.24 | 18.55 | 12.65 | 13.42 | 10.82 | 8.15 |
| Group 4 | 94.36 | 118.42 | 101.91 | 97.17 | 74.20 | 59.77 | 46.21 | 44.03 | 38.50 | 35.92 | 22.70 | 19.97 | 5.05 | 11.14 | 9.96 |
| Group 5 | 94.61 | 120.19 | 118.33 | 118.65 | 96.08 | 76.14 | 71.72 | 57.94 | 45.51 | 43.44 | 32.04 | 28.69 | 23.49 | 25.00 | 17.72 |

The curves of the corresponding tumour volume over time were shown in Fig. 11.

The experimental results showed that for at least 49 days, the AST-3424 5 mg/kg group, and the test drug Compound A 2.5 mg/kg and 5 mg/kg groups had excellent inhibitory activity against cancer cells and were all superior to the conventional DNA alkylating agent Isocyclophosphamide, and the relative tumor inhibition rate (TGI) of Compound A was more than 96%.

During the above experiments, after administration on day 14, the administration was stopped and the tumor volume was measured and the curve was plotted as shown in Fig. 12. The experimental results illustrated that even after stopping treatment for 1 month, both administration doses of Compound A still had a tumor inhibitory effect, and the tumor volume continued to decrease and either remained small or could not be measured. This showed a good therapeutic effect of Compound A, which was equal to that of AST-3424.

In order to verify the effect of Compound A on the body weight change of the experimental animals, the data of body weight change of the mice were recorded, and the rate of body weight change of the mice was calculated as shown in Fig. 13. The data of body weight change of the above experimental animals indicated that administration groups of Compound A at both doses were similar to the normal saline control group, the Isocyclophosphamide group, and the AST-3424 group, which had no effect on the body weights of the experimental animals. Compound A and AST-3424 at the same dose had equal tumor inhibitory efficacy and no toxic effects.

4.2 Comparison of *in vivo* pharmacodynamic experiment of Compound A and Carboplatin in human-derived non-small cell lung cancer NCI-H460 subcutaneous xenograft model

BALB/c nude mice were subcutaneously inoculated with human lung cancer NCI-H460 cells to establish a subcutaneous transplantation tumor model of human lung cancer. The experiment was divided into the groups of test drug Carboplatin 100 mg/kg group, test single drug Compound A 5 mg/kg, 10 mg/kg and 15 mg/kg groups, and 10% ethanol + 10% polyoxyethylene castor oil + 80% glucose injection D5W (pH 7.4) vehicle control group, a total of 5 groups, with 6 mice per group. Among them, 10% ethanol + 10% polyoxyethylene castor oil + 80% glucose injection D5W (pH 7.4) vehicle control group, test drug Compound A 5 mg/kg, 10 mg/kg and 15 mg/kg groups were administered by tail vein injection once a week for three weeks and observed for one week. Test drug Carboplatin 100 mg/kg group was administered by intraperitoneal injection once a week for three weeks and observed for one week. The efficacy was evaluated according to the relative tumor inhibition rate TGI (%), and the safety was evaluated according to the change in body weight and the death of the animals.

The pharmacodynamic analysis of each group in the NCI-H460 CDX model was performed on Day 31 after tumor cell inoculation, and the results were shown in Table 10. The size of tumour volume on different days was recorded and the results were shown in Table 11. The curve of corresponding tumor volume over time was plotted, as shown in Fig. 14.

Table 10: Table of pharmacodynamic analysis of each group in the human-derived lung cancer NCI-H460 model

| **Experimental group** | **Day 31 after tumor cell inoculation (i.e., Day31, 24th day after first administration)** | | | | |
|---|---|---|---|---|---|
| | **Tumor volume** ($\bar{x}$+S) | **Relative tumor volume** ($\bar{x}$+S) | **TGI (%)** | **T/C (%)** | **P-value (compared with control group)** |
| Group 1<br>10% ethanol + 10% polyoxyethylene castor oil + 80% glucose injection D5W (pH 7.4) QWx3, i.v. | 1818.22±235.32 | 13.24±2.49 | - | - | - |
| Group 2<br>Carboplatin, 100mg/kg, QWx3, i.p. | 1230.93±258.17 | 8.58±1.16 | 35.19 | 64.81 | 0.683 |
| Group 3<br>Compound A, Smg/kg, QWx3, i.v. | 337.78±54.37 | 2.45±0.29 | 81.49 | 18.51 | 0.0000233 |
| Group 4<br>Compound A, 10mg/kg, QWx3, i.v. | 164.13±34.01 | 1.18±0.27 | 91.06 | 8.94 | 0.0000000000975 |

(continued)

| Experimental group | Day 31 after tumor cell inoculation (i.e., Day31, 24th day after first administration) | | | | |
|---|---|---|---|---|---|
| | Tumor volume ($\bar{x}$+S) | Relative tumor volume ($\bar{x}$+S) | TGI (%) | T/C (%) | P-value (compared with control group) |
| Group 5<br>Compound A, 15mg/kg, QWx3, i.v. | 125.90±25.46 | 0.92±0.22 | 93.06 | 6.94 | 0.0000000000988 |
| Note: 1. Data were expressed as "mean ± standard error", i.e., $\bar{x}$±S;<br>2. $T/C\ \% = T_{RTV} / C_{RTV} \times 100\%$ or $T/C\ \% = T_{TV} / C_{TV} \times 100\%$; $TGI\% = (1-T/C) \times 100\%$;<br>3. Group 3 vs. Group 4, p=0.168; Group 3 vs. Group 5, p=0.0317; Group 4 vs. Group 5, p=0.998. | | | | | |

Table 11: Size of tumor volume in different experimental group on different days ($mm^3$)

| Experimental group | Day 7 | Day 10 | Day 14 | Day 17 | Day 21 | Day 24 | Day 28 | Day 31 | Day 35 |
|---|---|---|---|---|---|---|---|---|---|
| Group 1 | 144.68 | 226.31 | 339.60 | 493.01 | 774.49 | 977.77 | 1370.47 | 1818.22 | 2299.37 |
| Group 2 | 145.46 | 262.15 | 409.97 | 529.44 | 761.56 | 960.42 | 1073.28 | 1230.93 | 1577.99 |
| Group 3 | 144.35 | 183.15 | 198.80 | 228.87 | 253.30 | 310.83 | 279.31 | 337.78 | 452.75 |
| Group 4 | 144.21 | 164.75 | 151.95 | 140.29 | 138.66 | 144.36 | 149.16 | 164.13 | 168.68 |
| Group 5 | 145.41 | 171.22 | 167.54 | 161.80 | 157.66 | 151.20 | 134.73 | 125.90 | 108.70 |

In order to verify the effect of Compound A on the body weight change of experimental animals, the body weight change of experimental animals was recorded at different time points and the growth rate of their body weight was calculated and plotted as curves as shown in Fig. 15.

The above experimental data showed that Compound A showed better tumor inhibitory effects than that of carboplatin during 31 consecutive days of observation, and the relative tumor inhibitory rate (TGI%) of Compound A reached 81.49%, 91.06% and 93.06% at the three administration doses of 5 mg/kg, 10 mg/kg and 15 mg/kg, respectively. Compound A at the three administration doses had no significant effect on the body weights of the experimental animals for at least 31 days, indicating that Compound A had excellent tumor inhibitory effects without toxic side effects.

4.3 Comparison of *in vivo* pharmacodynamic experiment of Compound A and Sorafenib in human liver cancer LI6652 subcutaneous xenograft PDX model

Experiment Instruction: BALB/c nude mice were subcutaneously inoculated with human liver cancer LI6652 tumour blocks to establish a subcutaneous xenograft tumour model of human liver cancer. The experiment was divided into groups of test drug Sorafenib 30 mg/kg (administered once a day for 21 consecutive days) group, single drug groups of test drug Compound A 1.25 mg/kg (administered every day for 5 consecutive days, followed by a 2 days off, and then a further 2 weeks off for a total of 2 dosing cycles), 2.5 mg/kg (administered every day for 5 consecutive days, followed by a 2 days off, and then a further 2 weeks off for a total of 2 dosing cycles) , 5 mg/kg (administered every day for 5 consecutive days, followed by a 2 days off, and then a further 2 weeks off for a total of 2 dosing cycles) , and 7.5% anhydrous ethanol + 7.5% polyoxyethylene (35) castor oil + 80% glucose injection D5W (pH 7.4) control group, a total of 5 groups, with 6 mice per group. Test drug Compound A 1.25 mg/kg, 2.5 mg/kg, 5 mg/kg single drug groups and 7.5% anhydrous ethanol + 7.5% polyoxyethylene (35) castor oil + 80% glucose injection D5W (pH 7.4) control group were administered by intraperitoneal injection, administered every day for 5 consecutive days, followed by 2 days off, and then a further 2 weeks off for a total of 2 dosing cycles. The test drug Sorafenib 30 mg/kg group was administered by gavage, administered once daily for 21 consecutive days. The efficacy was evaluated according to the relative tumor inhibition rate TGI (%), and the safety was evaluated according to the change in body weight and the death of the animals.

The pharmacodynamic analysis of each group in liver cancer LI6652 model was performed on Day 31 after administration, and the results were shown in Table 12. The size of tumour volume on different days was recorded and

the results were shown in Table 13. The curve of corresponding tumor volume over time was plotted, as shown in Fig. 16.

Table 12: Table of pharmacodynamic analysis of each group in liver cancer LI6652 model

| Experimental group | Day 3 after the end of all administrations (i.e., Day28) | | | | |
|---|---|---|---|---|---|
| | Tumor volume ($\bar{x}\pm$) | Relative tumor volume ($\bar{x}\pm$S) | TGI (%) | T/C (%) | P-value (compared with control group) |
| Group 1<br>7.5% anhydrous ethanol + 7.5% polyoxyethylene (35) castor oil + 85% glucose injection D5W (pH7.4) | 1590.50±470.81 | 14.67±3.14 | - | - | - |
| Group 2<br>Sorafenib 30mg/kg | 315.93±81.35 | 2.50±0.57 | 82.96 | 17.04 | 0.108 |
| Group 3<br>Compound A<br>1.25 mg/kg | 24.72±9.57 | 0.18±0.07 | 98.77 | 1.23 | 0.00000291 |
| Group 4<br>Compound A 2.5mg/kg | 18.28±8.14 | 0.17±0.10 | 98.84 | 1.16 | 0.000000535 |
| Group 5<br>Compound A Smg/kg | 3.38±3.38 | 0.02±0.02 | 99.86 | 0.14 | 0.00000000575 |
| Note: 1. Data were expressed as "mean ± standard error" i.e., $\bar{x}\pm$S;<br>2.<br>$T/C\ \% = T_{RTV} / C_{RTV} \times 100\%$ or $T/C\ \% = T_{TV} / C_{TV} \times 100\%$; $TGI\% = (1-T/C)\times 100\%$. | | | | | |

Table 13: Size of tumor volume in different experimental group on different days ($mm^3$)

| Experimental group | Day 0 | Day 3 | Day 7 | Day 10 | Day 14 | Day 17 | Day 21 | Day 24 | Day 28 | Day 31 |
|---|---|---|---|---|---|---|---|---|---|---|
| Group 1 | 125.10 | 219.19 | 322.75 | 504.30 | 740.03 | 925.35 | 1284.19 | 1658.67 | 1590.50 | 2112.46 |
| Group 2 | 125.84 | 146.95 | 196.51 | 212.41 | 217.19 | 191.72 | 178.69 | 209.42 | 315.93 | 441.67 |
| Group 3 | 126.30 | 184.11 | 194.19 | 135.63 | 51.98 | 28.94 | 29.42 | 28.38 | 24.72 | 18.12 |
| Group 4 | 126.29 | 179.46 | 189.33 | 145.19 | 43.64 | 29.90 | 21.49 | 17.91 | 18.28 | 5.56 |
| Group 5 | 124.83 | 174.91 | 190.90 | 130.55 | 34.29 | 19.84 | 13.80 | 12.20 | 3.38 | 2.62 |

In order to verify the effect of Compound A on the body weight change of experimental animals, based on the above experimental method, the body weight change of experimental animals was recorded at different time points and the growth rate of their body weight was calculated and plotted as curves as shown in Fig. 17.

The above results showed that Compound A at the three administration doses showed excellent tumor inhibitory effects, with relative tumor inhibition TGI (%) exceeding 98%, and superior to the tumor inhibitory effects of Sorafenib. Compound A at the three administration doses had no significant effect on the body weight of the experimental animals for at least 28 days, indicating that Compound A had excellent tumor inhibitory effects without toxic side effects.

4.4 Comparison of *in vivo* pharmacodynamic experiment of Compound A and Paclitaxel in human-derived non-small cell lung cancer NCI-H460-Luc2 intracranial inoculation CDX model

Experiment Instruction: BALB/c nude mice were intracranially inoculated with human lung cancer NCI-H460-Luc2 cells to establish an intracranial transplantation tumor model of human lung cancer. The experiment was divided into groups of test drug Paclitaxel 20 mg/kg group, Compound A 2 mg/kg, 4 mg/kg and 8 mg/kg groups and 10% ethanol + 10% polyoxyethylene castor oil + 80% glucose injection D5W (pH 7.42) vehicle control group (Vehicle group), with 6 mice per group. Among them, 10% ethanol + 10% polyoxyethylene castor oil + 80% glucose injection D5W (pH 7.42) vehicle control group, test drug Compound A 2 mg/kg group, 4 mg/kg and 8 mg/kg groups were administered by tail vein injection once a week for 3 weeks. The positive drug Paclitaxel 20 mg/kg group was administered by intraperitoneal injection and administered once a week for 3 weeks. The relative tumor inhibition rate TGI (%) was calculated according to the tumor biofluorescence value and the efficacy was evaluated, and the safety was evaluated according to the change in body weight and death of the animals.

The pharmacodynamic analysis of each group in the human NCI-H460-luc intracranial inoculation model was performed based on the measured bioluminescence values on day 11 after administration, and the results were shown in Table 14.

Table 14: Table of pharmacodynamic analysis of each group in human-derived NCI-H460-Luc2 intracranial inoculation model

| **Group** | **Bioluminescence Value ($\times 10^5$ photon/second)[a] (Day 11)** | **TGI [b] (%)** | **T/C [c] (%)** | ***p value*[d]** |
|---|---|---|---|---|
| Vehicle (n=5) | 2576.80 ± 680.69 | -- | -- | -- |
| Paclitaxel 20 mg/kg, i.p. (n=5) | 4184.52 ± 1679.77 | -62.45 | 130.82 | 0.2278 |
| Compound A 2 mg/kg, i.v. (n=6) | 7.62 ± 2.10 | 99.80 | 0.24 | 0.0089 |
| Compound A 4 mg/kg, i.v. (n=6) | 1.62 ± 0.30 | 100.03 | 0.05 | 0.0087 |
| Compound A 8 mg/kg, i.v. (n=6) | 1.78 ± 0.42 | 100.03 | 0.06 | 0.0087 |

Curves regarding the number of treatment days were plotted based on the biofluorescence values measured in each treatment and control group, as shown in Fig. 18. The biofluorescence values of Compound A at the three administration doses were low and significantly lower than those of Paclitaxel at the administration dose, indicating that Compound A was able to cross the blood-brain barrier into the brain to exert a tumor inhibitory effect, which was a pharmacodynamic effect that Paclitaxel was unable to exert.

In addition, in order to verify the effect of Compound A on the body weight change of experimental animals, based on the above experimental method, the body weight change of experimental animals was recorded at different time points and the growth rate of their body weight was calculated and plotted as curves as shown in Fig. 19. In Figure, the body weight of mice in the two control groups decreased significantly, indicating that Paclitaxel could not cross the blood-brain barrier into the brain to exert tumor inhibitory effects. By observing the change rate of body weight corresponding to Compound A at the three administration doses, the body weights of the mice had little change, indicating that Compound A could enter the brain to exert tumor inhibitory effects while showing virtually no toxic side effect, and from the side, also indicating that it had crossed the blood-brain barrier and had exerted tumor inhibitory effects.

4.5 Comparison of *in vivo* pharmacodynamic experiment of Compound A and Gemcitabine in pancreatic cancer PA1222PDX model

Experiment Instruction: BALB/c nude mice were subcutaneously inoculated with HuPrime® model PA1222tumor blocks to establish a human pancreatic cancer subcutaneous transplantation tumor model. The test was divided into groups of test drug Gemcitabine 120 mg/kg group, test drug Compound A 10 mg/kg and 7.5% anhydrous ethanol + 7.5% polyoxyethylene (35) castor oil + 85% glucose injection D5W (pH 7.4) vehicle control group, a total of 3 groups, with 5 mice per group. Among them, 7.5% anhydrous ethanol + 7.5% polyoxyethylene (35) castor oil + 85% glucose injection D5W (pH 7.4) vehicle control group and test drug Compound A 10 mg/kg group were administered by tail vein injection once a week for three consecutive weeks. Test drug Gemcitabine (Gemcitabine) 120 mg/kg group was administered intraperitoneally, once a week for three consecutive weeks. The efficacy was evaluated according to the relative tumor inhibition rate TGI (%), and the safety was evaluated according to the change in body weight and the death of the animals.

The pharmacodynamic analysis of each group in the pancreatic cancer PA1222model was performed on Day 28 after administration and the results were shown in Table 15. The size of tumour volume on different days was recorded and the results were shown in Table 16.

Table 15: Table of pharmacodynamic analysis of each group in HuPrime® Pancreatic Cancer PA1222 model.

| **Experimental group** | **Day 28 after the first administration (i.e., Day 28)** | | | | |
|---|---|---|---|---|---|
| | **Tumor volume** ($\overline{x}\pm S$) | **Relative tumor volume** ($\overline{x}\pm S$) | **TGI (%)** | **T/C (%)** | **P-value (compared with control group)** |
| Group 1<br>7.5% anhydrous ethanol + 7.5% polyoxyethylene (35) castor oil + 85% glucose | 395.45±76.89 | 3.49±0.62 | - | - | - |
| injection D5W (pH7.4), 0mg/kg, QWx3, i.v. | | | | | |
| Group 2<br>Gemcitabine, 120mg/kg, QWx3, i.p. | 166.11±24.40 | 1.49±0.19 | 57.17% | 42.83% | 7.78e-4 |
| Group 3<br>Compound A, 10mg/kg, QWx3, i.v. | 12.51±5.25 | 0.10±0.04 | 97.14% | 2.86% | 9.14e-7 |
| Note: 1. Data were expressed as "mean ± standard error", i.e., $\overline{x}\pm S$;<br>2.<br>$T/C\ \% = T_{RTV} / C_{RTV} \times 100\%;\ TGI\% = (1-T/C)\times 100\%.$ | | | | | |

Table 16: Size of tumor volume in different experimental group on different days ($mm^3$)

| Experime ntal group | Day 0 | Day 3 | Day 7 | Day 10 | Day 14 | Day 17 | Day 21 | Day 24 | Day 28 |
|---|---|---|---|---|---|---|---|---|---|
| Group 1 | 112.01 | 139.75 | 177.43 | 199.20 | 249.21 | 306.79 | 339.57 | 362.58 | 395.45 |
| Group 2 | 111.91 | 136.17 | 153.75 | 157.55 | 147.44 | 136.71 | 138.01 | 153.69 | 166.11 |
| Group 3 | 111.50 | 117.62 | 113.91 | 107.17 | 66.08 | 42.94 | 23.22 | 21.20 | 12.51 |

The tumour volumes of different groups of mice were measured on different days and the mean values were obtained and plotted as curves as shown in Fig. 20.

In order to verify the effect of Compound A on the body weight change of experimental animals, based on the above experimental method, the body weight change of experimental animals was recorded at different time points and the growth rate of their body weight was calculated and plotted as curves as shown in Fig. 21.

The model was a Gemcitabine-sensitive model. The results of the above experiments showed that Compound A at the administration dose of 10 mg/kg had good anti-tumor activity, and its tumor inhibition activity was superior to

that of Gemcitabine at the administration dose of 120 mg/kg. Meanwhile, in comparison with the body weight change of experimental animals in the control group, Compound A showed excellent tumor inhibitory effect without toxic side effects.

4.6 Comparison of *in vivo* pharmacodynamic experiment of Compound A and Paclitaxel in lung cancer LU2505 PDX model

Experiment Instruction: BALB/c nude mice were subcutaneously inoculated with HuPrime® model LU2505 tumor blocks to establish a subcutaneous transplantation tumor model of human lung cancer. The experiment was divided into groups of test drug Paclitaxel 20 mg/kg group, test drug Compound A 1.25 mg/kg, 2.5 mg/kg and 5 mg/kg groups and 7.5% anhydrous ethanol + 7.5% polyoxyethylene (35) castor oil + 85% glucose injection D5W (pH 7.4) vehicle control group, a total of 5 groups, with 5 mice per group. Among them, the test drug Paclitaxel 20 mg/kg group was administered by intraperitoneal injection every four days for a total of four dosing cycles and observed for 13 days. 7.5% anhydrous ethanol + 7.5% polyoxyethylene (35) castor oil + 85% glucose injection D5W (pH 7.4) vehicle control group, the test drug Compound A 1.25 mg/kg, 2.5 mg/kg and 5 mg/kg groups were administered by tail vein injection once a week for three consecutive weeks and observed for 8 days. The efficacy was evaluated according to the relative tumor inhibition rate TGI (%), and the safety was evaluated according to the change in body weight and the death of the animals.

The pharmacodynamic analysis of each group in the lung cancer LU2505 model was performed on Day 28 after administration and the results were shown in Table 17. The size of tumour volume on different days was recorded and the results were shown in Table 18.

Table 17: Table of pharmacodynamic analysis of each group in HuPrime® Lung Cancer LU2505 model

| **Experimental group** | **The 8th day after the end of the 3-week dosing cycle (i.e., Day28)** | | | | |
|---|---|---|---|---|---|
| | **Tumor volume** ($\bar{x}\pm$S) | **Relative tumor volume** ($\bar{x}\pm$S) | **TGI (%)** | **T/C (%)** | **P-value (compared with control group)** |
| Group 1 vehicle control group | 1126.00±354.60 | 10.68±3.44 | - | - | - |
| Group 2 Paclitaxel 20 mg/kg | 250.47±157.81 | 2.29±1.37 | 78.60 | 21.40 | 0.0322 |
| Group 3 Compound A 1.25 mg/kg | 485.95±175.25 | 4.61±1.57 | 56.86 | 43.14 | 0.7120 |
| Group 4 Compound A 2.5 mg/kg | 102.06±47.51 | 1.11±0.56 | 89.57 | 10.43 | 0.0322 |
| Group 5 Compound A 5 mg/kg | 19.00±8.08 | 0.17±0.07 | 98.40 | 1.60 | 0.00112 |

Table 18: Size of tumor volume in different experimental group on different days ($mm^3$)

| Experiment al group | Day 0 | Day 4 | Day 7 | Day 11 | Day 14 | Day 18 | Day 21 | Day 25 | Day 28 |
|---|---|---|---|---|---|---|---|---|---|
| Group 1 | 103.58 | 196.01 | 291.37 | 390.10 | 486.55 | 670.52 | 824.93 | 902.96 | 1126.00 |
| Group 2 | 103.12 | 133.63 | 133.14 | 132.14 | 164.42 | 207.89 | 238.63 | 231.63 | 250.47 |
| Group 3 | 103.28 | 156.32 | 231.55 | 242.12 | 291.72 | 383.24 | 434.92 | 478.15 | 485.95 |
| Group 4 | 103.26 | 149.02 | 131.34 | 109.62 | 116.57 | 113.09 | 103.18 | 92.38 | 102.06 |
| Group 5 | 103.23 | 139.41 | 111.31 | 62.41 | 42.40 | 34.32 | 30.63 | 20.52 | 19.00 |

The tumour volumes of different groups of mice were measured on different days and the mean values were obtained and plotted as curves as shown in Fig. 22.

In order to verify the effect of Compound A on the body weight change of experimental animals, based on the above experimental method, the body weight change of experimental animals was recorded at different time points and the growth rate of their body weight was calculated and plotted as curves as shown in Fig. 23.

The above experimental results showed that Compound A at the administration doses of 1.25 mg/kg, 2.5 mg/kg and 5 mg/kg had good antitumor activity against the lung cancer LU2505 PDX model, and the antitumor activity of Compound A at the administration doses of 2.5 mg/kg and 5 mg/kg was higher than that of Paclitaxel at the administration dose of 20 mg/kg. The therapeutic effect of Compound A (1.25 mg/kg, 2.5 mg/kg and 5 mg/kg) was dose-dependent, with a statistically significant difference (p=0.0192) in the Compound A (5 mg/kg) treatment group as compared with the

Compound A (1.25 mg/kg) treatment group, and there was no statistically significant difference among the remaining groups. Meanwhile, in comparison with the body weight changes of experimental animals in the control group, Compound A showed excellent tumor inhibitory effects without toxic side effects.

4.7 Comparison of *in vivo* pharmacodynamic experiment of Compound A, Sorafenib and AST-3424 in liver cancer LI6643 PDX model

Experiment Instruction: BALB/c nude mice were subcutaneously inoculated with HuPrime® model LI6643 tumor blocks to establish a subcutaneous grafted tumor model of human liver cancer. The experiment was divided into groups of test drug Sorafenib (Sorafenib) 30 mg/kg group, test drug AST-3424 0.3 mg/kg, 1 mg/kg, 1.25 mg/kg groups, test drug Compound A 0.5 mg/kg, 1 mg/kg, 2 mg/kg groups, as well as 7.5% anhydrous ethanol + 7.5% polyoxyethylene (35) castor oil + 85% normal saline (pH 7.5) vehicle control group, a total of 8 groups, with 6 mice per group. Among them, 7.5% anhydrous ethanol + 7.5% polyoxyethylene (35) castor oil + 85% normal saline (pH 7.5) vehicle control group was administered by tail vein once a week for 3 consecutive weeks; test drug AST-3424 0.3 mg/kg and 1 mg/kg groups were administered by tail vein injection once a day for 5 consecutive days, followed by 2 days off, followed by 2 weeks off, and then continuously administered for 5 consecutive days; test drug AST-3424 1.25 mg/kg group was administered by tail vein injection once a week for 2 consecutive weeks, followed by one week off, and then continuously administered for 2 consecutive weeks; Compound A 0.5 mg/kg, 1 mg/kg, and 2 mg/kg groups were administered by tail vein injection once a day for 5 consecutive days, followed by 2 days off, followed by 2 weeks off, and then continuously administered for 5 consecutive days. The test drug Sorafenib 30 mg/kg group was administered by gavage, once daily for 21 consecutive days. The efficacy was evaluated according to the relative tumor inhibition rate TGI (%), and the safety was evaluated according to the change in body weight and the death of the animals.

The pharmacodynamic analysis of each group in the liver cancer LI6643 model was performed on Day 21 after administration and the results were shown in Table 19. Tumor volume size on different days was recorded and the results were shown in Table 20.

Table 19: Table of pharmacodynamic analysis of each group in HuPrime® liver cancer LI6643 Model

| Experimental group | Day 21 after the first administration (i.e., Day 21) | | | | |
|---|---|---|---|---|---|
| | **Tumor volume** ($\bar{x}\pm S$) | **Relative tumor volume** ($\bar{x}\pm S$) | **TGI (%)** | **T/C (%)** | **P-value (compared with control group)** |
| Group 1<br>7.5% anhydrous ethanol + 7.5% polyoxyethylene (35) castor oil + 85% normal saline | 605.52±161.19 | 4.82±1.36 | - | - | - |
| Group 2<br>Sorafenib<br>(30 mg/kg) | 214.75±31.53 | 1.83±0.2 | 62.12% | 37.88% | 0.29 |
| Group 3<br>AST-3424<br>(0.3 mg/kg) | 250.47±44.00 | 1.92±0.2 | 60.16% | 39.84% | 0.403 |
| Group 4<br>AST-3424<br>(1 mg/kg) | 75.89±21.82 | 0.66±0.22 | 86.32% | 13.68% | <0.001 |
| Group 5<br>AST-3424<br>(1.25 mg/kg) | 169.64±41.89 | 1.36±0.22 | 71.86% | 28.14% | <0.01 |
| Group 6<br>Compound A<br>(0.5 mg/kg) | 242.85±50.74 | 1.84±0.25 | 61.88% | 38.12% | 0.29 |

(continued)

| Experimental group | Day 21 after the first administration (i.e., Day 21) | | | | |
|---|---|---|---|---|---|
| | Tumor volume ($\bar{x}$±S) | Relative tumor volume ($\bar{x}$±S) | TGI (%) | T/C (%) | P-value (compared with control group) |
| Group 7 Compound A (1 mg/kg) | 174.86±17.43 | 1.44±0.16 | 70.09% | 29.91% | <0.01 |
| Group 8 Compound A (2 mg/kg) | 116.38±20.17 | 0.97±0.14 | 79.81% | 20.19% | <0.001 |
| Note: 1. Data were expressed as "mean + standard error"; 2. $T/C\ \% = T_{RTV} / C_{RTV} \times 100\%$ or $T/C\ \% = T_{TV} / C_{TV} \times 100\%$; $TGI\% = (1-T/C) \times 100\%$. | | | | | |

Table 20: Size of tumor volume in different experimental group on different days (mm$^3$)

| Experimental group | Day 0 | Day 3 | Day 7 | Day 10 | Day 14 | Day 17 | Day 21 | Day 24 | Day 28 |
|---|---|---|---|---|---|---|---|---|---|
| Group 1 | 124.93 | 190.19 | 249.09 | 351.57 | 421.67 | 493.35 | 605.52 | 796.73 | 996.53 |
| Group 2 | 124.67 | 170.03 | 185.13 | 193.95 | 189.87 | 171.25 | 214.75 | 238.48 | 309.08 |
| Group 3 | 124.74 | 210.50 | 242.31 | 227.43 | 208.00 | 223.43 | 250.47 | 302.15 | 386.93 |
| Group 4 | 124.32 | 156.70 | 144.82 | 110.44 | 90.12 | 97.62 | 75.89 | 89.34 | 98.43 |
| Group 5 | 124.62 | 205.56 | 217.23 | 216.04 | 187.94 | 176.52 | 169.64 | 174.54 | 146.31 |
| Group 6 | 124.86 | 239.56 | 282.74 | 266.35 | 231.35 | 241.82 | 242.85 | 173.56 | 188.68 |
| Group 7 | 124.32 | 205.79 | 246.90 | 225.40 | 195.33 | 191.98 | 174.86 | 217.19 | 231.64 |
| Group 8 | 124.40 | 182.25 | 182.74 | 162.65 | 137.87 | 117.10 | 116.38 | 122.78 | 95.87 |

The tumour volumes of different groups of mice were measured on different days and the mean values were obtained and plotted as curves as shown in Fig. 24.

The above experimental results showed that Compound A at the administration doses of 0.5 mg/kg, 1 mg/kg, and 2 mg/kg and AST-3424 at the administration doses of 0.3 mg/kg, 1 mg/kg, and 1.25 mg/kg had similarly positive antitumour activity against the human LI6643 PDX model and their antitumour activities were comparable to that of Sorafenib at the administration dose of 20 mg/kg.

4.8 Comparison of *in vivo* pharmacodynamic experiment of Compound A and Sorafenib in liver cancer LI6643 PDX model

Experiment Instruction: BALB/c nude mice were subcutaneously inoculated with HuPrime® model LI6643 tumor blocks to establish a subcutaneous transplantation tumor model of human liver cancer. The experiment was divided into groups of test drug Sorafenib 20-30 mg/kg group, Compound A 1.25 mg/kg, 2.5 mg/kg, 5 mg/kg, 10 mg/kg groups and 7.5% anhydrous ethanol + 7.5% polyoxymethylene (35) castor oil + 85% glucose injection solution D5W (pH 7.4) vehicle control group, a total of 6 groups, with 5 mice per group. Among them, 7.5% anhydrous ethanol + 7.5% polyoxyethylene (35) castor oil + 85% glucose injection D5W (pH 7.4) vehicle control group, test drug Compound A 1.25 mg/kg, 2.5 mg/kg, 5 mg/kg groups were administered by intraperitoneal injection, administered every day for 5 consecutive days, followed by 2 days off, and then a further 2 weeks off for a total of 2 dosing cycles, observed for 28 days. The test drug Compound A 10 mg/kg group was administered by tail vein injection and administered once a week for 2 consecutive weeks, followed by 1 week off for a total of 2 dosing cycles and observed for 25 days. Sorafenib 20-30 mg/kg group was administered by gavage, during day0-day11, 30 mg/kg dose were administered once a day for 12 consecutive days, during day12-day20, due to the significant weight loss of the mice, the doses were adjusted from 30 mg/kg to 20 mg/kg, administered once a day for 9 consecutive days and observed for 33 days. The efficacy was evaluated according to the

relative tumor inhibition rate TGI (%), and the safety was evaluated according to the change in body weight and the death of the animals.

The pharmacodynamic analysis of each group in the liver cancer LI6643 model was performed on day 35 after administration and the results were shown in Table 21. Tumour volume size was recorded on different days and the results were shown in Table 22.

Table 21: Table of pharmacodynamic analysis of each group in the HuPrime® liver cancer LI6643 model

| **Experimental group** | **7th day after the end of all administrations (i.e., Day 35).** | | | | |
|---|---|---|---|---|---|
| | **Tumor volume** ($\bar{x}\pm$S) | **Relative tumor volume** ($\bar{x}\pm$S) | **TGI (%)** | **T/C (%)** | **P-value (compared with control group)** |
| Group 1<br>7.5%anhydrous ethanol+ 7.5% polyoxyethylene (35) castor oil +85% glucose injection D5W(pH7.4) | 1780.45±371.56 | 14.15±2.84 | - | - | - |
| Group 2<br>Sorafenib 20-30mg/kg | 392.20±64.05 | 3.09±0.45 | 78.19% | 21.81% | 0.174 |
| Group 3<br>Compound A<br>10mg/kg, QWx2; 1 week off; QWx2, i.v. | 0±0 | 0±0 | 100.00% | 0.00% | 0.00000000138 |
| Group 4<br>Compound A<br>1.25mg/kg, QDx5; 2 days off, 2 weeks off; QDx5, i.p. | 35.61±18.63 | 0.27±0.14 | 98.11% | 1.89% | 0.000000555 |
| Group 5<br>Compound A<br>2.5mg/kg, QDx5; 2 days off, 2 weeks off; QDx5, i.p. | 56.62±9.62 | 0.45±0.07 | 96.81% | 3.19% | 0.0000895 |
| Group 6<br>Compound A<br>5mg/kg, QDx5; 2 days off, 2 weeks off; QDx5, i.p. | 7.12±7.12 | 0.05±0.05 | 99.66% | 0.34% | 0.00000000613 |

Table 22: Size of tumor volume in different experimental group on different days ($mm^3$)

| Experimental group | Day 0 | Day 4 | Day 7 | Day 11 | Day 14 | Day 18 | Day 21 | Day 25 |
|---|---|---|---|---|---|---|---|---|
| Group 1 | 127.06 | 184.53 | 321.39 | 445.25 | 595.14 | 856.01 | 1053.14 | 1249.58 |
| Group 2 | 126.16 | 163.51 | 205.85 | 235.50 | 279.42 | 291.41 | 287.76 | 326.85 |
| Group 3 | 126.11 | 160.85 | 216.99 | 94.21 | 72.94 | 59.48 | 25.80 | 21.28 |
| Group 4 | 126.02 | 181.97 | 270.85 | 264.04 | 197.40 | 177.65 | 102.13 | 90.41 |
| Group 5 | 125.92 | 199.12 | 278.32 | 191.74 | 167.14 | 154.33 | 100.94 | 103.28 |
| Group 6 | 126.93 | 178.97 | 214.95 | 132.13 | 63.74 | 60.36 | 40.93 | 24.91 |
| Laboratory | Day 28 | Day 32 | Day 35 | Day 39 | Day 42 | Day 46 | Day 49 | Day 53 |
| Group 1 | 1445.52 | 1574.72 | 1780.45 | 2218.93 | 1938.47 | 2147.64 | / | / |
| Group 2 | 346.56 | 385.09 | 392.20 | 431.87 | 451.48 | 521.44 | 585.72 | 622.46 |
| Group 3 | 10.93 | 7.11 | 0.00 | 0.00 | 0.00 | 0.00 | 0.00 | 0.00 |

(continued)

| Experimental group | Day 0 | Day 4 | Day 7 | Day 11 | Day 14 | Day 18 | Day 21 | Day 25 |
|---|---|---|---|---|---|---|---|---|
| Group 4 | 73.75 | 49.39 | 35.61 | 31.79 | 25.59 | 19.34 | 12.51 | 9.04 |
| Group 5 | 93.12 | 74.85 | 56.62 | 39.85 | 27.34 | 14.83 | 12.00 | 12.86 |
| Group 6 | 21.48 | 8.71 | 7.12 | 6.77 | 5.57 | 4.27 | 4.12 | 3.81 |

The tumour volumes of different groups of mice were measured on different days and the mean values were obtained and plotted as curves as shown in Fig. 25. In order to verify the effect of Compound A on the body weight change of experimental animals, based on the above experimental method, the body weight change of experimental animals was recorded at different time points and the growth rate of their body weight was calculated and plotted as curves as shown in Fig. 26.

The above experimental results showed that Compound A at the administration doses of 1.25 mg/kg, 2.5 mg/kg, 5 mg/kg and 10 mg/kg had good anti-tumor activity against LI6643 cells, and all of their anti-tumor activity was higher than that of Sorafenib at the administration dose of 30 mg/kg. Meanwhile, Compound A showed an excellent tumor inhibitory effect without toxic side effects as compared with the weight change of experimental animals in the control group.

4.9 Comparison of *in vivo* pharmacodynamic experiment of Compound A and Sorafenib in the liver cancer LI1005 PDX model

Experiment Instruction: BALB/c nude mice were subcutaneously inoculated with HuPrime® model LI1005 tumor blocks to establish a subcutaneous transplantation tumor model of human liver cancer. The experiment was divided into groups of test drug Sorafenib 30 mg/kg group, Compound A 1.25 mg/kg, 2.5 mg/kg, 5 mg/kg, 10 mg/kg groups and 7.5% anhydrous ethanol + 7.5% polyoxymethylene (35) castor oil + 85% glucose injection D5W (pH 7.4) vehicle control group, a total of 6 groups, with 5 mice per group. Among them, 7.5% anhydrous ethanol + 7.5% polyoxyethylene (35) castor oil + 85% glucose injection D5W (pH 7.4) vehicle control group, test drug Compound A 1.25 mg/kg, 2.5 mg/kg, 5 mg/kg groups were administered by intraperitoneal injection, administered every day for 5 consecutive days, followed by 2 days off, and then a further 2 weeks off for a total of 2 dosing cycles, observed for 10 days. Compound A 10 mg/kg group was administered by tail vein injection once a week for 2 consecutive weeks, followed by 1 week off for a total of 2 dosing cycles and observed for 7 days. Sorafenib 30 mg/kg group was administered by gavage, once a day for 21 consecutive days and observed for 15 days. The efficacy was evaluated according to the relative tumor inhibition rate TGI (%), and the safety was evaluated according to the change in body weight and the death of the animals.

The pharmacodynamic analysis of each group in liver cancer LI1005 model was performed on Day 35 after administration and the results were shown in Table 23. The size of tumor volume on different days was recorded and the results were shown in Table 24.

Table 23: Table of pharmacodynamic analysis of each group in HuPrime® liver cancer LI1005 model

| **Experimental group** | **35th day after administration (i.e., Day35, 9/29/2020)** | | | | |
|---|---|---|---|---|---|
| | **Tumor volume** ($\bar{x}\pm$S) | **Relative tumor volume** ($\bar{x}\pm$S) | **TGI (%)** | **T/C (%)** | **P-value (compared with control group)** |
| Group 1<br>7.5%anhydrous ethanol+ 7.5% polyoxyethylene (35) castor oil +85% glucose injection D5W (pH7.4) | 1090.50±293.57 | 10.09±1.78 | - | - | - |
| Group 2<br>Sorafenib 30mg/kg | 294.09±60.93 | 2.85±0.49 | 71.74% | 28.26% | 5.06E-02 |
| Group 3<br>Compound A | 179.97±51.38 | 1.87±0.54 | 81.47% | 18.53% | 9.15E-04 |
| 10mg/kg, QWx2; 1 week off; QWx2, i.v. | | | | | |

(continued)

| Experimental group | 35th day after administration (i.e., Day35, 9/29/2020) | | | | |
|---|---|---|---|---|---|
| | **Tumor volume** ($\bar{x}\pm$S) | **Relative tumor volume** ($\bar{x}\pm$S) | **TGI (%)** | **T/C (%)** | **P-value (compared with control group)** |
| Group 4<br>Compound A 1.25mg/kg, QDx5; 2 days off, 2 weeks off; QDx5, i.p. | 334.61±61.24 | 3.28±0.53 | 67.53% | 32.47% | 1.20E-01 |
| Group 5<br>Compound A 2.5mg/kg, QDx5; 2 days off, 2 weeks off; QDx5, i.p. | 178.69±65.98 | 1.60±0.42 | 84.12% | 15.88% | 3.21E-04 |
| Group 6<br>Compound A 5mg/kg, QDx5; 2 days off, 2 weeks off; QDx5, i.p. | 85.00±26.32 | 0.77±0.18 | 92.38% | 7.62% | 4.65E-06 |
| Note: 1. Data were expressed as "mean ± standard error";<br>2.<br>$T/C\ \% = T_{RTV} / C_{RTV} \times 100\%$ or $T/C\ \% = T_{TV} / C_{TV} \times 100\%$; $TGI\% = (1\text{-}T/C)\times 100\%$. | | | | | |

Table 24: Size of tumor volume in different experimental group on different days ($mm^3$)

| Experimental group | Day 0 | Day 3 | Day 7 | Day 10 | Day 14 | Day 17 | Day 21 | Day 24 | Day 28 | Day 31 | Day 35 |
|---|---|---|---|---|---|---|---|---|---|---|---|
| Group 1 | 102.76 | 180.44 | 290.97 | 342.24 | 413.26 | 516.71 | 630.34 | 736.61 | 923.45 | 1008.22 | 1090.50 |
| Group 2 | 102.89 | 121.64 | 136.21 | 136.21 | 136.89 | 140.09 | 142.56 | 161.17 | 200.97 | 227.65 | 294.09 |
| Group 3 | 102.58 | 127.72 | 172.58 | 138.61 | 127.60 | 132.95 | 151.00 | 181.36 | 191.25 | 186.25 | 179.97 |
| Group 4 | 102.66 | 157.94 | 198.68 | 188.83 | 196.25 | 204.85 | 228.87 | 256.49 | 246.26 | 282.93 | 334.61 |
| Group 5 | 103.02 | 156.63 | 197.27 | 156.39 | 147.34 | 148.94 | 173.92 | 180.83 | 178.96 | 182.98 | 178.69 |
| Group 6 | 103.15 | 160.80 | 167.82 | 126.93 | 97.35 | 90.25 | 106.81 | 115.29 | 110.01 | 102.22 | 85.00 |

The tumour volumes of different groups of mice were measured on different days and the mean values were obtained and plotted as curves as shown in Fig. 27. In order to verify the effect of Compound A on the body weight change of experimental animals, based on the above experimental method, the body weight change of experimental animals was recorded at different time points and the growth rate of their body weight was calculated and plotted as curves as shown in Fig. 28.

Experimental Conclusions: Compound A at the doses of 10 mg/kg, 2.5 mg/kg and 5 mg/kg had significant tumor growth inhibitory effects against HuPrime® liver cancer LI1005, with statistically significant differences compared with the control group, and the efficacy thereof was superior to that of Sorafenib. Compound A at the dose of 1.25 mg/kg and Sorafenib at the dose of 30 mg/kg had an inhibitory effect on tumor growth against HuPrime® liver cancer LI1005, with no statistically significant difference compared with the control group and no difference in efficacy compared with the Sorafenib group. Tumor-bearing mice were well tolerated to Compound A at tested doses of 10 mg/kg, 1.25 mg/kg, 2.5 mg/kg, and 5 mg/kg. Sorafenib caused a certain weight loss in mice at tested dose of 30 mg/kg, and the body weight gradually returned to normal after the end of the dosing period. Meanwhile, Compound A showed an excellent tumor inhibitory effect without toxic side effects as compared with the weight change of experimental animals in the control group.

4.10 Comparison of *in vivo* pharmacodynamic experiment of Compound A and Sorafenib in the liver cancer HepG2 CDX model

Experiment Instruction: BALB/c nude mice were subcutaneously inoculated with human liver cancer HepG2

cells to establish a subcutaneous transplantation tumor model of human liver cancer. The experiment was divided into groups of test drug Sorafenib 30 mg/kg group, test drug Compound A 5 mg/kg (QWx2; 1 week off; QWx2, i.v.), 10 mg/kg (QWx2; 1 week off; QWx2, i.v.), 1.25 mg/kg (QDx5; 2 days off, 2 weeks off; QDx5, i.p.), 2.5 mg/kg (QDx5; 2 days off, 2 weeks off; QDx5, i.p.), 5 mg/kg (QDx5; 2 days off, 2 weeks off; QDx5, i.p.) single drug groups and 7.5% anhydrous ethanol + 7.5% polyoxyethylene (35 ) castor oil + 85% glucose injection D5W (pH 7.4) vehicle control group, a total of 7 groups, with 6 mice per group. Among them, 7.5% anhydrous ethanol + 7.5% polyoxyethylene (35) castor oil + 85% glucose injection D5W (pH 7.4) vehicle control group, test drug Compound A 1.25 mg/kg (QDx5; 2 days off, 2 weeks off; QDx5, i.p.), 2.5 mg/kg (QDx5; 2 days off, 2 weeks off; QDx5, i.p.) and 5 mg/kg (QDx5; 2 days off, 2 weeks off; QDx5, i.p.) groups were administered by intraperitoneal injection, administered once a day for 5 consecutive days followed by 2 days off and 2 weeks off, and then once a day for 5 consecutive days, followed by 9 days of observation. Test drug Compound A 5 mg/kg (QWx2; 1 week off; QWx2, i.v.), 10 mg/kg (QWx2; 1 week off; QWx2, i.v.) groups were administered by tail vein injection, administered once a week for 2 consecutive weeks, followed by 1 week off, and then once a week for 2 consecutive weeks, followed by 6 days of observation after the final administration. The test drug Sorafenib 30 mg/kg group was administered by gavage, once a day for 21 consecutive days, followed by 14 days of observation. The efficacy was evaluated according to the relative tumor inhibition rate TGI (%), and the safety was evaluated according to the change in body weight and the death of the animals.

The pharmacodynamic analysis of each group in the liver cancer HepG2 model was performed on Day 53 after cell inoculation and the results were shown in Table 25. The size of tumor volume on different days was recorded and the results were shown in Table 26.

Table 25: Table of pharmacodynamic analysis of each group in human liver cancer HepG2 model

| **Experimental group** | **Day 53rd after tumor cell inoculation (i.e., Day53, day 34th after the first administration)** | | | | |
|---|---|---|---|---|---|
| | **Tumor volume** ($x \pm S$) | **Relative tumor volume** ($\bar{x} \pm S$) | **TGI (%)** | **T/C (%)** | **P-value (compared with control group)** |
| Group 1<br>7.5% anhydrous ethanol+ 7.5% polyoxyethylene (35) castor oil +85% glucose injection D5W (pH7.4) QDx5; 2 days off, 2 weeks off; QDx5, i.p. | 2293.53±358.48 | 11.85±1.76 | - | - | - |
| Group 2<br>Sorafenib, 30mg/kg, QDx21 Days, p.o. | 1484.78±304.89 | 7.82±1.58 | 34.05 | 65.95 | 0.833 |
| Group 3<br>Compound A, 5mg/kg, QWx2; 1 week off; QWx2, i.v. | 136.44±66.47 | 0.70±0.34 | 94.08 | 5.92 | 0.0000000978 |
| Group 4<br>Compound A, 10mg/kg, QWx2; 1 week off; QWx2, i.v. | 88.20±29.95 | 0.45±0.15 | 96.17 | 3.83 | 0.00000000582 |
| Group 5<br>Compound A, 1.25mg/kg, QDx5; 2 days off, 2 weeks off; QDx5, i.p. | 1066.23±252.18 | 5.57±1.26 | 52.97 | 47.03 | 0.192 |
| Group 6<br>Compound A, 2.5mg/kg, | 417.76±105.16 | 2.19±0.52 | 81.54 | 18.46 | 0.000184 |
| QDx5; 2 days off, 2 weeks off; QDx5, i.p. | | | | | |

(continued)

| Experimental group | Day 53rd after tumor cell inoculation (i.e., Day53, day 34th after the first administration) | | | | |
|---|---|---|---|---|---|
| | **Tumor volume** ($x \pm S$) | **Relative tumor volume** ($\bar{x} \pm S$) | **TGI (%)** | **T/C (%)** | **P-value (compared with control group)** |
| Group 7<br>Compound A, 5mg/kg, QDx5; 2 days off, 2 weeks off; QDx5, i.p. | 189.84±72.81 | 0.99±0.38 | 91.67 | 8.33 | 0.000000381 |
| Note: 1. Data were expressed as "mean ± standard error";<br>2.<br>$T/C\ \% = T_{RTV} / C_{RTV} \times 100\%$ or $T/C\ \% = T_{TV} / C_{TV} \times 100\%$; $TGI\% = (1\text{-}T/C) \times 100\%$. | | | | | |

Table 26: Size of tumor volume in different experimental group on different days ($mm^3$)

| Experimental group | Day 19 | Day 22 | Day 24 | Day 27 | Day 31 | Day 36 | Day 39 | Day 42 | Day 46 | Day 49 | Day 53 |
|---|---|---|---|---|---|---|---|---|---|---|---|
| Group 1 | 192.64 | 336.01 | 345.38 | 430.57 | 617.10 | 771.64 | 969.06 | 1325.75 | 1574.22 | 1939.71 | 2293.53 |
| Group 2 | 189.76 | 291.62 | 311.64 | 340.46 | 398.84 | 491.06 | 509.41 | 657.51 | 816.63 | 1012.20 | 1484.78 |
| Group 3 | 191.48 | 315.19 | 360.12 | 390.21 | 373.82 | 314.44 | 283.08 | 276.88 | 233.11 | 145.58 | 136.44 |
| Group 4 | 191.97 | 314.32 | 375.81 | 427.75 | 386.68 | 347.85 | 312.31 | 252.78 | 197.45 | 142.21 | 88.20 |
| Group 5 | 189.54 | 315.41 | 351.74 | 383.10 | 366.64 | 416.27 | 586.80 | 782.39 | 886.97 | 975.41 | 1066.23 |
| Group 6 | 189.31 | 292.03 | 360.14 | 373.23 | 405.67 | 392.90 | 342.04 | 399.84 | 417.54 | 456.60 | 417.76 |
| Group 7 | 189.64 | 259.29 | 280.24 | 286.22 | 290.75 | 293.88 | 247.49 | 224.58 | 215.32 | 234.89 | 189.84 |

The tumour volumes of different groups of mice were measured on different days and the mean values were obtained and plotted as curves as shown in Fig. 29.

In order to verify the effect of Compound A on the body weight change of experimental animals, based on the above experimental method, the body weight change of experimental animals was recorded at different time points and the growth rate of their body weight was calculated and plotted as curves as shown in Fig. 30.

Experimental Conclusions: the efficacy of Compound A is superior to that of Sorafenib. Since HepG2 was a model of cachexia, which was characterized by spontaneous weight loss in tumor-bearing mice. Compound A at the doses of 10 mg/kg (QWx2; 1 week off; QWx2, i.v., Group 4) and 2.5 mg/kg (QDx5; 2 days off, 2 weeks off; QDx5, i.p., Group 6) had a significant inhibitory effect on tumor growth in the human liver cancer HepG2 model, both with statistically significant differences as compared with the control group. Compound A did not cause severe weight loss or death in tumor-bearing mice at the above-mentioned 2 tested doses and modes of administration. The test Compound A single drug at the dose of 5 mg/kg (QWx2; 1 week off; QWx2, i.v., Group 3) had a statistically significant inhibitory effect on tumor growth in the human liver cancer HepG2 model, with a statistically significant difference compared with the control group, and one mouse in this group suffered death during the dosing cycles. Compound A at the dose of 5 mg/kg (QDx5; 2 days off, 2 weeks off; QDx5, i.p., Group 7) had a significant inhibitory effect on tumor growth in the human liver cancer HepG2 model, with a statistically significant difference compared with the control group, and one mouse was euthanized within the dosing cycles owing to BWL >20%. One mouse in the 5 mg/kg of Compound A group was completely cured. Moreover, 1.25 mg/kg (QDx5; 2 days off, 2 weeks off; QDx5, i.p., Group 5), 2.5 mg/kg (QDx5; 2 days off, 2 weeks off; QDx5, i.p., Group 6), and 5 mg/kg (QDx5; 2 days off, 2 weeks off; QDx5, i.p., Group 7) of Compound A treatment groups exhibited a dose-dependent dose-response relationship. One mouse in each of Groups 3, 4, and 7 was completely cured, and Sorafenib at the tested dose of 30 mg/kg exhibited tumor growth inhibition against the human liver cancer HepG2 model, with no statistically significant difference compared with the control group, and caused significant weight loss in mice at the tested dose.

4.11 Comparison of *in vivo* pharmacodynamic experiment of Compound A and Paclitaxel in the lung cancer LU0884 PDX model

Experiment Instruction: BALB/c nude mice were subcutaneously inoculated with HuPrime® model LU0884 tumor blocks to establish a subcutaneous transplantation tumor model of human lung cancer. The experiment was divided into groups of test drug Paclitaxel 20 mg/kg group, Compound A 2.5 mg/kg, 5 mg/kg and 10 mg/kg groups and 7.5% anhydrous ethanol + 7.5% polyoxyethylene (35) castor oil + 85% glucose injection D5W (pH 7.4) vehicle control group, a total of 6 groups, with 6 mice per group. Among them, 7.5% anhydrous ethanol + 7.5% polyoxyethylene (35) castor oil + 85% glucose injection D5W (pH 7.4) vehicle control group, Compound A 2.5 mg/kg, 5 mg/kg, and 10 mg/kg groups were administered by tail vein injection once a week for 3 consecutive weeks and observed for 8 days. The test drug Paclitaxel 20 mg/kg group was administered by intraperitoneal injection, once every 4 days for 4 consecutive doses and observed for 13 days. The efficacy was evaluated according to the relative tumor inhibition rate TGI (%), and the safety was evaluated according to the change in body weight and the death of the animals.

The pharmacodynamic analysis of each group in the lung cancer LU0884 model was performed on Day 28 after administration and the results were shown in Table 27. The size of tumor volume on different days was recorded and the results were shown in Table 28.

Table 27: Table of pharmacodynamic analysis of each group in the HuPrime® lung cancer LU0884 model

| **Experimental group** | **Day 8th after the end of all dosing cycles (i.e., Day28)** | | | | |
|---|---|---|---|---|---|
| | **Tumor volume** ($\bar{x}$±S) | **Relative tumor volume** ($\bar{x}$±S) | **TGI (%)** | **T/C (%)** | **P-value (compared with control group)** |
| Group 1<br>7.5% anhydrous ethanol+ 7.5% polyoxyethylene (35) castor oil +85% glucose injection D5W (pH7.4), 0 mg/kg, QWx3, i.v. | 664.59±121.31 | 5.99±0.80 | - | - | - |
| Group 2<br>Paclitaxel, 20mg/kg, Q4Dx4, i.p. | 528.88±147.69 | 4.72±1.26 | 21.21% | 78.79% | 0.957 |
| Group 4<br>Compound A, 2.5mg/kg, QWx3, i.v. | 360.43±44.42 | 3.47±0.56 | 42.15% | 57.85% | 0.560 |
| Group 5<br>Compound A, 5mg/kg, QWx3, i.v. | 291.60±103.85 | 2.61±0.94 | 56.38% | 43.62% | 0.252 |
| Group 6<br>Compound A, 10mg/kg, QWx3, i.v. | 434.80±160.03 | 3.78±1.19 | 36.98% | 63.02% | 0.687 |
| 1. Data were expressed as "mean ± standard error";<br>2.<br>$T/C\ \% = T_{RTV} / C_{RTV} \times 100\%$ or $T/C\ \% = T_{TV} / C_{TV} \times 100\%$; $TGI\% = (1\text{-}T/C) \times 100\%$. | | | | | |

Table 28: Size of tumor volume in different experimental group on different days ($mm^3$)

| Experimental group | Day 0 | Day 4 | Day 7 | Day 11 | Day 14 | Day 18 | Day 21 | Day 25 | Day 28 |
|---|---|---|---|---|---|---|---|---|---|
| Group 1 | 109.73 | 180.12 | 262.62 | 314.65 | 366.16 | 476.97 | 510.27 | 599.15 | 664.59 |
| Group 2 | 110.11 | 157.57 | 229.87 | 250.46 | 292.52 | 381.53 | 402.62 | 492.50 | 528.88 |
| Group 4 | 109.86 | 138.21 | 177.96 | 194.38 | 216.81 | 261.94 | 287.79 | 328.61 | 360.43 |
| Group 5 | 110.67 | 173.28 | 221.81 | 229.45 | 236.47 | 260.75 | 271.59 | 289.40 | 291.60 |
| Group 6 | 109.04 | 202.09 | 257.40 | 283.55 | 293.14 | 343.92 | 359.85 | 411.01 | 434.80 |

The tumour volumes of different groups of mice were measured on different days and the mean values were obtained and plotted as curves as shown in Fig. 31.

In order to verify the effect of Compound A on the body weight change of experimental animals, based on the above experimental method, the body weight change of experimental animals was recorded at different time points and the growth rate of their body weight was calculated and plotted as curves as shown in Fig. 32.

Experimental Conclusions: the test drug Paclitaxel (20 mg/kg, Group 2) showed weak tumor inhibitory effect on HuPrime® lung cancer LU0884 at the tested dose, with no statistically significant difference compared with the control group. Compound A 2.5 mg/kg (Group 4), 5 mg/kg (Group 5) and 10 mg/kg (Group 6) all showed certain tumor inhibitory effects against HuPrime® Lung Cancer LU0884 at the tested doses, with no statistically significantly difference compared with the control group. Therefore, the efficacy of the test drug Paclitaxel and Compound A were not significantly different in this model. The tumor-bearing mice were well tolerated to Paclitaxel at the tested dose of 20 mg/kg (Group 2) and Compound A at the tested doses of 2.5 mg/kg (Group 4), 5 mg/kg (Group 5), and 10 mg/kg (Group 6) without significant weight loss.

4.12 Comparison of *in vivo* pharmacodynamic experiment of Compound A, AST-3424 and Sorafenib in liver cancer LI6664 PDX model

Experiment Instruction: BALB/c nude mice were subcutaneously inoculated with HuPrime® model LI6664 tumor blocks to establish a subcutaneous transplantation tumor model of human liver cancer. The experiment was divided into groups of test drug Sorafenib 30 mg/kg group, test drug AST-3424 0.3 mg/kg, 1 mg/kg, 1.25 mg/kg group, Compound A 0.5 mg/kg, 1.5 mg/kg, 4. 5 mg/kg group, and 7.5% anhydrous ethanol + 7.5% polyoxyethylene (35) castor oil + 85% glucose injection D5W (pH 7.4) vehicle control group, a total of 8 groups, with 6 mice per group. Among them, 7.5% anhydrous ethanol + 7.5% polyoxyethylene (35) castor oil + 85% glucose injection D5W (pH 7.4) vehicle control group was administered by tail vein once a week for 3 consecutive weeks; test drug AST-3424 0.3 mg/kg and 1 mg/kg groups were administered by tail vein injection once a day for 5 consecutive days, followed by 2 days off, followed by 2 weeks off, and then continuously administered for 5 consecutive days; the test drug AST-3424 1.25 mg/kg group was administered by tail vein injection once a week for 2 consecutive weeks, followed by one week off, and then continuously administered for 2 consecutive weeks; Compound A 0.5 mg/kg, 1.5 mg/kg, and 4.5 mg/kg groups were administered by tail vein injection once a week for three consecutive weeks, followed by 1 week off, and then administered for three consecutive weeks. The test drug Sorafenib 30 mg/kg group was administered by gavage once daily for 21 consecutive days. The efficacy was evaluated according to the relative tumor inhibition rate TGI (%), and the safety was evaluated according to the change in body weight and the death of the animals.

The pharmacodynamics analysis of each group in liver cancer LI6664 PDX model was performed on Day 28 after administration and the results were shown in Table 29. The size of tumor volume on different days was recorded and the results were shown in Table 30.

Table 29: Table of pharmacodynamic analysis of each group in the HuPrime® liver cancer LI6664 model

| **Experimental group** | **Day 28 after the initial administration (i.e., Day 28)** | | | | |
|---|---|---|---|---|---|
| | **Tumor volume** ($\bar{x}\pm$S) | **Relative tumor volume** ($\bar{x}\pm$S) | **TGI (%)** | **T/C** (%) | **P-value (compared with control group)** |
| Group 1 7.5% anhydrous ethanol+ 7.5% polyoxyethylene (35) castor oil +85% glucose injection D5W (pH7.4) | 1506.35±457.93 | 12.91±2.94 | - | - | - |
| Group 2 Sorafenib (30 mg/kg) | 1003.42±128.88 | 9.17±0.47 | 28.99% | 71.01% | 1 |
| Group 3 AST-3424 (0.3 mg/kg) | 746.71±130.55 | 6.99±1.31 | 45.85% | 54.15% | 0.79 |
| Group 4 AST-3424 (1 mg/kg) | 463.09±99.32 | 4.51±1.21 | 65.05% | 34.95% | 0.0329 |
| Group 5 AST-3424 (1.25 mg/kg) | 687.95±147.16 | 6.14±0.94 | 52.42% | 47.58% | 0.479 |
| Group 6 Compound A | 920.46±215.96 | 8.51±2.07 | 34.07% | 65.93% | 0.855 |
| (0.5 mg/kg) | | | | | |
| Group 7 Compound A (1.5 mg/kg) | 466.31±77.63 | 4.31±0.54 | 66.57% | 33.43% | 0.0457 |

(continued)

| Experimental group | Day 28 after the initial administration (i.e., Day 28) | | | | |
|---|---|---|---|---|---|
| | Tumor volume ($\bar{x}\pm S$) | Relative tumor volume ($\bar{x}\pm S$) | TGI (%) | T/C (%) | P-value (compared with control group) |
| Group 8 Compound A (4.5 mg/kg) | 320.72±40.03 | 2.96±0.27 | 77.05% | 22.95% | 0.000856 |
| Note: 1. Data were expressed as "mean ± standard error"; 2. $T/C\ \% = T_{RTV} / C_{RTV} \times 100\%$ or $T/C\ \% = T_{TV} / C_{TV} \times 100\%$; $TGI\% = (1-T/C)\times 100\%$. | | | | | |

Table 30: Size of tumor volume in different experimental group on different days ($mm^3$)

| Experi mental group | Day 0 | Day 4 | Day 7 | Day 11 | Day 14 | Day 18 | Day 21 | Day 25 |
|---|---|---|---|---|---|---|---|---|
| Group 1 | 108.31 | 224.91 | 303.09 | 414.35 | 597.93 | 762.00 | 892.78 | 1214.79 |
| Group2 | 107.93 | 170.15 | 197.93 | 218.34 | 321.83 | 391.52 | 481.85 | 730.68 |
| Group3 | 108.20 | 162.32 | 177.51 | 208.59 | 306.95 | 391.40 | 499.34 | 631.08 |
| Group4 | 107.53 | 131.59 | 145.23 | 158.03 | 233.54 | 291.97 | 371.35 | 438.00 |
| Group5 | 108.46 | 174.65 | 210.25 | 222.97 | 291.02 | 361.03 | 467.94 | 581.63 |
| Group6 | 108.44 | 201.76 | 277.38 | 301.39 | 347.30 | 434.18 | 518.23 | 746.19 |
| Group7 | 108.25 | 127.56 | 152.00 | 161.35 | 206.44 | 225.71 | 287.02 | 398.93 |
| Group8 | 108.26 | 125.30 | 142.58 | 150.82 | 176.69 | 180.29 | 201.42 | 282.76 |
| Experi mental group | Day 28 | Day 32 | Day 35 | Day 39 | Day 42 | Day 46 | Day 49 | / |
| Group 1 | 1506.35 | 1441.57 | 1671.99 | 2081.93 | 1409.66 | 649.08 | 695.40 | / |
| Group2 | 1003.42 | 1333.07 | 1595.00 | 2102.94 | 2729.04 | 2892.15 | 2600.00 | / |
| Group3 | 746.71 | 958.09 | 1059.71 | 1226.51 | 1094.69 | 1268.82 | 1486.77 | / |
| Group4 | 463.09 | 622.48 | 748.13 | 929.60 | 1226.79 | 1496.83 | 1717.60 | / |
| Group5 | 687.95 | 746.71 | 871.84 | 1081.64 | 1307.36 | 1675.14 | 1923.31 | / |
| Group6 | 920.46 | 1036.44 | 1258.05 | 1426.66 | 1661.81 | 2037.97 | 1746.71 | / |
| Group7 | 466.31 | 543.94 | 643.84 | 805.84 | 1060.20 | 1179.82 | 1407.10 | / |
| Group8 | 320.72 | 339.73 | 360.45 | 391.38 | 417.79 | 451.01 | 538.20 | / |

The tumour volumes of different groups of mice were measured on different days and the mean values were obtained and plotted as curves as shown in Fig. 33.

In order to verify the effect of Compound A on the body weight change of experimental animals, based on the above experimental method, the body weight change of experimental animals was recorded at different time points and the growth rate of their body weight was calculated and plotted as curves as shown in Fig. 34.

Experimental Conclusions: the test drug Sorafenib 30 mg/kg treatment group had no tumor inhibitory effect on Day 28 after the first administration, and the relative tumor inhibition rate TGI (%) was 28.99%. The test drug AST-3424 0.3 mg/kg (administered once daily for five consecutive days, followed by 2 days off, followed by 2 weeks off, and then continuously administered for 5 consecutive days) treatment group had a certain tumor inhibitory effect on Day 28 after the first administration, with no statistically significant difference compared with the control group (p=0.79), and the relative tumor inhibition rate TGI (%) was 45.85%. The test drug AST-3424 1 mg/kg (administered once daily for five consecutive days, followed by 2 days off, followed by 2 weeks off, and then continuously administered for 5 consecutive days) treatment group had a significant tumor inhibitory effect on Day 28 after the first administration, with statistically significant difference compared with the control group (p=0.0329), and the relative tumor inhibition rate TGI(%) was

65.05%.The test drug AST- 3424 1.25 mg/kg (administered once weekly for two consecutive weeks, followed by one week off, and then administered continuously for two weeks) treatment group showed a certain tumor inhibitory effect on Day 28 (Day 28) after the first administration, with no statistically significant difference compared with the control group (p=0.479), and the relative tumor inhibition rate TGI (%) was 52.42%.

Compound A 0.5 mg/kg (administered once weekly for 3 consecutive weeks, followed by one week off, and then continuously administered for 3 consecutive weeks) treatment group showed a slight tumor inhibitory effect on Day 28 after the first administration, with no statistically significant difference (p=0.855) compared with the control group, and the relative tumor inhibition rate TGI(%) was 34.07%. Compound A 1.5 mg/kg and 4.5 mg/kg (administered once weekly for 3 consecutive weeks, followed by one week off, and then continuously administered for 3 consecutive weeks) treatment group showed a significant tumor inhibitory effect on Day 28 after the first administration, with statistically significant difference compared with the control group (p-value of 0.0457 and 0.000856, respectively), and the relative tumor inhibition rate TGI(%) was 66.57% and 77.05%, respectively. The tumor inhibitory effect of Compound A was dose-dependent and there was a statistically significant difference (p=0.0428) in the test drug Compound A 4.5 mg/kg treatment group (Group 8) compared with the 0.5 mg/kg treatment group (Group 6).

Compound A and AST-3424 had similar tumor inhibitory effects and both were superior to that of Sorafenib.

Sorafenib 30 mg/kg treatment group showed weight loss in individual mice. There was no weight loss in the AST-3424 and Compound A treatment groups during the treatment period, and there was good tolerance in the groups.

**V. Pgp inhibition assay and BBB blood-brain barrier assay**

P-glycoprotein (P-GP, also known as multidrug resistance protein) is a high-molecular-weight protein discovered on the plasma membrane of the multidrug resistant tumor cell and has a transport-pump-like structure. P-GP pumps out a variety of chemotherapy drugs out of cells and reduces intracellular drug concentration. P-GP is closely related to the resistance in clinical chemotherapy.

The role of the chemotherapy drug for treating the tumor or cancer of the central nervous system (such as the brain) is limited. The limitation is mainly due to the blood-brain barrier (BBB), which leads to the low permeability of the chemotherapy drug to the tumor system or the tumor tissue, i.e., the chemotherapy drug may not kill the cancer cells by passing through the blood-brain barrier to enter the brain.

The process that the small molecules (e.g., the chemotherapy drug) pass through the blood-brain barrier is more complicated. The blood-brain barrier, located between the systemic blood circulation and the cerebrospinal fluid, is formed by specialized brain microvascular endothelial cells, along with surrounding cells and perivascular astrocytes through tight junctions between adjacent cells, and it forced to form selective barriers when most molecules pass through, rather than forming a physical barrier around vascular endothelial cells. The small hydrophilic molecules are allowed to pass through the membrane transportation system of the BBB, whereas the large hydrophilic molecules, such as many chemotherapy drugs and macromolecular drugs, are excluded from the central nervous system unless they may be actively transported by certain proteins. More importantly, the "drug efflux pumps" of BBB for protecting the brain tissue (e.g., P-GP) may actively exclude some chemotherapy drugs and macromolecular drugs from the brain. Therefore, even that the small hydrophilic molecular chemotherapy drugs, including small molecule targeted antitumor drug molecule, may pass through the blood-brain barrier to enter the brain to exert their effect. However, the small hydrophilic molecular chemotherapy drugs may not work because they are excluded from the central nervous system under the action of the P-GP. In other words, the transmembrane structure of P-GP has the function of the energy dependent "drug pump" and may pump the hydrophobic lipophilic drugs (e.g., Vincristine (VCR), adriamycin (Dox) or etoposide (VP-16)) out of the cell and decrease the intracellular drug concentration, so that the cytotoxicity is decreased or completely lost.

For the reasons described above, the experimental data of the interaction between P-GP and the compound is used to evaluate the effective degree of the compound on the inhibitory activity of the proliferation of the tumor cells in the central nervous system.

5.1 The evaluation of the potential of the Compound A as the P-gp substrate, which took MDCKII-MDR1 cells as the model and Verapamil as the inhibitor.

5.1.1 Preparation of the cells for the penetration test:

(1) MDCKII-MDR1 cells were cultured in the cell culture bottle. The conditions of the incubator were set as 37°C, 5% $CO_2$, and 95% of the relative humidity. When the cell growth confluence was up to 70-90%, the cells were inoculated to the transwell.

(2) Before the cell inoculation, 50 μL of the culture medium was added to every well of the upper compartment of

the transwell, and 25 mL of the culture medium was added to the lower culture plate. After the culture plate was incubated in the incubator under 37°C and 5% $CO_2$ for 1 hour, it may be used to inoculate the cells.

(3) After the cells were incubated, the cell suspension was sucked and transferred to a round bottom centrifuge tube, and the tube was centrifuged at 120 g for 5 min.

(4) Cells were resuspended in the culture medium, and the final concentration was $1.56\times10^6$ cells/mL. The cell suspension was transferred to the upper compartment of culture plate of the 96-well transwell, with50 μL of the cell suspension per well. The final inoculum density was $5.45\times10^5$ cells/$cm^2$.

(5) The culture medium was replaced after inoculating for 48 h. After culturing for 4-8 days, the medium was changed every other day.

(6) The process of replacing the culture medium was as the follow: the transwell and the receiving plate were separated; the medium in the receiving plate was discarded, and then the medium in the transwell was discarded; finally, 75 μL of fresh medium was added to each transwell and 25 mL of fresh medium was added to the receiving plate.

5.1.2 Evaluation of the cell monolayer membrane integrity

(1) The MDCKII-MDR1 cells should be totally confluent and complete the differentiation after culturing for 4-8 days. After that, the MDCKII-MDR1 cells can be used for the penetration test.

(2) Millipore was used to measure the resistance of monolayer membrane, and the resistance of every well was recorded.

(3) After the measuring, the transwell culture plate was put back to the incubator.

(4) The calculation of the resistance: measured resistance (ohms) $\times$ area of the membrane ($cm^2$) = TEER value (ohm·$cm^2$). If TERR value was < 42 ohm·$cm^2$, the well would not be used in the penetration test.

5.1.3 Penetration test of the drug:

(1) MDCKII-MDR1 transwell culture plate was taken out from the incubator. The cell monolayer membrane was rinsed two times with the buffer solution and incubated at 37°C for 30 min.

(2) The transport rate that the compound was transported from the top-end to the base-end was tested. 75 μL of the buffer solution containing the test substance was added to each well of upper compartment (top-end), and 235 μL of the buffer solution was added to each well of lower compartment (base-end).

(3) The transport rate that the compound was transported from the base-end to the top-end was tested. 75 μL of the buffer solution was added to each well of upper compartment (top-end), and 235μL of the buffer solution containing the test substance was added to each well of lower compartment (base-end).

(4) 50 μL of the sample was transferred from the working solution preparation plate, and added to 200 μL of the acetonitrile containing the internal standard (100 nM Alprazolam, 200 nM Labetalol, 200 nM Caffeine and 2 μM Ketoprofen), and the mixture was tested as the dosing sample at 0 min.

(5) For testing the transportation of the test substance under the condition that the P-GP inhibitor Verapamil was added, it is necessary to add Verapamil to the buffer salts of the donor end and acceptor end of MDCKII-MDR1 transwell, and the final concentration was 100 μM.

(6) The upper and lower transportations were pooled and then incubated at 37°C for 2 h.

(7) After the incubation was finished, 50μL of the samples were taken out from each well of the upper compartment and the lower compartment of the transwell culture plate, respectively, and added to new sample tubes. 200 μL of the acetonitrile containing the internal standard (100 nM Alprazolam, 200 nM Labetalol, 200 nM Caffeine and 2 μM Ketoprofen) was added to the sample tubes. After the sample tubes were vortexed for 10 min, and centrifuged at 3220 g for 30 min. 100 μL of the supernatant was taken and diluted with the same volume of the water to perform the LC-MS/MS analysis. Each sample was adopted to the three parallel incubation.

(8) After the cell monolayer membrane was incubated for 2 h, its integrity was evaluated by the leakage assay using the fluorescence yellow, wherein the fluorescence yellow stock solution was diluted by the buffer solution to the final concentration of 100 μM. 100 μL of the fluorescence yellow solution was added to each well of the upper compartment of the transwell, and 300 μL of the buffer was added to each well of the lower compartment of the transwell. After the transwell was incubated at 37°C for 30 min, 80 μL of the solution from each well of the upper compartment and the lower compartment was sucked respectively to a new 96-well plate. Microplate reader was used to perform the fluorimetry with the excitation wavelength of 480 nm and the emission wavelength of 530 nm.

5.1.4 Data analysis:

The peak area was calculated by the result of the ion chromatography. The apparent permeability coefficient (Papp, unit: cm/s×10$^{-6}$) of the compound was calculated by the following formula:

$$P_{app} = \frac{V_A}{Area \times time} \times \frac{[drug]_{acceptor}}{[drug]_{initial,donor}}$$

In the formula, "VA" refers to the volume of the solution on the acceptor end (Ap→B1 was 0.235 mL, and B1→Ap was 0.075 mL), "Area" refers to the area of the membrane of Transwell-96 well plate (0.143 cm$^2$), "time" refers to the incubation time (unit: s), "[drug]acceptor" refers to the drug concentration of the acceptor end after the incubation, and "[drug]initial, donor" refers to the initial concentration of the administration before the incubation.

Efflux Ratio was calculated by the following formula:

$$Efflux\ \ Ratio = \frac{P_{app\ (B-A)}}{P_{app\ (A-B)}}$$

In the formula, "Papp (B-A)" refers to the apparent permeability coefficient from the base-end to the top-end, and "Papp (A-B)" refers to the apparent permeability coefficient from the top-end to the base-end.

Recovery was calculated by the following formula:

$$Re\,cov\,ery\% = \frac{[drug]_{acceptor} \times V_A + [drug]_{donor} \times V_D}{[drug]_{initial,donor} \times V_D} \times 100$$

In the formula, "VA" refers to the volume of the solution on the acceptor end (unit: mL), "VD" refers to the volume of the solution on the donor end (unit: mL), "[drug]acceptor" refers to the drug concentration of the acceptor end after the incubation, "[drug]donor" refers to the drug concentration of the donor end after the incubation, and "[drug]initial, donor" refers to the initial concentration of the administration before the incubation.

The fluorescence value LY Leakage of the cell monolayer membrane was calculated by the following formula:

$$\text{LY Leakage} = \left( \frac{I_{acceptor} \times 0.3}{I_{acceptor} \times 0.3 + I_{donor} \times 0.1} \right) \times 100$$

In the formula, "Iacceptor" refers to the fluorescence intensity of the acceptor well (0.3 mL), "Idonor" refers to the fluorescence intensity of the donor well (0.1 mL), expressed by %LY. LY<1.5% denotes that the cell monolayer membrane is intact.

The efflux ratios of different compounds that were in the presence of or in the absence of P-glycoprotein inhibitor,

Verapamil, were obtained after respectively testing a part of the prepared compounds. The experimental data were shown in Table 31.

Table 31: Penetration experimental data for drugs

| Compound | Verapamil (μM) | $P_{app\ (A-B)}$ ($10^{-6}$, cm/s) | $P_{app\ (B-A)}$ ($10^{-6}$, cm/s) | Efflux Ratio | Recovery (%) AP-BL | Recovery (%) BL-AP |
|---|---|---|---|---|---|---|
| Metoprolol | 0 | 27.57 | 26.51 | 0.96 | 96.18 | 98.46 |
| Prazosin | 0 | 10.99 | 31.84 | 2.90 | 90.69 | 78.53 |
| Prazosin | 100 | 30.36 | 17.65 | 0.58 | 94.19 | 88.52 |
| Imatinib | 0 | 1.89 | 30.49 | 16.13 | 79.04 | 63.40 |
| Imatinib | 100 | 21.14 | 19.68 | 0.93 | 83.02 | 87.60 |
| A | 0 | 0.18 | 0.24 | 1.36 | 5.03 | 4.34 |
| A | 100 | 0.27 | 0.31 | 1.12 | 5.70 | 12.56 |
| AST-3424 | 0 | 1.95 | 34.51 | 17.71 | 79.11 | 90.60 |
| AST-3424 | 100 | 7.50 | 21.51 | 2.87 | 92.56 | 96.29 |

Experimental Conclusions: the three key parameters of Papp (A-B), Papp (B-A), Efflux Ratio of Compound A in the presence and absence of P-gp inhibitor Verapamil were very close to each other; therefore, Compound A was not substrates for Pgp and its efficacy was weakly affected by the blood-brain barrier.

As a control, AST-3424, which was also activated by AKR1C3 enzyme, was tested and the three key parameters, Papp (A-B), Papp (B-A), and Efflux Ratio, were obtained with large differences, showing that AST-3424 was a Pgp substrate and its efficacy was strongly affected by the blood-brain barrier.

5.2 Blood-brain barrier assay

5.2.1 Blood-brain barrier assays of Compound A

Three CD-1 mice were administered 1 mg/kg of the test Compound A intravenously at each time point. Blood and brain tissue were taken at 5 min, 15 min, 1 h, 2 h, 4 h and 8 h after administration. The concentrations of the test substances in the samples were determined by LC/MS method and the results were shown in Table 32.

Table 32: Concentrations of test substances in blood and brain tissues at different time points after intravenous administration of Compound A to CD-1 mice

| Route | Time (h) | Animal ID | Brain (ng/g) | Plasma (ng/mL) | Ratio (Brain/ Plasma) | Mean | SD | CV (%) |
|---|---|---|---|---|---|---|---|---|
| IV | 0.083 | 1 | 538.05 | 459.53 | 1.17 | 1.28 | 0.10 | 7.51 |
| | | 2 | 540.75 | 400.55 | 1.35 | | | |
| | | 3 | 549.95 | 416.19 | 1.32 | | | |
| | 0.25 | 4 | 419.34 | 371.76 | 1.13 | 1.15 | 0.19 | 16.87 |
| | | 5 | 489.60 | 362.24 | 1.35 | | | |
| | | 6 | 371.38 | 384.54 | 0.97 | | | |
| | 1 | 7 | 180.29 | 226.51 | 0.80 | 6.76 | 0.06 | 8.49 |
| | | 8 | 188.68 | 238.84 | 0.79 | | | |
| | | 9 | 169.85 | 249.07 | 0.68 | | | |
| | 2 | 10 | 107.09 | 150.39 | 0.71 | 0.67 | 0.07 | 10.32 |
| | | 11 | 94.27 | 134.42 | 0.70 | | | |
| | | 12 | 77.50 | 131.84 | 0.59 | | | |
| | 4 | 13 | 71.20 | 96.41 | 0.74 | 0.78 | 0.12 | 15.87 |
| | | 14 | 68.47 | 74.91 | 0.91 | | | |
| | | 15 | 42.73 | 63.17 | 0.68 | | | |
| | 8 | 16 | 23.60 | 48.84 | 0.48 | 0.52 | 0.10 | 18.78 |
| | | 17 | 21.85 | 49.05 | 0.45 | | | |
| | | 18 | 18.85 | 29.91 | 0.63 | | | |

The corresponding results of the concentrations of Compound A in blood and brain tissue versus time were made into a curve graph, as shown in Fig. 35.

The corresponding test results of the ratio of the concentration of Compound A in brain tissues and blood versus time were made into a curve graph, as shown in Fig. 36.

The PK (Pharmacokinetic) parameters obtained from the above assays were listed as follows, as shown in Table 33 and Table 34.

PK parameters, pharmacokinetic parameters.
T1/2, half-life.
Cl-obs, plasma clearance.
C0, starting concentration.
AUClast, area under the concentration-time curve from start of administration time to the last point.
AUCInf, area under the concentration-time curve from start of administration to the time at infinity. Tmax, time to maximum observed plasma concentration.
Cmax, concentration to maximum observed plasma concentration.
MRTInf-obs, represents the mean residence time from the start of administration to the time of theoretical extrapolation to infinity.
AUCInf Ration (Brain/Plasma), the ratio of the area under the concentration-time curve of serum and brain tissue from the start of administration to the time at infinity.
Unit, unit.
Mean, average value.

Table 33: PK parameters obtained from tests in plasma

| PK parameters | Unit | Mean |
|---|---|---|
| Cl_obs | mL/min/kg | 13.85 |

(continued)

| PK parameters | Unit | Mean |
|---|---|---|
| T1/2 | h | 3.64 |
| C0 | ng/mL | 453.48 |
| AUClast | h*ng/mL | 979.51 |
| AUCInf | h*ng/mL | 1202.97 |
| MRTInf_obs | h | 4.37 |
| Vss_obs | L/kg | 3.63 |

Table 34: PK parameters obtained from tests in brain tissues

| PK parameters | Unit | Mean |
|---|---|---|
| T1/2 | h | 2.80 |
| Tmax | h | 0.08 |
| Cmax | ng/g | 543.00 |
| AUClast | h*ng/g | 785.85 |
| AUCInf | h*ng/g | 872.43 |
| MRTInf_obs | h | 2.99 |
| AUCInf Ratio (Brain/Plasma) | - | 0.80 |

The above experimental results showed that Compound A can be detected in brain tissue, and Compound A can cross the blood-brain barrier to carry out normal physiological metabolism and exert tumour inhibitory effects.

5.2.2 Blood-brain barrier assay of Compound AST-2870

Compound AST-2870 is a racemate of Compound AST-3424 and has similar tumour inhibitory activity as that of AST-3424.

Three CD-1 mice were administered 10 mg/kg of test Compound AST-2870 by intraperitoneal injection at each time point. Blood and brain tissues were taken at 5 min, 15 min, 30 min, 1 h, 1.5 h, and 2 h after administration, and the concentration of the test substance in the samples was determined by LC/MS method. The corresponding results of the concentrations of Compound AST-2870 in blood and brain tissue versus time were made into a curve graph, as shown in Fig. 37. The corresponding test results of the ratio of the concentration of the Compound AST-2870 in brain tissue and blood versus time were made into a curve graph, as shown in Fig. 38.

The PK (Pharmacokinetic) parameters obtained from the above assays were listed as follows, as shown in Table 35 and Table 36.

Table 35: PK parameters of AST-2870 obtained from tests in plasma

| PK parameters | Unit | Mean |
|---|---|---|
| Cl_obs | mL/min/kg | NC |
| T1/2 | h | 0.15 |
| Cmax | ng/mL | 4023 |
| AUClast | h*ng/mL | 725 |
| AUCInf | h*ng/mL | 725 |
| MRTInf_obs | h | NC |
| Vss_obs | L/kg | NC |

Table 36: PK parameters of AST-2870 obtained from tests in brain tissues

| PK parameters | Unit | Mean |
|---|---|---|
| T1/2 | h | 0.0747 |
| Tmax | h | 0.08 |
| Cmax | ng/g | 88.7 |
| AUClast | h*ng/g | 16.3 |
| AUCInf | h*ng/g | 16.5 |
| MRTInf_obs | h | NC |
| AUCInf Ratio (Brain/Plasma) | - | 0.0227 |

Experimental Conclusions: combined with the experimental data in section 5.2.1, it can be concluded that the half-life of Compound A was significantly longer than that of AST-2870; the concentration of Compound A in brain tissue was similar to that in plasma within 8 hours of the determination, indicating that this compound can effectively penetrate the blood-brain barrier; whereas, the concentration of AST-2870 determined in brain was significantly lower than that in plasma, and after 30 minutes of the administration, the concentration of AST-2870 was lower than the lower limit of quantitation, indicating that AST-2870 could not cross the blood-brain barrier.

**VI. Metabolic stability evaluation assay**

6.1 Cytoplasmic solution stability assay

(I) Solution preparation

(1) Compound A for test, progesterone positive control, and 100mM stock solutions of AST-3021 inhibitor were prepared and stored in the freezer (-10°C to -30°C).

2) 2.5 mM sample for test, intermediate solution of positive control and 1.5 mM intermediate solution of inhibitor were prepared.

3) A 10mg/mL of monkey cell cytosol solution was prepared.

4) A 1.5 mg/mL hepatocyte cytosol suspension containing 7.5μM of positive control was prepared.

5) A 1.5 mg/mL hepatocyte cytosol suspension containing 7.5μM of sample for test was prepared.

6) A 1.5 mg/mL hepatocyte cytosol suspension containing 7.5μM of sample for test and 4.5μM of the inhibitor was prepared.

7) An appropriate amount of NADPH powder was weighed and then dissolved in potassium phosphate buffer to formulate 6 mM of NADPH working solution and preheated at 37°C.

(II) Incubation assay
1) Negative control group: at different time points (0 and 60 min), 30 μL/well of hepatocyte cytosol suspension containing 7.5 μM of sample for test was distributed in a 96-well plate. Three replicate wells were prepared in parallel.
2) Positive control group: at different time points (0, 5, 15, 30, 45 and 60 min), 30 μL/well of hepatocyte cytosol suspension containing 7.5 μM of the positive control was distributed in a 96-well plate. Three replicate wells were prepared in parallel.
3) Normal reaction group: at different time points (0, 5, 15, 30, 45 and 60 min), 30 μL/well of hepatocyte cytosol suspension containing 7.5 μM of sample for test was distributed in a 96-well plate. Three replicate wells were prepared in parallel.
4) Inhibition reaction group: at different time points (0, 5, 15, 30, 45 and 60 min), 30 μL/well of hepatocyte cytosol suspension containing 7.5 μM of sample for test and 4.5 μM of the inhibitor was distributed in a 96-well plate. Three

replicate wells were prepared in parallel.

5) The 96-well plate was incubated in a 37°C water bath for 5 min.

6) 0 min samples: after 150 μL/well of acetonitrile containing the internal standard was added, then 15 μL/well of pre-heated NADPH working solution was added to the samples of normal reaction group, inhibition reaction group and positive control group, and 15 μL/well of pre-heated potassium phosphate buffer was added to the samples of negative control group.

7) Other samples (5, 15, 30, 45 and 60 min): for normal reaction group, inhibition reaction group and positive control group, 15 μL/well of pre-heated NADPH working solution was added to start the reaction; for negative control group, 15 μL/well of pre-heated potassium phosphate buffer was added. The reaction plates were then continued to incubate in a 37°C water bath and the reactions were stopped by the addition of 150 μL/well of stop solution at the corresponding time points (5, 15, 30, 45 and 60 min). The final incubation system was shown in Table 25 below.

8) After all the reactions were stopped, the plate was sealed, shaken well at 600 rpm for about 10 min, and then cryopreserved (-10°C ~ -30°C) until analysis.

Table 37: Data of incubation system

| Species | Group | Substrate | Substrate concentrat ion (μM) | Inhibitor (AST - 3021) (μM) | NADPH (mM) | Cytosol (mg/mL) | Incubation time |
|---|---|---|---|---|---|---|---|
| Mice, rats, monkeys and humans | Negative control | Compound A | 5 | 0 | 0 | 1 | 0 and 60 min |
| | Normal reaction | Compound A | 5 | 0 | 2 | 1 | 0, 5, 15, 30, 45 and 60 min |
| | Inhibition reaction | Compound A | 5 | 3 | 2 | 1 | 0, 5, 15, 30, 45 and 60 min |
| | Positive control | progesterone | 5 | 0 | 2 | 1 | 0, 5, 15, 30, 45 and 60 min |

(III) Sample analysis

The samples were centrifuged at 4°C, 4000 rpm for 15 min, and the supernatant was taken and mixed with an equal volume of ultrapure water, then the concentrations of Compound A, AST-2660 and progesterone were analysed by LC-MS/MS.

AST-2660 is a DNA alkylating agent that is released after metabolism of prodrug Compound A by the AKR1C3 enzyme and is effective in killing tumor cells, and the structural formula of AST-2660 is as follows:

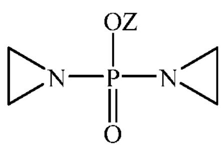,

wherein Z could be hydrogen, sodium or potassium.

(IV) Data analysis

The concentration of analyte in each sample was expressed as a peak area ratio (peak area $_{analyte}$/peak area$_{internal\ standard}$), then the peak area ratio at 0 min was used as a reference to calculate the %Remaining at each time point of the incubation, and the ln logarithmic value of the %Remaining was linearly fitted to the incubation time and the half-life was calculated according to the following formula.

$$\%Remaining,\ \%Remaining = \text{average of } PAR_{appointed\ time}/PAR_{0\text{-}min} \times 100\ (PAR\text{: Peak Area Ratio});$$

Elimination rate constant (k) = (-slope value);
Half-life ($t_{1/2}$) (minutes) = 0.693/k.

In this assay, the metabolic stability of the compounds to be tested (Compound A and Compound AST-3424) was investigated *in vitro* in the presence or absence of the AKR1C3 inhibitor (AST-3021) in hepatocyte cytosol of CD-1 mice, SD rats, machins and humans: in the presence or absence of AST-3021, 5 μM of the compounds to be tested (Compound A and Compound AST-3424) were co-incubated with 2 mM of NADPH and 1 mg/mL of hepatocyte cytosol for 0-60 min. In addition, in the absence of NADPH and AST-3021, 5 μM of the compounds to be tested (Compound A and Compound AST-3424) were co-incubated with 1 mg/mL of hepatocyte cytosol for 0, 60 min as a negative control. In the absence of AST-3021, 5 μM of progesterone was co-incubated with 2 mM of NADPH and 1 mg/mL of hepatocyte cytosol for 0-60 min as a positive control. The parameters of positive control progesterone, compounds to be tested (Compound A and Compound AST-3424) and AST-2660 in the post-incubation samples were then determined by LC-MS/MS, and the experimental data were shown in Tables 38 and 39.

Table 38: Metabolic parameters based on Compound A clearance in hepatocyte cytosol of different species

| Substrate | Substrate concentration (μM) | Species | Group | Analyte | average % Remaining | | | | | | Metabolic parameter | |
|---|---|---|---|---|---|---|---|---|---|---|---|---|
| | | | | | 0min | 5min | 15min | 30min | 45min | 60min | k | $t_{1/2}$ (min) |
| Compound A | 5 | mouse | Negative control group | Compound A | 100.00% | NA | NA | NA | NA | 83.01 % | 0.0031 | 223.40 |
| Compound A | 5 | rat | | Compound A | 100.00% | NA | NA | NA | NA | 68.42% | 0.0063 | 109.60 |
| Compound A | 5 | monkey | | Compound A | 100.00% | NA | NA | NA | NA | 78.57% | 0.0040 | 172.44 |
| Compound A | 5 | human | | Compound A | 100.00% | NA | NA | NA | NA | 38.96% | 0.0157 | 44.12 |
| Compound A | 5 | mouse | Normal reaction group | Compound A | 100.00% | 99.18% | 109.17% | 95.86% | 95.38% | 88.89% | 0.0014 | 511.07 |
| Compound A | 5 | | Inhibition reaction group | Compound A | 100.00% | 90.76% | 87.29% | 85.00% | 82.33% | 74.94% | 0.0041 | 168.42 |
| Compound A | 5 | rat | Normal reaction group | Compound A | 100.00% | 90.07% | 90.26% | 84.80% | 81.99% | 71.72% | 0.0053 | 130.84 |
| Compound A | 5 | | Inhibition reaction group | Compound A | 100.00% | 88.10% | 86.23% | 83.84% | 78.06% | 63.81% | 0.0068 | 101.55 |
| Compound A | 5 | monkey | Normal reaction group | Compound A | 100.00% | 98.89% | 94.52% | 89.81% | 83.43% | 73.28% | 0.0046 | 151.55 |
| Compound A | 5 | | Inhibition reaction group | Compound A | 100.00% | 96.83% | 101.19% | 90.97% | 88.36% | 69.52% | 0.0045 | 155.47 |
| Compound A | 5 | human | Normal reaction group | Compound A | 100.00% | 88.27% | 79.04% | 70.02% | 52.35% | 43.34% | 0.0139 | 49.88 |
| Compound A | 5 | | Inhibition reaction group | Compound A | 100.00% | 83.74% | 78.58% | 67.01% | 66.39% | 53.65% | 0.0107 | 64.94 |
| Progesterone | 5 | mouse | Positive Control group | Progesterone | 100.00% | 89.61% | 78.52% | 62.61% | 44.84% | 31.03% | 0.0184 | 37.71 |
| Progesterone | 5 | rat | | Progesterone | 100.00% | 84.80% | 69.77% | 45.51% | 21.61% | 4.70% | 0.0311 | 22.28 |
| Progesterone | 5 | monkey | | Progesterone | 100.00% | 82.47% | 59.82% | 28.19% | 10.01% | 3.26% | 0.0525 | 13.20 |
| Progesterone | 5 | human | | Progesterone | 100.00% | 80.76% | 55.69% | 20.59% | 7.72% | 0.46% | 0.0543 | 12.76 |

Table 39: Average peak area ratios of metabolite AST-2660 in hepatocyte cytosol of different species

| Substrate | Substrate concentration (μM) | Species | Group | Analyte | Average peak area ratio of metabolites | | | | | |
|---|---|---|---|---|---|---|---|---|---|---|
| | | | | | 0min | 5min | 15min | 30min | 45min | 60min |
| Compound A | 5 | mouse | Negative control group | AST-2660 | 0.00E+00 | NA | NA | NA | NA | 0.00E+00 |
| Compound A | 5 | rat | | AST-2660 | 0.00E+00 | NA | NA | NA | NA | 0.00E+00 |
| Compound A | 5 | monkey | | AST-2660 | 0.00E+00 | NA | NA | NA | NA | 0.00E+00 |
| Compound A | 5 | human | | AST-2660 | 0.00E+00 | NA | NA | NA | NA | 0.00E+00 |
| Compound A | 5 | mouse | Normal reaction group | AST-2660 | 0.00E+00 | 0.00E+00 | 0.00E+00 | 0.00E+00 | 0.00E+00 | 0.00E+00 |
| Compound A | 5 | | Inhibition reaction group | AST-2660 | 0.00E+00 | 0.00E+00 | 0.00E+00 | 0.00E+00 | 0.00E+00 | 0.00E+00 |
| Compound A | 5 | rat | Normal reaction group | AST-2660 | 0.00E+00 | 0.00E+00 | 0.00E+00 | 0.00E+00 | 0.00E+00 | 0.00E+00 |
| Compound A | 5 | | Inhibition reaction group | AST-2660 | 0.00E+00 | 0.00E+00 | 0.00E+00 | 0.00E+00 | 0.00E+00 | 0.00E+00 |
| Compound A | 5 | monkey | Normal reaction group | AST-2660 | 0.00E+00 | 0.00E+00 | 0.00E+00 | 1.24E-05 | 2.71E-05 | 4.27E-05 |
| Compound A | 5 | | Inhibition reaction group | AST-2660 | 0.00E+00 | 0.00E+00 | 0.00E+00 | 0.00E+00 | 0.00E+00 | 3.68E-06 |
| Compound A | 5 | human | Normal reaction group | AST-2660 | 0.00E+00 | 0.00E+00 | 8.73E-06 | 2.29E-05 | 6.26E-05 | 8.33E-05 |
| Compound A | 5 | | Inhibition reaction group | AST-2660 | 0.00E+00 | 0.00E+00 | 0.00E+00 | 0.00E+00 | 0.00E+00 | 0.00E+00 |

Compound A and AST-3424 were tested in the above-mentioned method, respectively, and the experimental result data were analyzed and plotted to obtain Fig. 39, showing the curves of the remaining amount of Compound A after reaction in mouse, monkey, rat, and human hepatocyte cytosol solution with the addition of NADPH, without the addition of NADPH, with the addition of AKR1C3 inhibitor and of control drug progesterone over time. Fig. 40 was obtained to show the curves of the remaining amount of AST-3424 after reaction in mouse, monkey, rat, and human hepatocyte cytosol solution with the addition of NADPH, without the addition of NADPH, with the addition of AKR1C3 inhibitor and of control drug progesterone over time.

Experimental analysis of Compound A:

1. In the absence of NADPH and in the absence of AST-3021, the average %Remaining of Compound A after co-preincubation with mouse, rat, monkey and human hepatocyte cytosol for 60 min was 83.01%, 68.42%, 78.57%, and 38.96%, respectively (see Table 38). These data indicated that Compound A may be subjected to a non-NADPH-dependent metabolic pathway. The metabolite AST-2660 was not detected in any of the samples (see Table 39).

2. In the absence of AST-3021, the average %Remaining of Compound A after co-preincubation with mouse, rat, monkey and human hepatocyte cytosol and NADPH for 60 min was 88.89%, 71.72%, 73.28%, and 43.34%, respectively (see Table 38), corresponding to half-lives of 511.07 min, 130.84 min, 151.55 min and 49.88 min, respectively. AST-2660 was not detected in mouse and rat samples, and a small amount of AST-2660 was detected in monkey and human samples.

3. In the presence of the AKR1C3 inhibitor AST-3021, %Remaining of Compound A in mouse, rat, monkey and human hepatocyte cytosol was 74.94%, 63.81%, 69.52%, and 53.65%, respectively, corresponding to half-lives of 140.38 min, 101.55 min, 155.47 min, and 64.94 min, respectively (see Table 38). AST-2660 was not detected in mouse, rat, and human samples, and was detected in only one monkey 60-min sample. AST-3021 did not significantly inhibit the metabolism of Compound A in mouse, rat, and monkey hepatocyte cytosol, but it slightly inhibited the metabolism of Compound A in human hepatocyte cytosol. In the presence of AST-3021, AST-2660 was not detected in mouse and rat cytosol, but in most monkey and human cytosol samples, the concentration of AST-2660 was reduced to undetectable levels in the presence of AST-3021.

The above experiments demonstrated that:

i. Compound A has a similar metabolic mechanism as that of AST-3424: it is metabolized in the cytoplasm; it is metabolized in the presence of NADPH and AKR1C3 enzyme to release the DNA alkylating agent AST-2660; it is metabolized in the absence of NADPH or in the absence of AKR1C3 enzyme, but it is not metabolized to release the DNA alkylating agent AST-2660.

ii. In human and monkey hepatocyte cytosol, the metabolic stability of Compound A in the normal group was better than that of AST-3424, and its half-life was longer, and when acting on cancer cells, Compound A was capable of exerting a longer-lasting inhibitory effect on cancer than that of AST-3424.

6.2 Stability experiments of liver microsomes

1) Two individual experiments were performed.

1a) With NADPH: 10 μL of 20 mg/mL liver microsomes and 40 μL of 10 mM NADPH were added to the culture solution. The final concentrations of microsomes and NADPH were 0.5 mg/mL and 1 mM, respectively.

1b) Without NADPH: 10 μL of 20 mg/mL liver microsomes and 40 μL of ultrapure $H_2O$ were added to the culture solution. The final concentration of microsomes was 0.5 mg/mL.

2) The reaction was started by adding 4 μL of 200 μM test compound solution at a final concentration of 2 μM or control compound (Verapamil) solution and incubated at 37°C.
3) 50 μL of aliquots were taken from the reaction solution at 0 min and 30 min. The reaction was stopped by adding 4

volumes of cold acetonitrile and four internal standards (IS, 100 nM Alprazolam, 200 nM Caffeine, 200 nM Labetalol and 2 μM Ketoprofen). The samples were centrifuged at 3, 220 × g for 40 min. The aliquots of 100 μL of supernatant were mixed with 100 μL of ultrapure $H_2O$ and then used for LC-MS/MS analysis. All experiments were performed in duplicate. The test results were shown in Table 40 below.

Table 40: Remaining amount of Compound A in the stability experiments of liver microsomes after 30 minutes

| Compound | Percentage remaining after 30 minutes | | | | | | | | | |
|---|---|---|---|---|---|---|---|---|---|---|
| | Human | | Monkey | | Dog | | Rat | | Mouse | |
| | With the addition of NADPH | without the addition of NADPH | With the addition of NADPH | without the addition of NADPH | With the addition of NADPH | without the addition of NADPH | With the addition of NADPH | without the addition of NADPH | With the addition of NADPH | without the addition of NADPH |
| Verapamil | 15.55 | 98.27 | 0.19 | 92.44 | 15.73 | 97.58 | 1.13 | 89.24 | 2.91 | 93.95 |
| Compound A | 115.25 | 111.93 | 82.29 | 81.30 | - | - | 92.10 | 114.62 | 98.59 | 104.30 |
| AST-3424 | 10.65 | - | 0.03 | - | - | - | 8.79 | - | 3.1 | - |

The above experimental results showed that Compound A was relatively stable in liver microsomes of human, monkey and mouse and relatively unstable in liver microsomes of rat. By comparing the data of Compound A with AST-3424, it was found that Compound A was more stable than that of AST-3424 in liver microsomes of human, monkey, rat and mouse.

6.3 Plasma stability test

1) Plasma preparation

The frozen plasma was immediately melted in a water bath of 37°C. The plasma was centrifuged at 3, 220g for 10 minutes to remove the blood clots and the supernatant was collected into a new test tube. The pH of the plasma was checked and recorded. Note: a. Only plasma that has been melted no more than twice since arrival was used; b. Only plasma in the range of pH 7 to pH 8 was used.

2) Preparation of experimental solutions

1 mM of working solutions of the test compound and the control compound mevinolin were prepared in DMSO. 1 mM of the working solution of the control compound solution in propane was prepared in acetonitrile. 4 μL of the working solution was added to 796 μL of pre-incubated plasma to achieve 5 μM of final concentration. The final concentration of solvent was 0.5%.

3) Test procedure for plasma stability

The aliquots of 50 μL of the standard addition plasma were added to new test tubes and incubated in the water bath of 37°C at approximately 60 rpm for 2 hours. The assay was performed in duplicate. At the end of the two hours of incubation, 300 μL of quenched solution at room temperature (acetonitrile containing internal standards (acetonitrile, 100 nM Alprazolam, 500 nM Labetalol and 2 μM Ketoprofen)) was added to stop the reaction. Samples at time 0 were prepared by adding 50 μL of standard addition plasma to the new tubes with 300 μL of quenched solution at room temperature. The tubes were mixed by vortex for 5 minutes. The samples in the plates were centrifuged at 3, 220 × g for 30 minutes at 4°C to precipitate the proteins. 100 μL of supernatant was transferred to a new plate. The supernatant was diluted with 100 μL of water, mixed well and analysed using LC-MS/MS.

The test results of Compound A after 2 hours in the plasma of different species were shown in Table 41 below.

Table 41: Percentage remaining after 2 hours in plasma of different species

| Compound | Species | Percentage remaining |
|---|---|---|
| Compound A | Human | 60.06 |
| Compound A | Rat | 33.03 |
| Compound A | Mouse | 38.19 |
| Compound A | Monkey | 79.20 |

The results of the above assays showed that Compound A remained 79.20% and 60.06% in monkey and human plasma after 2 hours, and its stability was superior to that in rat and mouse plasma.

**VII. Maximal tolerance dose (MTD) assay**

7.1 MTD assay of Compound A

The toxic response and toxicokinetic characteristics in machins after a single administration of Compound A by intravenous infusion for 30 minutes were evaluated.

7.1.1 Experimental procedure:

Eight healthy and suitably fit machins, half male and half female, were randomly divided into four groups according to sex and body weight, with one animal of each sex in each group. Group 1 was the vehicle control group and Groups 2 to 4 were the Compound A low (1 mg/kg), medium (3 mg/kg) and high (6 mg/kg) dose groups, respectively. All animals were administered by single intravenous infusion for 30 minutes and observed for 14 days, and necropsy was performed on day 15.

All animals were subjected to 1 detailed clinical observation and physical examination prior to administration. Except for once on the planned dissection day, cage-side observation was performed twice a day (once in the morning and once in the afternoon) during the adaptation period and the experimental period. During the experimental period, all animals in Groups 1~4 were subjected to detailed clinical observation 1~2 times daily; weighed once before grouping, once before D1 (before administration), once before D3, once before D7, once before D10, once before D14 and once before dissection on D15, respectively; food consumption for 24±1 h was determined once a day, except for D3 and D14; temperature was measured once during the adaptation period, before administration on D1, 2-4 h after administration on D1, and 6-8 h after administration on D1, and D2 (24±1 h after administration), respectively; electrocardiogram was measured once during the adaptation period and 2-4 h after administration, respectively; and blood pressure was measured once during the adaptation period, 2-4 h after administration, and D14, respectively.

Animals were fasted overnight but not watered before the blood sample collection for clinical examination. Blood samples (approximately 5 mL) were collected once through the femoral vein for biochemical analyses of haematological, blood coagulation and serum on adaptation period, D4 and D15 (prior to planned dissection), respectively. Urine was also collected one day in advance for urinalysis.

Samples of toxicokinetics were collected from animals in Group 1 before and 1h after administration, and samples of toxicokinetics were collected from animals in Groups 2 to 4 at 0h (before administration), 0.5h (immediately after administration), 0.75h, 1h, 1.25h, 1.5h, 2h, 2.5h, 4.5h, and 8.5h. Blood samples of approximately 0.5mL/animal/time point were collected via the femoral vein, and then were placed in an anticoagulation tube containing sodium heparin after collection and kept on wet ice. Plasma was separated by centrifugation at 6800 g for 6 min at 2 to 8°C within 2 hours after collection. The concentration of Compound A in the plasma samples was determined by the Bioanalytical Laboratory of the research institution using an established LC-MS/MS method. Cmax, Tmax, AUC (0-t), AUC(O-oo), t1/2, Vss, Vz and Cl were calculated using the WinNonlin 7.0 non-compartmental model.

All animals were euthanized by intravenous injection of sodium pentobarbital on the day of planned dissection (D15), the death of the animals was ensured by bloodletting through the femoral artery/vein, then gross dissection examination was performed.

7.1.2 Experimental results:

Under the conditions of this assay, after the administration of 0 mg/kg, 1 mg/kg, 3 mg/kg, and 6 mg/kg Compound A by a single intravenous infusion to the machins, all animals survived until the day of the planned dissection. The

females in the 3 mg/kg and 6 mg/kg of Compound A groups showed slight erythema at the site of the administration. Soft stools were observed in both males and females in the 6 mg/kg Compound A group. All these symptoms recovered to normal during the observation period. The exposure to Compound A was dose proportional. Compound A showed moderate to high systemic clearance in the range of 1.34-2.72 L/hr/kg and moderate volume of distribution steady-state in the range of 1.80-3.64 L/kg. In summary, the MTD (maximal tolerance dose) of Compound A under the conditions of this assay was greater than 6 mg/kg.

7.2 MTD assay of Compound AST-3424

The maximal tolerance dose in machins after a single administration of AST-3424 injection by intravenous drip for 30 minutes was evaluated.

7.2.1 Experimental procedure:

Eight healthy and suitably fit machins, half male and half female, were randomly divided into four groups according to sex and body weight, with one animal of each sex in each group. Group 1 was the vehicle control group and Groups 2 to 4 were the AST-3424 (containing 3.75% ethanol/1.25% propylene glycol) low (0.5 mg/kg), medium (1.0 mg/kg) and high (1.5 mg/kg) dose groups, respectively. All animals were administered by single intravenous infusion for 30 minutes and observed for 28 days, and necropsy was performed on day 29.

The following parameters were evaluated during the trial: near-death and death conditions, clinical observations, body weight, food intake, biochemical analyses of haematological, blood coagulation and serum, ECG, body temperature, gross dissection and microscopic histopathological examination.

7.2.2 Experimental Results:

Male machins administered with AST-3424 at the doses of 1.5 mg/kg and 1.0 mg/kg, showed test substance-related death on day 8 and day 16, respectively. Body leanness and loose stools were observed prior to the death of the animals. In addition, the male machins of the 1.0 mg/kg dose group showed the following behaviors prior to sacrifice: reduced activity, body coolness, body weight loss, electrolyte disturbances (decreased $Na^+$, increased $K^+$), increased CK and BUN; and the following behaviors: it could be observed by gross dissection that the animal had a yellowish exudate seen in the pericardium, a dark red plaque seen in the local mucosa of the colon, and two white plaques seen in the left lobe of the lungs, and hardness at the plaques. Microscopically, it could be observed that the animal had a marked inflammatory reaction in the lungs and heart, haemorrhage in the mucosa and submucosa of the colon and lymphocyte necrosis. In female monkeys, no deaths related to sample for test occurred. However, female monkeys of the 1.5 mg/kg AST-3424 group showed weight loss, decreased food consumption, diarrhoea and vomiting.

After administration of AST-3424 at a dose of 1.5 mg/kg, a change of hematological indicators in female monkeys was observed, including a decrease of red blood cells (RBCs), haemoglobin, and erythrocyte specific volume, and an increase of the percentage of reticulocytes. The increase of percentage of reticulocytes was considered to be a regeneration reaction to the decrease of erythrocytes. After administration of AST-3424 at a dose of 1.0 mg/kg to male monkeys, leukocyte counts (percentage of neutrophils) and fibrinogen were increased, and the percentage of lymphocytes was decreased; the above phenomena were associated with inflammation in a variety of organs, including the heart and lungs. In addition, a decrease in total protein and albumin was seen in female monkeys in the 1.5 mg/kg dose group on day 14 and recovered on day 28.

In summary, after the administration of 0.5, 1.0, and 1.5 mg/kg of AST-3424 injection by a single intravenous drip for 30 minutes to machins, the death of male monkeys at doses $\geq$1.0 mg/kg was observed. Therefore, under the conditions of this assay, the maximal tolerance doses for single-dose administration were 0.5 mg/kg and 1.5 mg/kg for male and female machins, respectively.

Through the above series of assays, comparing Compound A and AST-3424, it can be found:

(1) Compound A is a solid, compared with AST-3424 which is a liquid, having the property of being easy to store and transport, measure, and more convenient in formulation operations;

(2) Compound A has a cancer cell killing effect that is not too dissimilar to that of AST-3424 and has a higher sensitivity and greater specificity for AKR1C3 enzyme activation; in fact, they both have similar therapeutic effects in the PDX animal model;

(3) As compared with AST-3424, which is a substrate of P-gp and cannot cross the blood-brain barrier, the new AKR1C3-activated Compound A is not a substrate of P-gp and is able to cross the blood-brain barrier. Therefore,

the new Compound A has therapeutic effects on the central nervous system, especially primary brain tumors or cancers as well as metastatic cancers and tumors metastasized to the brain, and the experimental data for the brain tumor model provided in the Examples (NCI-H460-Luc2 intracranially inoculated CDX model) also confirms that Compound A has a therapeutic effect in treating brain tumors (brain metastases), whereas AST-3424 has no therapeutic effect for the same model;

(4) Compound A has a longer half-life than that of AST-3424 in both plasma and brain tissue. Therefore, the new Compound A is better than AST-3424 in terms of the design of the dosing cycle and dosage, etc., and it can exert its efficacy by being present for a certain period of time in brain tissue;

(5) The maximal tolerance dose (MTD) of Compound A is larger than that of AST-3424, indicating that Compound A may have a wider dosage of administration and be more tolerated by patients;

(6) Compounds B and C, the R/S isomers of Compound A, have *in vitro* proliferation inhibitory activity for cancer cells equal to that of Compound A. Moreover, Compounds A, B, and C have the same mechanism of release of DNA alkylating agents through the action of AKR1C3 enzyme, which is independent of the R/S configuration. Therefore, it can be determined that Compounds B and C, like Compound A, could also cross the blood-brain barrier and enter the central nervous system, especially the primary brain tumors or cancer tissues, as well as metastatic cancers and tumor tissues metastasized to the brain, and exert an inhibitory effect of cancer.

## Claims

1. A compound with any one of the following structural formulae, or a pharmaceutically acceptable salt, a prodrug, a solvate, or an isotopic variant thereof:

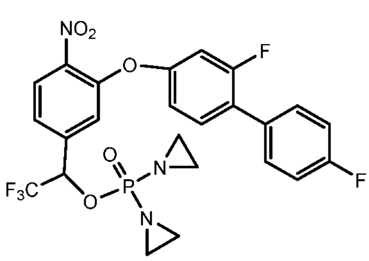
,
A

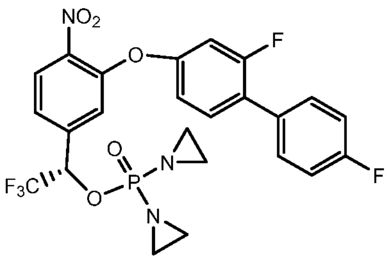
,
B

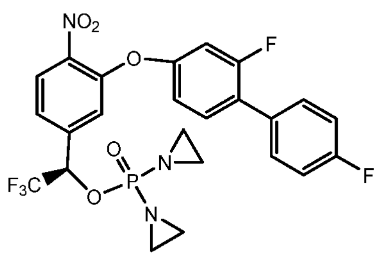
,
C .

2. The compound according to claim 1, wherein the salt is a basic salt or an acid salt, and the solvate is a hydrate or alcoholate.

3. The compound according to claim 2, wherein the alcoholate is an ethanolate.

4. The compound according to claim 1, wherein the isotopic variant has the following structural formulae:

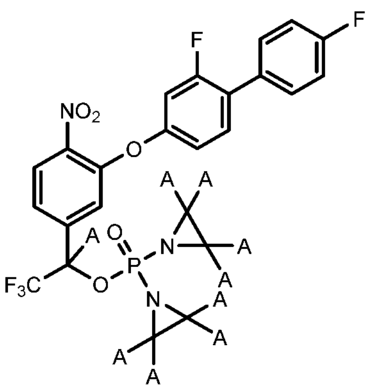
,

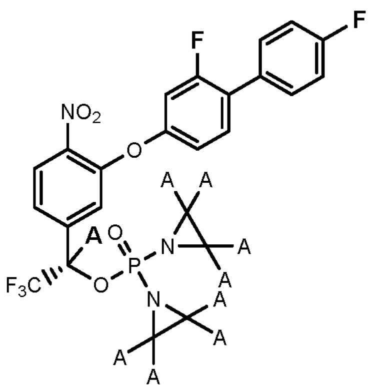
,

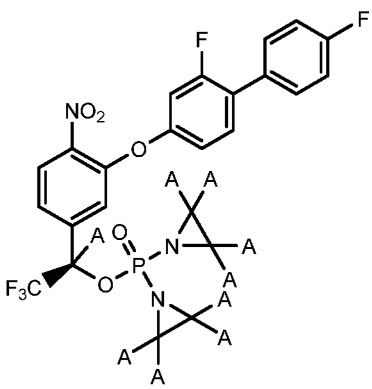
,

wherein As are each independently H or D, and at least one of the nine As is D.

**5.** The compound according to claim 1 or 4, wherein the isotopic variant corresponds to a deuterated compound with the following structures:

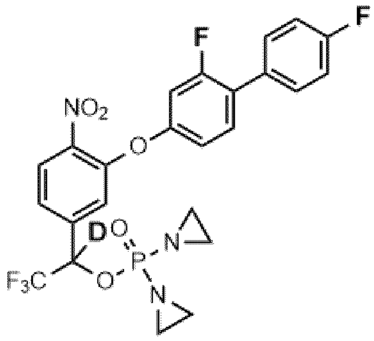
,
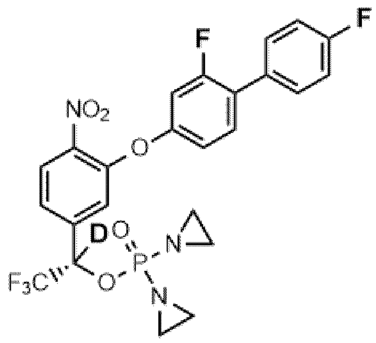
,
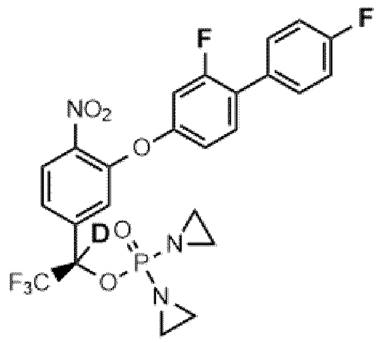
,
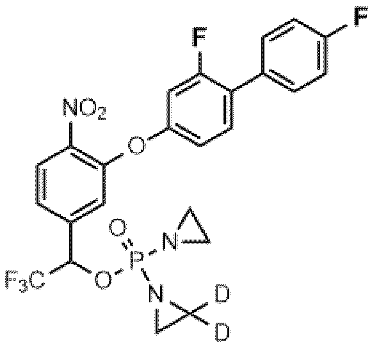
,
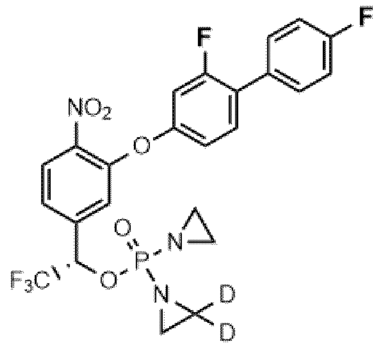
,
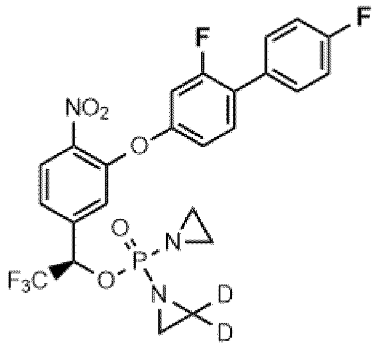
,
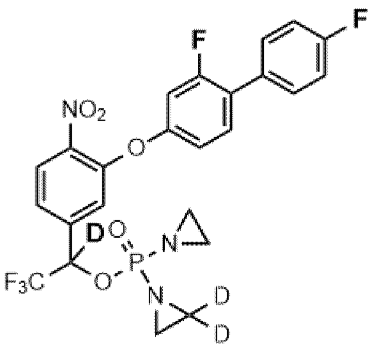
,
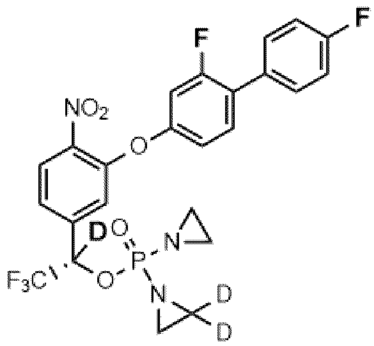
,
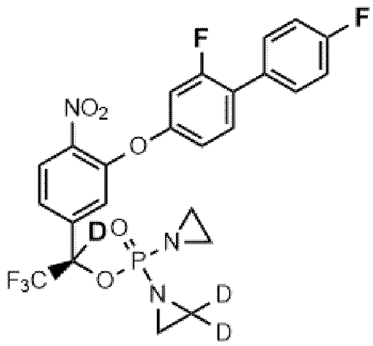
,
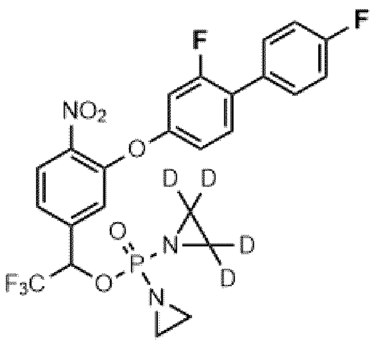
,
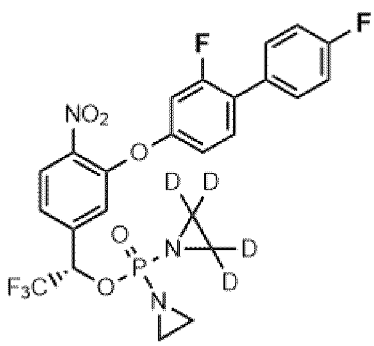
,
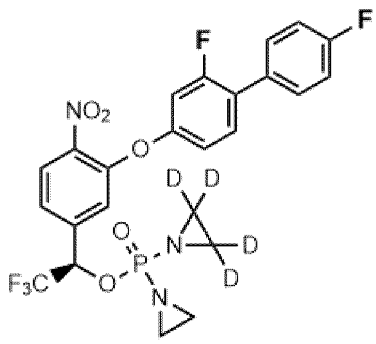
,

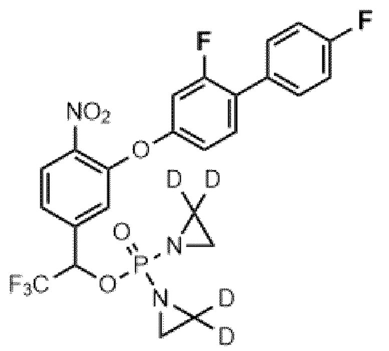 , 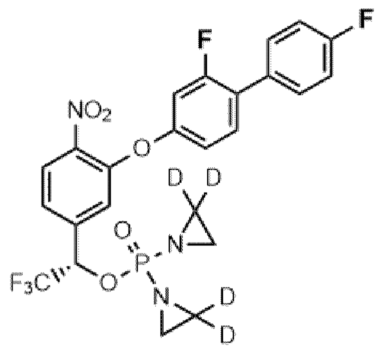 , 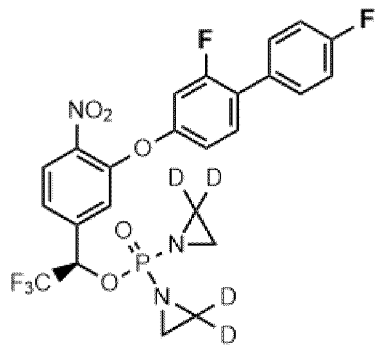 ,

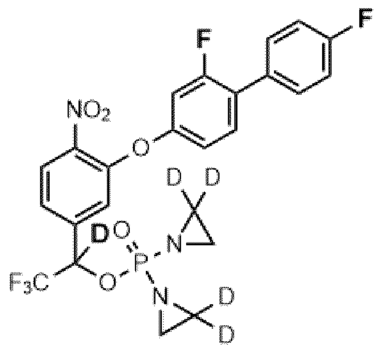 , 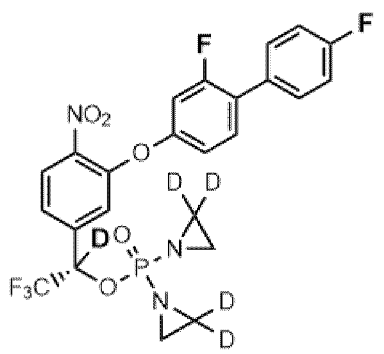 , 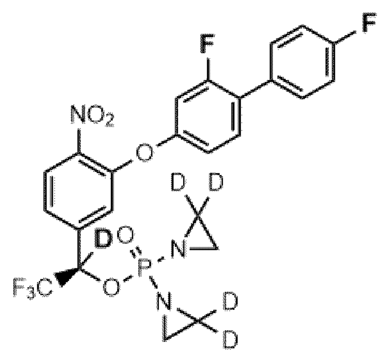 ,

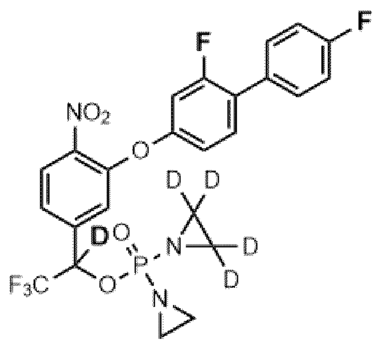 , 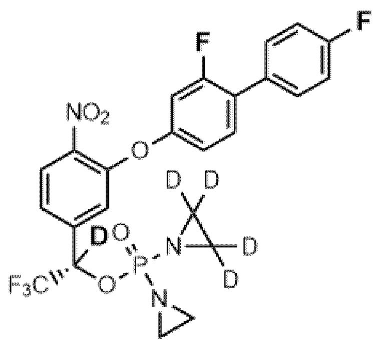 , 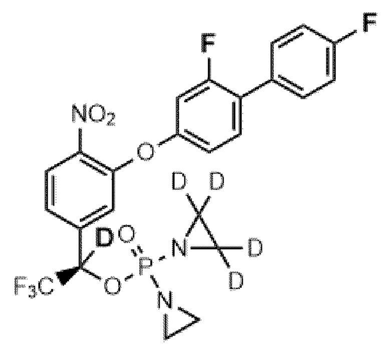 ,

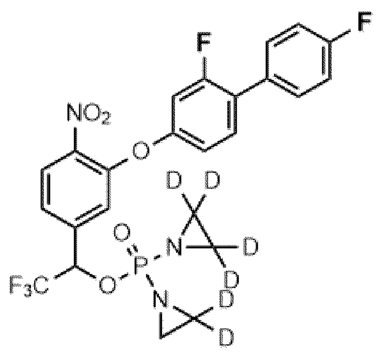 , 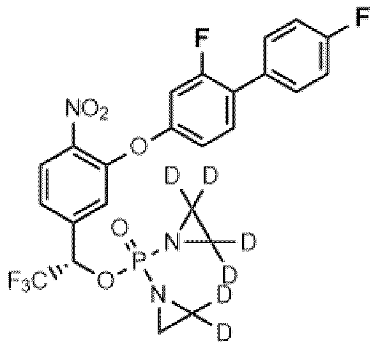 , 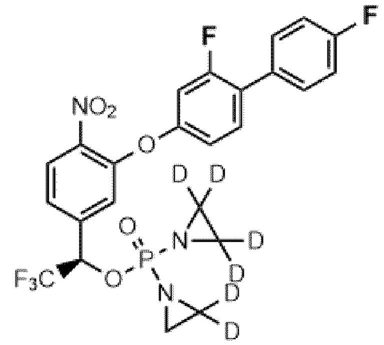 ,

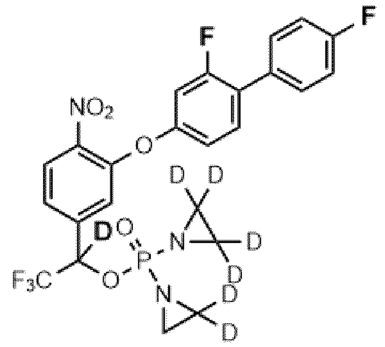
,
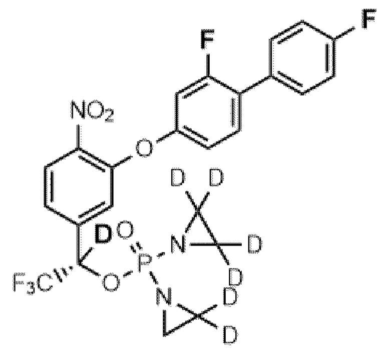
,
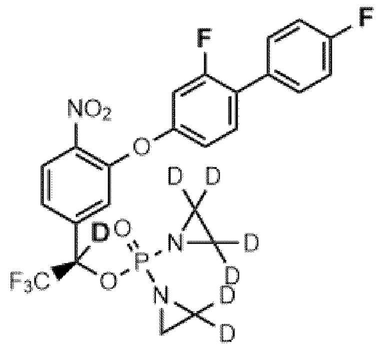
,

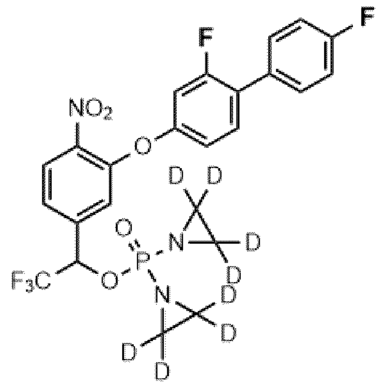
,
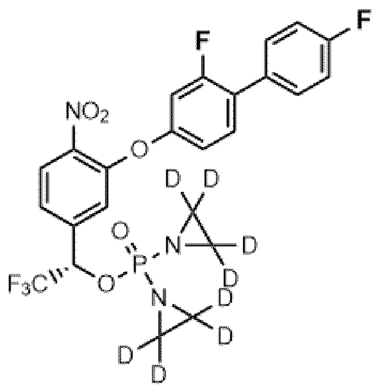
,
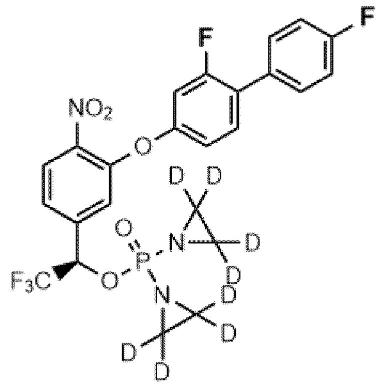
,

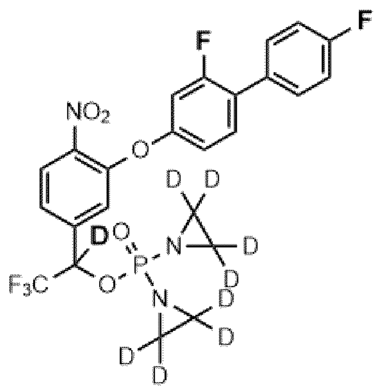
,
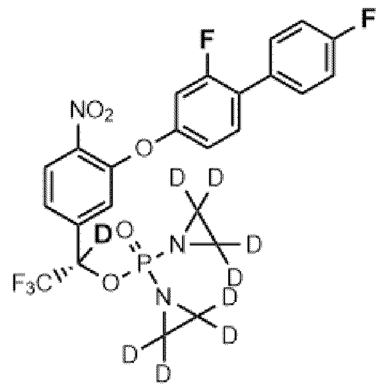
,
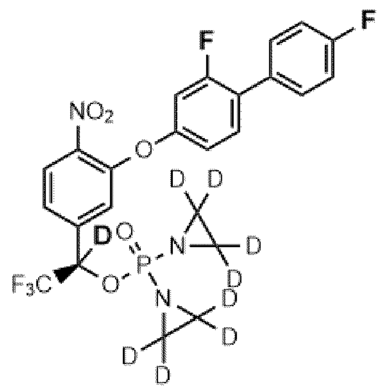
.

6. A drug containing the compound according to any one of claims 1-5, or a pharmaceutically acceptable salt, a prodrug, a solvate, or an isotopic variant thereof.

7. Use of the drug according to claim 6 or the compound according to any one of claims 1-5 for the treatment of cancer or tumor patients, conditions caused by cancer or tumor, or cell proliferative diseases.

8. The use according to claim 7, wherein the cancer or tumor is lung cancer, pancreatic cancer, liver cancer, or gastric cancer.

9. The use according to claim 7, wherein the cancer or tumor is primary brain cancer, brain tumor, or metastatic cancer or tumor that metastasizes to the brain.

10. Use of the compound according to any one of claims 1-5, or a pharmaceutically acceptable salt, a prodrug, a solvate, or an isotopic variant thereof in the preparation of a drug for the treatment of cancer or tumor patients, conditions caused by cancer or tumor, or cell proliferative diseases.

11. The use according to claim 10, wherein the cancer or tumor is lung cancer, pancreatic cancer, liver cancer, or gastric

cancer.

**12.** The use according to claim 8 or 11, wherein the lung cancer is non-small cell lung cancer.

**13.** The use according to claim 10, wherein the cancer or tumor is primary brain cancer, brain tumor, or metastatic cancer or tumor that metastasizes to the brain.

**14.** A method for treating cancer or tumor, comprising step a or step b:

a. determining the AKR1C3 reductase content of cancer cells or tissues in a patient, and administering the drug according to claim 6 to the patient if the measured AKR1C3 reductase content is equal to or greater than a predetermined value;
b. determining the corresponding RNA expression level of AKR1C3 reductase of cancer cells or tissues in a patient, and administering the drug according to claim 6 to the patient if the measured RNA expression level is in a predetermined range.

**15.** A method for treating cancer or tumor, comprising a step of adjusting the AKR1C3 reductase content or expression level, wherein the drug according to claim 6 is administered to the patient when the AKR1C3 reductase content or expression level is adjusted to be equal to or greater than a predetermined value.

**16.** A method for preparing any one of the compounds with the following structures, comprising subjecting compound I-1, I-2, or I-3 to a ring-closure reaction to obtain the corresponding compound A, B, or C, respectively:

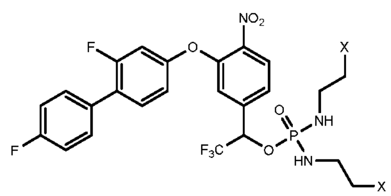
I-1

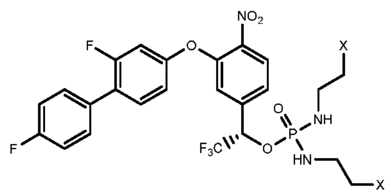
I-2

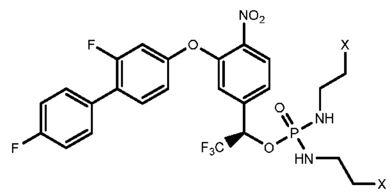
I-3

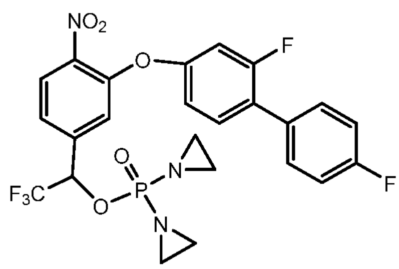
,
A

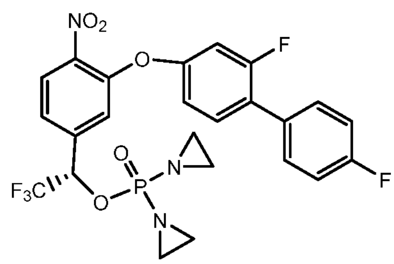
,
B

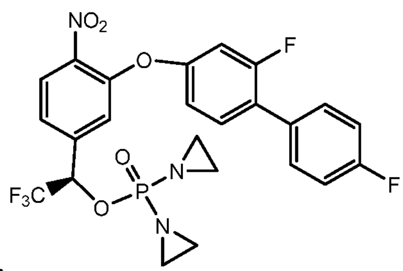
,
C

wherein Xs are each independently fluorine, chlorine, bromine, or iodine.

**17.** The method according to claim 16, wherein silver oxide or silver nitrate is used as a catalyst in the ring-closure reaction, with or without the addition of an organic base.

**18.** The method according to claim 16, wherein Xs are both bromine.

**19.** The method according to claim 17, wherein the organic base is N,N-diisopropylethylamine or triethylamine.

**20.** A compound with any one of the following structures:

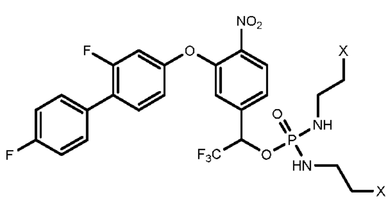

I-1

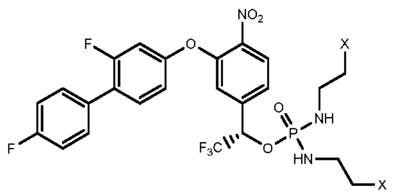

I-2

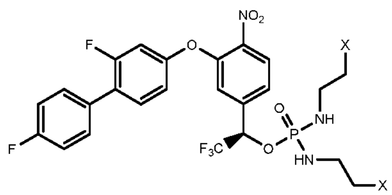

I-3

wherein Xs are each independently fluorine, chlorine, bromine, or iodine.

**21.** Use of the compound according to claim 20 as an intermediate for the synthesis of the compound according to claim 1, or in the preparation of a drug for the treatment of cancer or tumor patients, conditions caused by cancer or tumor, or cell proliferative diseases.

**22.** A method for preparing any one of the compounds with the following structures, comprising performing the first reaction of compound II-1, II-2, or II-3 as the starting reactant with phosphorus oxyhalide to obtain an intermediate, respectively, then performing the second reaction of the intermediate with haloethylamine or haloethylamine hydrohalide, and finally obtaining the corresponding compound I-1, I-2, or I-3, respectively:

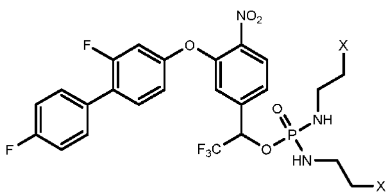

I-1

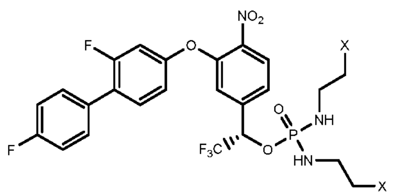

I-2

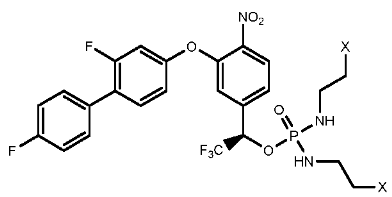

I-3

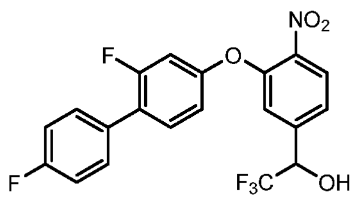

II-1

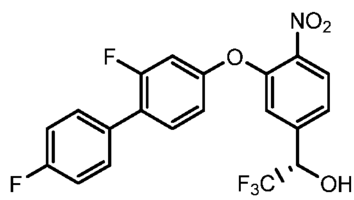

II-2

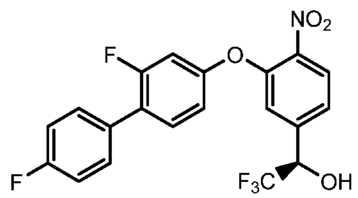

II-3

wherein the two Xs in compound I-1, I-2, or I-3 are each independently fluorine, chlorine, bromine, or iodine; wherein the phosphorus oxyhalide is selected from phosphorus oxyfluoride, phosphorus oxychloride, phosphorus oxybromide, and phosphorus oxyiodide, and is preferably phosphorus oxychloride;
wherein the halogen atoms in the phosphorus oxyhalide and the two Xs in compound I-1, I-2, or I-3 may be the same or different.

**23.** The method according to claim 22, wherein Xs in compound I-1, I-2, or I-3 are bromine, and the haloethylamine is bromoethylamine, and the haloethylamine hydrohalide is bromoethylamine hydrobromide.

**24.** A compound with any one of the following structures:

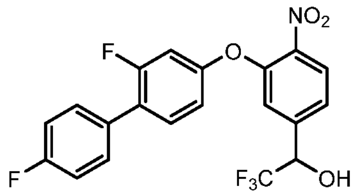

II-1

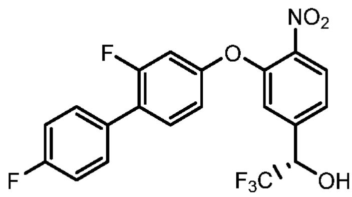

II-2

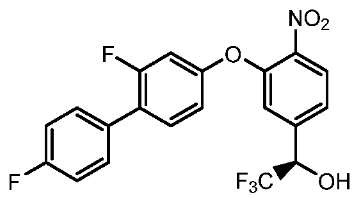

II-3

**25.** A method for preparing any one of the compounds with the following structures, wherein compound III-3 is reacted with $TMSCF_3$ in contact and hydrolyzed after deprotection to obtain racemic alcohol II-1, and chiral resolution operation is performed on racemic alcohol II-1 to obtain chiral alcohol II-2 and chiral alcohol II-3, respectively;

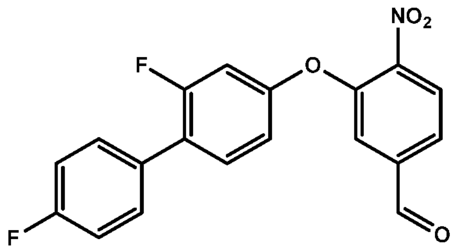

III-3

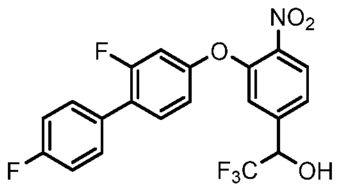

II-1

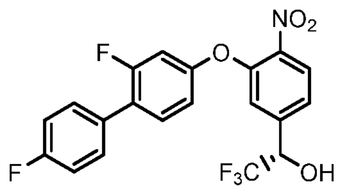

II-2

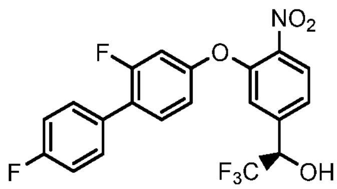

II-3 .

**26.** The method according to claim 25, wherein the deprotection reagent used for the deprotection operation is tetrabutylammonium fluoride, and ammonium fluoride, aqueous ammonium chloride or hydrochloric acid is used for hydrolysis operation.

**27.** The method according to claim 25, wherein methods of the chiral resolution include crystallization method and chemical resolution method.

**28.** A method for separating the racemate of the compound according to claim 1: to obtain an enantiomer or to increase the concentration of either of the enantiomers of the racemate in the compound to be excessive, comprising the following steps:

subjecting the racemic compound to optical resolution operation, wherein the operation is performed by chiral chromatography comprising stationary phase and mobile phase, wherein the

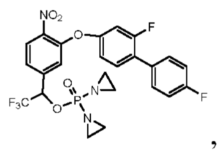

,

stationary phase comprises silica gel impregnated with a functionalized polysaccharide, and wherein the mobile phase comprises an alcohol and a further solvent;
wherein the alcohol is ethanol, and the further solvent is n-hexane;

wherein the temperature of the chromatography column during the operation of the chiral chromatography is 38°C;
wherein the functionalized polysaccharide is amylose tris(3,5-dimethylphenylcarbamate).

**29.** An HPLC method for separating the racemate of the compound according to claim 1:

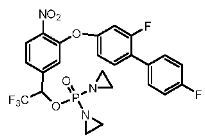

,

to obtain an enantiomer or to increase the concentration of either of the enantiomers of the racemate in the compound to be excessive, wherein the chromatography column is CHIRALPAK® AD, and the mobile phase is ethanol, and the column temperature is 35°C.

**30.** A method for preparing any one of the deuterated compounds with the following structures, comprising subjecting compounds IV-1 to IV-21 to a ring-closure reaction to obtain the corresponding deuterated compounds V-1 to V-15, respectively:

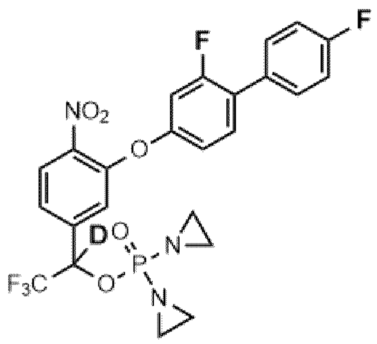

,

V-1

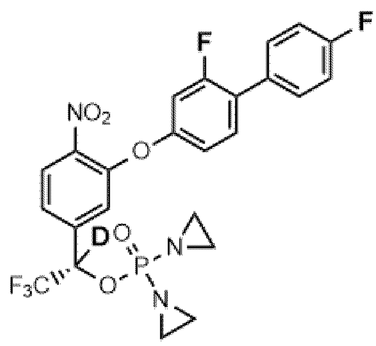

,

V-2

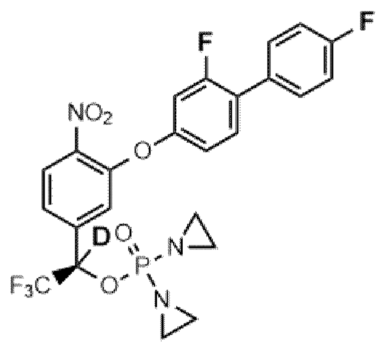

,

V-3

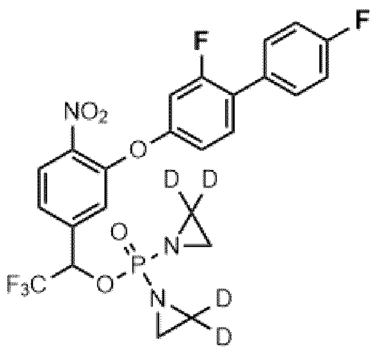

,

V-4

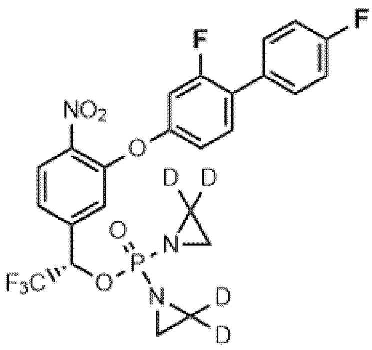

,

V-5

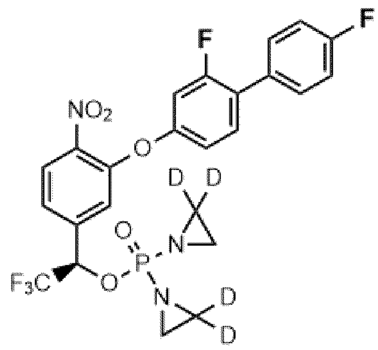

,

V-6

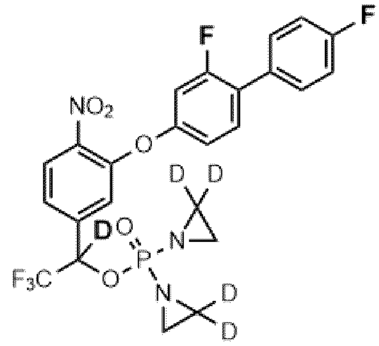

,

V-7

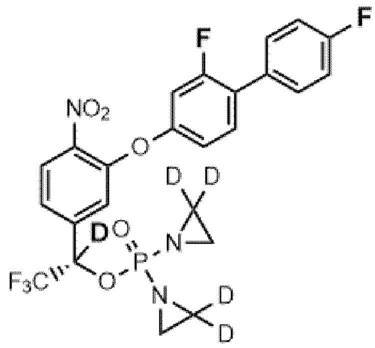

,

V-8

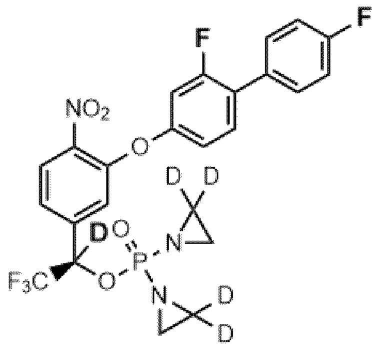

,

V-9

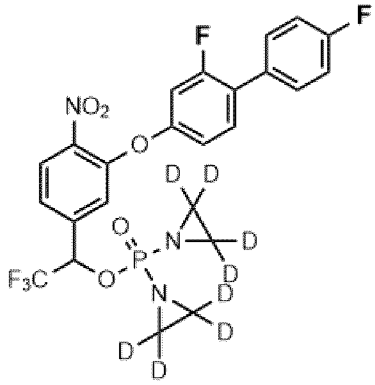

,

V-10

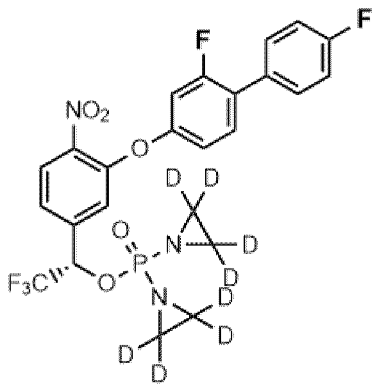

,

V-11

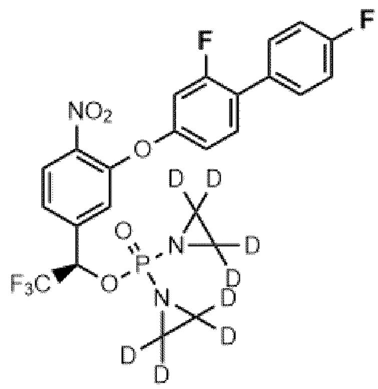

,

V-12

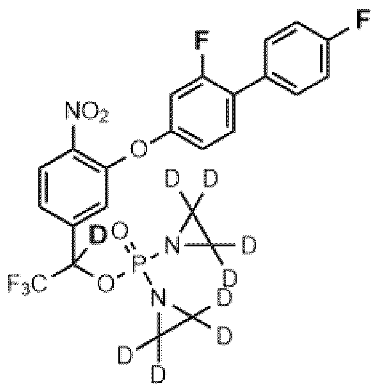

,

V-13

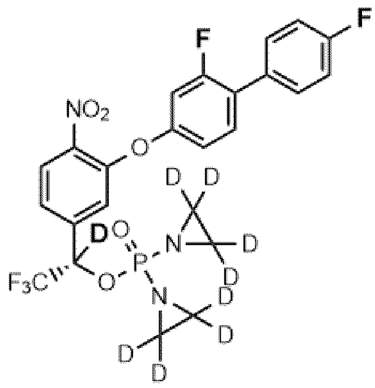

,

V-14

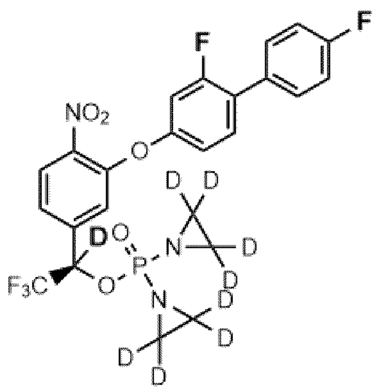

,

V-15

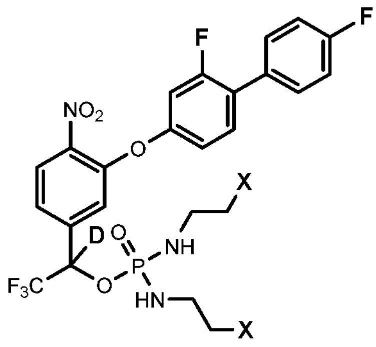 ,

IV-1

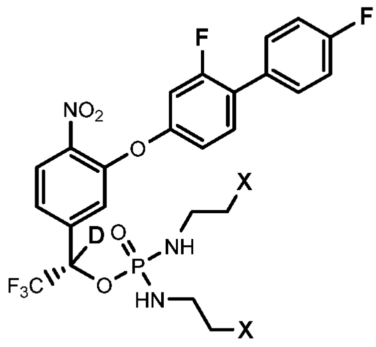 ,

IV-2

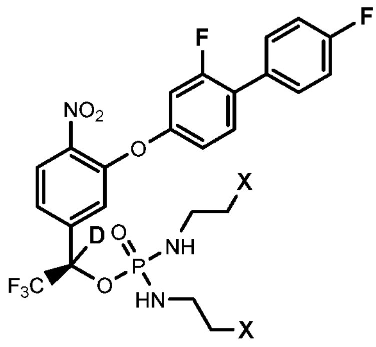 ,

IV-3

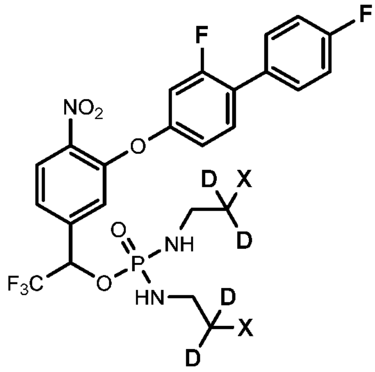 ,

IV-4

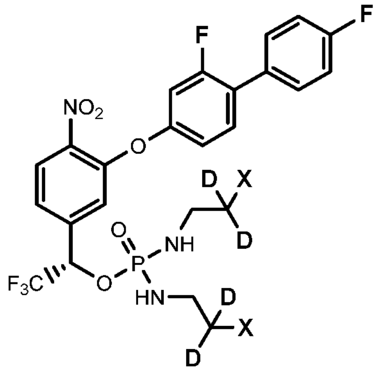 ,

IV-5

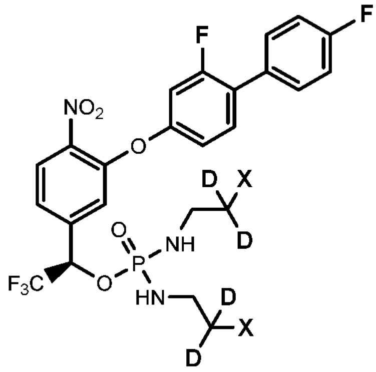 ,

IV-6

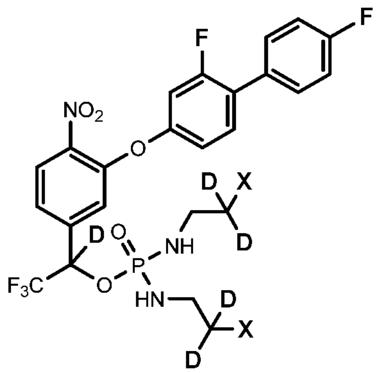 ,

IV-7

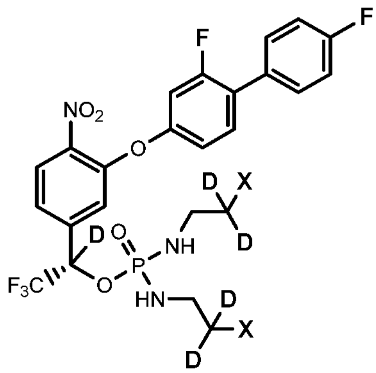 ,

IV-8

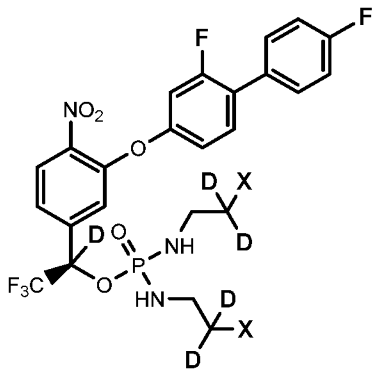 ,

IV-9

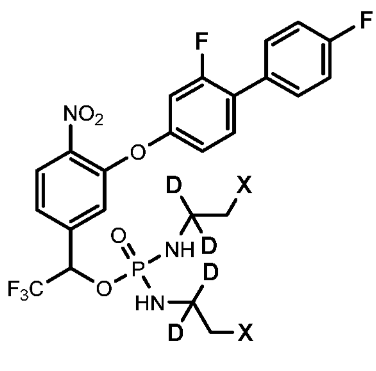 ,

IV-10

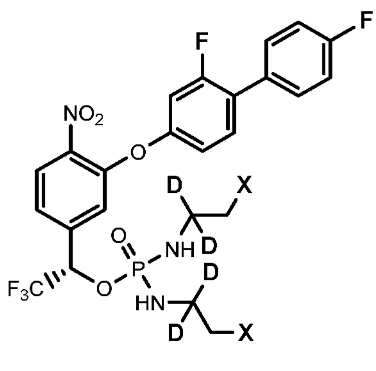 ,

IV-11

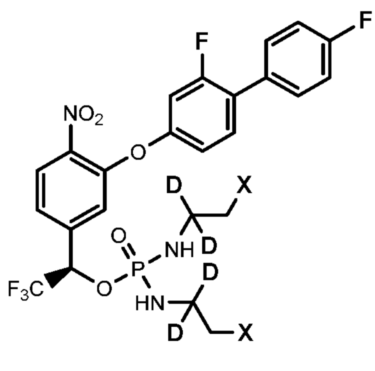 ,

IV-12

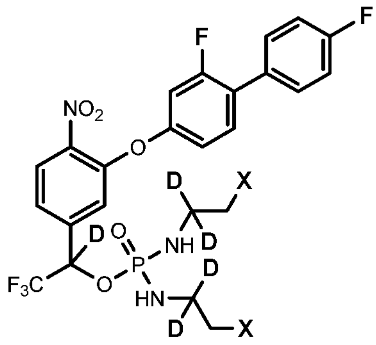,

IV-13

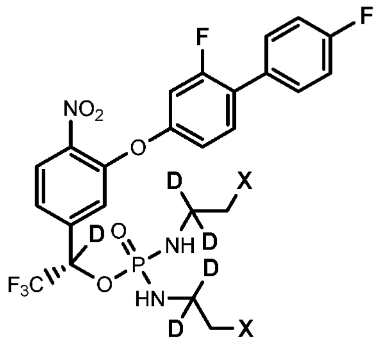,

IV-14

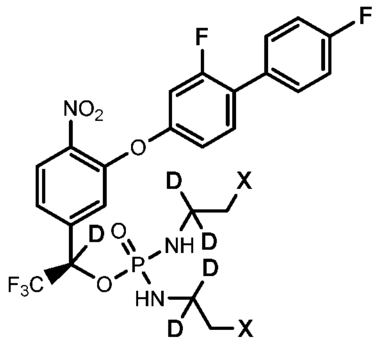,

IV-15

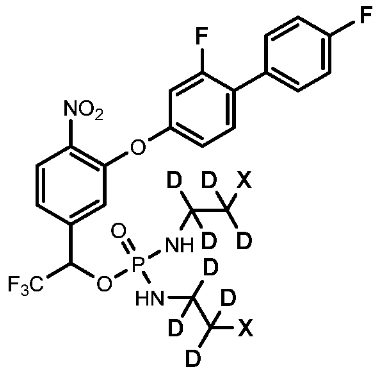,

IV-16

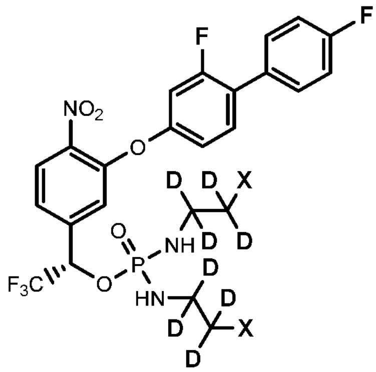,

IV-17

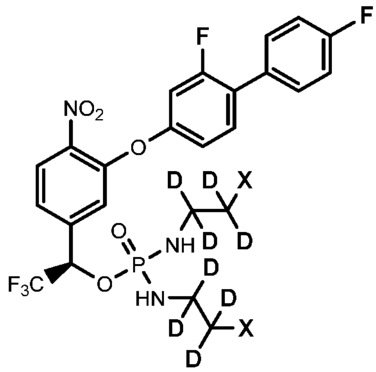,

IV-18

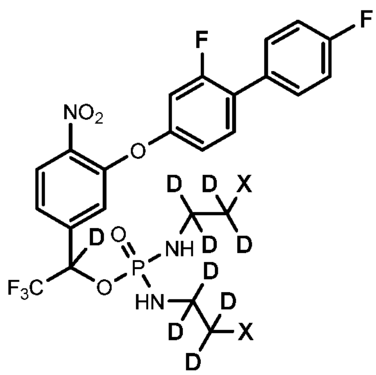,

IV-19

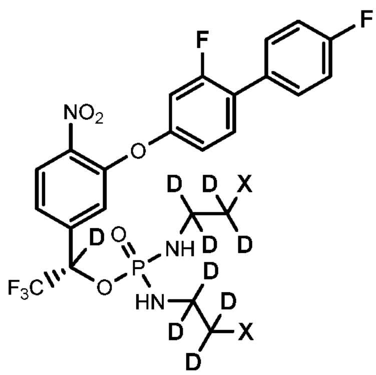,

IV-20

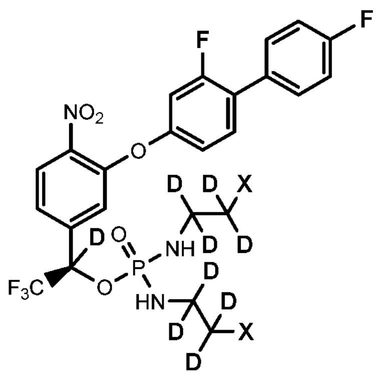,

IV-21

wherein the two X atoms in any one of compounds IV-1 to IV-21 are each independently fluorine, chlorine, bromine, or iodine, preferably bromine;
wherein silver oxide or silver nitrate is used as a catalyst in the ring-closure reaction, with or without the addition of a base.

**31.** A method for preparing any one of compounds IV-1 to IV-21 according to claim 30, comprising performing the first reaction of compound II-1, II-2, or II-3 or the deuterated compound thereof II-1a, II-2a, or II-3a as the starting reactant with phosphorus oxyhalide to obtain an intermediate, respectively, then performing the second reaction of the intermediate with the corresponding deuterated haloethylamine VI-1 or VI-2 or hydrohalide of the deuterated haloethylamine VI-1 or VI-2, and finally obtaining the corresponding compounds IV-1 to IV-21:

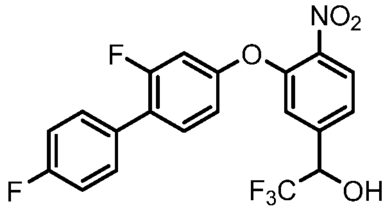,
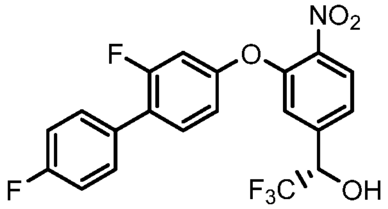,
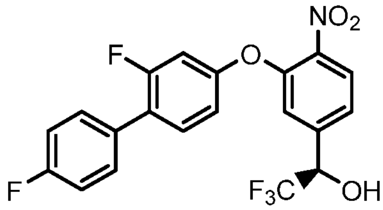,

II-1 II-2 II-3

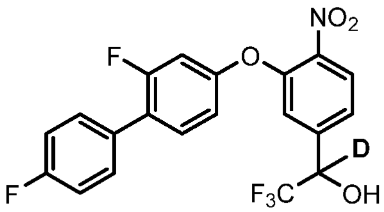,
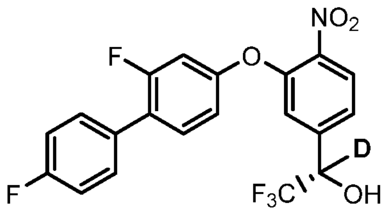,
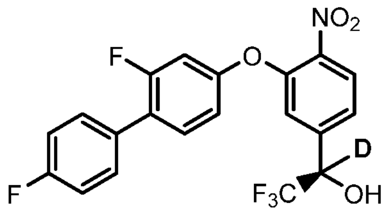

II-1a II-2a II-3a

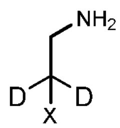
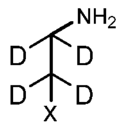

VI-1 VI-2

wherein the X atom in compound VI-1 or VI-2 is independently fluorine, chlorine, bromine, or iodine, and corresponds to the X atoms in compounds IV-1 to IV-21;
wherein the phosphorus oxyhalide is selected from phosphorus oxyfluoride, phosphorus oxychloride, phosphorus oxybromide, and phosphorus oxyiodide, and is preferably phosphorus oxychloride;
wherein the halogen in the phosphorus oxyhalide and the two X atoms in any one of compounds IV-1 to IV-21 or the X atom in compound VI-1 or VI-2 may be the same or different;
wherein in the hydrohalide of the deuterated haloethylamine, the halogen in hydrohalide and the halogen in deuterated haloethylamine are the same;
preferably, the haloethylamine is bromoethylamine, and the haloethylamine hydrohalide is bromoethylamine hydrobromide.

**32.** A method for preparing any one of the compounds with the following structures, comprising the following steps: reacting compound

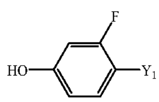

as the starting material to obtain compound

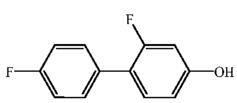,

then reacting the compound

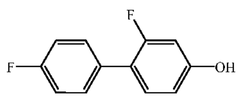

with formula I-7-1, I-7-2, or I-7-3 to obtain the corresponding compound of formula A, B, or C, respectively:

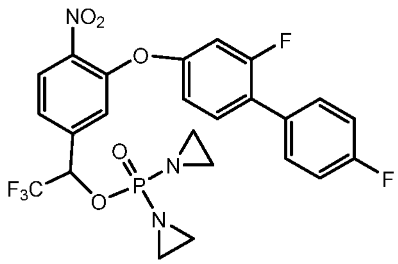
,
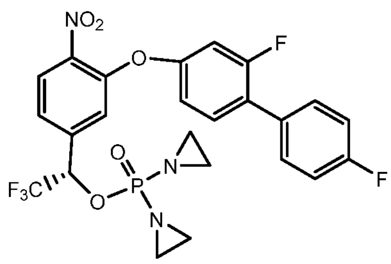
,
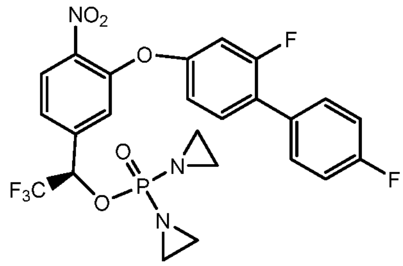

A B C

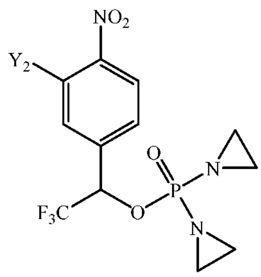
,
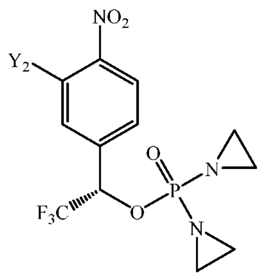
,
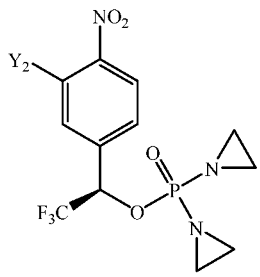
,

I-7-1 I-7-2 I-7-3

wherein $Y_1$ and $Y_2$ are halogen or OM, and M is hydrogen, sodium, potassium, magnesium, or calcium, and halogen is preferably bromine.

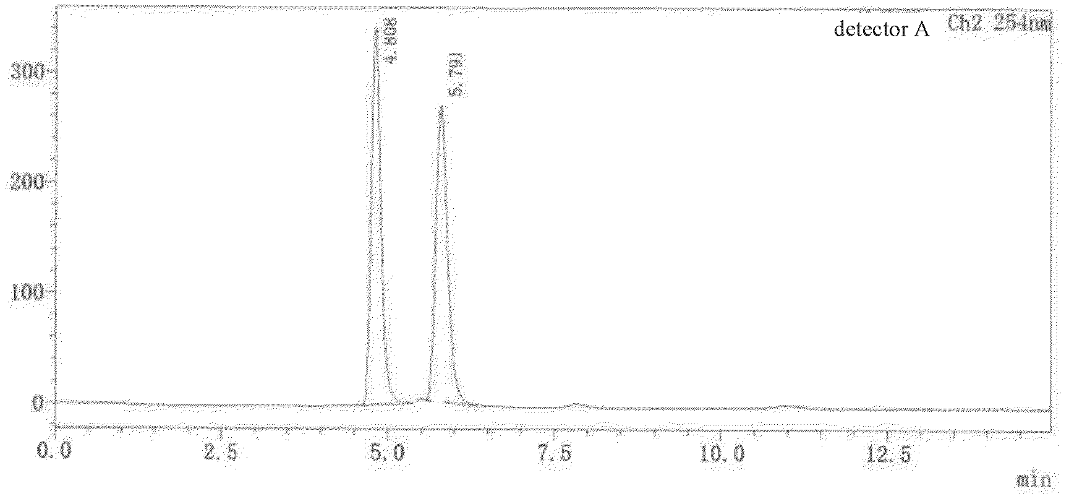


Fig. 1

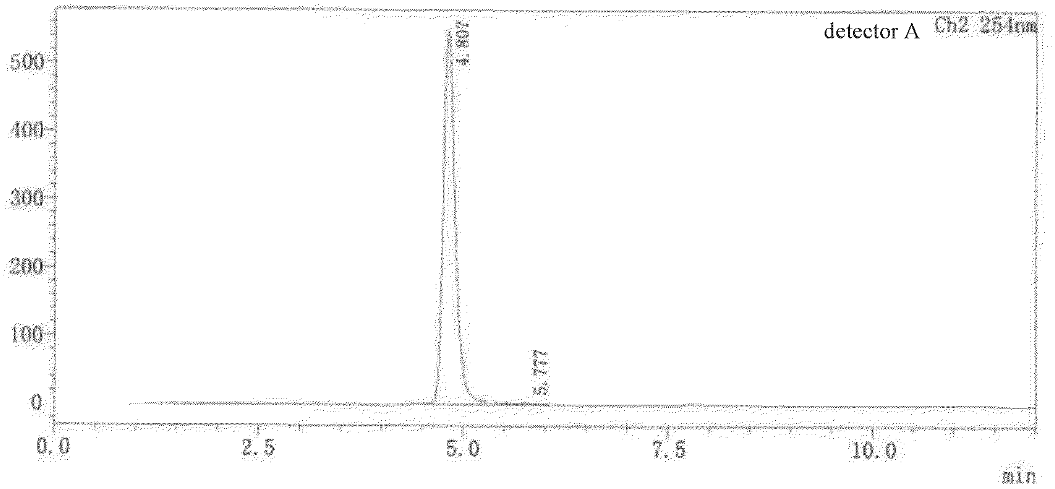


Fig. 2

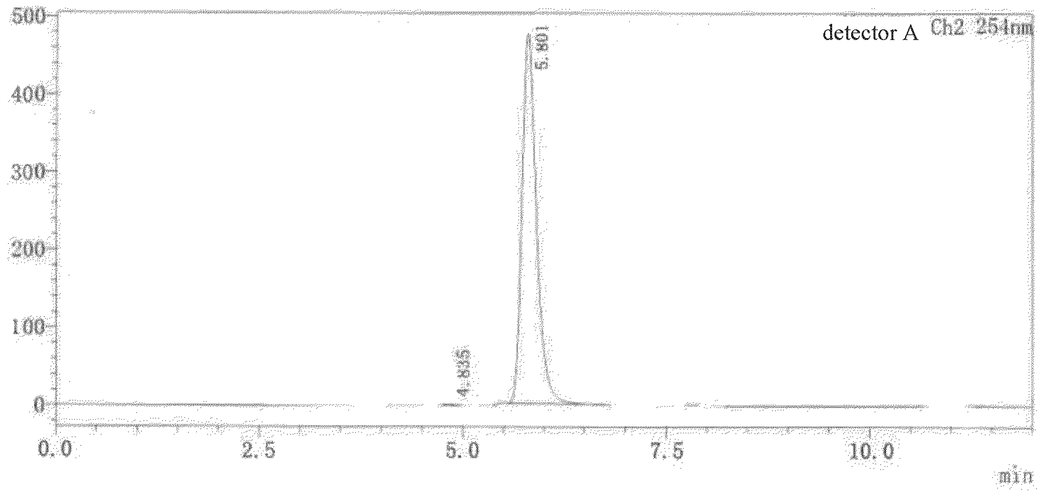


Fig. 3

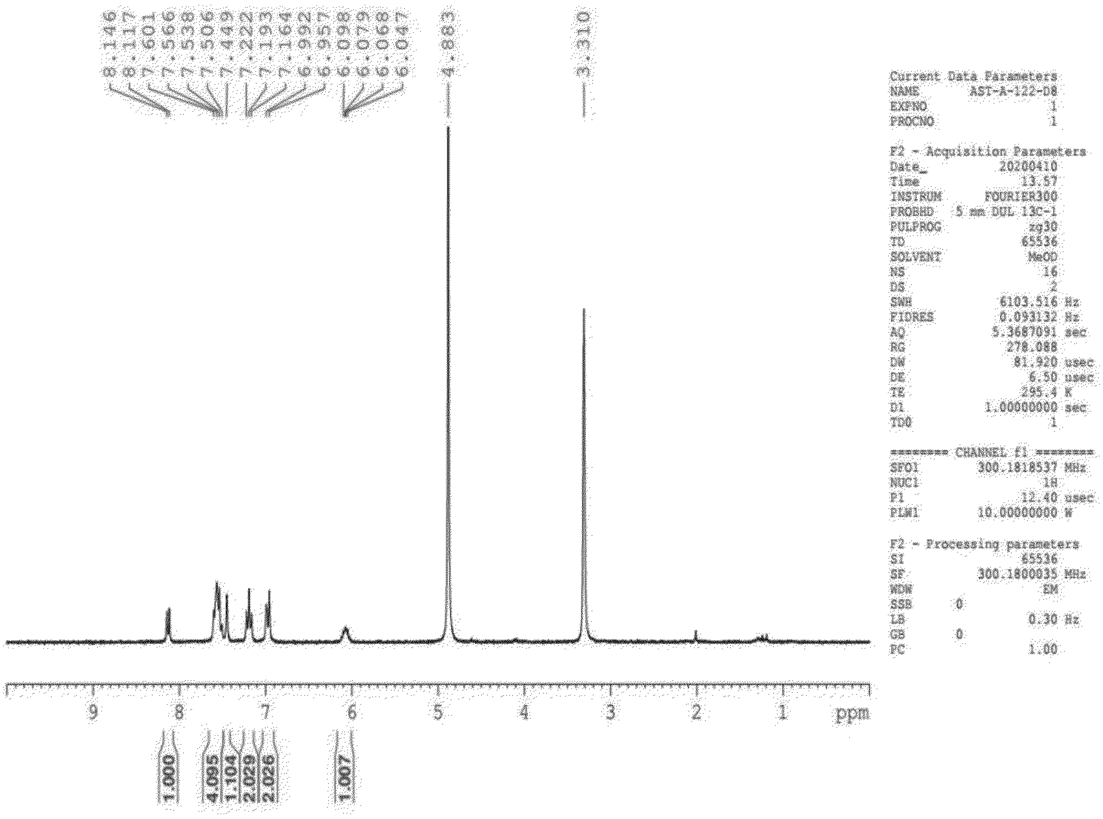
8.146
8.117
7.601
7.566
7.538
7.506
7.449
7.222
7.193
7.164
6.992
6.957
6.098
6.079
6.068
6.047
4.883
3.310
Current Data Parameters
NAME AST-A-122-D8
EXPNO 1
PROCNO 1
F2 - Acquisition Parameters
Date_ 20200410
Time 13.57
INSTRUM FOURIER300
PROBHD 5 mm DUL 13C-1
PULPROG zg30
TD 65536
SOLVENT MeOD
NS 16
DS 2
SWH 6103.516 Hz
FIDRES 0.093132 Hz
AQ 5.3687091 sec
RG 278.088
DW 81.920 usec
DE 6.50 usec
TE 295.4 K
D1 1.00000000 sec
TD0 1
======== CHANNEL f1 ========
SFO1 300.1818537 MHz
NUC1 1H
P1 12.40 usec
PLW1 10.00000000 W
F2 - Processing parameters
SI 65536
SF 300.1800035 MHz
WDW EM
SSB 0
LB 0.30 Hz
GB 0
PC 1.00
9
8
7
6
5
4
3
2
1
ppm
1.000
4.095
1.104
2.029
2.026
1.007

Fig. 4

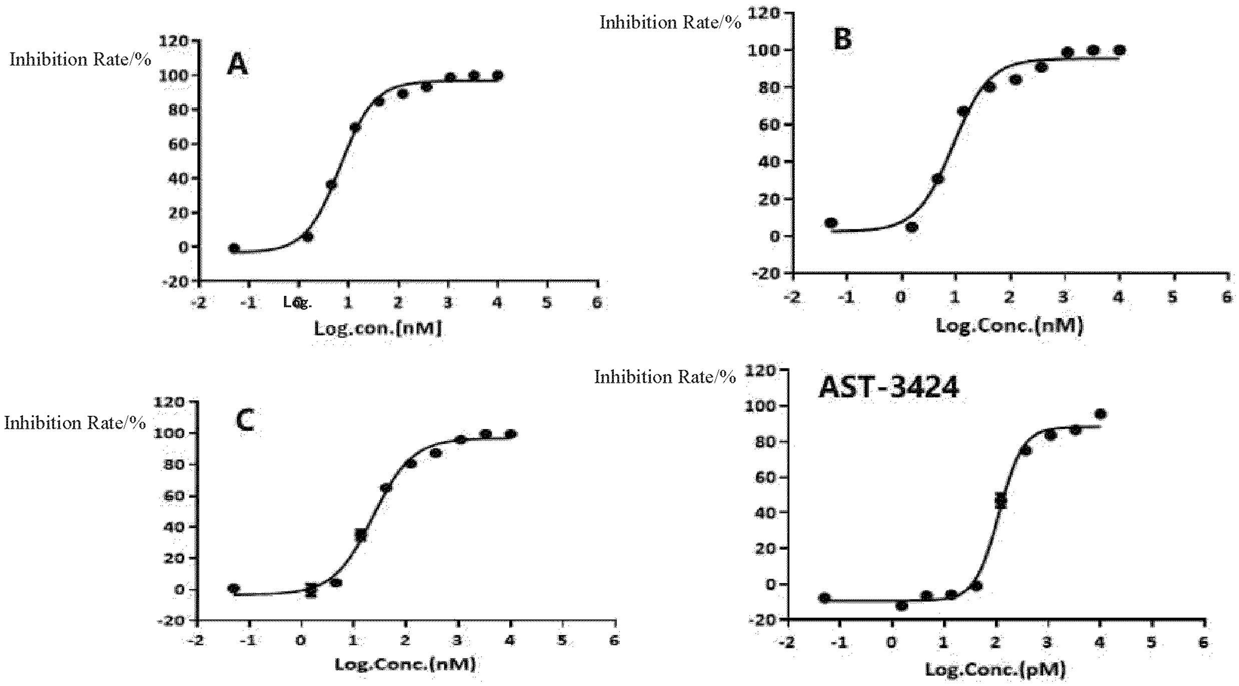
Inhibition Rate/%
A
Log.con.[nM]
Inhibition Rate/%
B
Log.Conc.(nM)
Inhibition Rate/%
C
Log.Conc.(nM)
Inhibition Rate/%
AST-3424
Log.Conc.(pM)

Fig. 5

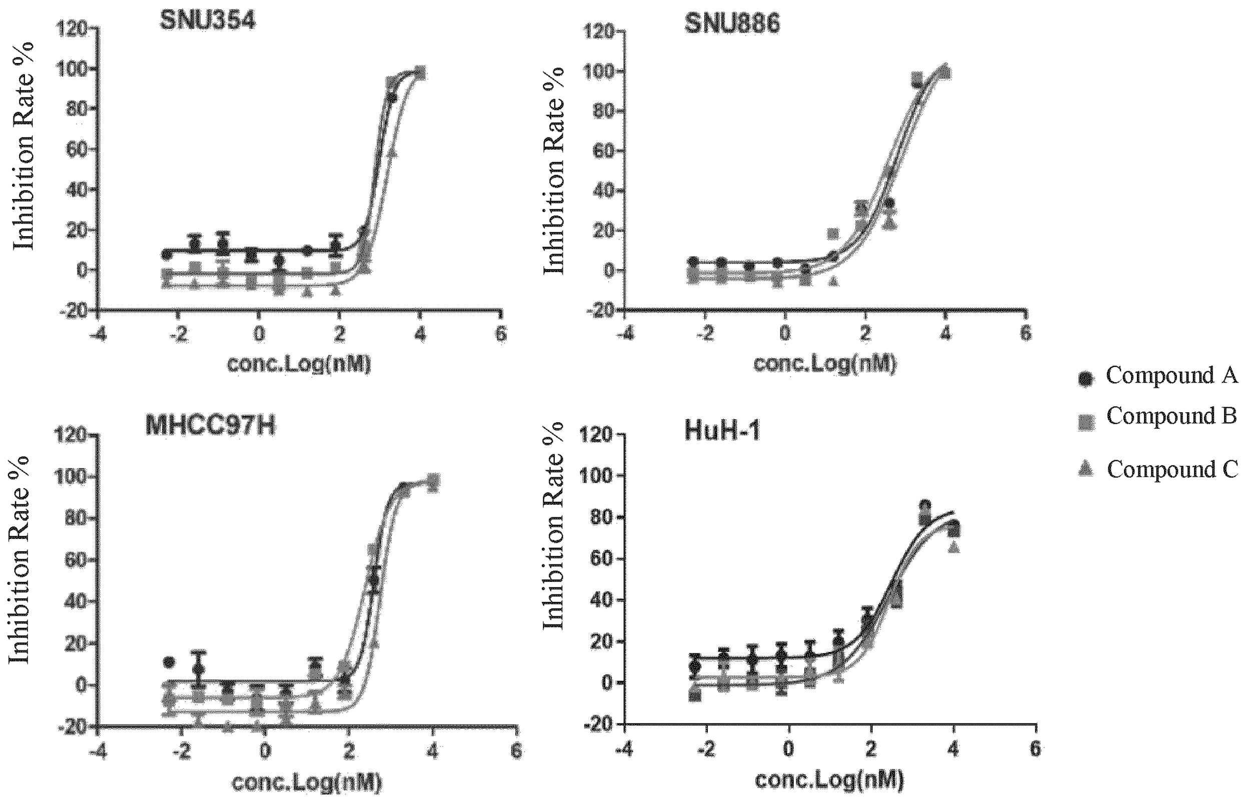
SNU354
SNU886
MHCC97H
HuH-1
Inhibition Rate %
conc.Log(nM)
Compound A
Compound B
Compound C

Fig. 6

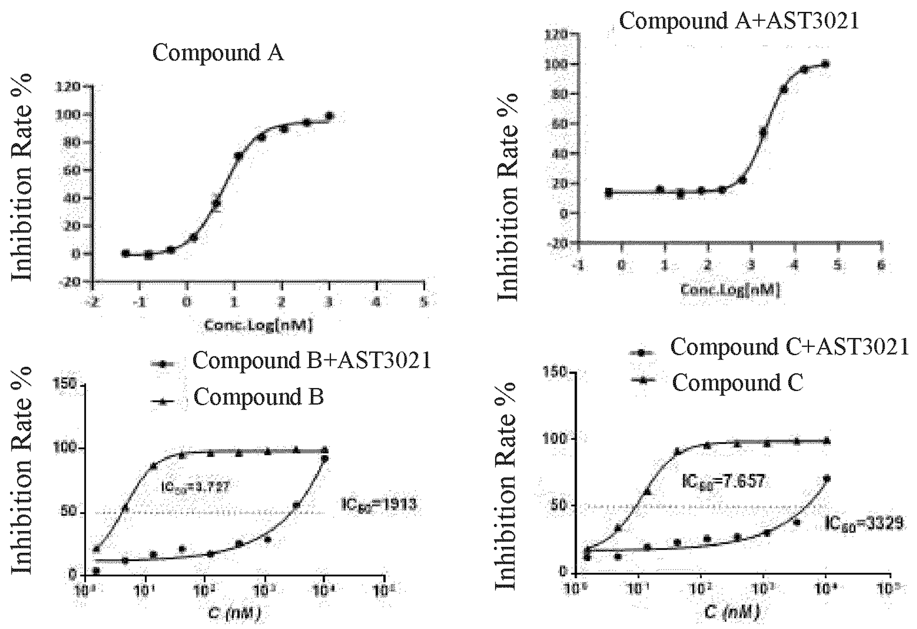
Compound A
Compound A+AST3021
Inhibition Rate %
Conc.Log[nM]
Compound B+AST3021
Compound B
$IC_{50}$=3.727
$IC_{50}$=1913
Compound C+AST3021
Compound C
$IC_{50}$=7.657
$IC_{50}$=3329
C (nM)

Fig. 7

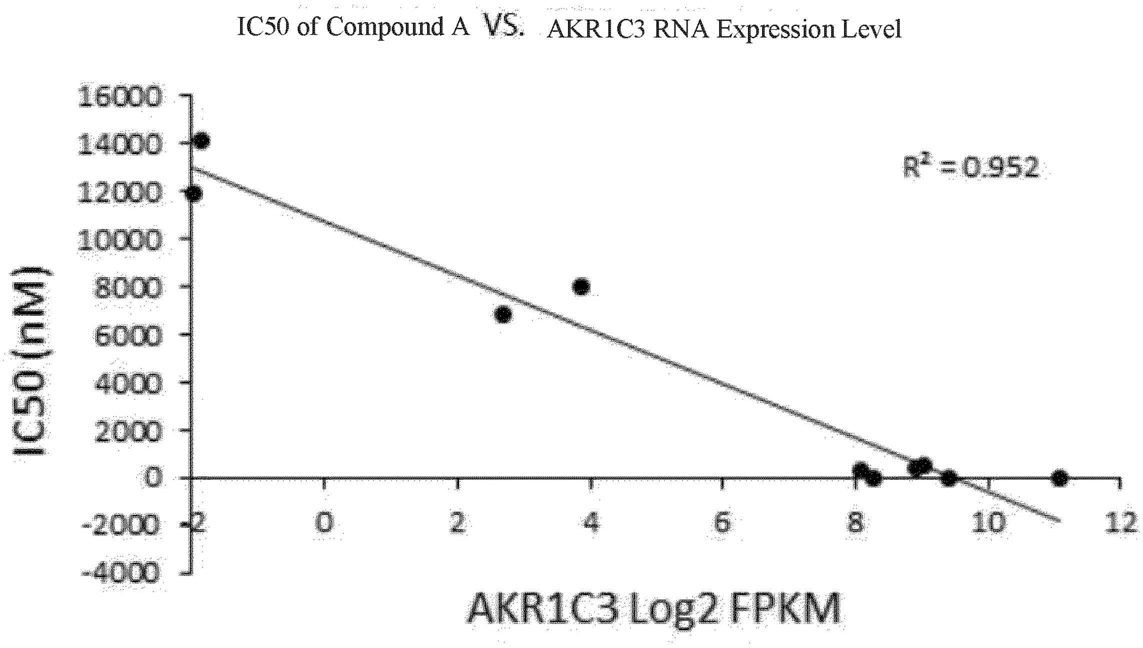
IC50 of Compound A VS. AKR1C3 RNA Expression Level
R² = 0.952
IC50 (nM)
16000
14000
12000
10000
8000
6000
4000
2000
0
-2000
-4000
-2
0
2
4
6
8
10
12
AKR1C3 Log2 FPKM

Fig. 8

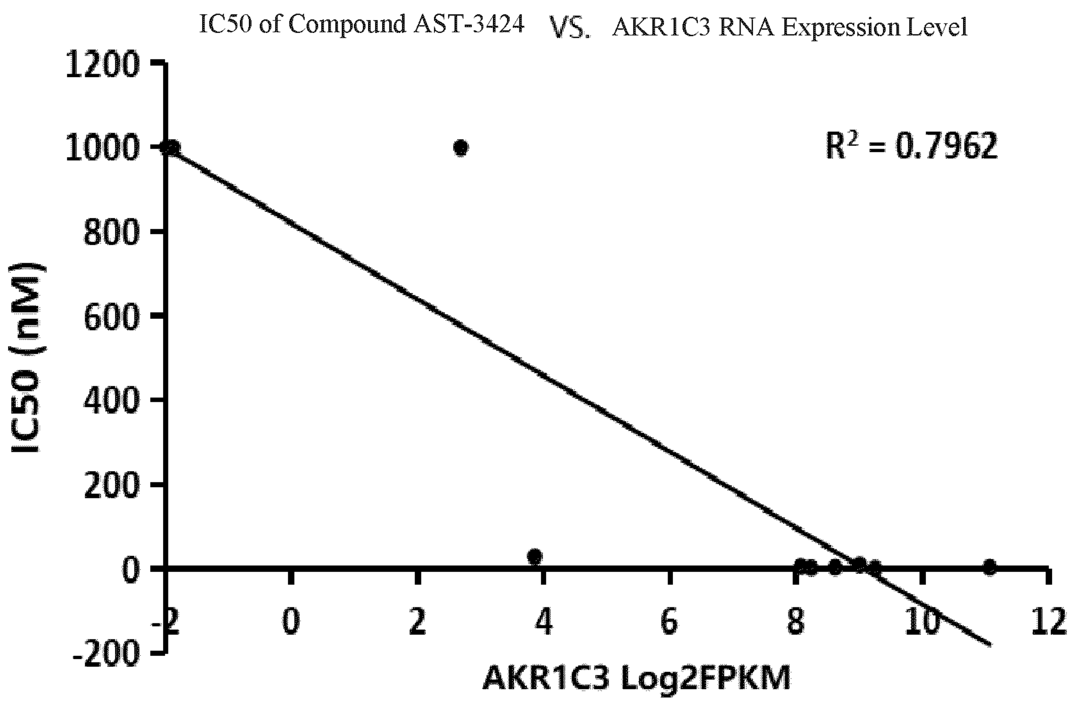
IC50 of Compound AST-3424 VS. AKR1C3 RNA Expression Level
R² = 0.7962
IC50 (nM)
1200
1000
800
600
400
200
0
-200
-2
0
2
4
6
8
10
12
AKR1C3 Log2FPKM

Fig. 9

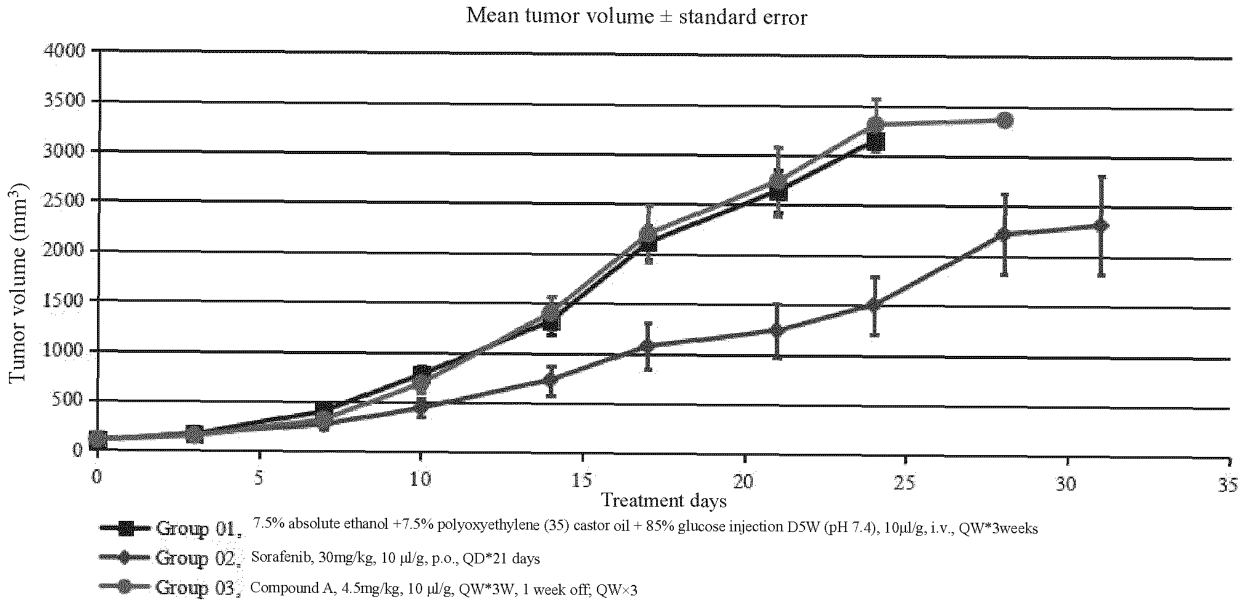
Mean tumor volume ± standard error
Tumor volume (mm³)
4000
3500
3000
2500
2000
1500
1000
500
0
0
5
10
15
20
25
30
35
Treatment days
Group 01, 7.5% absolute ethanol +7.5% polyoxyethylene (35) castor oil + 85% glucose injection D5W (pH 7.4), 10μl/g, i.v., QW*3weeks
Group 02, Sorafenib, 30mg/kg, 10 μl/g, p.o., QD*21 days
Group 03, Compound A, 4.5mg/kg, 10 μl/g, QW*3W, 1 week off, QW×3

Fig. 10

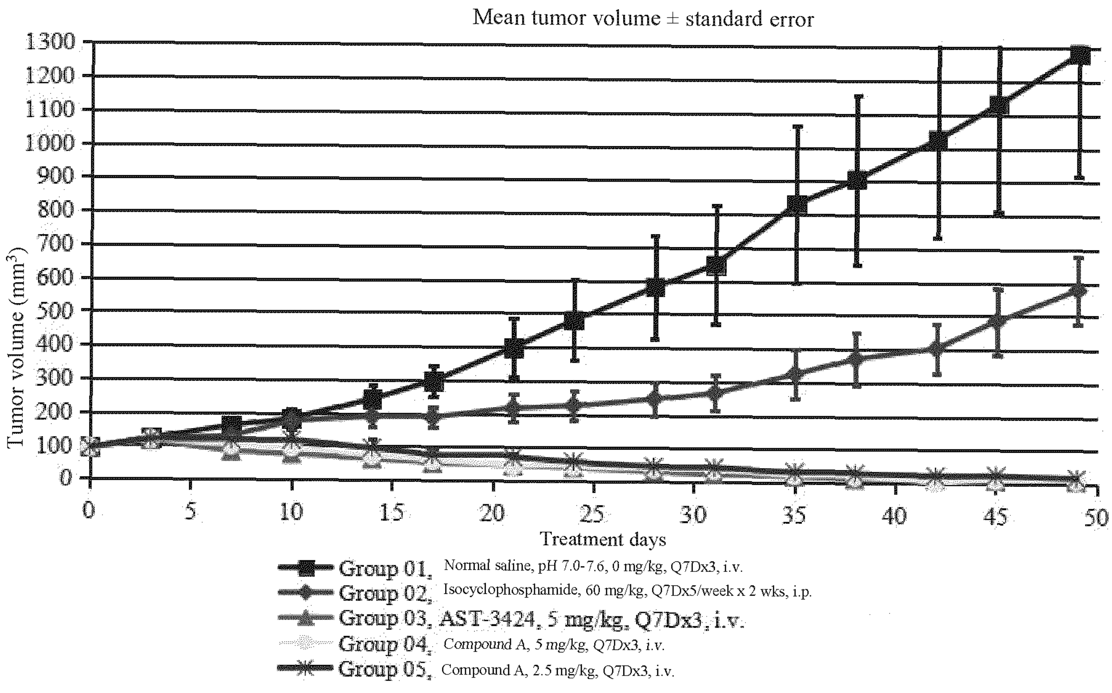
Mean tumor volume ± standard error
Tumor volume (mm³)
1300
1200
1100
1000
900
800
700
600
500
400
300
200
100
0
0
5
10
15
20
25
30
35
40
45
50
Treatment days
Group 01, Normal saline, pH 7.0-7.6, 0 mg/kg, Q7Dx3, i.v.
Group 02, Isocyclophosphamide, 60 mg/kg, Q7Dx5/week x 2 wks, i.p.
Group 03, AST-3424, 5 mg/kg, Q7Dx3, i.v.
Group 04, Compound A, 5 mg/kg, Q7Dx3, i.v.
Group 05, Compound A, 2.5 mg/kg, Q7Dx3, i.v.

Fig. 11

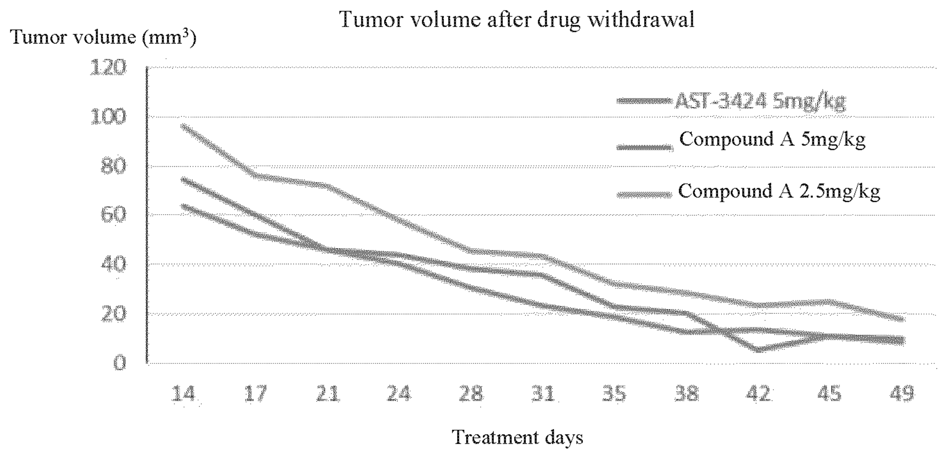
Tumor volume after drug withdrawal
Tumor volume (mm3)
120
100
80
60
40
20
0
AST-3424 5mg/kg
Compound A 5mg/kg
Compound A 2.5mg/kg
14
17
21
24
28
31
35
38
42
45
49
Treatment days

Fig. 12

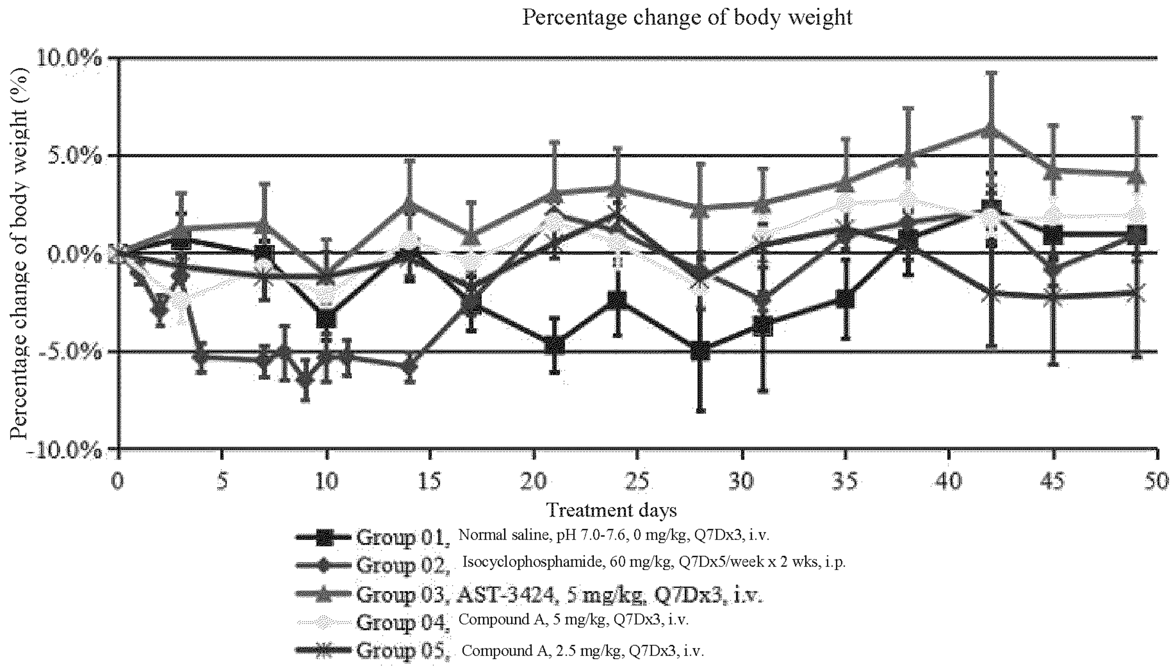
Percentage change of body weight
Percentage change of body weight (%)
10.0%
5.0%
0.0%
-5.0%
-10.0%
0
5
10
15
20
25
30
35
40
45
50
Treatment days
Group 01, Normal saline, pH 7.0-7.6, 0 mg/kg, Q7Dx3, i.v.
Group 02, Isocyclophosphamide, 60 mg/kg, Q7Dx5/week x 2 wks, i.p.
Group 03, AST-3424, 5 mg/kg, Q7Dx3, i.v.
Group 04, Compound A, 5 mg/kg, Q7Dx3, i.v.
Group 05, Compound A, 2.5 mg/kg, Q7Dx3, i.v.

Fig. 13

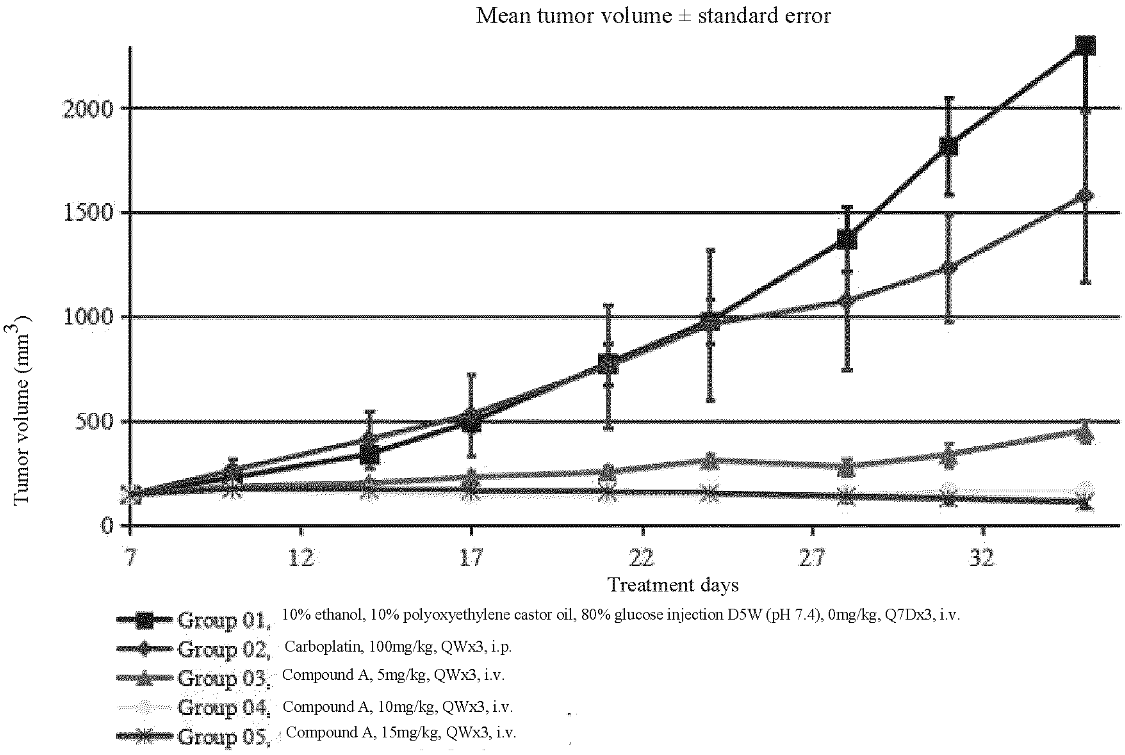
Mean tumor volume ± standard error
Tumor volume (mm3)
2000
1500
1000
500
0
7
12
17
22
27
32
Treatment days
Group 01, 10% ethanol, 10% polyoxyethylene castor oil, 80% glucose injection D5W (pH 7.4), 0mg/kg, Q7Dx3, i.v.
Group 02, Carboplatin, 100mg/kg, QWx3, i.p.
Group 03, Compound A, 5mg/kg, QWx3, i.v.
Group 04, Compound A, 10mg/kg, QWx3, i.v.
Group 05, Compound A, 15mg/kg, QWx3, i.v.

Fig. 14

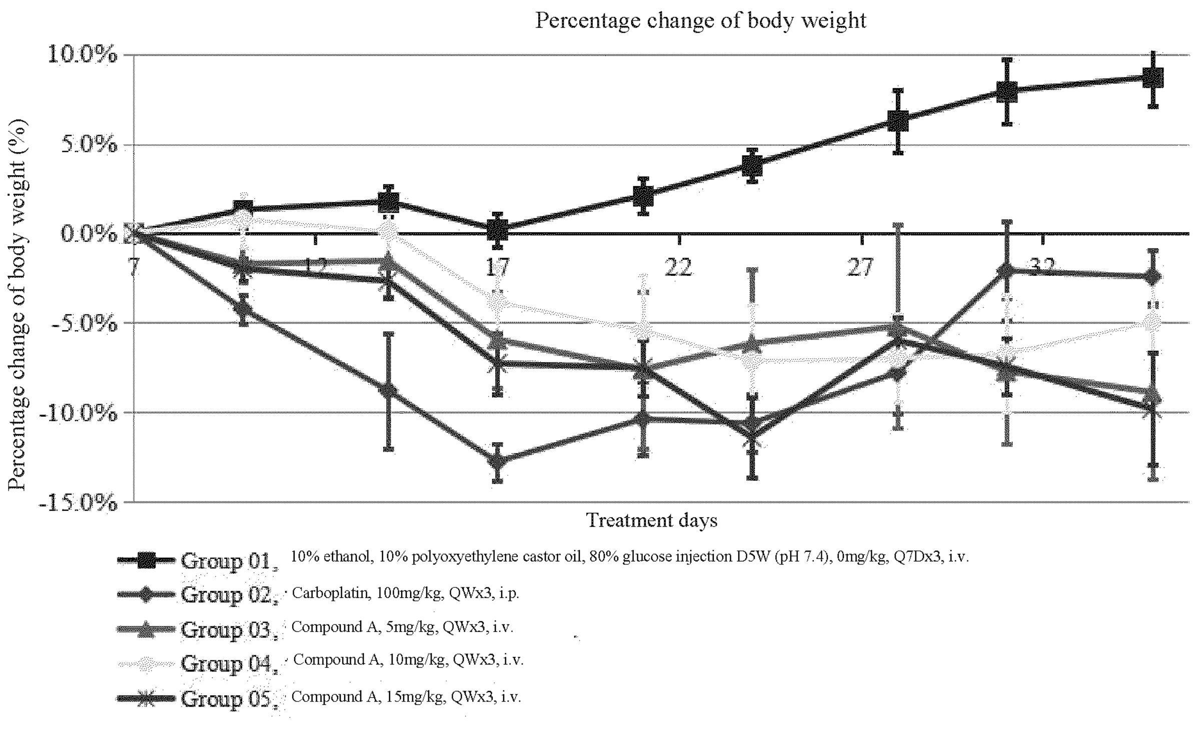
Percentage change of body weight
Percentage change of body weight (%)
10.0%
5.0%
0.0%
-5.0%
-10.0%
-15.0%
7
17
22
27
Treatment days
Group 01, 10% ethanol, 10% polyoxyethylene castor oil, 80% glucose injection D5W (pH 7.4), 0mg/kg, Q7Dx3, i.v.
Group 02, Carboplatin, 100mg/kg, QWx3, i.p.
Group 03, Compound A, 5mg/kg, QWx3, i.v.
Group 04, Compound A, 10mg/kg, QWx3, i.v.
Group 05, Compound A, 15mg/kg, QWx3, i.v.

Fig. 15

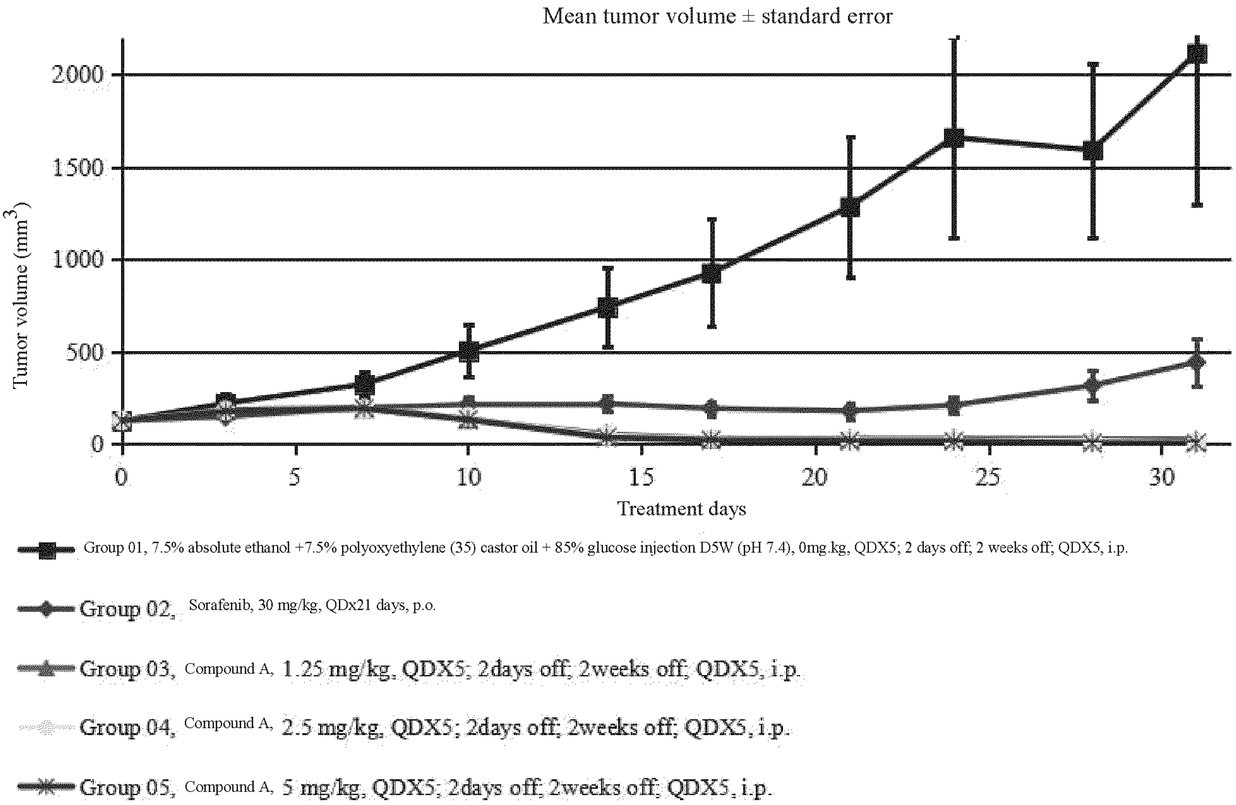
Mean tumor volume ± standard error
Tumor volume (mm3)
2000
1500
1000
500
0
0
5
10
15
20
25
30
Treatment days
Group 01, 7.5% absolute ethanol +7.5% polyoxyethylene (35) castor oil + 85% glucose injection D5W (pH 7.4), 0mg.kg, QDX5; 2 days off; 2 weeks off; QDX5, i.p.
Group 02, Sorafenib, 30 mg/kg, QDx21 days, p.o.
Group 03, Compound A, 1.25 mg/kg, QDX5; 2days off; 2weeks off; QDX5, i.p.
Group 04, Compound A, 2.5 mg/kg, QDX5; 2days off; 2weeks off; QDX5, i.p.
Group 05, Compound A, 5 mg/kg, QDX5; 2days off; 2weeks off; QDX5, i.p.

Fig. 16

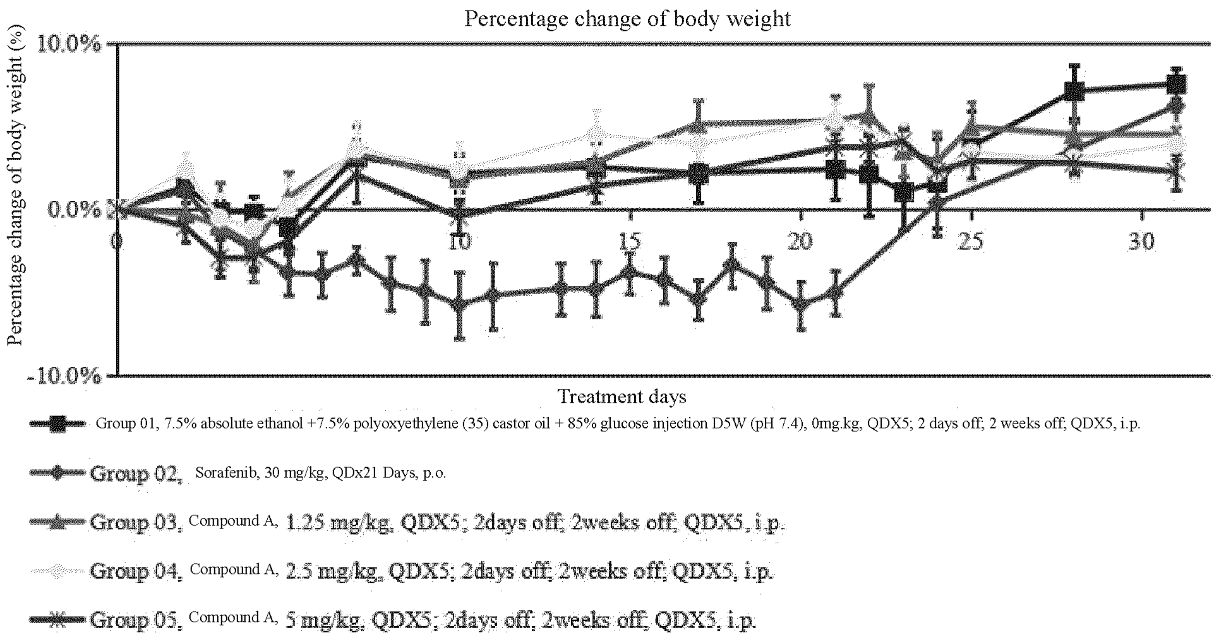
Percentage change of body weight
Percentage change of body weight (%)
10.0%
0.0%
-10.0%
0
10
15
20
25
30
Treatment days
Group 01, 7.5% absolute ethanol +7.5% polyoxyethylene (35) castor oil + 85% glucose injection D5W (pH 7.4), 0mg.kg, QDX5; 2 days off; 2 weeks off; QDX5, i.p.
Group 02, Sorafenib, 30 mg/kg, QDx21 Days, p.o.
Group 03, Compound A, 1.25 mg/kg, QDX5; 2days off; 2weeks off; QDX5, i.p.
Group 04, Compound A, 2.5 mg/kg, QDX5; 2days off; 2weeks off; QDX5, i.p.
Group 05, Compound A, 5 mg/kg, QDX5; 2days off; 2weeks off; QDX5, i.p.

Fig. 17

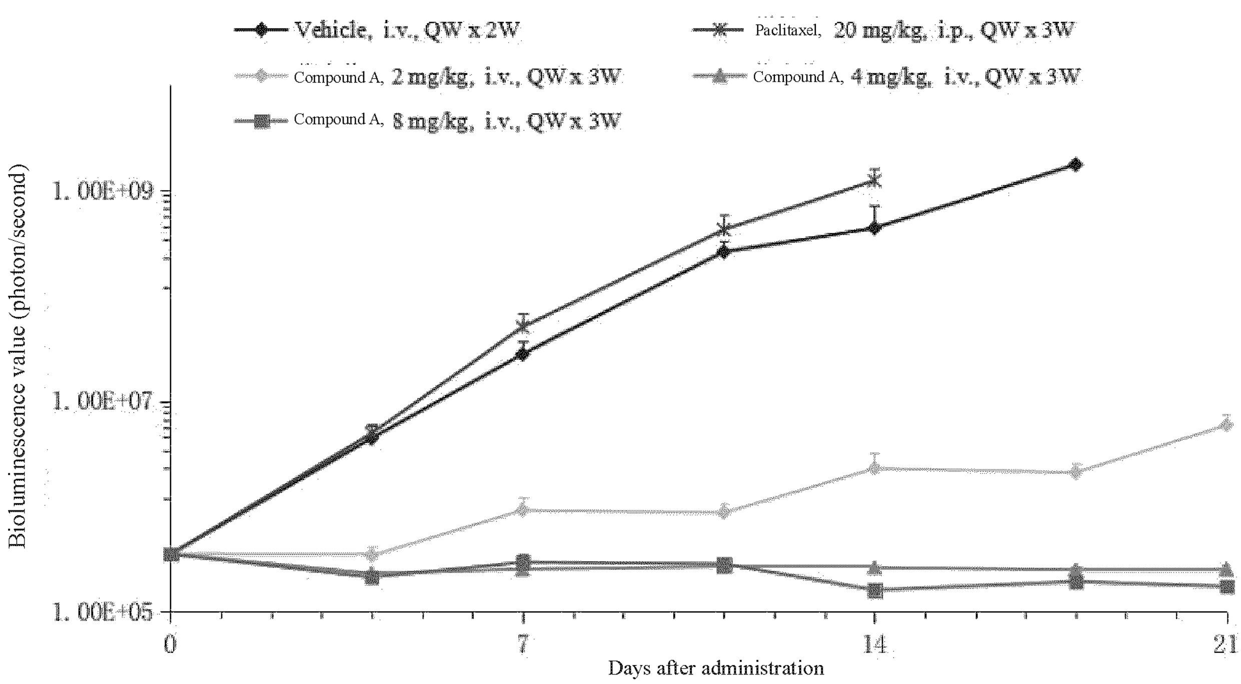
Vehicle, i.v., QW x 2W
Paclitaxel, 20 mg/kg, i.p., QW x 3W
Compound A, 2 mg/kg, i.v., QW x 3W
Compound A, 4 mg/kg, i.v., QW x 3W
Compound A, 8 mg/kg, i.v., QW x 3W
Bioluminescence value (photon/second)
1. 00E+09
1. 00E+07
1. 00E+05
0
7
14
21
Days after administration

Fig. 18

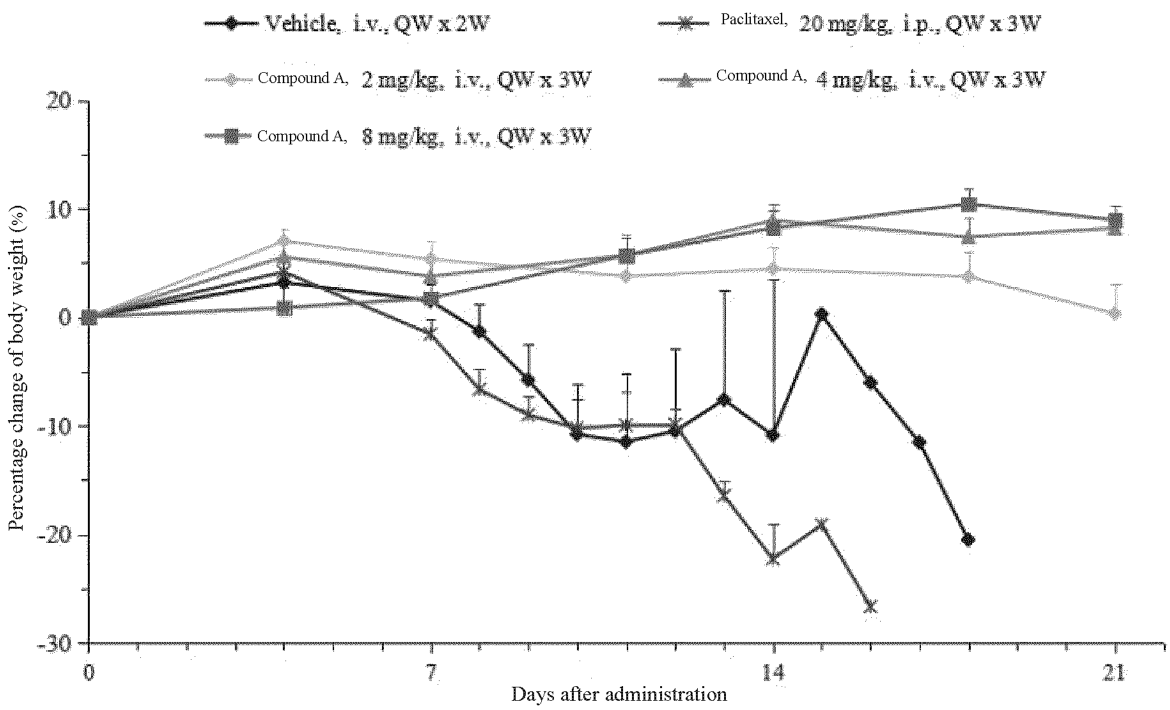
Vehicle, i.v., QW x 2W
Paclitaxel, 20 mg/kg, i.p., QW x 3W
Compound A, 2 mg/kg, i.v., QW x 3W
Compound A, 4 mg/kg, i.v., QW x 3W
Compound A, 8 mg/kg, i.v., QW x 3W
Percentage change of body weight (%)
20
10
0
-10
-20
-30
0
7
14
21
Days after administration

Fig. 19

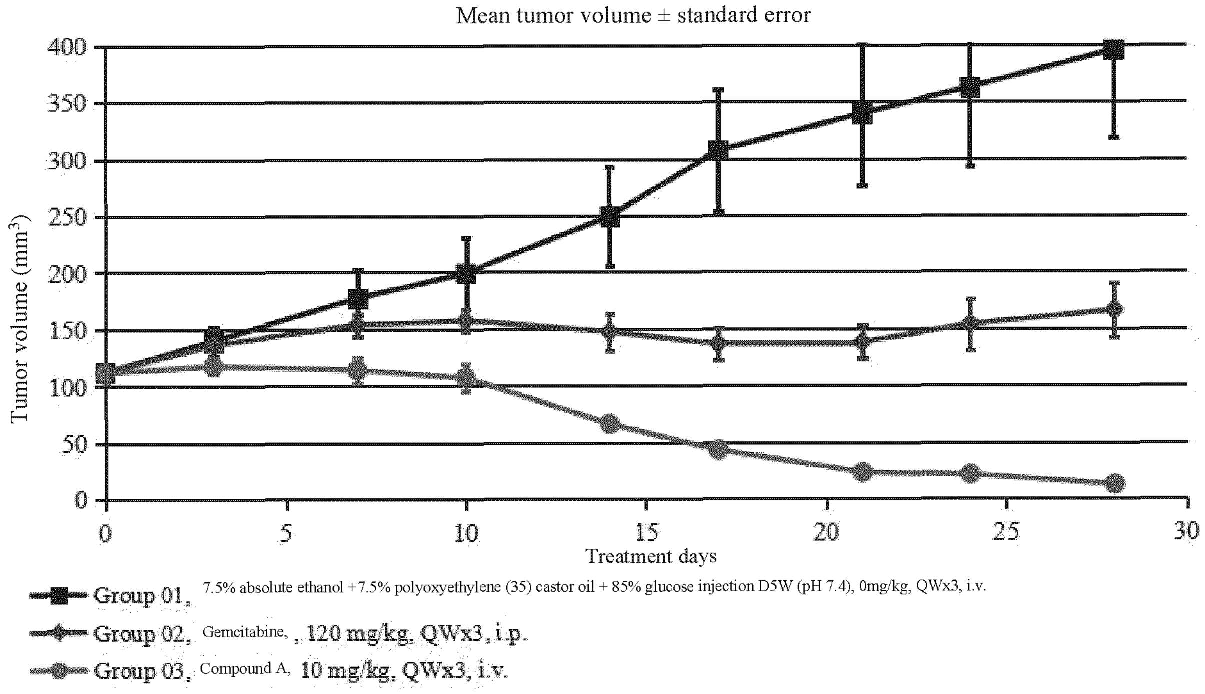
Mean tumor volume ± standard error
Tumor volume (mm³)
400
350
300
250
200
150
100
50
0
0
5
10
15
20
25
30
Treatment days
Group 01, 7.5% absolute ethanol +7.5% polyoxyethylene (35) castor oil + 85% glucose injection D5W (pH 7.4), 0mg/kg, QWx3, i.v.
Group 02, Gemcitabine, , 120 mg/kg, QWx3, i.p.
Group 03, Compound A, 10 mg/kg, QWx3, i.v.

Fig. 20

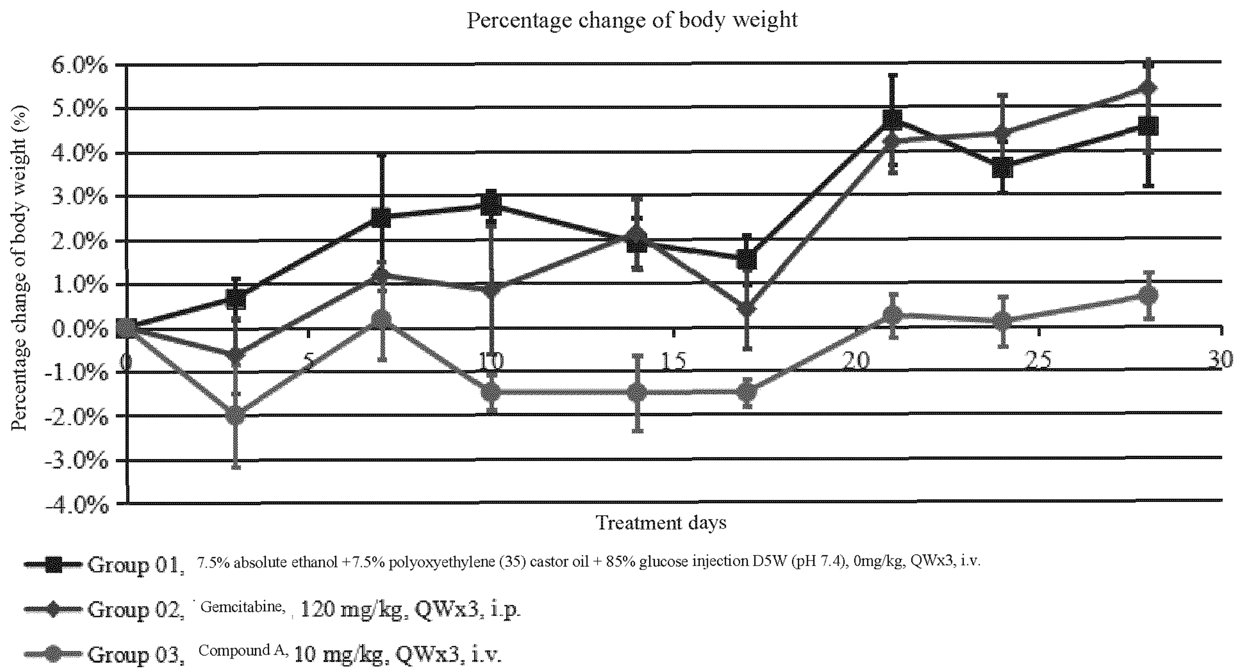
Percentage change of body weight
Percentage change of body weight (%)
6.0%
5.0%
4.0%
3.0%
2.0%
1.0%
0.0%
-1.0%
-2.0%
-3.0%
-4.0%
0
5
10
15
20
25
30
Treatment days
Group 01, 7.5% absolute ethanol +7.5% polyoxyethylene (35) castor oil + 85% glucose injection D5W (pH 7.4), 0mg/kg, QWx3, i.v.
Group 02, Gemcitabine, , 120 mg/kg, QWx3, i.p.
Group 03, Compound A, 10 mg/kg, QWx3, i.v.

Fig. 21

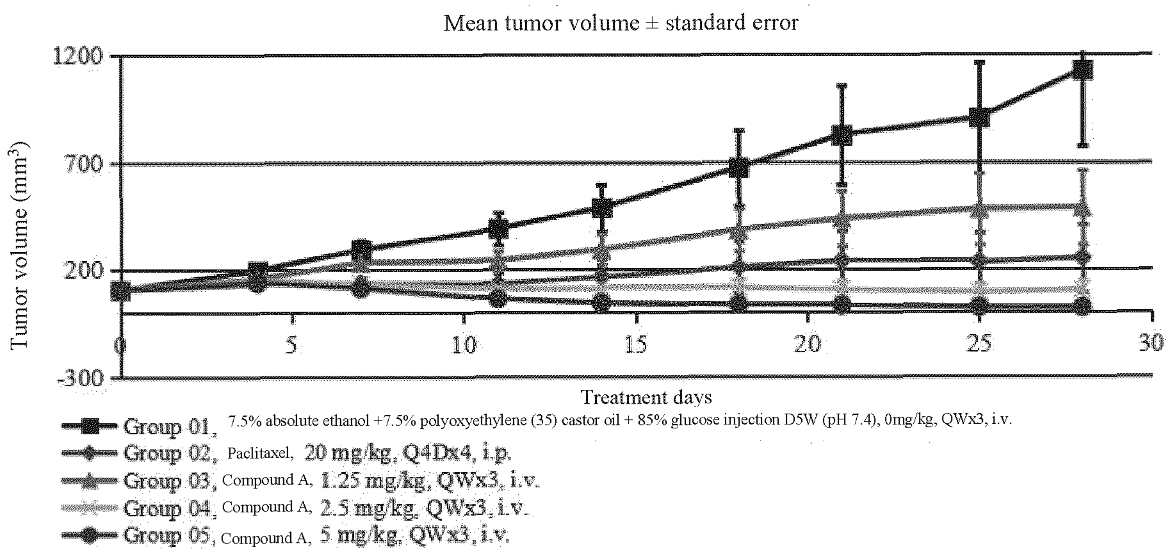
Mean tumor volume ± standard error
1200
700
200
-300
Tumor volume (mm3)
0
5
10
15
20
25
30
Treatment days
Group 01, 7.5% absolute ethanol +7.5% polyoxyethylene (35) castor oil + 85% glucose injection D5W (pH 7.4), 0mg/kg, QWx3, i.v.
Group 02, Paclitaxel, 20 mg/kg, Q4Dx4, i.p.
Group 03, Compound A, 1.25 mg/kg, QWx3, i.v.
Group 04, Compound A, 2.5 mg/kg, QWx3, i.v.
Group 05, Compound A, 5 mg/kg, QWx3, i.v.

Fig. 22

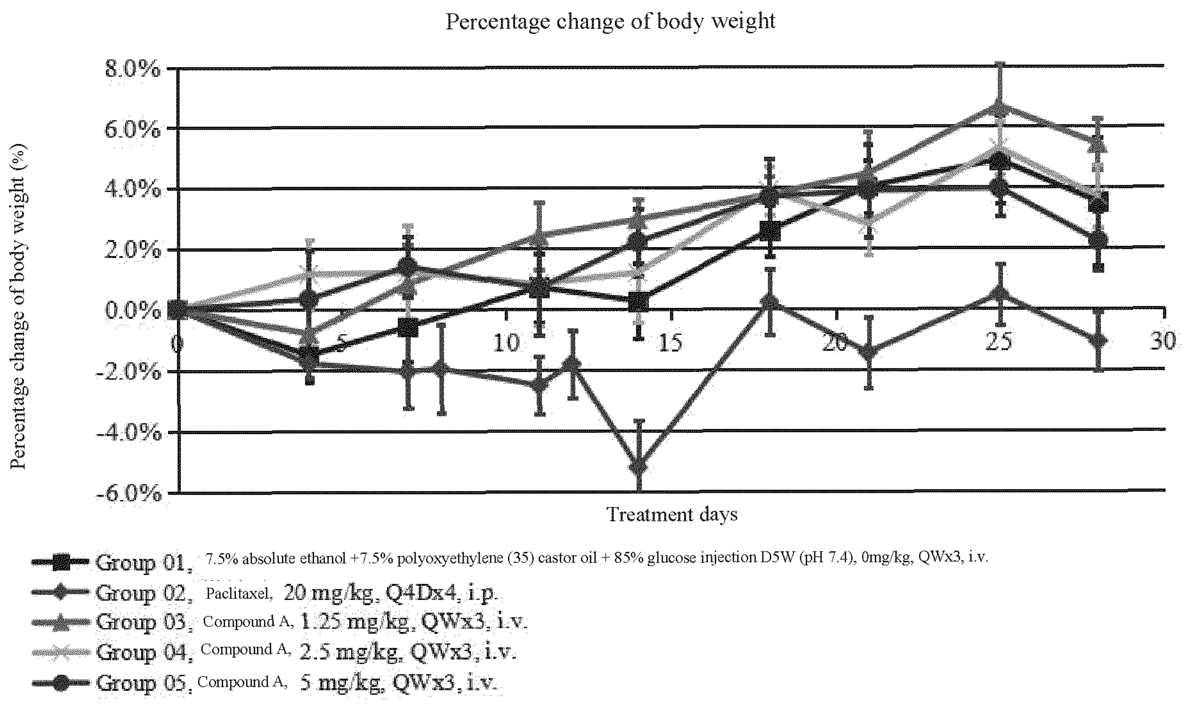
Percentage change of body weight
8.0%
6.0%
4.0%
2.0%
0.0%
-2.0%
-4.0%
-6.0%
Percentage change of body weight (%)
0
5
10
15
20
25
30
Treatment days
Group 01, 7.5% absolute ethanol +7.5% polyoxyethylene (35) castor oil + 85% glucose injection D5W (pH 7.4), 0mg/kg, QWx3, i.v.
Group 02, Paclitaxel, 20 mg/kg, Q4Dx4, i.p.
Group 03, Compound A, 1.25 mg/kg, QWx3, i.v.
Group 04, Compound A, 2.5 mg/kg, QWx3, i.v.
Group 05, Compound A, 5 mg/kg, QWx3, i.v.

Fig. 23

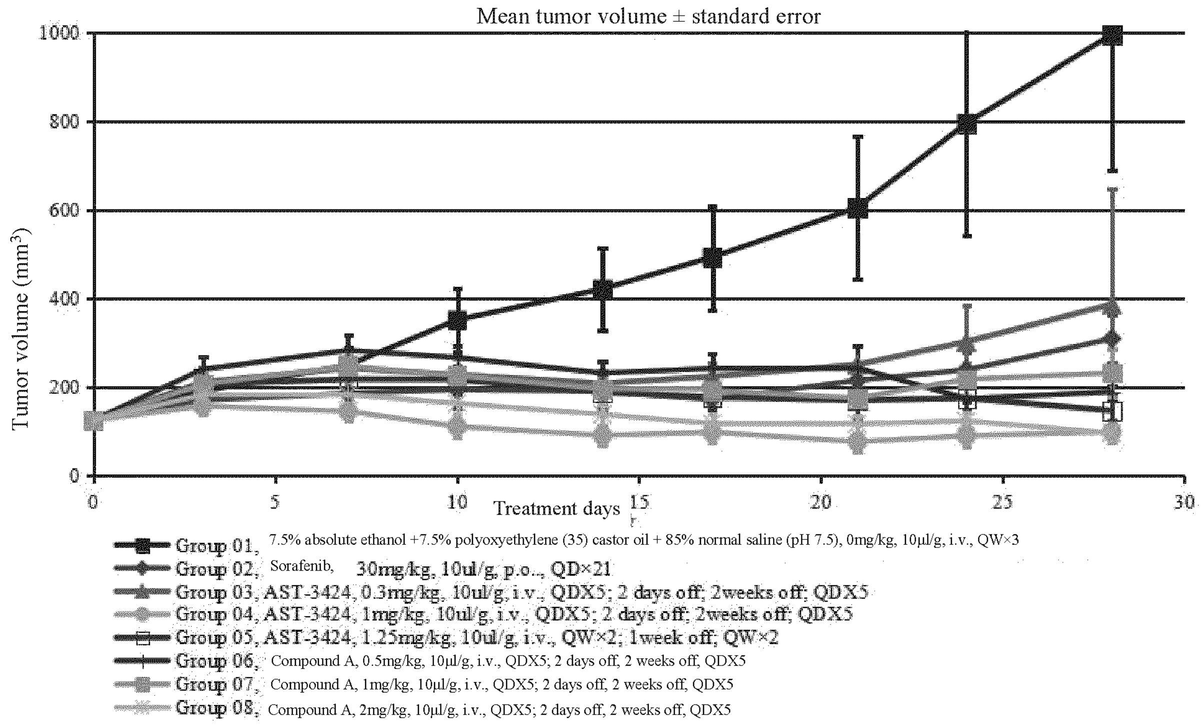
Mean tumor volume ± standard error
Tumor volume (mm$^3$)
1000
800
600
400
200
0
0
5
10
15
20
25
30
Treatment days
Group 01, 7.5% absolute ethanol +7.5% polyoxyethylene (35) castor oil + 85% normal saline (pH 7.5), 0mg/kg, 10μl/g, i.v., QW×3
Group 02, Sorafenib, 30mg/kg, 10ul/g, p.o.., QD×21
Group 03, AST-3424, 0.3mg/kg, 10ul/g, i.v., QDX5; 2 days off; 2weeks off; QDX5
Group 04, AST-3424, 1mg/kg, 10ul/g, i.v., QDX5; 2 days off; 2weeks off; QDX5
Group 05, AST-3424, 1.25mg/kg, 10ul/g, i.v., QW×2; 1week off; QW×2
Group 06, Compound A, 0.5mg/kg, 10μl/g, i.v., QDX5; 2 days off, 2 weeks off, QDX5
Group 07, Compound A, 1mg/kg, 10μl/g, i.v., QDX5; 2 days off, 2 weeks off, QDX5
Group 08, Compound A, 2mg/kg, 10μl/g, i.v., QDX5; 2 days off, 2 weeks off, QDX5

Fig. 24

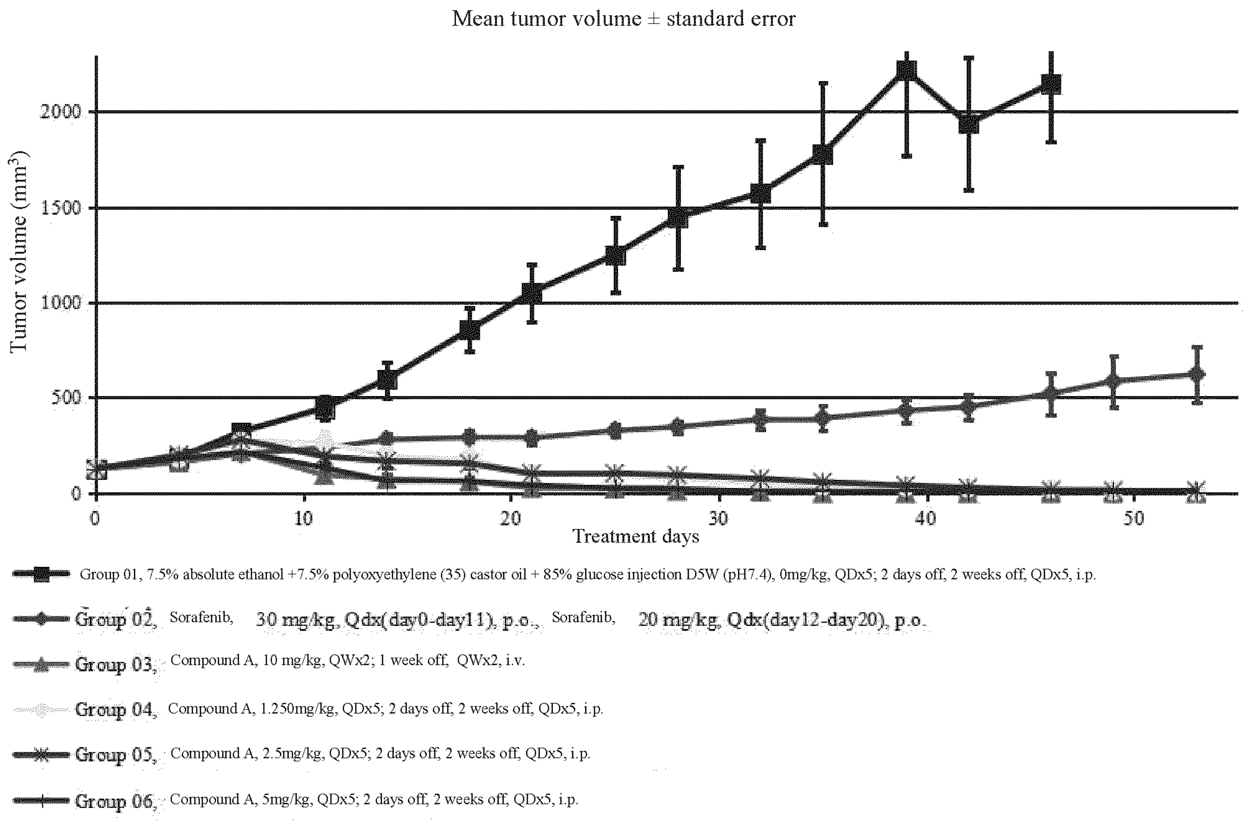
Mean tumor volume ± standard error
Tumor volume (mm$^3$)
2000
1500
1000
500
0
0
10
20
30
40
50
Treatment days
Group 01, 7.5% absolute ethanol +7.5% polyoxyethylene (35) castor oil + 85% glucose injection D5W (pH7.4), 0mg/kg, QDx5; 2 days off, 2 weeks off, QDx5, i.p.
Group 02, Sorafenib, 30 mg/kg, Qdx(day0-day11), p.o., Sorafenib, 20 mg/kg, Qdx(day12-day20), p.o.
Group 03, Compound A, 10 mg/kg, QWx2; 1 week off, QWx2, i.v.
Group 04, Compound A, 1.250mg/kg, QDx5; 2 days off, 2 weeks off, QDx5, i.p.
Group 05, Compound A, 2.5mg/kg, QDx5; 2 days off, 2 weeks off, QDx5, i.p.
Group 06, Compound A, 5mg/kg, QDx5; 2 days off, 2 weeks off, QDx5, i.p.

Fig. 25

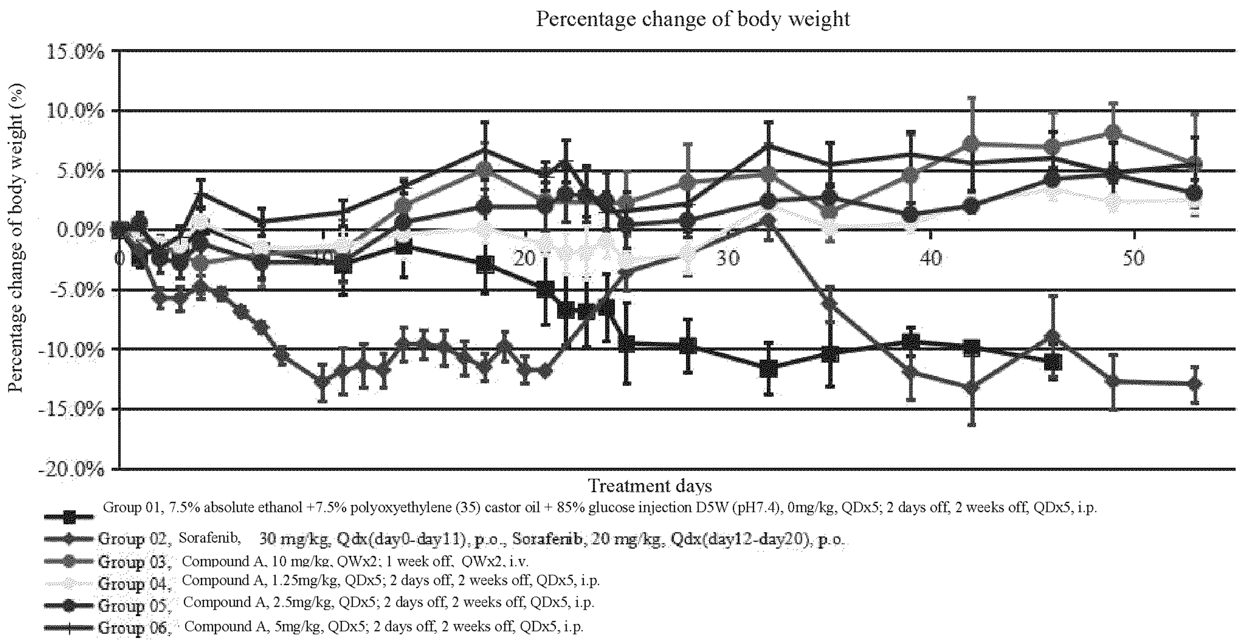
Percentage change of body weight
15.0%
10.0%
5.0%
0.0%
-5.0%
-10.0%
-15.0%
-20.0%
Percentage change of body weight (%)
0
10
20
30
40
50
Treatment days
Group 01, 7.5% absolute ethanol +7.5% polyoxyethylene (35) castor oil + 85% glucose injection D5W (pH7.4), 0mg/kg, QDx5; 2 days off, 2 weeks off, QDx5, i.p.
Group 02, Sorafenib, 30 mg/kg, Qdx(day0-day11), p.o., Sorafenib, 20 mg/kg, Qdx(day12-day20), p.o.
Group 03, Compound A, 10 mg/kg, QWx2; 1 week off, QWx2, i.v.
Group 04, Compound A, 1.25mg/kg, QDx5; 2 days off, 2 weeks off, QDx5, i.p.
Group 05, Compound A, 2.5mg/kg, QDx5; 2 days off, 2 weeks off, QDx5, i.p.
Group 06, Compound A, 5mg/kg, QDx5; 2 days off, 2 weeks off, QDx5, i.p.

Fig. 26

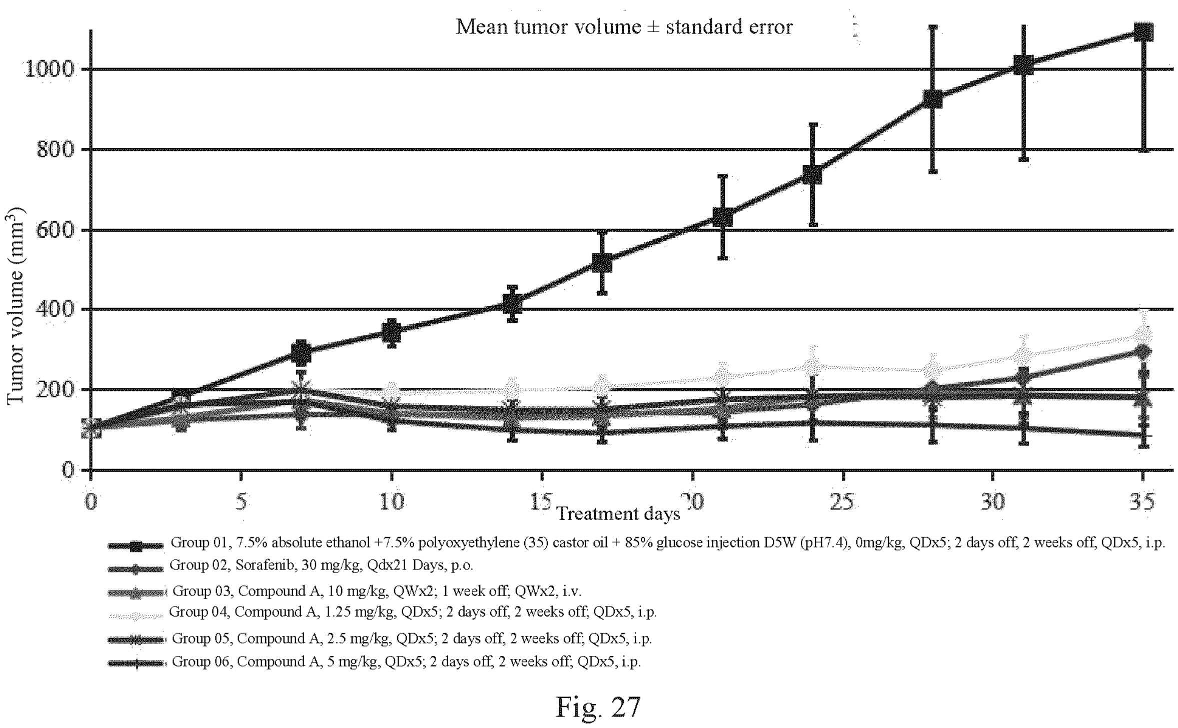
Mean tumor volume ± standard error
1000
800
600
400
200
0
Tumor volume (mm3)
0
5
10
15
20
25
30
35
Treatment days
Group 01, 7.5% absolute ethanol +7.5% polyoxyethylene (35) castor oil + 85% glucose injection D5W (pH7.4), 0mg/kg, QDx5; 2 days off, 2 weeks off, QDx5, i.p.
Group 02, Sorafenib, 30 mg/kg, Qdx21 Days, p.o.
Group 03, Compound A, 10 mg/kg, QWx2; 1 week off; QWx2, i.v.
Group 04, Compound A, 1.25 mg/kg, QDx5; 2 days off, 2 weeks off; QDx5, i.p.
Group 05, Compound A, 2.5 mg/kg, QDx5; 2 days off, 2 weeks off; QDx5, i.p.
Group 06, Compound A, 5 mg/kg, QDx5; 2 days off, 2 weeks off; QDx5, i.p.

Fig. 27

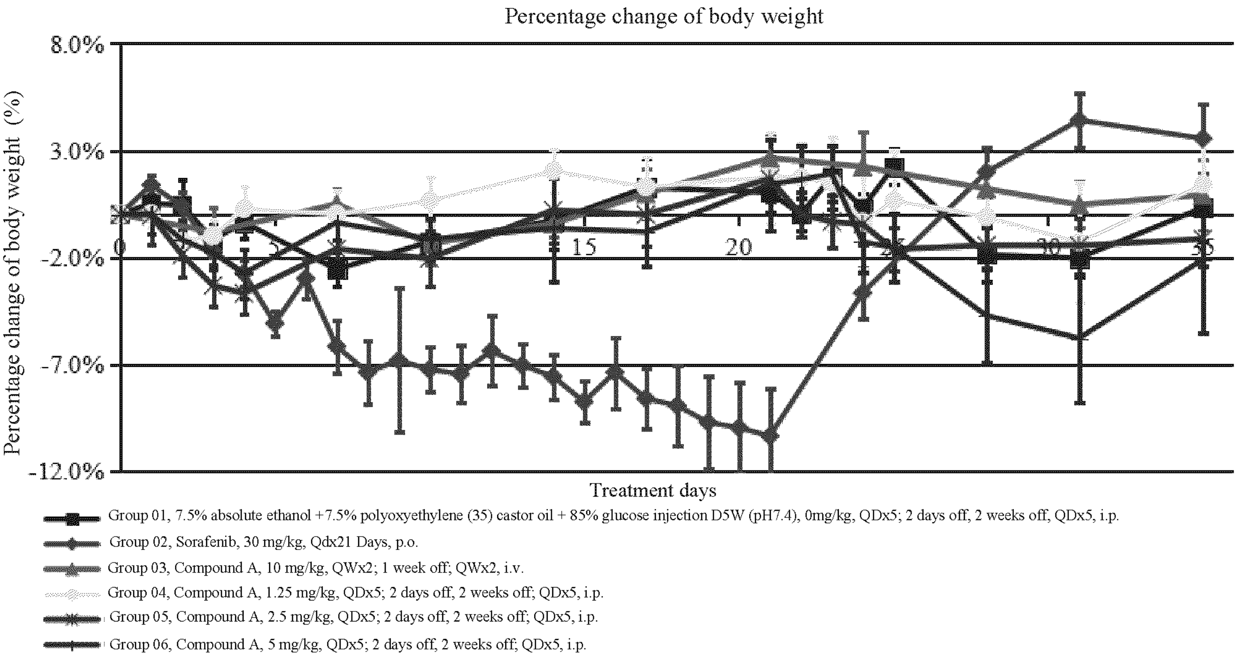
Percentage change of body weight
8.0%
3.0%
-2.0%
-7.0%
-12.0%
Percentage change of body weight (%)
0
15
20
Treatment days
Group 01, 7.5% absolute ethanol +7.5% polyoxyethylene (35) castor oil + 85% glucose injection D5W (pH7.4), 0mg/kg, QDx5; 2 days off, 2 weeks off, QDx5, i.p.
Group 02, Sorafenib, 30 mg/kg, Qdx21 Days, p.o.
Group 03, Compound A, 10 mg/kg, QWx2; 1 week off; QWx2, i.v.
Group 04, Compound A, 1.25 mg/kg, QDx5; 2 days off, 2 weeks off; QDx5, i.p.
Group 05, Compound A, 2.5 mg/kg, QDx5; 2 days off, 2 weeks off; QDx5, i.p.
Group 06, Compound A, 5 mg/kg, QDx5; 2 days off, 2 weeks off; QDx5, i.p.

Fig. 28

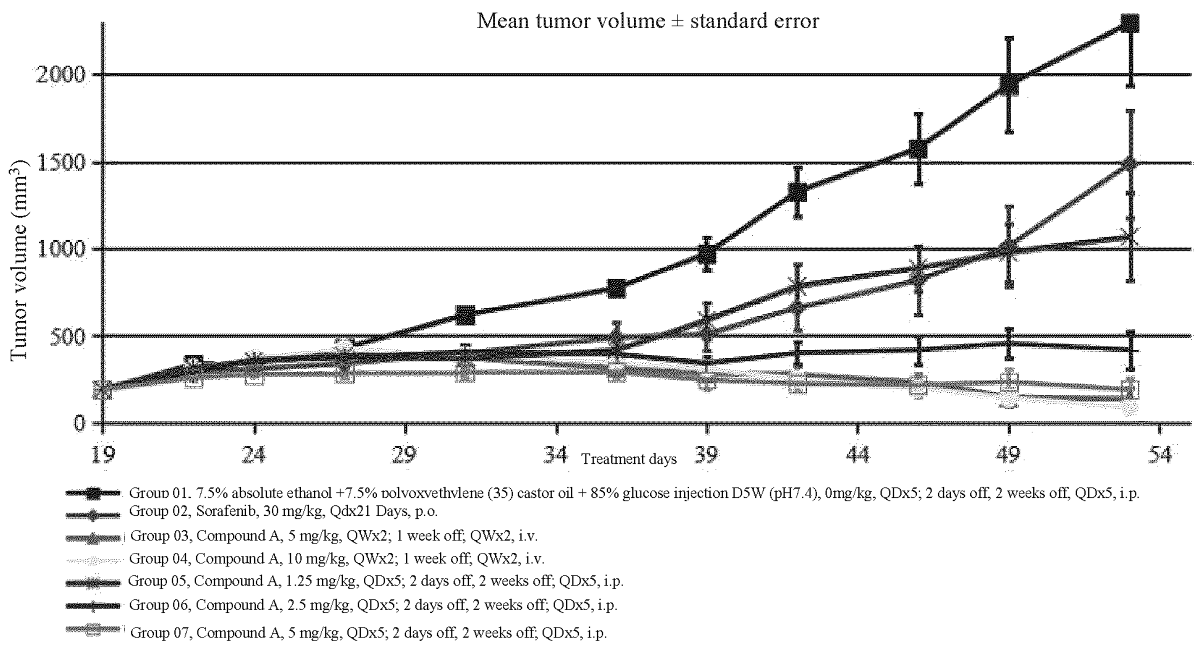
Mean tumor volume ± standard error
2000
1500
1000
500
0
Tumor volume (mm3)
19
24
29
34
Treatment days
39
44
49
54
Group 01, 7.5% absolute ethanol +7.5% polyoxyethylene (35) castor oil + 85% glucose injection D5W (pH7.4), 0mg/kg, QDx5; 2 days off, 2 weeks off, QDx5, i.p.
Group 02, Sorafenib, 30 mg/kg, Qdx21 Days, p.o.
Group 03, Compound A, 5 mg/kg, QWx2; 1 week off; QWx2, i.v.
Group 04, Compound A, 10 mg/kg, QWx2; 1 week off; QWx2, i.v.
Group 05, Compound A, 1.25 mg/kg, QDx5; 2 days off, 2 weeks off; QDx5, i.p.
Group 06, Compound A, 2.5 mg/kg, QDx5; 2 days off, 2 weeks off; QDx5, i.p.
Group 07, Compound A, 5 mg/kg, QDx5; 2 days off, 2 weeks off; QDx5, i.p.

Fig. 29

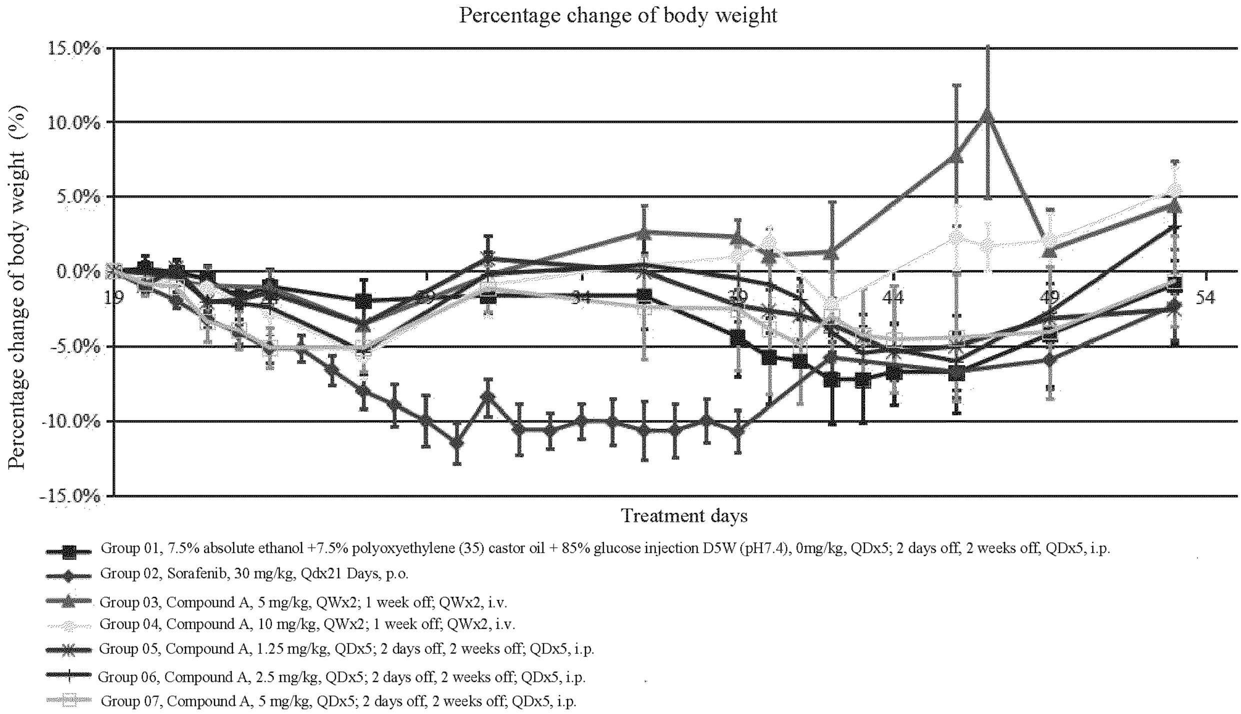

Percentage change of body weight
Percentage change of body weight (%)
Treatment days
Group 01, 7.5% absolute ethanol +7.5% polyoxyethylene (35) castor oil + 85% glucose injection D5W (pH7.4), 0mg/kg, QDx5; 2 days off, 2 weeks off, QDx5, i.p.
Group 02, Sorafenib, 30 mg/kg, Qdx21 Days, p.o.
Group 03, Compound A, 5 mg/kg, QWx2; 1 week off; QWx2, i.v.
Group 04, Compound A, 10 mg/kg, QWx2; 1 week off; QWx2, i.v.
Group 05, Compound A, 1.25 mg/kg, QDx5; 2 days off, 2 weeks off; QDx5, i.p.
Group 06, Compound A, 2.5 mg/kg, QDx5; 2 days off, 2 weeks off; QDx5, i.p.
Group 07, Compound A, 5 mg/kg, QDx5; 2 days off, 2 weeks off; QDx5, i.p.


Fig. 30

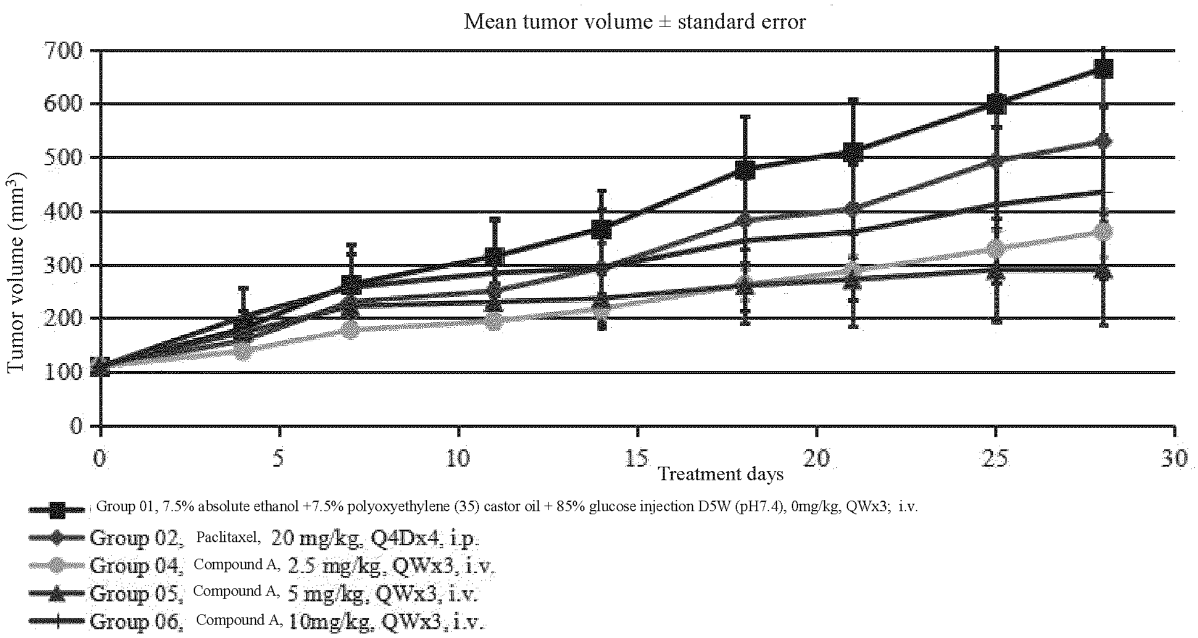

Mean tumor volume ± standard error
Tumor volume (mm³)
Treatment days
Group 01, 7.5% absolute ethanol +7.5% polyoxyethylene (35) castor oil + 85% glucose injection D5W (pH7.4), 0mg/kg, QWx3; i.v.
Group 02, Paclitaxel, 20 mg/kg, Q4Dx4, i.p.
Group 04, Compound A, 2.5 mg/kg, QWx3, i.v.
Group 05, Compound A, 5 mg/kg, QWx3, i.v.
Group 06, Compound A, 10mg/kg, QWx3, i.v.


Fig. 31

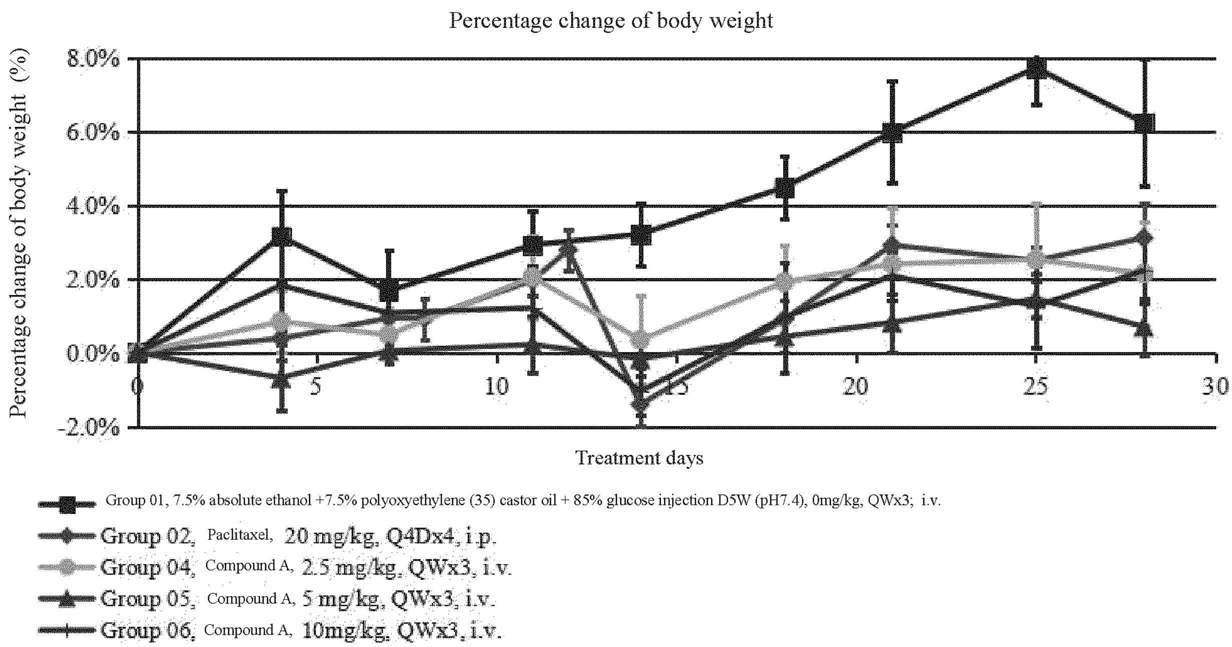
Percentage change of body weight
Percentage change of body weight (%)
8.0%
6.0%
4.0%
2.0%
0.0%
-2.0%
0
5
10
15
20
25
30
Treatment days
Group 01, 7.5% absolute ethanol +7.5% polyoxyethylene (35) castor oil + 85% glucose injection D5W (pH7.4), 0mg/kg, QWx3; i.v.
Group 02, Paclitaxel, 20 mg/kg, Q4Dx4, i.p.
Group 04, Compound A, 2.5 mg/kg, QWx3, i.v.
Group 05, Compound A, 5 mg/kg, QWx3, i.v.
Group 06, Compound A, 10mg/kg, QWx3, i.v.

Fig. 32

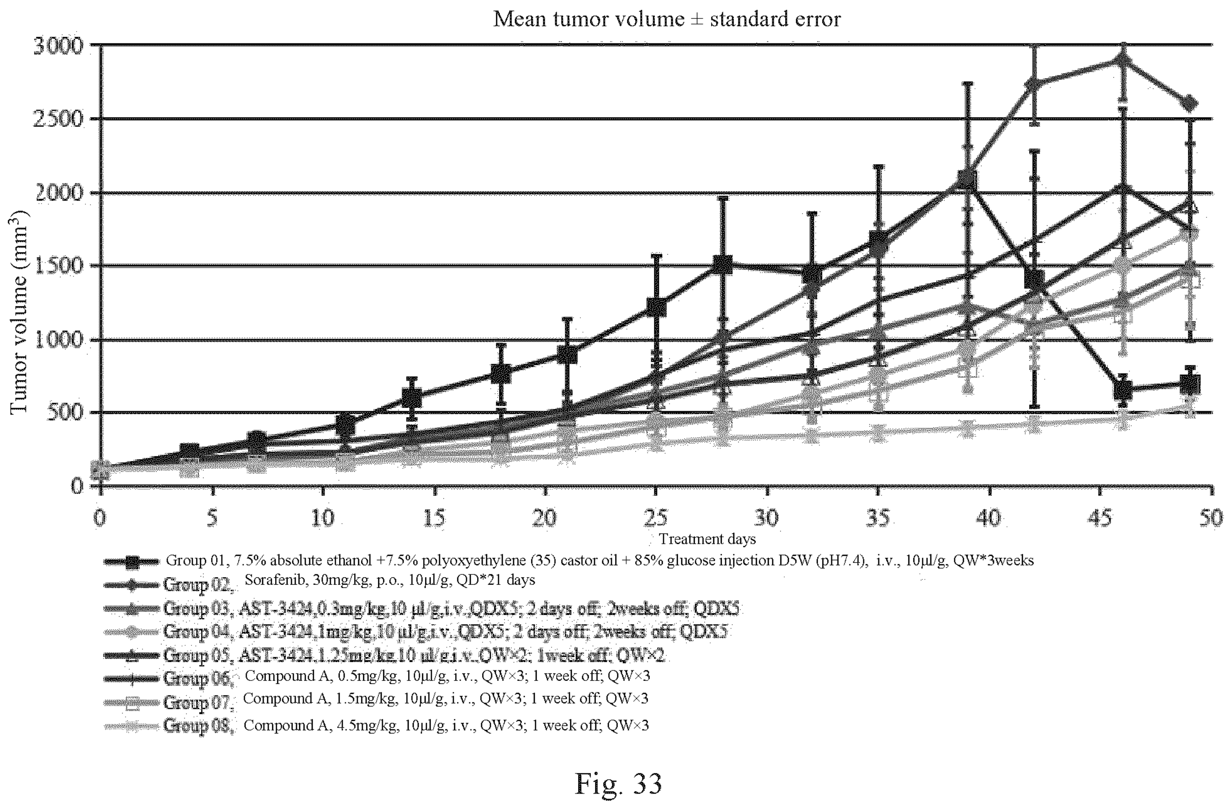
Mean tumor volume ± standard error
Tumor volume (mm3)
3000
2500
2000
1500
1000
500
0
0
5
10
15
20
25
30
35
40
45
50
Treatment days
Group 01, 7.5% absolute ethanol +7.5% polyoxyethylene (35) castor oil + 85% glucose injection D5W (pH7.4), i.v., 10μl/g, QW*3weeks
Group 02, Sorafenib, 30mg/kg, p.o., 10μl/g, QD*21 days
Group 03, AST-3424,0.3mg/kg,10 μl/g,i.v.,QDX5; 2 days off; 2weeks off; QDX5
Group 04, AST-3424,1mg/kg,10 μl/g,i.v.,QDX5; 2 days off; 2weeks off; QDX5
Group 05, AST-3424,1.25mg/kg,10 ul/g,i.v.,QW×2; 1week off; QW×2
Group 06, Compound A, 0.5mg/kg, 10μl/g, i.v., QW×3; 1 week off; QW×3
Group 07, Compound A, 1.5mg/kg, 10μl/g, i.v., QW×3; 1 week off; QW×3
Group 08, Compound A, 4.5mg/kg, 10μl/g, i.v., QW×3; 1 week off; QW×3

Fig. 33

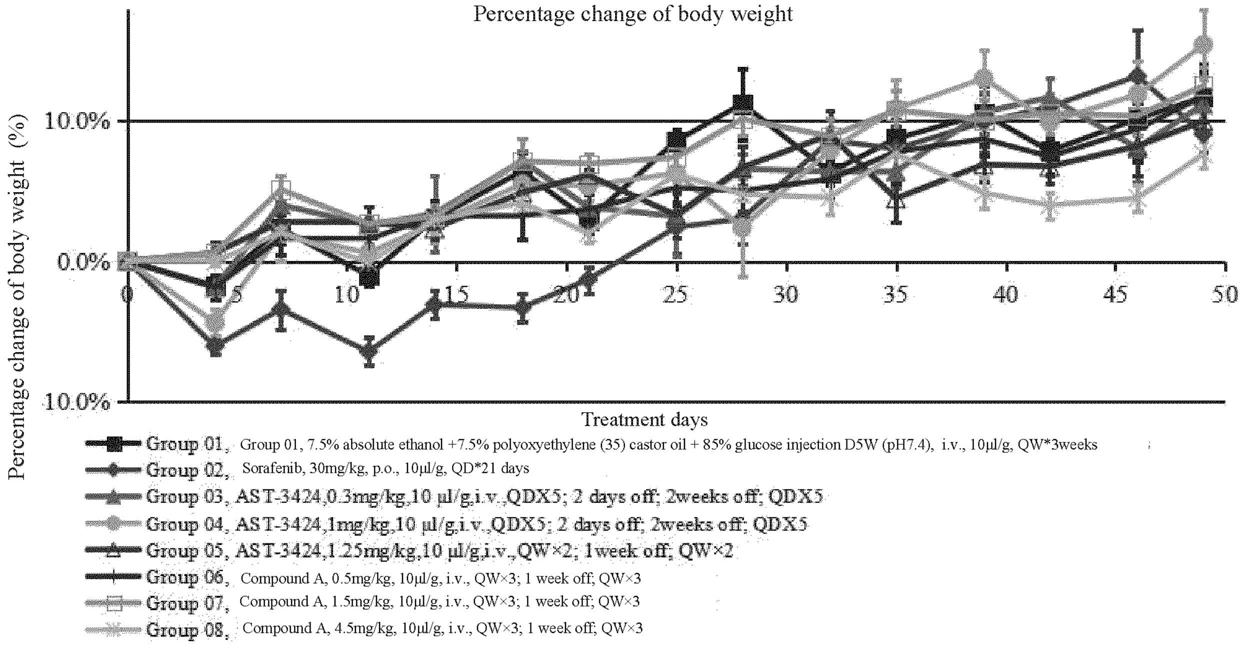
Percentage change of body weight
10.0%
0.0%
-10.0%
Percentage change of body weight (%)
0
5
10
15
20
25
30
35
40
45
50
Treatment days
Group 01, Group 01, 7.5% absolute ethanol +7.5% polyoxyethylene (35) castor oil + 85% glucose injection D5W (pH7.4), i.v., 10μl/g, QW*3weeks
Group 02, Sorafenib, 30mg/kg, p.o., 10μl/g, QD*21 days
Group 03, AST-3424,0.3mg/kg,10 μl/g,i.v.,QDX5; 2 days off; 2weeks off; QDX5
Group 04, AST-3424,1mg/kg,10 μl/g,i.v.,QDX5; 2 days off; 2weeks off; QDX5
Group 05, AST-3424,1.25mg/kg,10 μl/g,i.v.,QW×2; 1week off; QW×2
Group 06, Compound A, 0.5mg/kg, 10μl/g, i.v., QW×3; 1 week off; QW×3
Group 07, Compound A, 1.5mg/kg, 10μl/g, i.v., QW×3; 1 week off; QW×3
Group 08, Compound A, 4.5mg/kg, 10μl/g, i.v., QW×3; 1 week off; QW×3

Fig. 34

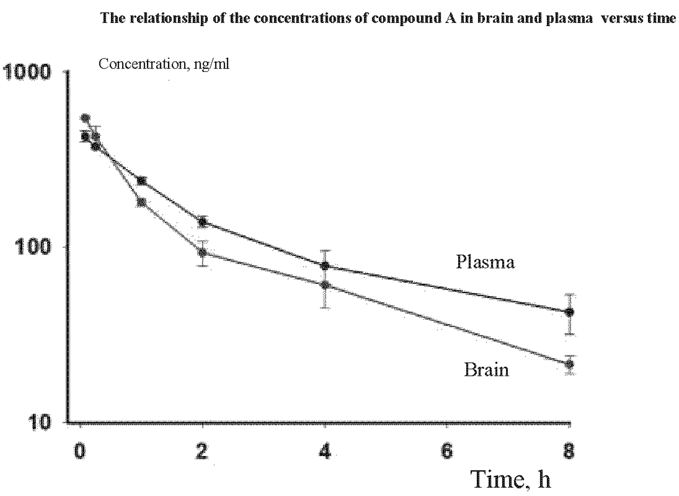
The relationship of the concentrations of compound A in brain and plasma versus time
Concentration, ng/ml
1000
100
10
0
2
4
6
8
Time, h
Plasma
Brain

Fig. 35

The relationship of the ratio between concentrations of compound A in brain and plasma versus time

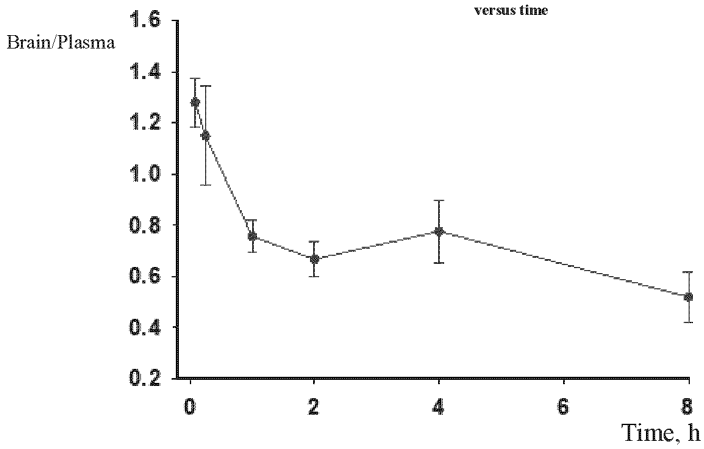


Fig. 36

The relationship of the concentrations of compound AST-2870 in brain and plasma versus time

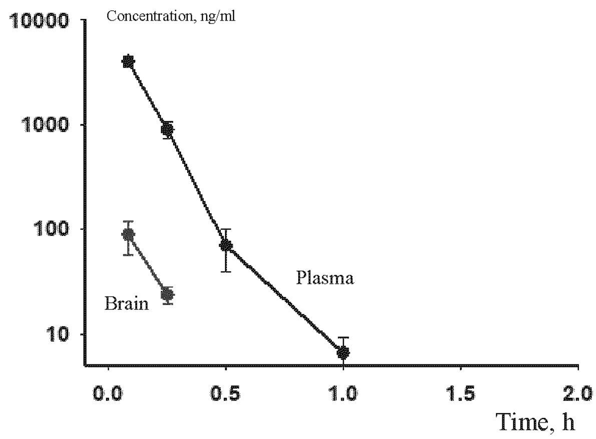


Fig. 37

The relationship of the ratio between concentrations of compound AST-2870 in brain and plasma versus time

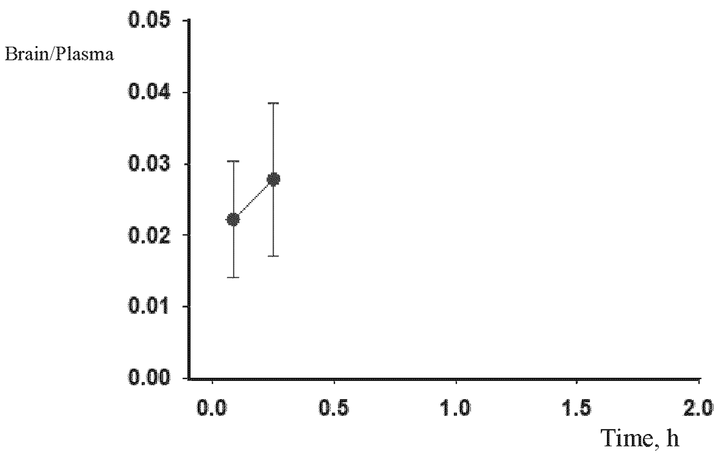


Fig. 38

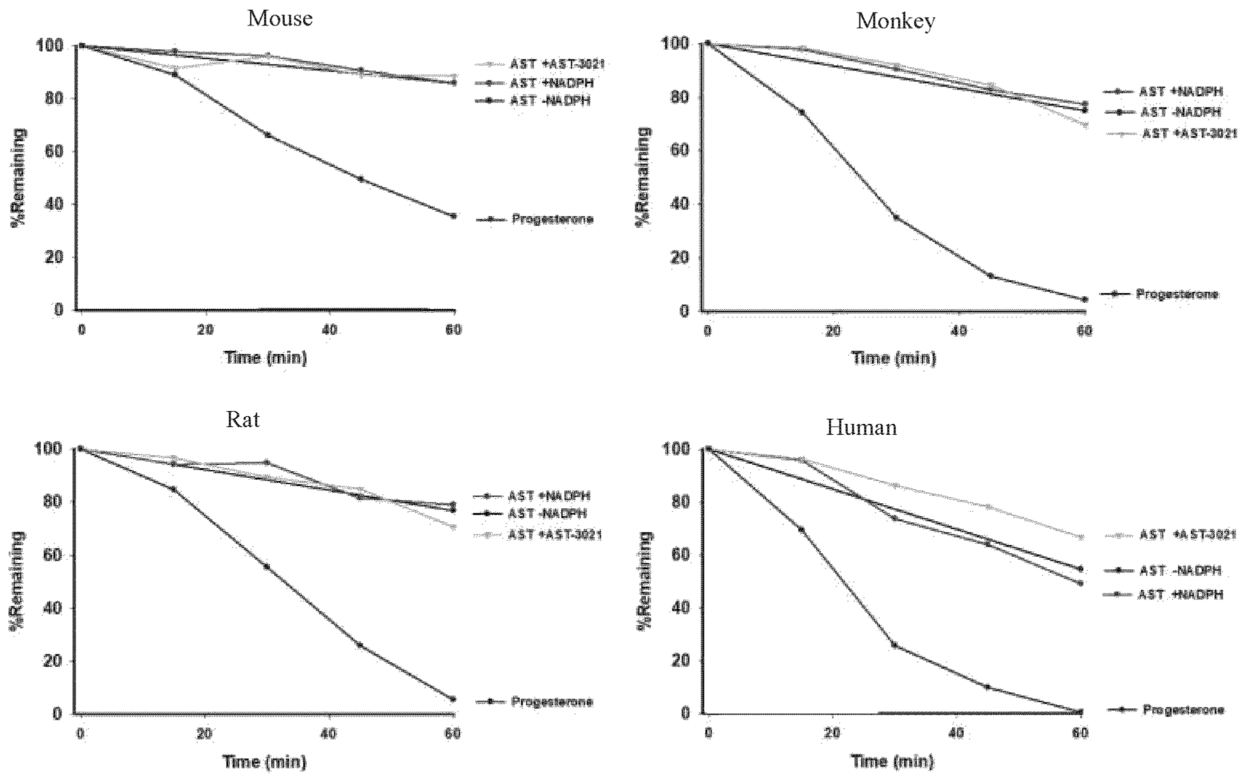
Mouse
AST +AST-3021
AST +NADPH
AST -NADPH
Progesterone
Monkey
AST +NADPH
AST -NADPH
AST +AST-3021
Progesterone
Rat
AST +NADPH
AST -NADPH
AST +AST-3021
Progesterone
Human
AST +AST-3021
AST -NADPH
AST +NADPH
Progesterone
%Remaining
Time (min)
0
20
40
60
80
100

Fig. 39

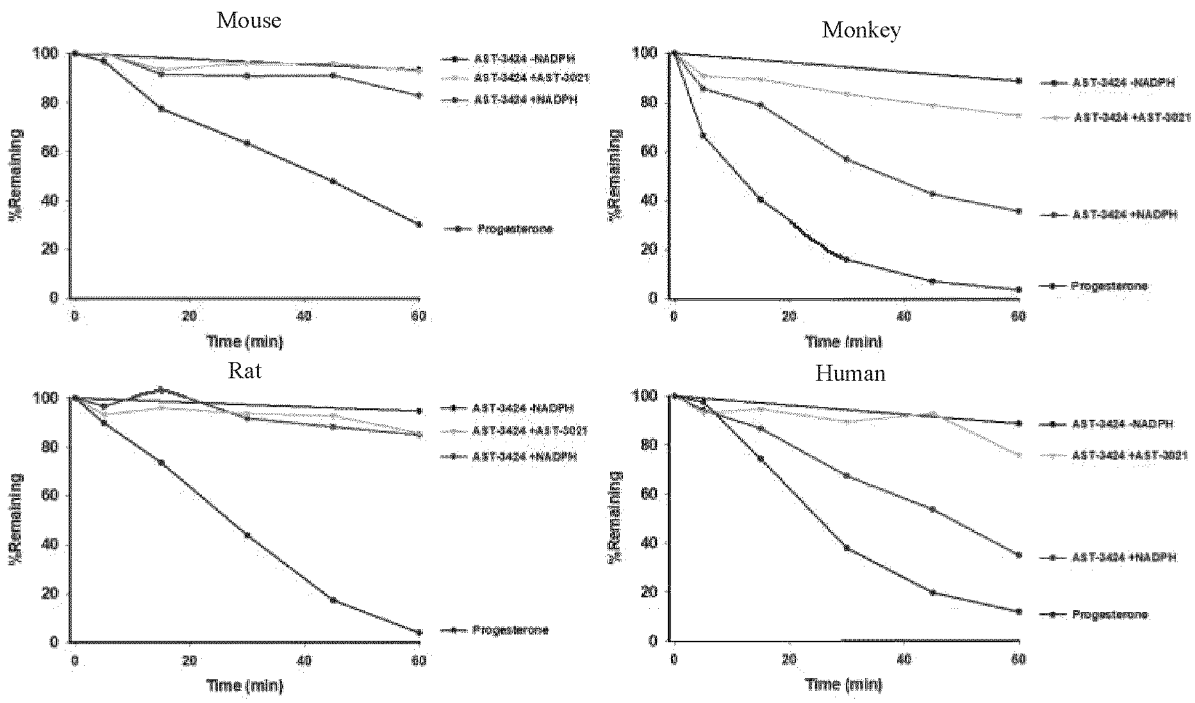
Mouse
AST-3424 -NADPH
AST-3424 +AST-3021
AST-3424 +NADPH
Progesterone
Monkey
AST-3424 -NADPH
AST-3424 +AST-3021
AST-3424 +NADPH
Progesterone
Rat
AST-3424 -NADPH
AST-3424 +AST-3021
AST-3424 +NADPH
Progesterone
Human
AST-3424 -NADPH
AST-3424 +AST-3021
AST-3424 +NADPH
Progesterone
%Remaining
Time (min)
0
20
40
60
80
100

Fig. 40

## INTERNATIONAL SEARCH REPORT

International application No.
**PCT/CN2021/129077**

**A. CLASSIFICATION OF SUBJECT MATTER**

C07F 9/24(2006.01)i； C07F 9/564(2006.01)i； A61K 31/664(2006.01)i； A61K 31/396(2006.01)i； A61K 31/04(2006.01)i； A61K 31/06(2006.01)i； A61P 35/00(2006.01)i

According to International Patent Classification (IPC) or to both national classification and IPC

**B. FIELDS SEARCHED**

Minimum documentation searched (classification system followed by classification symbols)

C07F； A61K； A61P

Documentation searched other than minimum documentation to the extent that such documents are included in the fields searched

Electronic data base consulted during the international search (name of data base and, where practicable, search terms used)

CNABS, CNTXT, DWPI, VEN, USTXT, EPTXT, WOTXT, CNKI: 深圳艾欣达伟, 李安蓉, 段建新, 孟繁英, 齐天阳, 孟腾, 张梦云, 醛基酮还原酶1C3, 醛酮还原酶1C3, DNA烷化剂, p-糖蛋白, 多药耐药蛋白, 血脑屏障, 同位素变体, 外消旋体, 颅内, 脑癌, 脑肿瘤, 脑转移, 肝癌, 肺癌, 胃癌, 胰腺癌, 直链淀粉-三(3, 5-二甲苯基氨基甲酸酯), Ascentawits, AKR1C3, aldo-keto reductase 1C3, DNA alkylating agent, p-gp, pgp, P-glycoprotein, blood brain barrier, BBB, racemate, brain, cancer, tumour, brain metastasis, AST-2660, AST-3424, TH2870, OBI-3424, TH-3424, CHIRALPAK, TGI, 2553207-65-9, 2553207-92-2, 2553207-66-0, 2553207-61-5, 2553207-58-0

**C. DOCUMENTS CONSIDERED TO BE RELEVANT**

| Category* | Citation of document, with indication, where appropriate, of the relevant passages | Relevant to claim No. |
|---|---|---|
| X | WO 2021083310 A1 (ASCENTAWITS PHARMACEUTICALS, LTD.) 06 May 2021 (2021-05-06)<br>description, page 1, lines 21-33, page 4, lines 15-16, page 7, line 1 to page 13, line 9, and page 32, line 8 to page 37, line 5 | 1-13, 16-27, 30-32 |
| X | WO 2020228685 A1 (ASCENTAWITS PHARMACEUTICALS, LTD.) 19 November 2020 (2020-11-19)<br>description, page 2, line 14, page 5, line 4, and page 7, lines 15-21 | 1-15 |
| Y | WO 2021083310 A1 (ASCENTAWITS PHARMACEUTICALS, LTD.) 06 May 2021 (2021-05-06)<br>description, page 1, lines 21-33, page 4, lines 15-16, page 7, line 1 to page 13, line 9, and page 32, line 8 to page 37, line 5 | 14, 15, 28, 29 |
| Y | WO 2020228685 A1 (ASCENTAWITS PHARMACEUTICALS, LTD.) 19 November 2020 (2020-11-19)<br>description, page 2, line 14, page 5, line 4, and page 7, lines 15-21 | 14, 15 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **13 June 2022** | **24 June 2022** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

International application No.
**PCT/CN2021/129077**

**C. DOCUMENTS CONSIDERED TO BE RELEVANT**

| Category* | Citation of document, with indication, where appropriate, of the relevant passages | Relevant to claim No. |
|---|---|---|
| Y | WO 2021120717 A1 (ASCENTAWITS PHARMACEUTICALS, LTD.) 24 June 2021 (2021-06-24) embodiments 1 and 23 | 28, 29 |
| A | CN 107530556 A (THRESHOLD PHARMACEUTICALS, INC.) 02 January 2018 (2018-01-02) entire document | 1-32 |
| A | CN 108136214 A (THRESHOLD PHARMACEUTICALS, INC.) 08 June 2018 (2018-06-08) entire document | 1-32 |
| A | CN 108290911 A (THRESHOLD PHARMACEUTICALS, INC.) 17 July 2018 (2018-07-17) entire document | 1-32 |

Form PCT/ISA/210 (second sheet) (January 2015)

**INTERNATIONAL SEARCH REPORT**

International application No.
**PCT/CN2021/129077**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **7-9, 12, 14, 15**
because they relate to subject matter not required to be searched by this Authority, namely:

[1] Claims 7-9 and 12 relate to a use of the drug according to claim 6 or the compound according to any one of claims 1-5 for the treatment of a cancer patient or tumor patient or a condition induced by cancer or a tumor, or a hyperplasia disease. Claims 14 and 15 relate to a method for the treatment of cancer or a tumor. The use or method relates to a method for the treatment of a disease, and therefore, said claims do not comply with PCT Rule 39.1(iv). The search report was made on the basis that the title of the subject matter of claims 7-9 and 12 is amended to be "a use of the drug according to claim 6 or the compound according to any one of claims 1-5 for in the preparation of a drug for treating a cancer patient or tumor patient or a condition induced by cancer or a tumor, or a hyperplasia disease" and that the title of the subject matter of claims 14 and 15 is amended to be "a use of the drug according to claim 6 in the preparation of a drug for treating cancer or a tumor".

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**INTERNATIONAL SEARCH REPORT**
**Information on patent family members**

International application No.
**PCT/CN2021/129077**

| Patent document cited in search report | Publication date (day/month/year) | Patent family member(s) | Publication date (day/month/year) |
|---|---|---|---|
| WO 2021083310 A1 | 06 May 2021 | CN 114555614 A | 27 May 2022 |
| WO 2020228685 A1 | 19 November 2020 | US 2022119429 A1 | 21 April 2022 |
| | | AU 2020275818 A1 | 25 November 2021 |
| | | CA 3140070 A1 | 19 November 2020 |
| | | EP 3971194 A1 | 23 March 2022 |
| | | KR 20220012274 A | 03 February 2022 |
| | | CN 113853379 A | 28 December 2021 |
| WO 2021120717 A1 | 24 June 2021 | TW 202128188 A | 01 August 2021 |
| | | TW 202135831 A | 01 October 2021 |
| | | WO 2022052564 A1 | 17 March 2022 |
| CN 107530556 A | 02 January 2018 | KR 20190072689 A | 25 June 2019 |
| | | US 2019225633 A1 | 25 July 2019 |
| | | IL 254377 D0 | 30 November 2017 |
| | | CA 2979251 A1 | 15 September 2016 |
| | | US 2018044360 A1 | 15 February 2018 |
| | | KR 20210095231 A | 30 July 2021 |
| | | EP 3277380 A1 | 07 February 2018 |
| | | SG 11201707293V A | 30 October 2017 |
| | | TW 201706262 A | 16 February 2017 |
| | | ES 2828026 T3 | 25 May 2021 |
| | | EP 3747508 A1 | 09 December 2020 |
| | | ES 2896960 T3 | 28 February 2022 |
| | | KR 20170128280 A | 22 November 2017 |
| | | BR 112017019287 A2 | 02 May 2018 |
| | | KR 20190072688 A | 25 June 2019 |
| | | WO 2016145092 A1 | 15 September 2016 |
| | | AU 2016229136 A1 | 05 October 2017 |
| | | JP 2018513876 A | 31 May 2018 |
| | | SG 10201913462 X A | 30 March 2020 |
| CN 108136214 A | 08 June 2018 | JP 2019178172 A | 17 October 2019 |
| | | US 2021017120 A1 | 21 January 2021 |
| | | KR 20170127463 A | 21 November 2017 |
| | | SG 10201913709 Q A | 30 March 2020 |
| | | WO 2016161342 A2 | 06 October 2016 |
| | | HK 1250959 A1 | 18 January 2019 |
| | | JP 2018511612 A | 26 April 2018 |
| | | US 2018086693 A1 | 29 March 2018 |
| | | CA 2981494 A1 | 06 October 2016 |
| | | EP 3277381 A2 | 07 February 2018 |
| | | SG CN 11201707375 U A | 30 October 2017 |
| | | IL 254769 D0 | 31 December 2017 |
| | | TW 201706267 A | 16 February 2017 |
| | | AU 2016244000 A1 | 12 October 2017 |
| | | CN 112142692 A | 29 December 2020 |
| | | BR 112017021167 A2 | 03 July 2018 |
| | | KR 20190075145 A | 28 June 2019 |
| CN 108290911 A | 17 July 2018 | JP 2018517710 A | 05 July 2018 |
| | | TW 201726695 A | 01 August 2017 |
| | | ES 2781398 T3 | 01 September 2020 |

Form PCT/ISA/210 (patent family annex) (January 2015)

**INTERNATIONAL SEARCH REPORT**
**Information on patent family members**

International application No.
**PCT/CN2021/129077**

| Patent document cited in search report | Publication date (day/month/year) | Patent family member(s) | | | Publication date (day/month/year) |
|---|---|---|---|---|---|
| | | EP | 3390415 | A1 | 24 October 2018 |
| | | IL | 256569 | D0 | 28 February 2018 |
| | | CA | 2990696 | A1 | 26 May 2017 |
| | | KR | 20170130615 | A | 28 November 2017 |
| | | AU | 2016357728 | A1 | 30 November 2017 |
| | | WO | 2017087428 | A1 | 26 May 2017 |
| | | BR | 112017025778 | A2 | 14 August 2018 |
| | | HK | 1250988 | A1 | 18 January 2019 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 2020089692 W, DUAN Jianxin, LI Anrong
- US 2016021581 W
- WO 2016145092 A1
- CN 201680015078
- CN 107530556 B
- CN 2021079299 W


### Non-patent literature cited in the description


- **FLANAGAN et al.** *Bioorganic and Medicinal Chemistry,* 2014, 962-977
- **JESSICA D. SUN ; QIAN LIU ; DHARMENDRA AHLUWALIA ; DAMIEN J. FERRARO ; YAN WANG ; DON JUNG ; MARK D. MATTEUCCI ; CHARLES P. HART.** Comparison of hypoxia-activated prodrug evofosfamide (TH-302) and ifosfamide in preclinical non-small cell lung cancer models [J. *Cancer Biology & Therapy,* 2016, vol. 17 (4), 371-380